# EUROPEAN PATENT APPLICATION

(11) **EP 3 967 711 A1**
(43) Date of publication of application: **16.03.2022**
(21) Application number: 21796353.7
(22) Date of filing: 19.04.2021
(51) Int. Cl.: C07K 16/28, C07K 16/46, A61K 39/395, A61P 35/00

(54) **TETRAVALENT BISPECIFIC ANTIBODY, PREPARATION METHOD THEREFOR, AND USE THEREOF**

(30) Priority: 29.04.2020 CN 202010357134
(60) Divisional application: 22152931.6
(71) Applicant: Sunshine Guojian Pharmaceutical (Shanghai) Co., Ltd., China (Shanghai) Pilot Free Trade Zone Shanghai 201203 (CN)
(72) Inventor: ZHAO, Jie, Shanghai 201203 (CN); HUANG, Haomin, Shanghai 201203 (CN); ZHU, Zhenping, Shanghai 201203 (CN); ZHU, Yunxia, Shanghai 201203 (CN)
(74) Representative: Plougmann Vingtoft a/s
(86) International application number: PCT/CN2021/088153
(87) International publication number: WO 2021/218684

(57) **Abstract**

Provided are a structurally symmetrical tetravalent bispecific antibody based on a common light chain and a construction method therefor. The prepared tetravalent bispecific antibody has similar or even better biological activity and physical and chemical properties than monoclonal antibodies, and can be used for the treatment of various inflammatory diseases, cancer, and other diseases.

## Description

### Field of the Invention

The present invention relates to the field of antibodies. More particularly, the present invention relates to a class of tetravalent bispecific antibodies, preparation method therefor and use thereof.

### Background of the Invention

A bispecific antibody refers to an antibody molecule that can specifically bind to two antigens or two epitopes simultaneously. According to the symmetry, bispecific antibodies can be divided into structurally symmetric and asymmetric molecules. According to the number of binding sites, bispecific antibodies can be divided into bivalent, trivalent, tetravalent and multivalent molecules.

An ScFv consists of a heavy chain variable region (VH) and a light chain variable region (VL), where VH and VL are connected by a flexible peptide linker. In the ScFv, the order of the domains may be VH-linker-VL or VL-linker-VH. Various linkers have been reported for connecting VH and VL, such as short alanine linkers, glycine-serine-rich linkers, linkers in helical conformation, and linkers derived from various immunoglobulin and non-immunoglobulin molecules (Ahmad Z A, Yeap S K, Ali A M, et al. scFv antibody: principles and clinical application[J]. Clinical and developmental immunology, 2012, 2012:980250.). A ScFv usually represents the smallest binding site of an antibody. A bispecific antibody can be constructed by connecting two ScFvs through a linker. The bispecific antibody molecule thus constructed is bivalent, having one binding site for each antigen, with a molecular weight of usually about 50-60 kDa. Two tandem ScFv fragments will fold independently and form their own antigen binding site. Bispecific tandem scFv format has been widely used in cancer immunotherapy to retarget T cells to tumor cells or tumor-associated cells in the tumor microenvironment. This form constitutes the molecular basis of bispecific T-cell engager (BiTE). The first BiTE molecule approved for marketing is Blinatumomab (Huehls A M, Coupet T A, Sentman C L. Bispecific T-cell engagers for cancer immunotherapy[J]. Immunology and cell biology, 2015, 93(3): 290-296.).

Diabody (Db) is a bivalent molecule composed of two chains, each of which contains a VH and VL domain, derived from the same or different antibodies. In a diabody, the two variable domains are connected by a short linker, usually 5 amino acid residues, such as GGGGS. Because the linker is significantly shorter than the length required to allow intra-chain assembly, this will cause the two ScFv chains to dimerize in a head-to-tail direction, resulting in a tightly packed diabody molecule with a molecular weight comparable to the tandem ScFvs (Wu C. Diabodies: molecular engineering and therapeutic applications[J]. Drug News Perspect, 2009, 22(8): 453.). When the two different chains of diabody are expressed in the cells, mispairing of variable domains will occur. In addition, since there is no non-covalent bond between the two chains, the diabody is not stable (Kipriyanov S M, Moldenhauer G, Braunagel M, et al. Effect of domain order on the activity of bacterially produced bispecific single-chain Fv antibodies[J]. Journal of molecular biology, 2003, 330(1): 99-111.).

Diabody can be fused with human Fc to produce an IgG-like molecule, called Di-diabody (Lu D, Zhang H, Koo H, et al. A fully human recombinant IgG-like bispecific antibody to both the epidermal growth factor receptor and the insulin-like growth factor receptor for enhanced antitumor activity[J]. Journal of Biological Chemistry, 2005, 280(20): 19665-19672.). Two tandem ScFvs can also be fused with human Fc to produce an IgG-like molecule, called TaFv-Fc (Chen Z, Xie W, Acheampong D O, et al. A human IgG-like bispecific antibody co-targeting epidermal growth factor receptor and the vascular endothelial growth factor receptor 2 for enhanced antitumor activity[J]. Cancer biology & therapy, 2016, 17(2): 139-150.).

In addition to the Di-diabody and TaFv-Fc described above, structurally symmetrical tetravalent bispecific antibodies also include (but are not limited to) the following types:
The N-terminus or C-terminus of the heavy chain or light chain of natural IgG is connected to ScFv through a polypeptide linker, so that bispecific antibodies in the IgG-ScFv format can be formed (Coloma M J, Morrison S L. Design and production of novel tetravalent bispecific antibodies[J]. Nature biotechnology, 1997, 15(2): 159.).

The N-terminus of the light chain and heavy chain of the natural antibody are connected to the VL and VH of another antibody, respectively through a polypeptide linker, so that bispecific antibodies in the DVD-Ig format can be formed (Wu C, Ying H, Grinnell C, et al. Simultaneous targeting of multiple disease mediators by a dual-variable-domain immunoglobulin[J]. Nature biotechnology, 2007, 25(11): 1290.).

CrossMAb is a technology for the interchange of functional regions of antibody Fab arms, and is a technology platform developed by Roche. This technology solves the problem of correct assembly of homologous light and heavy chains, and further improves the success rate of assembly. Various forms of bispecific antibodies based on CrossMAb have been published (Klein C, Schaefer W, Regula J T. The use of CrossMAb technology for the generation of bi-and multispecific antibodies[C]//MAbs. Taylor & Francis, 2016, 8(6): 1010-1020.).

The N-terminus of the heavy chain of the natural antibody is connected to the light chain of another antibody through a polypeptide linker, and then VH+CH1 is used as an independent short chain to pair with the light chain connected to the N-terminus, so that bispecific antibodies in the FIT-IgG format can be formed (US 10266608 B2).

IgG-TCR improves the pairing efficiency of the related heavy chain and light chain by replacing the CH1 of the heavy chain and the CL of the light chain with the constant regions of the α and β chains of T cell receptors (TCR) (Wu X, Sereno A J, Huang F, et al. Protein design of IgG/TCR chimeras for the co-expression of Fab-like moieties within bispecific antibodies[C]//MAbs. Taylor & Francis, 2015, 7(2): 364-376.). WuXiBody has been improved on the basis of IgG-TCR, which adds an engineered disulfide bond between the constant regions of the α and β chains, and further improves the efficiency of the related heavy chain and light chain pairing, as well as the stability of bispecific antibody molecules (WO 2019/057122 A1).

Recently, a method for constructing tetravalent bispecific antibodies has been reported. This method artificially designs the interface between the heavy chains and light chains of the Fab based on the crystal structure, and screens out an orthogonal Fab interface through experimental methods. The heavy chains and light chains of the orthogonal Fab can be specifically paired without mispairing with wild-type heavy chains and light chains (Lewis S M, Wu X, Pustilnik A, et al. Generation of bispecific IgG antibodies by structure-based design of an orthogonal Fab interface[J]. Nature biotechnology, 2014, 32(2): 191.; Wu X, Sereno A J, Huang F, et al. Fab-based bispecific antibody formats with robust biophysical properties and biological activity[C]//MAbs. Taylor & Francis, 2015, 7(3): 470-482.).

In addition, bispecific antibodies with asymmetric structures may adopt the following structures (but is not limited to):
The main function of the knob-in-hole (KIH) structure is to promote the heterodimerization of the two different heavy chains of a bispecific antibody. The structural characteristics are as follows:
the CH3 region of one heavy chain of the bispecific antibody is mutated to form a protruding "knob" structure, and the CH3 region of the other heavy chain is mutated to form a recessed "hole" structure, and the knob-into-hole (KIH) design facilitates the correct assembly of the heavy chains of the two heterologous antibodies (Merchant A M, Zhu Z, Yuan J Q, et al. An efficient route to human bispecific IgG[J]. Nature biotechnology, 1998, 16(7): 677.). DuetMab uses KIH technology for heterodimerization of 2 distinct heavy chains and increases the efficiency of related heavy chain and light chain pairing by replacing the native disulfide bond in one of the CH1-CL interfaces with an engineered disulfide bond (Mazor Y, Oganesyan V, Yang C, et al. Improving target cell specificity using a novel monovalent bispecific IgG design[C]//MAbs. Taylor & Francis, 2015, 7(2): 377-389.).

Another method for enhancing the heterodimerization of different heavy chains is the electrostatic steering mutation technique, which is based on charged residues with electrostatic interactions. This method chooses to change the polarity of the charge in the CH3 interface, so that the electrostatically matched Fc domain facilitates the formation of heterodimers due to the favorable charge attraction effect, while the unfavorable charge repulsion effect inhibits homodimerization (US 10011858 B2; Gunasekaran K, Pentony M, Shen M, et al. Enhancing antibody Fc heterodimer formation through electrostatic steering effects applications to bispecific molecules and monovalent IgG[J]. Journal of Biological Chemistry, 2010, 285(25): 19637-19646.).

Common light chain is often used in the construction of bispecific antibodies. The common light chain can reduce the production of by-products caused by mispairing of heavy chains and light chains, and increase the yield of bispecific antibodies (Brinkmann U, Kontermann R E. The making of bispecific antibodies[C]//MAbs. Taylor & Francis, 2017, 9(2): 182-212.). Under normal circumstances, the common light chain needs to be screened out by experimental methods, which can generally be obtained by hybridoma or phage display technology. Under normal circumstances, high-throughput screening of antibody libraries for anti-A antibodies and anti-B antibodies is required, which requires that the capacity of the two antibody libraries should not be too small (Sampei Z, Igawa T, Soeda T, et al. Identification and multidimensional optimization of an asymmetric bispecific IgG antibody mimicking the function of factor VIII cofactor activity[J]. PloS one, 2013, 8(2): e57479.; US 9657102 B2).

Bispecific antibodies are gradually becoming a new class of therapeutic antibodies that can be used to treat various inflammatory diseases, cancers and other diseases. Although a large number of new bispecific antibody structures have been reported recently, the main technical difficulty in producing bispecific antibodies lies in obtaining the correctly paired molecules. The above bispecific antibody forms all have the problem of mispairing, so one or more by-products or aggregates caused by mispairing will be produced, which will affect the yield, purity and physical and chemical stability of the bispecific antibodies of interest, and further affect the safety and effectiveness of the bispecific antibodies in the body.

### Summary of the Invention

The present invention describes a structurally symmetrical tetravalent bispecific antibody based on a common light chain as well as a construction method thereof. Under normal circumstances, there will be mispairing between heavy chains and light chains, as well as mispairing between heavy chains and heavy chains of bispecific antibodies. The higher the efficiency of correct assembly is, the lower the degree of mispairing is. Here, the present invention solves the problem of mispairing between heavy chains and light chains using a common light chain, and connects the heavy chain variable region VH-B of one antibody to CH1, and then connects to the heavy chain variable region VH-A of another antibody through a peptide linker, and then connects to the heavy chain constant region CH1-CH2-CH3 to form a long heavy chain. When the genes of the long heavy chain and the common light chain are expressed in the same cells, each long heavy chain will be paired with two common light chains, and there will be no mispairing between the long heavy chain and the common light chain, because the two light chains paired with each long heavy chain are the same; the long heavy chains will undergo homodimerization instead of heterodimerization, so there will be no mispairing between the long heavy chains. The tetravalent bispecific antibody of the present invention does not require Fc modification, has a simple preparation method, and has similar or even better biological activity and physical and chemical properties than those of monoclonal antibodies.

Furthermore, the inventors of the present application unexpectedly discovered during the longterm study of the above tetravalent bispecific antibody that the anti-human PD-1 antibody mAb1-25-Hu (609) binds to the PD-1 molecule mainly through the heavy chain, but is less dependent on the light chain. Therefore, the heavy chain variable region/heavy chain of 609 may be combined with the heavy chain variable region/heavy chain and light chain variable region/light chain (as a common light chain) of other target antibodies to construct a bispecific antibody that binds to PD-1 and other targets, and may be applied to, including but not limited to, the tetravalent bispecific antibody structure of the present invention or other forms of bispecific antibody structure containing a common light chain known in the art.

Therefore, the first object of the present invention is to provide a tetravalent bispecific antibody.

The second object of the present invention is to provide an isolated nucleotide encoding the tetravalent bispecific antibody.

The third object of the present invention is to provide an expression vector comprising the nucleotide.

The fourth object of the present invention is to provide a host cell comprising the expression vector.

The fifth object of the present invention is to provide a method for preparing the tetravalent bispecific antibody.

The sixth object of the present invention is to provide a pharmaceutical composition comprising the tetravalent bispecific antibody.

The seventh object of the present invention is to provide the use of the tetravalent bispecific antibody or of the pharmaceutical composition in the preparation of a medicine for the treatment of cancers, inflammatory diseases, autoimmune diseases and other disorders.

The eighth object of the present invention is to provide a method for constructing a tetravalent bispecific antibody.

The ninth object of the present invention is to provide a bispecific antibody comprising the heavy chain variable region comprising the amino acid sequence as shown in SEQ ID NO:83.

The tenth object of the present invention is to provide the use of the heavy chain variable region comprising the amino acid sequence as shown in SEQ ID NO: 83 for the construction of a bispecific antibody.

In order to achieve the above objects, the present invention provides the following technical solutions.

The first aspect of the present invention provides a tetravalent bispecific antibody, the tetravalent bispecific antibody comprises:
two polypeptide chains, the polypeptide chain comprising VH-B-CH1-peptide linker-VH-A-CH1-CH2-CH3 from N-terminus to C-terminus, the VH-A being a heavy chain variable region of a first antibody, the VH-B being a heavy chain variable region of a second antibody, the CH1 being a first domain of the heavy chain constant region, the CH2 being a second domain of the heavy chain constant region, the CH3 being a third domain of the heavy chain constant region, the first antibody specifically binding to a first antigen, the second antibody specifically binding to a second antigen;
four common light chains, the common light chain comprising VL-CL from N-terminus to C-terminus, the VL being the light chain variable region, the CL being the light chain constant region, and the VH-A-CH1 of the polypeptide chain and the VH-B-CH1 of the polypeptide chain being paired with the VL-CL of the common light chain, respectively, the VH-A and the VL forming a first antigen binding site, and the VH-B and the VL forming a second antigen binding site.

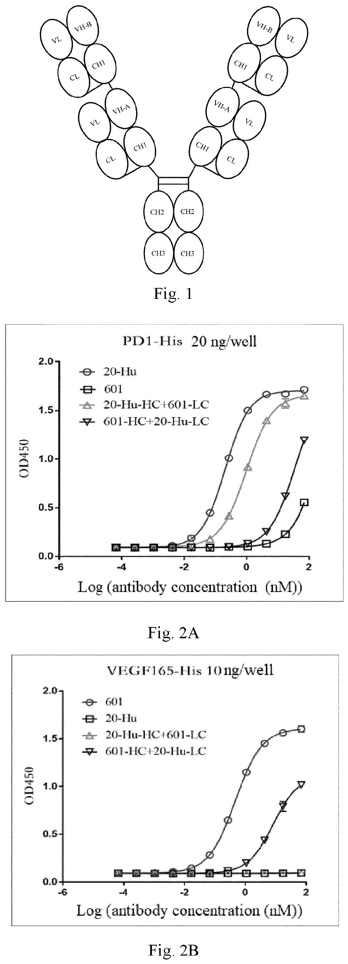

The structure of the bispecific antibody of the present invention is shown in Fig. 1.

According to the present invention, the common light chain is selected and obtained by the following method:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and :
(a) selecting the light chain of the second antibody as the common light chain, if the hybrid antibody formed by the combination of the heavy chain of the first antibody and the light chain of the second antibody can specifically bind to the first antigen;
(b) selecting the light chain of the first antibody as the common light chain, if the hybrid antibody formed by the combination of the heavy chain of the second antibody and the light chain of the first antibody can specifically bind to the second antigen;
(c) back-mutating the light chain of the first antibody so that the hybrid antibody formed by the combination of the heavy chain of the second antibody and the mutant light chain of the first antibody can specifically bind to the second antigen, and then selecting the mutant light chain of the first antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen; or
(d) back-mutating the light chain of the second antibody so that the hybrid antibody formed by the combination of the heavy chain of the first antibody and the mutant light chain of the second antibody can specifically bind to the first antigen, and then selecting the mutant light chain of the second antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen.

For the above cases of (a) and (b), when the hybrid antibody can specifically bind to the antigen, as a preferred embodiment, the light chain may also be back-mutated to further improve the binding affinity of the hybrid antibody to the antigen. Therefore, further, the common light chain is selected and obtained by the following method:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and
(a') back-mutating the light chain of the second antibody so that the binding affinity of the hybrid antibody formed by the combination of the heavy chain of the first antibody and the mutant light chain of the second antibody to the first antigen is better than the binding affinity of the hybrid antibody formed by the combination of the heavy chain of the first antibody and the light chain of the second antibody to the first antigen, and slecting the mutant light chain of the second antibody as the common light chain, if the hybrid antibody formed by the combination of the heavy chain of the first antibody and the light chain of the second antibody can specifically bind to the first antigen;
(b') back-mutating the light chain of the first antibody so that the binding affinity of the hybrid antibody formed by the combination of the heavy chain of the second antibody and the mutant light chain of the first antibody to the second antigen is better than the binding affinity of the hybrid antibody formed by the combination of the heavy chain of the second antibody and the light chain of the first antibody to the second antigen, and then selecting the mutant light chain of the first antibody as the common light chain, if the hybrid antibody formed by the combination of the heavy chain of the second antibody and the light chain of the first antibody can specifically bind to the second antigen.

According to the present invention, the peptide linker is an artificial linker. Preferably, the artificial linker is selected from the group consisting of: G, GS, SG, GGS, GSG, SGG, GGG, GGGS, SGGG, GGGGS, GGGGSGS, GGGGSGGS, GGGGSGGGGS, GGGGSGGGGSGGGGS (SEQ ID NO: 150), AKTTPKLEEGEFSEAR, AKTTPKLEEGEFSEARV, AKTTPKLGG, SAKTTPKLGG, SAKTTP, RADAAP, RADAAPTVS, RADAAAAGGPGS, SAKTTPKLEEGEFSEARV, ADAAP, ADAAPTVSIFPP, TVAAP, TVAAPSVFIFPP, QPKAAP, QPKAAPSVTLFPP, AKTTPP, AKTTPPSVTPLAP, AKTTAPSVYPLAP, ASTKGP, ASTKGPSVFPLAP, GENKVEYAPALMALS, GPAKELTPLKEAKVS, GHEAAAVMQVQYPAS, etc. More preferably, the artificial linker is GGGGSGGGGSGGGGS (SEQ ID NO: 150).

According to the present invention, the CH1 domain which is near the N-terminus of the polypeptide chain and CH1-CH2-CH3 may be derived from heavy chain constant regions of the same or different subtypes, including the heavy chain constant region of the group consisting of: IgG1, IgG2, IgG3, and IgG4, and preferably derived from the heavy chain constant region of IgG1 or IgG4. Preferably, the IgG4 comprises the S228P mutation (according to EU numbering).

According to the present invention, the CL is κ light chain constant region or λ light chain constant region.

According to a preferred embodiment of the present invention, the first antigen and the second antigen are selected from the group consisting of: VEGF/PD-1, PD-1/VEGF, TGF-β/PD-1, PD-1/ TGF-β, HER2/CD47, CD47/HER2, HER2/CD137, CD137/HER2, PD-1/CD137, CD137/PD-1, PD-1/CD40, CD40/PD-1, PD-1/EGFR, EGFR/PD-1, PD-1/HER2, HER2/PD-1, PD-1/CTLA-4, CTLA-4/PD-1, PD-1/LAG-3, LAG-3/PD-1.

According to a preferred embodiment of the present invention, the VH-A or the VH-B has the sequence as shown in SEQ ID NO: 83.

According to a preferred embodiment of the present invention, the VH-A, the VH-B and the VL have the sequences selected from the group consisting of: SEQ ID NOs: 1, 11, 15; SEQ ID NOs: 11, 1, 15; SEQ ID NOs: 20, 11, 15; SEQ ID NOs: 11, 20, 15; SEQ ID NOs: 29, 41, 42; SEQ ID NOs: 41, 29, 42; SEQ ID NOs: 31, 41, 42; SEQ ID NOs: 41, 31, 42; SEQ ID NOs: 53, 61, 54; SEQ ID NOs: 61, 53, 54; SEQ ID NOs: 53, 77, 54; SEQ ID NOs: 77, 53, 54; SEQ ID NOs: 83, 91, 92; SEQ ID NOs: 91, 83, 92; SEQ ID NOs: 83, 105, 106; SEQ ID NOs: 105, 83, 106; SEQ ID NOs: 83, 113, 114; SEQ ID NOs: 113, 83, 114; SEQ ID NOs: 83, 117, 118; SEQ ID NOs: 117, 83, 118; SEQ ID NOs: 83, 119, 120; SEQ ID NOs: 119, 83, 120; SEQ ID NOs: 83, 121, 122 and SEQ ID NOs: 121, 83, 122.

According to a preferred embodiment of the present invention, the polypeptide chain and the common light chain have the sequences selected from the group consisting of: SEQ ID NOs: 16, 13; SEQ ID NOs: 18, 13; SEQ ID NOs: 21, 13; SEQ ID NOs: 45, 43; SEQ ID NOs: 47, 43; SEQ ID NOs: 49, 43; SEQ ID NOs: 51, 43; SEQ ID NOs: 65, 63; SEQ ID NOs: 67, 63; SEQ ID NOs: 79, 63; SEQ ID NOs: 81, 63; SEQ ID NOs: 95, 93; SEQ ID NOs: 97, 93; SEQ ID NOs: 109, 107; SEQ ID NOs: 111, 107; SEQ ID NOs: 131, 123; SEQ ID NOs: 133, 125; SEQ ID NOs: 135, 127; SEQ ID NOs: 137, 127; SEQ ID NOs: 139, 127; SEQ ID NOs: 141, 127; SEQ ID NOs: 143, 129 and SEQ ID NOs: 145, 129.

The second aspect of the present invention provides an isolated nucleotide, which encodes the tetravalent bispecific antibody.

The third aspect of the present invention provides an expression vector, which comprises the nucleotide.

According to a preferred embodiment of the present invention, the nucleotide encodes the polypeptide chain and the common light chain, and the nucleotide has the sequence selected from the group consisting of: SEQ ID NOs: 17, 14; SEQ ID NOs: 19, 14; SEQ ID NOs: 22, 14; SEQ ID NOs: 46, 44; SEQ ID NOs: 48, 44; SEQ ID NOs: 50, 44; SEQ ID NOs: 52, 44; SEQ ID NOs: 66, 64; SEQ ID NOs: 68, 64; SEQ ID NOs: 80, 64; SEQ ID NOs: 82, 64; SEQ ID NOs: 96, 94; SEQ ID NOs: 98, 94; SEQ ID NOs: 110, 108; SEQ ID NOs: 112, 108; SEQ ID NOs: 132, 124; SEQ ID NOs: 134, 126; SEQ ID NOs: 136, 128; SEQ ID NOs: 138, 128; SEQ ID NOs: 140, 128; SEQ ID NOs: 142, 128; SEQ ID NOs: 144, 130 and SEQ ID NOs: 146, 130.

The fourth aspect of the present invention provides a host cell, which comprises the expression vector.

The fifth aspect of the present invention provides a method for preparing the tetravalent bispecific antibody, which comprises the following steps of:
a) culturing the host cell under expression conditions, to express the tetravalent bispecific antibody;
b) isolating and purifying the tetravalent bispecific antibody of step a).

The sixth aspect of the present invention provides a pharmaceutical composition, which comprises the tetravalent bispecific antibody as described above and a pharmaceutically acceptable carrier.

The seventh aspect of the present invention provides the use of the tetravalent bispecific antibody or of the pharmaceutical composition in the preparation of a medicine for the treatment of cancers, inflammatory diseases, autoimmune diseases and other disorders. The present invention also provides a method for treating cancers, inflammatory diseases, autoimmune diseases and other disorders, which comprises administering the tetravalent bispecific antibody or the pharmaceutical composition to a subject in need. The cancers include, but are not limited to: melanoma (e.g., metastatic malignant melanoma), kidney cancer (e.g., clear cell carcinoma), prostate cancer (e.g., hormone refractory prostate adenocarcinoma), pancreatic cancer, breast cancer, colon cancer, lung cancer (e.g., non-small cell lung cancer), esophageal cancer, head and neck squamous cell carcinoma, liver cancer, ovarian cancer, cervical cancer, thyroid cancer, glioblastoma, glioma, leukemia, lymphoma and other neoplastic malignant diseases. The inflammatory diseases, autoimmune diseases and other disorders include, but are not limited to: ophthalmological disorders, fibrosis, asthma, rheumatoid arthritis, systemic lupus erythematosus, multiple sclerosis, psoriasis, atopic dermatitis, etc. And, the subject includes but is not limited to human.

The eighth aspect of the present invention provides a method for constructing a tetravalent bispecific antibody, which comprises the following steps of:
(a) connecting a heavy chain variable region VH-B of a second antibody with a first domain CH1 of a heavy chain constant region, and the second antibody specifically binding to a second antigen;
(b) connecting (a) with a heavy chain variable region VH-A of a first antibody through a peptide linker, and the first antibody specifically binding to a first antigen;
(c) connecting (b) with a heavy chain constant region CH1-CH2-CH3, to form a polypeptide chain;
(d) respectively constructing (c) and the common light chain VL-CL into an expression vector, for combination expression, so as to obtain the tetravalent bispecific antibody of interest; and
   the CH1 is the first domain of the heavy chain constant region, the CH2 is a second domain of the heavy chain constant region, and the CH3 is a third domain of the heavy chain constant region, the VL is a light chain variable region, and the CL is a the light chain constant region, the VH-A-CH1 and the VH-B-CH1 are paired with the VL-CL, respectively, the VH-A and the VL form a first antigen binding site, the VH-B and the VL form a second antigen binding site.

The ninth aspect of the present invention provides a bispecific antibody, which comprises at least two different heavy chain variable regions and at least two common light chains, the common light chains comprise a same light chain variable region, and the heavy chain variable regions and the light chain variable regions form antigen binding sites, and the heavy chain variable regions comprise an amino acid sequence as shown in SEQ ID NO: 83.

The tenth aspect of the present invention provides the use of the heavy chain variable region comprising the amino acid sequence as shown in SEQ ID NO: 83 for constructing a bispecific antibody, the bispecific antibody comprises at least two different heavy chain variable regions and at least two common light chains, the common light chains comprising a same light chain variable region, and the heavy chain variable regions and the light chain variable regions form antigen binding sites.

In the present invention, the terms "antibody (abbreviated as Ab)" and "immunoglobulin G (abbreviated as IgG)" are heterotetrameric glycoproteins of about 150,000 daltons with identical structural characteristics, composed of two identical light chains (LC) and two identical heavy chains (HC). Each light chain is linked to a heavy chain by one covalent disulfide bond, while the number of disulfide linkages varies between the heavy chains of different immunoglobulin isotypes. Each heavy chain and light chain also has regularly spaced intrachain disulfide bridges. Each heavy chain has at one end a variable region (VH) followed by constant regions. The heavy chain constant region is composed of three structural domains, CH1, CH2, and CH3. Each light chain has a variable region (VL) at one end and a constant region at its other end, the light chain constant region includes a domain CL; the constant region of the light chain is paired with the CH1 domain of the constant region of the heavy chain, and the variable region of the light chain is paired with the variable region of the heavy chain. The constant regions are not involved directly in binding an antibody to an antigen, but they exhibit various effector functions, such as participation in antibodydependent cell-mediated cytotoxicity (ADCC). The heavy chain constant region includes IgG1, IgG2, IgG3, IgG4 subtypes; the light chain constant region includes κ (Kappa) or λ (Lambda). The heavy chain and light chain of the antibody are covalently linked together by the disulfide bond between the CH1 domain of the heavy chain and the CL domain of the light chain, and the two heavy chains of the antibody are covalently linked together by inter-polypeptide disulfide bonds formed between the hinge regions.

In the present invention, the term "bispecific antibody (BsAb)" refers to an antibody molecule that can specifically bind to two antigens (targets) or two epitopes at the same time.

In the present invention, the term "monoclonal antibody (mAb)" refers to an antibody obtained from a population of substantially homogeneous antibodies, that is, the individual antibodies contained in the population are the same, except for a few possible naturally occurring mutations. Monoclonal antibodies target a single antigen site with high specificity. Moreover, unlike conventional polyclonal antibody preparations (usually a mixture having different antibodies directed against different antigen determinants), each monoclonal antibody is directed against a single determinant on the antigen. Besides their specificity, the benefit of monoclonal antibodies is that they are synthesized by hybridoma culture and are not contaminated by other immunoglobulins. The modifier "monoclonal" indicates the characteristics of an antibody, which is obtained from a substantially uniform antibody population, and should not be interpreted as requiring any special method to produce the antibody.

In the present invention, the terms "Fab" and "Fc" mean that papain can cleave an antibody into two identical Fab segments and one Fc segment. The Fab segment is composed of the VH and CH1 of the heavy chain of the antibody and the VL and CL domains of the light chain. The Fc segment can be a fragment crystallizable (Fc), which is composed of the CH2 and CH3 domains of the antibody. The Fc segment has no antigen binding activity and is the site where the antibody interacts with effector molecules or cells.

In the present invention, the term "variable" refers to the fact that certain portions of the variable regions differ extensively in sequence among antibodies and are responsible for the binding specificity of each particular antibody for its particular antigen. However, the variability is not evenly distributed through the variable regions of antibodies. It is concentrated in three segments called complementarity determining regions (CDRs) or hypervariable regions both in the light chain variable regions and the heavy chain variable regions. The more highly conserved portions of the variable regions are called the framework regions (FR). The variable regions of native heavy chains and light chains each comprise four FR regions, largely adopting a β-sheet configuration, connected by three CDRs, which form loops connecting, and in some cases forming part of the β-sheet structure. The CDRs in each chain are held together in close proximity by the FR regions and, with the CDRs from the other chain, contribute to the formation of the antigen binding site of antibodies (see Kabat et al., NIH Publ.No.91-3242, Volume I, pages 647-669 (1991)).

In the present invention, the term "murine antibody" refers to an antibody derived from a rat or a mouse, preferably a mouse.

In the present invention, the term "humanized antibody" means that the CDRs are derived from a non-human (preferably, mouse) antibody, while the remaining parts (including framework regions and constant regions) are derived from a human antibody. In addition, framework region residues may be altered to preserve the binding affinity.

In the present invention, the terms "specifically bind/specific binding" and "bind/binding" refer to a non-random binding reaction between two molecules, such as the reaction between an antibody and the antigen it is directed against. Generally, the antibody binds to the antigen with an equilibrium dissociation constant (KD) of less than about 10⁻⁷M, for example, less than about 10⁻⁸M, 10⁻⁹M, 10⁻¹⁰M, 10⁻¹¹M or less. In the present invention, the term "KD" refers to the equilibrium dissociation constant of a specific antibody-antigen interaction, which is used to describe the binding affinity between the antibody and the antigen. The smaller the equilibrium dissociation constant is, the tighter the antibody-antigen binding is, and the higher the affinity between the antibody and the antigen is. For example, surface plasmon resonance (abbreviated as SPR) is used to measure the binding affinity of antibody to antigen in BIACORE instrument or ELISA is used to measure the relative binding affinity of antibody to antigen.

In the present invention, the term "valency" refers to the presence of a specified number of antigen binding sites in an antibody molecule. Preferably, the bispecific antibody of the present invention has four antigen binding sites and is tetravalent. In the present invention, the antigen binding site includes heavy chain variable region (VH) and light chain variable region (VL).

In the present invention, the term "epitope" refers to a polypeptide determinant that specifically binds to an antibody. The epitope of the present invention is a region of an antigen that is bound by an antibody.

In the present invention, the term "peptide linker" refers to a peptide having an amino acid sequence. The peptide linker of the present invention is a native linker or an artificial linker. Preferably, the peptide linker of the present invention is an artificial linker. The polypeptide linker of the present invention can be selected from G, GS, SG, GGS, GSG, SGG, GGG, GGGS, SGGG, GGGGS, GGGGSGS, GGGGSGGS, GGGGSGGGGS, GGGGSGGGGSGGGGS (SEQ ID NO: 150), AKTTPKLEEGEFSEAR, AKTTPKLEEGEFSEARV, AKTTPKLGG, SAKTTPKLGG, SAKTTP, RADAAP, RADAAPTVS, RADAAAAGGPGS, SAKTTPKLEEGEFSEARV, ADAAP, ADAAPTVSIFPP, TVAAP, TVAAPSVFIFPP, QPKAAP, QPKAAPSVTLFPP, AKTTPP, AKTTPPSVTPLAP, AKTTAPSVYPLAP, ASTKGP, ASTKGPSVFPLAP, GENKVEYAPALMALS, GPAKELTPLKEAKVS and GHEAAAVMQVQYPAS, etc. The linker can also be a peptide linker that may be cleavable in vivo, a protease (such as MMP) sensitive linker, a disulfide bond-based linker that may be cleaved by reduction, etc., see previously described "Fusion Protein Technologies for Biopharmaceuticals: Applications and Challenges, edited by Stefan R. Schmidt", or any cleavable linker known in the art. These cleavable linkers can be used to release the Fab at the top of the molecule in vivo to improve tissue penetration and distribution, enhance the binding to target, reduce potential side effects, and adjust in vivo functions and half-lifves of two different Fab regions. Most preferably, the artificial linker of the present invention is GGGGSGGGGSGGGGS (SEQ ID NO: 150).

In the present invention, the term "common light chain" refers to a light chain comprising the same light chain variable region and light chain constant region, which can pair with the heavy chain of a first antibody that binds to a first antigen, to form a binding site that specifically binds to the first antigen, and can also pair with the heavy chain of a second antibody that binds to a second antigen, to form a binding site that specifically binds to the second antigen. Further, the light chain variable region of the common light chain and the heavy chain variable region of the first antibody form a first antigen binding site, and the light chain variable region of the common light chain and the heavy chain variable region of the second antibody form a second antigen binding site.

In the present invention, the term "expression vector" may be pTT5, pSECtag series, pCGS3 series, pCDNA series vectors, as well as other vectors used in mammalian expression systems, etc. The expression vector comprises a fusion DNA sequence connected with appropriate transcription and translation regulatory sequences.

In the present invention, the term "host cell" refers to a cell suitable for expressing the expression vector as described above, which may be a eukaryotic cell, for example, mammalian or insect host cell culture system may be used to express the fusion protein of the present invention, CHO (Chinese Hamster Ovary), HEK293, COS, BHK, as well as derived cells of the above-mentioned cells are applicable to the present invention.

In the present invention, the term "pharmaceutical composition" means that the tetravalent bispecific antibody of the present invention can be combined with a pharmaceutically acceptable carrier to form a pharmaceutical preparation composition, so as to exert a therapeutic effect more stably. These preparations can ensure the conformational integrity of the amino acid core sequences of the tetravalent bispecific antibody disclosed in the present invention, and meanwhile, protect the multifunctional groups of the protein from degradation (including but not limited to aggregation, deamination or oxidation).

The present invention describes a tetravalent bispecific antibody having symmetrical structure based on common light chains and a construction method therefor. The bispecific antibody prepared by the present invention has similar or even better biological activity and physical and chemical properties than those of monoclonal antibodies, and can be used for the treatment of various inflammatory diseases, cancers, and other diseases.

### Description of the Drawings

Fig. 1 is a schematic diagram of the structure of the bispecific antibody of the present invention, where VH-A represents the heavy chain variable region of the first antibody, VH-B represents the heavy chain variable region of the second antibody, VL represents the light chain variable region of the common light chain; CH1, CH2, and CH3 represent the three domains of the heavy chain constant region, CL represents the light chain constant region of the common light chain, the line between two heavy chains represents a disulfide bond, the line between heavy chain and light chain also represents a disulfide bond, the line between CH1 which is near the N-terminus of the polypeptide chain and VH-A represents an artificially designed linker, and the line between CH1 which is near the C-terminus of the polypeptide chain and CH2 represents a native linker and hinge region of the antibody (if the heavy chain is human IgG4 subtype, the hinge region will contain the S228P mutation).
Figs. 2A and 2B show the ELISA results of 601 and 20-Hu and their hybrid antibodies.
Figs. 3A and 3B show the ELISA results of 20-Fab-601-IgG4-V94L and 601-Fab-20-IgG4-V94L.
Figs. 4A and 4B show the HPLC-SEC patterns of 20-Fab-601-IgG4-V94L.
Figs. 5A and 5B show the HPLC-IEC patterns of 20-Fab-601-IgG4-V94L.
Figs. 6A ~ 6D show the CE-SDS patterns of 20-Fab-601-IgG4-V94L.
Figs. 7A and Fig. 7B show the DSC patterns of 20-Fab-601-IgG4-V94L.
Fig. 8 shows the determination results of the ability of 20-Fab-0313-IgG4-V94L to neutralize the biological activity of VEGF.
Figs. 9A and 9B show the evaluatation results of the functional activity of 20-Fab-0313-IgG4-V94L to enhance MLR.
Fig. 10 shows an evaluation of the ability of 20-Fab-0313-IgG4-V94L to simultaneously bind to PD-1 and VEGF 165.
Fig. 11 shows the pharmacokinetic properties of 20-Fab-0313-IgG4-V94L.
Fig. 12 shows the anti-tumor growth curves of the bispecific antibodies against PD-1 and VEGF in mice.
Figs. 13A and 13B show the ELISA results of 1D11-Hu and 14-Hu and their hybrid antibodies.
Fig. 14 shows the ELISA results of mAb127, 14-Hu and their hybrid antibodies.
Figs. 15A and 15B show the ELISA results of 14-Fab-1D11-IgG4, 1D11-Fab-14-IgG4, 14-Fab-127-IgG4 and 127-Fab-14-IgG4.
Figs. 16A and 16B show the evaluatation results of the functional activity of 14-Fab-127-IgG4 to enhance MLR.
Fig. 17 shows the evaluatation of the ability of 14-Fab-127-IgG4 to simultaneously bind to TGF-β1 and PD-1.
Fig. 18 shows the anti-tumor growth curves of the bispecific antibodies against PD-1 and TGF-beta in mice.
Figs. 19A and 19B show the ELISA results of 19H6-Hu, Anti-CD47B-Hu and their hybrid antibodies.
Figs. 20A and 20B show the ELISA results of CD47B-Fab-19H6-IgG1 and 19H6-Fab-CD47B-IgG1.
Figs. 21A and 21B show the ELISA results of 19H6-Hu, 94-Hu and their hybrid antibodies.
Figs. 22A and 22B show the ELISA results of 94-Fab-19H6-IgG1-LALA and 19H6-Fab-94-IgG1-LALA.
Figs. 23A and 23B show the ELISA results of 609, Anti-CD137-Hu and their hybrid antibodies.
Figs. 24A and 24B show the ELISA results of 609-Fab-137-IgG4 and 137-Fab-609-IgG4.
Figs. 25A and 25B show the ELISA results of 609 and Anti-CD40-Hu and their hybrid antibodies.
Figs. 26A and 26B show the ELISA results of 609-Fab-40-IgG4 and 40-Fab-609-IgG4.
Fig. 27 shows the ELISA results of 609-HC+Cetuximab-LC, 609-HC+Bevacizumab-LC, 609-HC+Trastuzumab-LC, 609-HC+Pertuzumab-LC, 609-HC+Ipilimumab-LC and 609-HC+5E7-Hu-LC.
Fig. 28 shows the results of the ability of 609-HC+Cetuximab-LC, 609-HC+Bevacizumab-LC, 609-HC+Trastuzumab-LC, 609-HC+Pertuzumab-LC, 609-HC+Ipilimumab-LC and 609-HC+5E7-Hu-LC to block the PD-1/PD-L1 interaction.
Fig. 29 shows the results of the ability of 609-HC+Cetuximab-LC, 609-HC+Bevacizumab-LC, 609-HC+Trastuzumab-LC, 609-HC+Pertuzumab-LC, 609-HC+Ipilimumab-LC and 609-HC+5E7-Hu-LC to enhance mixed lymphocyte reaction.
Fig. 30 shows the determination of the ability of 609, 609-HC+Cetuximab-LC, 609-HC+Bevacizumab-LC, 609-HC+Pertuzumab-LC, 609-HC+Ipilimumab-LC and 609-HC+5E7-Hu-LC to bind PD-1 on the cell surface by flow cytometry.
Fig. 31 shows the alanine scanning results of the light chain variable region of 609.
Figs. 32A and 32B show the ELISA results of 609-Fab-Cetuximab-IgG4.
Figs. 33A and 33B show the ELISA results of 609-Fab-Pertuzumab-IgG4.
Figs. 34A and 34B show the ELISA results of 609-Fab-Ipilimumab-IgG1, Ipilimumab-Fab-609-IgG1, 609-Fab-Ipilimumab-IgG4 and Ipilimumab-Fab-609-IgG4.
Fig. 35 shows the determination results of the functional activity of the bispecific antibodies against PD-1 and CTLA-4.
Fig. 36 shows the determination results of the ADCC activity of the bispecific antibodies against PD-1 and CTLA-4.
Fig. 37 shows the pharmacokinetics of the bispecific antibodies against PD-1 and CTLA-4 in rats.
Fig. 38 shows the anti-tumor growth curves of the bispecific antibodies against PD-1 and CTLA-4 in mice.
Figs. 39A and 39B show the ELISA results of 609-Fab-5E7-IgG4 and 5E7-Fab-609-IgG4.
Fig. 40 shows the determination of the ability of the bispecific antibodies against PD-1 and LAG-3 to simultaneously bind to two antigens.
Fig. 41 shows the determination of the functional activity of the bispecific antibodies against PD-1 and LAG-3.
Fig. 42 shows the anti-tumor growth curves of the bispecific antibodies against PD-1 and LAG-3 in mice.
Figs. 43A ~ 43E show the specificity tests of hybrid antibodies.
Figs. 44A ~ 44H show the HPLC-IEC patterns of 609-Fab-Cetuximab-IgG4, 609-Fab-Pertuzumab-IgG4, 609-Fab-Ipilimumab-IgG1, Ipilimumab-Fab-609-IgG1, 609-Fab-Ipilimumab-IgG4, Ipilimumab-Fab-609-IgG4, 609-Fab-5E7-IgG4 and 5E7-Fab-609-IgG4.
Figs. 45A and 45B show the HPLC-IEC patterns of 609-Fab-Ipilimumab-IgG1 and 609-Fab-5E7-IgG4.
Figs. 46A ~ 46D show the NR-CE-SDS and R-CE-SDS patterns of 609-Fab-Ipilimumab-IgG1 and 609-Fab-5E7-IgG4.

### Detailed Description of the Invention

The method of expressing and purifying antibodies used in the following examples is described as follows: Exogenous genes were constructed into the pcDNA3.4 (purchased from Thermo Fisher Scientific) expression vector, and the combination of expression vectors was transferred into HEK293F cells (purchased from Thermo Fisher Scientific) by the PEI (Polyethylenimine) transfection method to express the antibody, and then the antibody was purified by ProteinA affinity chromatography.

The ELISA detection method used in the following examples is described as follows: An ELISA plate was coated with recombinant proteins, respectively, and blocked with PBST containing 1% bovine serum albumin (PBST: phosphate buffer containing 0.05% Tween-20). The antibody to be tested was serially diluted, and then transferred to the above plate coated with recombinant protein, incubated at room temperature for half an hour and then the plate was washed; an appropriately diluted HRP (Horseradish Peroxidase)-labeled goat anti-human antibody (Fc- or Fab Specific, purchased from Sigma) was added, incubated at room temperature for half an hour and then the plate was washed; 100 µl of chromogenic solution with TMB (3,3',5,5'-Tetramethylbenzidine) as a substrate was added to each well, incubated at room temperature for 1~5 min; 50 µl of stop solution (2M H₂SO₄) was added to stop the reaction. OD450 was read with a microplate reader (SpectraMax 190), and graphing and data analysis were performed using GraphPad Prism6, and EC50 was calculated.

The method used in the following examples to evaluate the ability to enhance the mixed lymphocyte reaction (MLR) is described as follows: Peripheral blood mononuclear cells (PBMC) were separated from human blood using Histopaque (purchased from Sigma), and the monocytes in the PBMC were separated by adherence method, and then the monocytes were induced with IL-4 (25 ng/ml) and GM-CSF (25 ng/ml) to differentiate into dendritic cells. Seven days later, the aboveinduced dendritic cells were digested and collected. PBMCs were separated from the blood of other donors by the above method, and then CD4⁺ T cells were separated from PBMCs with MACS magnet and CD4 MicroBeads (purchased from Miltenyibiotec). The induced dendritic cells (10⁴/well) and the seperated CD4⁺ T cells (10⁵/well) were mixed in proportion and then inoculated into a 96-well plate, 150 µl per well; a few hours later, 50 µl of serially diluted antibody was added into the above 96-well plate; the 96-well plate was incubated in a 37°C cell incubator for 3 days. During the above experiment, AIM-V medium (purchased from Thermo Fisher Scientific) was used to culture the cells. Then, the secretion of IL-2 and IFN-γ was determined according to standard operating procedures. IL-2 and IFN-γ were determined using standard double-antibody sandwich ELISA (the paired antibodies for related detection were purchased from BD Biosciences). OD450 was read with a microplate reader (SpectraMax 190). Graphing was performed by GraphPad Prism6 and EC50 was calculated.

The methods for detecting physical and chemical properties used in the following examples are described as follows:

### HPLC-SEC

Antibodies are high molecular weight proteins with highly complex secondary and tertiary structures. Due to changes such as post-translational modification, aggregation, and degradation, antibodies are heterogeneous in their biochemical and biophysical properties. Variants, aggregates, and degraded fragments are commonly observed when bispecific antibodies are analyzed by separation techniques, and their presence may compromise safety and effectiveness. Aggregates, degraded fragments and incompletely assembled molecules are prone to appear during production and storage of an antibody. In the present invention, high-performance liquid chromatography - size exclusion chromatography (HPLC-SEC) was used to detect the content of the above impurities in a sample. The molecular weight of the aggregate is larger than that of the monomer, so the retention time of the corresponding peak is shorter; the molecular weight of the degraded fragment or the incompletely assembled molecule is smaller than that of the monomer, so the retention time of the corresponding peak is longer. Chromatograph used for HPLC-SEC: Dionex Ultimate 3000; the method for the preparation of mobile phase is as follows: an appropriate amount of 20 mM sodium dihydrogen phosphate mother liquor is adjusted with 20 mM sodium dihydrogen phosphate to a pH of 6.8 ± 0.1; injection volume: 20 µg; chromatograph column: TSK G3000SWXL, specification: 7.8×300 mm 5 µm; flow rate: 0.5 ml/min, elution time: 30 min; column temperature: 25°C, sample room temperature: 10°C; detection wavelength: 214 nm.

### HPLC-IEC

Many post-translational modifications (such as N-glycosylation, C-terminal lysine residue modification, N-terminal glutamine or glutamate cyclization, asparagine deamidation, aspartic acid isomerization, amino acid residue oxidation, etc.) will directly or indirectly change the surface charge of the antibody, leading to the generation of charge heterogeneity. The charge variants can be separated and analyzed based on the charge. Commonly used analysis methods include cation exchange chromatography (CEX) and anion exchange chromatography (AEX). When analyzed by a chromatography-based method, acidic species and basic species are defined based on their retention time relative to the main peak. The acidic species are the variants that eluted earlier than the main peak of CEX or later than the main peak of AEX, while the basic species are the variants that eluted later than the main peak of CEX or earlier than the main peak of AEX. The peaks corresponding to the acidic species and the basic species are called acidic peaks and basic peaks, respectively. Charge variants are easily generated during the production and storage of antibodies. Here, high-performance liquid chromatography-ion exchange chromatography (HPLC-IEC) was used to analyze the charge heterogeneity of the samples. Chromatograph used in HPLC-IEC was Dionex Ultimate 3000; mobile phase A: 20 mM PB pH 6.3, mobile phase B: 20 mM PB + 200 mM NaCl pH 6.3, the mixing ratio of the two mobile phases changed with time according to the preset program, flow rate 1.0 ml/min; chromatographic column: Thermo Propac^{™} WCX-10; column temperature: 30°C, sample room temperature: 10°C; injection volume: 20 µg; detection wavelength: 214 nm.

### CE-SDS

In the present invention, CE-SDS (Capillary Electrophoresis-Sodium Dodecyl Sulfate) was used to analyze the content of degraded fragments or incompletely assembled molecules in the sample. CE is divided into two types: non-reduced and reduced; for the former, when the sample is denatured, the reducing agent DTT is not needed to destroy the disulfide bond in the molecule; for the latter, when the sample is denatured, the reducing agent DTT is needed to destroy the disulfide bond in the molecule. Non-reduced and reduced CE-SDS are denoted as NR-CE-SDS and R-CE-SDS, respectively. The capillary electrophoresis instrument used was ProteomeLab^{™} PA800 plus (Beckman Coulter), equipped with a UV 214 nm detector, capillary model: Bare Fused-Silica Capillary, specification: 30.7 cm×50 µm, effective length: 20.5 cm; other related reagents were purchased from Beckman Coulter. The key parameters of the instrument were set as follows: temperature of capillary and sample chamber: 20±2°C, separation voltage: 15 kV.

### DSC

Differential Scanning Calorimeter (DSC) reflects the thermal stability of the sample mainly by detecting the heat change in biomolecules in a controlled heating or cooling process. By heating, the unfolding of the protein sample will absorb heat, and the supplementary energy required to eliminate the temperature difference in the sample pool will be recorded by the device. These heat changes will form a peak shape in the spectrum. The peak top temperature corresponding to the unfolding of the protein sample is taken as the melting temperature Tm. Tm is an important indicator of protein thermal stability. The higher the Tm is, the better the stability of the protein is.

The sequence information involved in the present invention is summarized in Table 1.

**Table 1. Sequence information of the antibodies of the present invention**

| SEQ ID NO: | Sequence name |
|---|---|
| 1 | Amino acid sequence of heavy chain variable region of Bevacizumab (601) |
| 2 | Amino acid sequence of light chain variable region of Bevacizumab (601) |
| 3 | Amino acid sequence of heavy chain variable region of murine antibody No.20 |
| 4 | Amino acid sequence of light chain variable region of murine antibody No.20 |
| 5 | Amino acid sequence of heavy chain complementarity determining region H-CDR1 of murine antibody No.20 |
| 6 | Amino acid sequence of heavy chain complementarity determining region H-CDR2 of murine antibody No.20 |
| 7 | Amino acid sequence of heavy chain complementarity determining region H-CDR3 of murine antibody No.20 |
| 8 | Amino acid sequence of light chain complementarity determining region L-CDR1 of murine antibody No.20 |
| 9 | Amino acid sequence of light chain complementarity determining region L-CDR2 of murine antibody No.20 |
| 10 | Amino acid sequence of light chain complementarity determining region L-CDR3 of murine antibody No.20 |
| 11 | Amino acid sequence of heavy chain variable region of 20-Hu |
| 12 | Amino acid sequence of light chain variable region of 20-Hu |
| 13 | Amino acid sequence of 601-LC-V94L |
| 14 | Nucleotide sequence of 601-LC-V94L |
| 15 | Amino acid sequence of light chain variable region of 601-LC-V94L |
| 16 | Amino acid sequence of 20-Fab-601-IgG4 |
| 17 | Nucleotide sequence of 20-Fab-601-IgG4 |
| 18 | Amino acid sequence of 601-Fab-20-IgG4 |
| 19 | Nucleotide sequence of 601-Fab-20-IgG4 |
| 20 | Amino acid sequence of heavy chain variable region of Y0313-1 |
| 21 | Amino acid sequence of 20-Fab-0313-IgG4 |
| 22 | Nucleotide sequence of 20-Fab-0313-IgG4 |
| 23 | Amino acid sequence of heavy chain complementarity determining region H-CDR1 of murine antibody No. 1D11 |
| 24 | Amino acid sequence of heavy chain complementarity determining region H-CDR2 of murine antibody No. 1D11 |
| 25 | Amino acid sequence of heavy chain complementarity determining region H-CDR3 of murine antibody No. 1D11 |
| 26 | Amino acid sequence of light chain complementarity determining region L-CDR1 of murine antibody No. 1D11 |
| 27 | Amino acid sequence of light chain complementarity determining region L-CDR2 of murine antibody No. 1D11 |
| 28 | Amino acid sequence of light chain complementarity determining region L-CDR3 of murine antibody No. 1D11 |
| 29 | Amino acid sequence of heavy chain variable region of 1D11-Hu |
| 30 | Amino acid sequence of light chain variable region of 1D11-Hu |
| 31 | Amino acid sequence of heavy chain variable region of mAb127 |
| 32 | Amino acid sequence of light chain variable region of mAb127 |
| 33 | Amino acid sequence of heavy chain variable region of murine antibody No. 14 |
| 34 | Amino acid sequence of light chain variable region of murine antibody No. 14 |
| 35 | Amino acid sequence of heavy chain complementarity determining region H-CDR1 of murine antibody No. 14 |
| 36 | Amino acid sequence of heavy chain complementarity determining region H-CDR2 of murine antibody No. 14 |
| 37 | Amino acid sequence of heavy chain complementarity determining region H-CDR3 of murine antibody No. 14 |
| 38 | Amino acid sequence of light chain complementarity determining region L-CDR1 of murine antibody No. 14 |
| 39 | Amino acid sequence of light chain complementarity determining region L-CDR2 of murine antibody No. 14 |
| 40 | Amino acid sequence of light chain complementarity determining region L-CDR3 of murine antibody No. 14 |
| 41 | Amino acid sequence of heavy chain variable region of 14-Hu |
| 42 | Amino acid sequence of light chain variable region of 14-Hu |
| 43 | Amino acid sequence of 14-Hu-LC |
| 44 | Nucleotide sequence of 14-Hu-LC |
| 45 | Amino acid sequence of 14-Fab-1D11-IgG4 |
| 46 | Nucleotide sequence of 14-Fab-1D11-IgG4 |
| 47 | Amino acid sequence of 1D11-Fab-14-IgG4 |
| 48 | Nucleotide sequence of 1D11-Fab-14-IgG4 |
| 49 | Amino acid sequence of 14-Fab-127-IgG4 |
| 50 | Nucleotide sequence of 14-Fab-127-IgG4 |
| 51 | Amino acid sequence of 127-Fab-14-IgG4 |
| 52 | Nucleotide sequence of 127-Fab-14-IgG4 |
| 53 | Amino acid sequence of heavy chain variable region of 19H6-Hu |
| 54 | Amino acid sequence of light chain variable region of 19H6-Hu |
| 55 | Amino acid sequence of heavy chain complementarity determining region H-CDR1 of Anti-CD47B |
| 56 | Amino acid sequence of heavy chain complementarity determining region H-CDR2 of Anti-CD47B |
| 57 | Amino acid sequence of heavy chain complementarity determining region H-CDR3 of Anti-CD47B |
| 58 | Amino acid sequence of light chain complementarity determining region L-CDR1 of Anti-CD47B |
| 59 | Amino acid sequence of light chain complementarity determining region L-CDR2 of Anti-CD47B |
| 60 | Amino acid sequence of light chain complementarity determining region L-CDR3 of Anti-CD47B |
| 61 | Amino acid sequence of heavy chain variable region of Anti-CD47B-Hu |
| 62 | Amino acid sequence of light chain variable region of Anti-CD47B-Hu |
| 63 | Amino acid sequence of 19H6-Hu-LC |
| 64 | Nucleotide sequence of 19H6-Hu-LC |
| 65 | Amino acid sequence of CD47B-Fab-19H6-IgG1 |
| 66 | Nucleotide sequence of CD47B-Fab-19H6-IgG1 |
| 67 | Amino acid sequence of 19H6-Fab-CD47B-IgG1 |
| 68 | Nucleotide sequence of 19H6-Fab-CD47B-IgG1 |
| 69 | Amino acid sequence of heavy chain variable region of murine antibody No. 94 |
| 70 | Amino acid sequence of light chain variable region of murine antibody No. 94 |
| 71 | Amino acid sequence of heavy chain complementarity determining region H-CDR1 of murine antibody No. 94 |
| 72 | Amino acid sequence of heavy chain complementarity determining region H-CDR2 of murine antibody No. 94 |
| 73 | Amino acid sequence of heavy chain complementarity determining region H-CDR3 of murine antibody No. 94 |
| 74 | Amino acid sequence of light chain complementarity determining region L-CDR1 |
| | of murine antibody No. 94 |
| 75 | Amino acid sequence of light chain complementarity determining region L-CDR2 of murine antibody No. 94 |
| 76 | Amino acid sequence of light chain complementarity determining region L-CDR3 of murine antibody No. 94 |
| 77 | Amino acid sequence of heavy chain variable region of 94-Hu |
| 78 | Amino acid sequence of light chain variable region of 94-Hu |
| 79 | Amino acid sequence of 94-Fab-19H6-IgG1-LALA |
| 80 | Nucleotide sequence of 94-Fab-19H6-IgG1-LALA |
| 81 | Amino acid sequence of 19H6-Fab-94-IgG1-LALA |
| 82 | Nucleotide sequence of 19H6-Fab-94-IgG1-LALA |
| 83 | Amino acid sequence of heavy chain variable region of mAb1-25-Hu (609) |
| 84 | Amino acid sequence of light chain variable region of mAb1-25-Hu (609) |
| 85 | Amino acid sequence of heavy chain complementarity determining region H-CDR1 of Anti-CD 13 7 |
| 86 | Amino acid sequence of heavy chain complementarity determining region H-CDR2 of Anti-CD 13 7 |
| 87 | Amino acid sequence of heavy chain complementarity determining region H-CDR3 of Anti-CD 13 7 |
| 88 | Amino acid sequence of light chain complementarity determining region L-CDR1 of Anti-CD 13 7 |
| 89 | Amino acid sequence of light chain complementarity determining region L-CDR2 of Anti-CD 13 7 |
| 90 | Amino acid sequence of light chain complementarity determining region L-CDR3 of Anti-CD 13 7 |
| 91 | Amino acid sequence of heavy chain variable region of Anti-CD 137-Hu |
| 92 | Amino acid sequence of light chain variable region of Anti-CD137-Hu |
| 93 | Amino acid sequence of Anti-CD 13 7-Hu-LC |
| 94 | Nucleotide sequence of Anti-CD 13 7-Hu-LC |
| 95 | Amino acid sequence of 609-Fab-137-IgG4 |
| 96 | Nucleotide sequence of 609-Fab-137-IgG4 |
| 97 | Amino acid sequence of 137-Fab-609-IgG4 |
| 98 | Nucleotide sequence of 137-Fab-609-IgG4 |
| 99 | Amino acid sequence of heavy chain complementarity determining region H-CDR1 of Anti-CD40 |
| 100 | Amino acid sequence of heavy chain complementarity determining region H-CDR2 of Anti-CD40 |
| 101 | Amino acid sequence of heavy chain complementarity determining region H-CDR3 of Anti-CD40 |
| 102 | Amino acid sequence of light chain complementarity determining region L-CDR1 of Anti-CD40 |
| 103 | Amino acid sequence of light chain complementarity determining region L-CDR2 of Anti-CD40 |
| 104 | Amino acid sequence of light chain complementarity determining region L-CDR3 of Anti-CD40 |
| 105 | Amino acid sequence of heavy chain variable region of Anti-CD40-Hu |
| 106 | Amino acid sequence of light chain variable region of Anti-CD40-Hu |
| 107 | Amino acid sequence of Anti-CD40-Hu-LC |
| 108 | Nucleotide sequence of Anti-CD40-Hu-LC |
| 109 | Amino acid sequence of 609-Fab-40-IgG4 |
| 110 | Nucleotide sequence of 609-Fab-40-IgG4 |
| 111 | Amino acid sequence of 40-Fab-609-IgG4 |
| 112 | Nucleotide sequence of 40-Fab-609-IgG4 |
| 113 | Amino acid sequence of heavy chain variable region of Cetuximab |
| 114 | Amino acid sequence of light chain variable region of Cetuximab |
| 115 | Amino acid sequence of heavy chain variable region of Trastuzumab |
| 116 | Amino acid sequence of light chain variable region of Trastuzumab |
| 117 | Amino acid sequence of heavy chain variable region of Pertuzumab |
| 118 | Amino acid sequence of light chain variable region of Pertuzumab |
| 119 | Amino acid sequence of heavy chain variable region of 10D1 (Ipilimumab) |
| 120 | Amino acid sequence of light chain variable region of 10D1 (Ipilimumab) |
| 121 | Amino acid sequence of heavy chain variable region of 5E7-Hu |
| 122 | Amino acid sequence of light chain variable region of 5E7-Hu |
| 123 | Amino acid sequence of Cetuximab-LC |
| 124 | Nucleotide sequence of Cetuximab-LC |
| 125 | Amino acid sequence of Pertuzumab-LC |
| 126 | Nucleotide sequence of Pertuzumab-LC |
| 127 | Amino acid sequence of Ipilimumab-LC |
| 128 | Nucleotide sequence of Ipilimumab-LC |
| 129 | Amino acid sequence of 5E7-Hu-LC |
| 130 | Nucleotide sequence of 5E7-Hu-LC |
| 131 | Amino acid sequence of 609-Fab-Cetuximab-IgG4 |
| 132 | Nucleotide sequence of 609-Fab-Cetuximab-IgG4 |
| 133 | Amino acid sequence of 609-Fab-Pertuzumab-IgG4 |
| 134 | Nucleotide sequence of 609-Fab-Pertuzumab-IgG4 |
| 135 | Amino acid sequence of 609-Fab-Ipilimumab-IgG1 |
| 136 | Nucleotide sequence of 609-Fab-Ipilimumab-IgG1 |
| 137 | Amino acid sequence of Ipilimumb-Fab-609-IgG1 |
| 138 | Nucleotide sequence of Ipilimumab-Fab-609-IgG1 |
| 139 | Amino acid sequence of 609-Fab-Ipilimumab-IgG4 |
| 140 | Nucleotide sequence of 609-Fab-Ipilimumab-IgG4 |
| 141 | Amino acid sequence of Ipilimumab-Fab-609-IgG4 |
| 142 | Nucleotide sequence of Ipilimumab-Fab-609-IgG4 |
| 143 | Amino acid sequence of 609-Fab-5E7-IgG4 |
| 144 | Nucleotide sequence of 609-Fab-5E7-IgG4 |
| 145 | Amino acid sequence of 5E7-Fab-609-IgG4 |
| 146 | Nucleotide sequence of 5E7-Fab-609-IgG4 |
| 147 | Amino acid sequence of heavy chain constant region of IgG1 |
| 148 | Amino acid sequence of heavy chain constant region of IgG4 (S228P) |
| 149 | Amino acid sequence of kappa light chain constant region |
| 150 | Linker (GGGGSGGGGSGGGGS) |
| 151 | The fourth framework region of heavy chain (WGQGTLVTVSS) |
| 152 | The fourth framework region of light chain (FGQGTKVEIK) |
| 153 | The fourth framework region of light chain (FGGGTKVELK) |

The following examples and experimental examples are used to further illustrate the present invention and should not be construed as limiting the present invention. The examples do not include a detailed description of traditional methods, such as those methods of constructing expression vectors and plasmids, methods of inserting genes encoding proteins into such vectors and plasmids, or methods of transfecting plasmids into host cells. Such methods are well known to those of ordinary skill in the art, and are described in many publications, including Sambrook, J., Fritsch, E.F. and Maniais, T. (1989) Molecular Cloning: A Laboratory Manual, 2nd edition, Cold spring Harbor Laboratory Press.

### Example 1 Construction of bispecific antibodies against PD-1 and VEGF

### Example 1.1 Sequences

The sequences of heavy chain variable region and light chain variable region (SEQ ID NO: 1 and 2) of Bevacizumab (hereinafter referred to as 601) antibody were obtained from the publicly available literature (Magdelaine-Beuzelin C, Kaas Q, Wehbi V, et al. Structure-function relationships of the variable domains of monoclonal antibodies approved for cancer treatment[J]. Critical reviews in oncology/hematology, 2007, 64(3): 210-225). The DNAs encoding the above variable regions were synthesized by Shanghai Sangon Biotech Co., Ltd.. The heavy chain variable region (601-VH) and the light chain variable region (601-VL) of 601 were connected to the human IgG1 heavy chain constant region (SEQ ID NO: 147) and the human Kappa light chain constant region (SEQ ID NO: 149), respectively, to construct full-length heavy chain and light chain genes of the antibody 601, which were named as 601-HC and 601-LC, respectively.

According to Examples 1-5 in WO2018/137576A1, based on the selection results, the murine anti-human PD-1 monoclonal antibody No. 20 was finally selected as the lead antibody, and the nucleotide sequence of the heavy chain variable region and the nucleotide sequence of the light chain variable region were obtained, and translated into amino acid sequences (SEQ ID NOs: 3 and 4).

The amino acid sequences of the heavy chain variable region and the light chain variable region of the antibody No. 20 were analyzed, according to the Kabat scheme, the antigen complementarity determining regions and framework regions of the heavy chain and light chain of the antibody No. 20 were determined. The antibody No. 20 comprises the amino acid sequences of the heavy chain CDRs of H-CDR1: NYDMS (SEQ ID NO: 5), H-CDR2: TISGGGGYTYYSDSVKG (SEQ ID NO: 6) and H-CDR3: PYGHYGFEY (SEQ ID NO: 7), and the amino acid sequences of the light chain CDRs of L-CDR1: SASQGISNFLS (SEQ ID NO: 8), L-CDR2: YTSSLHS (SEQ ID NO: 9) and L-CDR3: QQYSNLPWT (SEQ ID NO: 10).

The homology comparison of the heavy chain variable region of the murine antibody No. 20 with the human IgG germline sequence was performed at https://www.ncbi.nlm.nih.gov/igblast/. IGHV3-21^{∗}01 was selected as the heavy chain CDR grafting template, the heavy chain CDRs of the murine antibody No. 20 were transplanted into the framework regions of IGHV3-21^{∗}01, and WGQGTLVTVSS (SEQ ID NO:151) was added following the H-CDR3 as the fourth framework region, to obtain a CDR-grafted heavy chain variable region sequence. Similarly, the homology comparison of the light chain variable region of the murine antibody No. 20 with the human IgG germline sequence was performed. IGKV1-39^{∗}01 was selected as the light chain CDR grafting template, the light chain CDRs of the murine antibody No. 20 were transplanted into the framework regions of IGKV1-39^{∗}01, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework regions were subjected to back-mutation (Back mutation is to mutate certain amino acids in human framework regions into the amino acids at the same position in murine framework region. The site of back mutation is generally criticial to maintain the structure and/or affinity of the antibody). When back-mutation was performed, the amino acid sequence was numbered according to Kabat and the position of each site was indicated by Kabat numbering.

Preferably, for the CDR-grafted heavy chain variable region, according to Kabat numbering, T at position 28 was back-mutated to murine V, G at position 44 was back-mutated to R, and R at position 94 was back-mutated to S. For the CDR-grafted light chain variable region, A at position 43 was back-mutated to T, P at position 44 was back-mutated to V, and F at position 71 was back-mutated to Y.

The above heavy chain variable region and light chain variable region with back mutation sites were defined as humanized heavy chain variable region and light chain variable region (SEQ ID NOs: 11 and 12), respectively. The DNAs encoding the humanized heavy chain and light chain variable regions were synthesized by Shanghai Sangon Biotech Co., Ltd.. The synthetic humanized heavy chain variable region was connected to the human IgG4 (with the S228P mutation in the hinge region) constant region (SEQ ID NO: 148) to obtain a full-length humanized heavy chain gene, named as 20-Hu-HC; the humanized light chain variable region was connected to the human Kappa chain constant region (SEQ ID NO: 149), to obtain a full-length humanized light chain gene, named as 20-Hu-LC.

The heavy chain and light chain genes of the above antibody were constructed into the pcDNA3.4 expression vector, respectively, and the resulting heavy chain and light chain expression vectors were transferred into HEK293F cells by PEI transfection method to express the antibody. After HEK293F cells were cultured in Free Style 293 Expression Medium for 5 days, the cell supernatant was collected, and the antibody was purified by Protein A affinity chromatography. The antibody obtained by the combination of 20-Hu-HC and 20-Hu-LC was named as 20-Hu.

### Example 1.2 Selection of common light chain

BLAST was used to compare and analyze the amino acid sequences of the light chain variable region of 20-Hu and the light chain variable region of 601. The results showed that between them, the identical amino acids accounted for 89% (Identities) and the amino acids with similar properties accounted for 94% (Positives).

The gene sequences of 601-HC and 601-LC were constructed into the pcDNA3.4 expression vector, respectively. The expression vectors of 20-Hu-HC, 20-Hu-LC, 601-HC and 601-LC were combined in the following manner: 20-Hu-HC+20-Hu-LC, 601-HC+601-LC, 20-Hu-HC+601-LC and 601-HC+20-Hu-LC, and the antibodies were expressed and purified. The obtained antibodies were named as 20-Hu, 601, 20-Hu-HC+601-LC and 601-HC+20-Hu-LC, respectively.

The ELISA detection method is as follows: the extracellular domain protein of human PD-1 with a 6^{∗}His tag was prepared by the inventors (the source of the extracellular domain of PD-1 is described in WO2018/137576A1), and this recombinant protein was denoted as PD1-His. Human VEGF165 with a 6^{∗}His tag (sequence from NCBI, Accession: AAM03108), and this recombinant protein was denoted as VEGF165-His. An ELISA plate was coated with PD1-His and VEGF165-His, with a coating concentration of 20 ng/well and 10 ng/well, respectively.

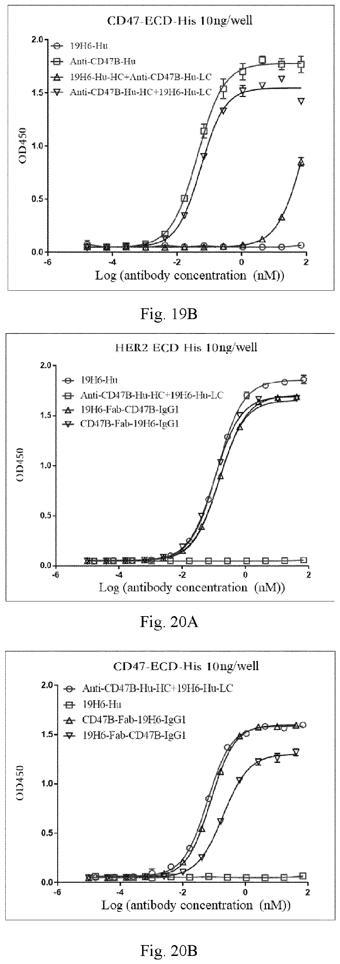

As shown in Fig. 2A, 20-Hu and 20-Hu-HC+601-LC can effectively bind to PD1-His, with EC50s of 0.2062 nM and 0.9747 nM, respectively; while 601 and 601-HC+20-Hu-LC cannot effectively bind to PD1-His, the EC50s cannot be calculated accurately. As shown in Fig. 2B, 601 and 601-HC+20-Hu-LC can effectively bind to VEGF165-His, with EC50s of 0.4681 nM and 8.217 nM, respectively; while 20-Hu and 20-Hu-HC+601-LC cannot effectively bind to VEGF165-His.

Compared to 20-Hu, the relative affinity of 20-Hu-HC+601-LC to PD1-His decreased significantly; compared to 601, the relative affinity of 601-HC+20-Hu-LC to VEGF165-His also decreased significantly. Here we tried to use back mutation to increase the relative affinity of 20-Hu-HC+601-LC to PD1-His. It was found by analysis that there were 12 amino acid residue differences between the light chain variable region of 601 and the light chain variable region of 20-Hu, among which the amino acid residues at positions 28, 32, 34, 46, 50, 71, 93 and 94 (according to the Kabat numbering scheme) may be critical to maintain the affinity of the antibody. Here, by site-directed mutagenesis, the amino acid residues at the above positions of 601-LC were mutated into amino acid residues at the corresponding positions of 20-Hu-LC, and these 601-LC with point mutations were denoted as 601-LC-D28G, 601-LC-Y32F, 601-LC-N34S, 601-LC-V46L, 601-LC-F50Y, 601-LC-F71Y, 601-LC-T93N and 601-LC-V94L, respectively.

The gene sequences of the above light chains were constructed into the pcDNA3.4 expression vector, respectively. 20-Hu-HC was combined with the above expression vector of 601-LC with point mutations, respectively, and the antibodies were expressed and purified. The obtained antibodies were named as 20-Hu-HC+601-LC-D28G, 20-Hu-HC+601-LC-Y32F, 20-Hu-HC+ 601-LC-N34S, 20-Hu-HC+601-LC-V46L, 20-Hu-HC+601-LC-F50Y, 20-Hu-HC+601-LC-F71Y, 20-Hu-HC+601-LC-T93N, and 20-Hu-HC+601-LC-V94L, respectively. The ELISA described in the above example was used to evaluate the relative affinity of the above antibodies to bind to PD-1, and 20-Hu-HC+601-LC was used as a reference. The ELISA results showed that the EC50s of the above mutant antibodies and 20-Hu-HC+601-LC were 0.4849 nM, 0.4561 nM, 0.1751 nM, 0.5333 nM, 0.5255 nM, 1.0345 nM, 0.4859 nM, 0.3079 nM and 0.6251 nM, respectively; compared to 20-Hu-HC+601-LC, 20-Hu-HC+601-LC-N34S and 20-Hu-HC+601-LC-V94L had significantly higher relative affinity to PD-1; other mutant antibodies had basically the same or even lower relative affinity than 20-Hu-HC+601-LC.

601-HC was combined with the above expression vector of 601-LC with point mutations and the antibodies were expressed and purified. The obtained antibodies were named as 601-HC+601-LC-D28G, 601-HC+601-LC-Y32F, 601-HC+601-LC-N34S, 601-HC+601-LC-V46L, 601-HC+601-LC-F50Y, 601-HC+601-LC-F71Y, 601-HC+601-LC-T93N, 601-HC+601-LC-V94L, respectively. The ELISA described in the above example was used to evaluate the relative affinity of the above antibodies to bind to VEGF165, and 601 was used as a reference. The ELISA results showed that the EC50s of the above mutant antibodies and 601 were 0.1328 nM, 0.1254 nM, 0.2081 nM, 0.3256 nM, 0.1400 nM, 0.1481 nM, 0.1259 nM, 0.1243 nM and 0.1291 nM, respectively; compared to 601, 601-HC+601-LC-N34S and 601-HC+601-LC-V46L had significantly lower relative affinity to VEGF165; other mutant antibodies had basically the same relative affinity as 601.

In summary, the V94L mutation can enhance the relative affinity of 20-Hu-HC+601-LC to PD-1 without reducing the relative affinity of 601 to VEGF165. Here, 601-LC-V94L (SEQ ID NOs: 13 and 14) was selected as the common light chain to construct a bispecific antibody.

### Example 1.3 Construction of bispecific antibodies

The heavy chain variable region of 20-Hu was connected to the CH1 domain of human IgG4, and then connected to the heavy chain variable region of 601 through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 20-Fab-601-IgG4 (SEQ ID NOs: 16 and 17). Similarly, the heavy chain variable region of 601 was connected to the CH1 domain of human IgG4, and then connected to the heavy chain variable region of 20-Hu through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 601-Fab-20-IgG4 (SEQ ID NOs: 18 and 19).

The above sequences were constructed into a pcDNA3.4 expression vector, respectively, the 20-Fab-601-IgG4 and 601-Fab-20-IgG4 expression vectors were combined with the 601-LC-V94L expression vector, and the antibodies were expressed and purified. The obtained antibodies were named as 20-Fab-601-IgG4-V94L and 601-Fab-20-IgG4-V94L, respectively.

### Example 1.4 Determination of relative affinity by ELISA

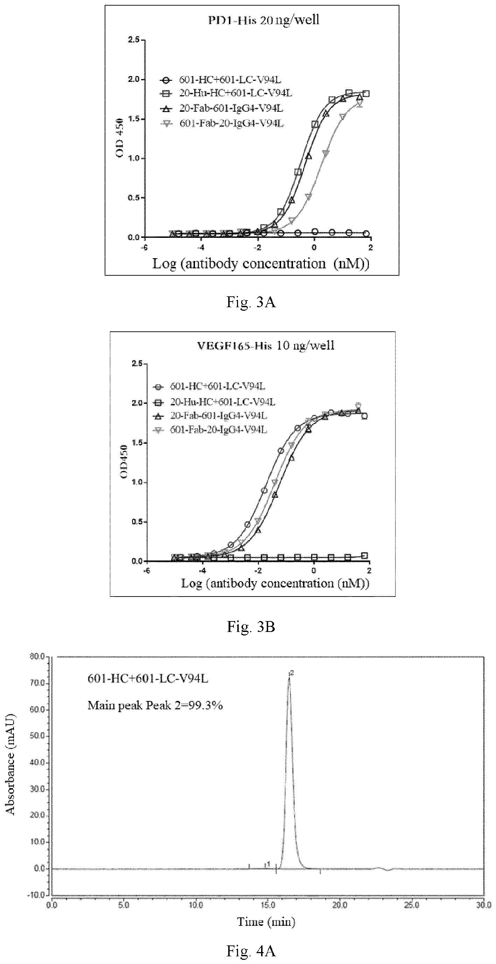

As shown in Fig. 3A, 20-Hu-HC+601-LC-V94L, 20-Fab-601-IgG4-V94L and 601-Fab-20-IgG4-V94L can effectively bind to PD1-His, with EC50s of 0.3314 nM, 0.4768 nM and 1.772 nM, respectively. As shown in Fig. 3B, 601-HC+601-LC-V94L, 20-Fab-601-IgG4-V94L and 601-Fab-20-IgG4-V94L can effectively bind to VEGF165-His, with EC50s of 0.01872 nM, 0.05859 nM and 0.03886 nM, respectively. 20-Fab-601-IgG4-V94L and 601-Fab-20-IgG4-V94L can bind to both PD-1 and VEGF, indicating that they are bispecific antibodies.

### Example 1.5 Determination of affinity by Biacore

Herein, the affinity of the above antibodies to PD-1 or VEGF was deterimined by Biacore 8K (GE healthcare). On Biacore 8K, the chip coupled with Protein A/G was used to capture various antibodies, respectively, and then the recombinant protein PD1-His or VEGF165-His was injected to obtain the binding-dissociation curve, which was eluted with 6M guanidine hydrochloride regeneration buffer for next cycle. Data were analyzed using the Biacore 8K Evaluation Software. The results are shown in Table 2.

**Table 2-1. Binding and dissociation kinetic parameter and equilibrium dissociation constant for PD-1**

| **Sample name** | Kon (1/Ms) | Koff (1/s) | KD (M) |
|---|---|---|---|
| 20-Hu-HC+601-LC-V94L | 2.86E+04 | 4.54E-05 | 15.9E-10 |
| 20-Fab-601-IgG4-V94L | 7.52E+04 | 6.66E-05 | 8.85E-10 |

**Table 2-2. Binding and dissociation kinetic parameter and equilibrium dissociation constant for VEGF**

| **Sample name** | Kon (1/Ms) | Koff (1/s) | KD (M) |
|---|---|---|---|
| 601-HC+601-LC-V94L | 3.90E+06 | 2.91E-05 | 7.46E-12 |
| 20-Fab-601-IgG4-V94L | 1.81E+06 | 1.67E-05 | 9.26E-12 |

Table 2-1 showed that 20-Hu-HC+601-LC-V94L and 20-Fab-601-IgG4-V94L had very similar equilibrium dissociation constants (KD) for PD-1, with KDs of 1.59E-09 and 8.85E-10, respectively.

Table 2-2 showed that 601-HC+601-LC-V94L and 20-Fab-601-IgG4-V94L had very similar binding constant (Kon) and dissociation constant (Koff) for VEGF165-His, and have basically equivalent equilibrium dissociation constants (KD), with KDs of 7.46E-12 and 9.26E-12, respectively. The equilibrium dissociation constant (KD) was inversely proportional to the affinity.

### Example 1.6 Characterization of physicochemical properties

### Example 1.6.1 HPLC-SEC

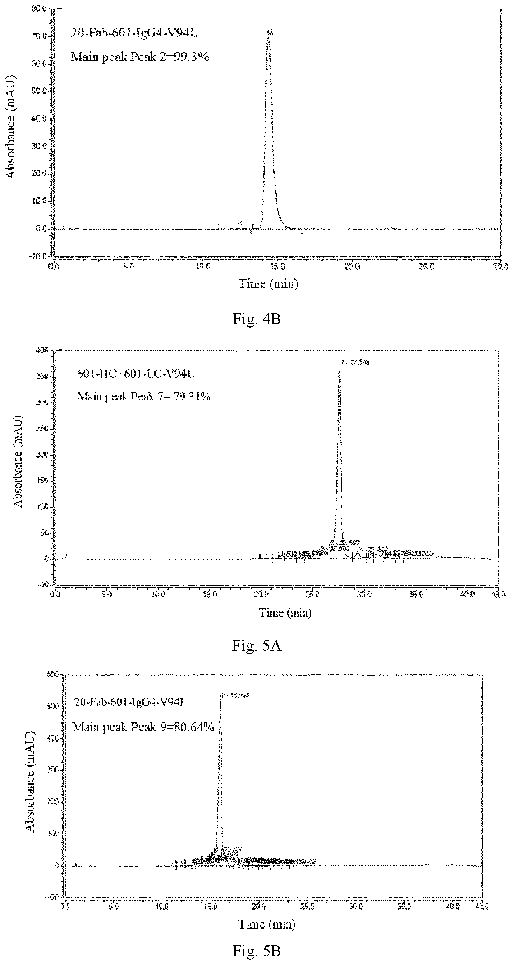

Fig. 4A shows the HPLC-SEC pattern of the monoclonal antibody 601-HC+601-LC-V94L, in which there were two obvious peaks, Peak 1 and Peak 2, accounting for 0.7% and 99.3%, respectively. Among them, the retention time of Peak 1 was shorter than that of the main peak Peak 2, indicating that Peak 1 may be caused by aggregates; there was no peaks that may represent degraded fragments or incompletely assembled molecules in the figure. Fig. 4B shows the HPLC-SEC pattern of 20-Fab-601-IgG4-V94L, in which there were two obvious peaks, Peak 1 and Peak 2, accounting for 0.7% and 99.3%, respectively. Among them, the retention time of Peak1 was shorter than that of the main peak Peak 2, indicating that Peak 1 may be caused by aggregates; there was no peaks that may represent degraded fragments or incompletely assembled molecules in the figure.

### Example 1.6.2 HPLC-IEC

Figs. 5A and 5B show the HPLC-IEC patterns of 601-HC+601-LC-V94L and 20-Fab-601-IgG4-V94L. Their main peaks accounted for 79.31% and 80.64%, respectively. The results indicate that 20-Fab-601-IgG4-V94L has a charge heterogeneity comparable to that of 601-HC+601-LC-V94L.

### Example 1.6.3 CE-SDS

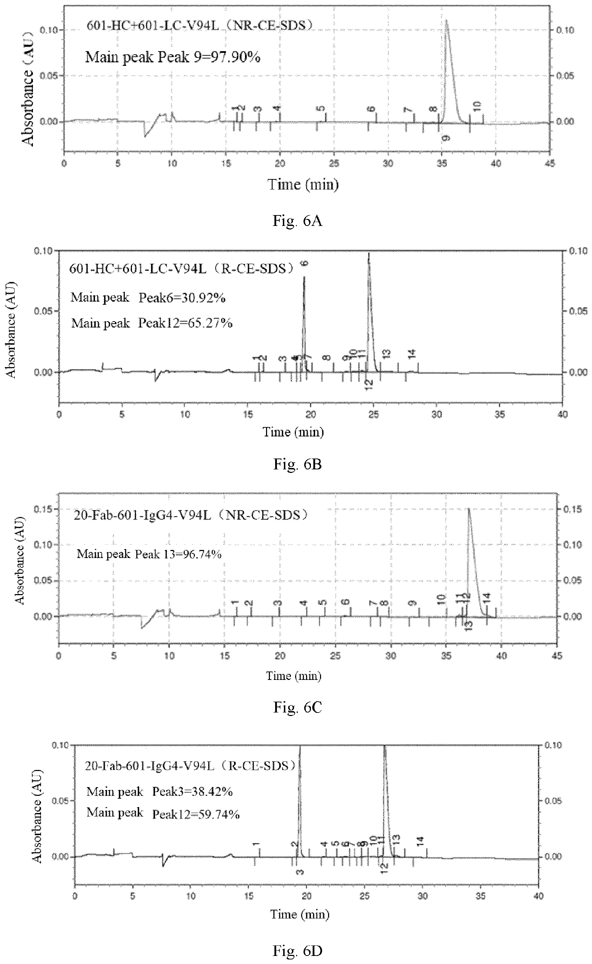

Figs. 6A and 6B show the NR-CE-SDS and R-CE-SDS patterns of 601-HC+601-LC-V94L, respectively. In the NR-CE-SDS pattern, the main peak Peak 9 accounted for 97.90%; In the R-CE-SDS pattern, the two main peaks Peak 6 (corresponding to the light chain) and Peak 12 (corresponding to the heavy chain) accounted for 30.92% and 65.27%, respectively, and the ratio of the two peak areas was 1:2.1. Figs. 6C and 6D show the NR-CE-SDS and R-CE-SDS patterns of 20-Fab-601-IgG4-V94L, respectively. In the NR-CE-SDS pattern, the main peak Peak 13 accounted for 96.74%; In the R-CE-SDS pattern, the two main peaks Peak 3 (corresponding to the light chain) and Peak 12 (corresponding to the heavy chain) accounted for 38.42% and 59.74%, respectively, and the ratio of the two peak areas was 2:3.1. In NR-CE-SDS, the proportions of main peaks of 601-HC+601-LC-V94L and 20-Fab-601-IgG4-V94L were very similar; in R-CE-SDS, the peak area ratios of light chain and heavy chain of both 601-HC+601-LC-V94L and 20-Fab-601-IgG4-V94L were consistent with expectations.

### Example 1.6.4 DSC

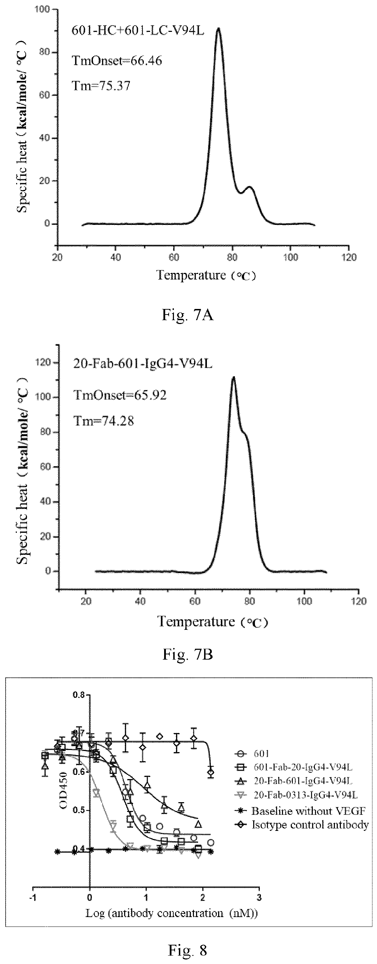

Figs. 7A and 7B show the DSC patterns of 601-HC+601-LC-V94L and 20-Fab-601-IgG4-V94L, respectively. Among them, the TmOnset and Tm of 601-HC+601-LC-V94L were 66.46°C and 75.37°C, respectively, and the TmOnset and Tm of 20-Fab-601-IgG4-V94L were 65.92°C and 74.28°C, respectively. The results indicate that 20-Fab-601-IgG4-V94L and 601-HC+601-LC-V94L have very similar thermal stability.

### Example 1.7 Construction of improved bispecific antibodies

### Example 1.7.1 Construction of bispecific antibodies

In the US patent application US20020032315A1, the related inventors use phage display method to modify the heavy chain variable region and the light chain variable region of Bevacizumab, and obtain the amino acid sequence Y0313-1 of heavy chain variable region with higher affinity and neutralizing activity (SEQ ID NO: 114 in US20020032315A1 is the same as SEQ ID NO: 20 in the present invention).

Herein, the heavy chain variable region of 601 (Bevacizumab) in 20-Fab-601-IgG4-V94L was replaced with Y0313-1. The method is as follows: the heavy chain variable region of 20-Hu was connected to the CH1 domain of human IgG4, and then connected to the heavy chain variable region of Y0313-1 through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 20-Fab-0313-IgG4 (SEQ ID NOs: 21 and 22).

The above sequences were constructed into the pcDNA3.4 expression vector, respectively, the 20-Fab-0313-IgG4 expression vector was combined with the 601-LC-V94L expression vector, and the antibody was expressed and purified. The obtained antibody was named as 20-Fab-0313-IgG4-V94L.

### Example 1.7.2 Determination of the ability to neutralize biological activity of VEGF

Human umbilical vein endothelial cells (HUVEC) were purchased from AllCells Biotechnology Co., Ltd. HUVECs were cultured and subcultured with complete medium for umbilical vein endothelial cells (purchased from AllCells, Cat. No./Specification: H-004/500ml). When HUVECs grew to the logarithmic growth phase, the cells were trypsinized to detach the cells. After centrifugation, the cells were resuspended in the complete medium, and then inoculated in a 96-well cell culture plate at a density of 8000 cells/well; after 24 hours, the complete medium in the 96-well plate was replaced with minimal medium (purchased from AllCells, Cat. No./Specification: H-004B/500ml), 150 µl/well; the antibody was serially diluted in the minimal medium containing 400 ng/ml of recombinant VEGF165 (purchased from Acrobiosystems, Cat. No.: VE5-H4210), and then the mixed solution of VEGF165 and the antibody was added to a 96-well plate, 50 µl/well; incubated for 3 days in a 37°C, 5% CO₂ cell incubator; and then 20 µl of CCK-8 (Dojindo) solution was added to each well, and incubated for another 4 hours in the incubator; OD450 was read with a microplate reader. Graphing and data analysis were performed using GraphPad Prism6 and IC50 was calculated.

As shown in Fig. 8, 601, 20-Fab-601-IgG4-V94L, 601-Fab-20-IgG4-V94L and 20-Fab-0313-IgG4-V94L can effectively inhibit the cell proliferation of HUVECs induced by VEGF165, with IC50s of 4.422 nM, 9.039 nM, 3.84 nM and 1.632 nM, respectively. The above results indicate that 20-Fab-0313-IgG4-V94L has the strongest ability to neutralize the biological activity of VEGF165.

### Example 1.7.3 Evaluation of the functional activity to enhance MLR

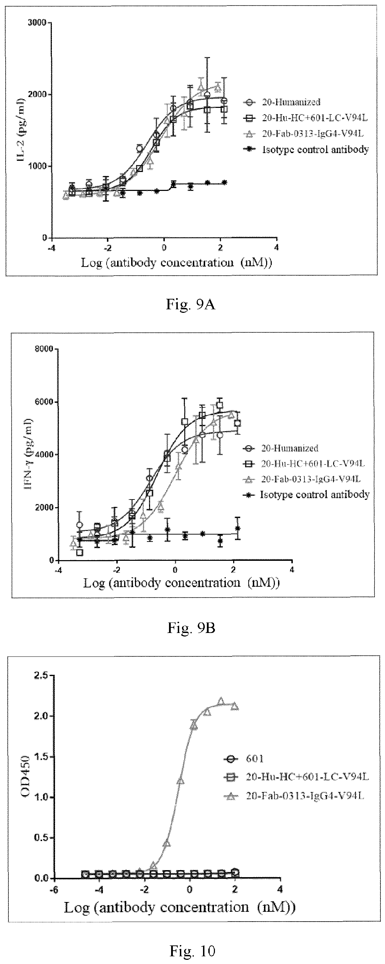

As shown in Figs. 9A and 9B, 20-Humanized (i.e., 20-Hu), 20-Hu-HC+601-LC-V94L and 20-Fab-0313-IgG4-V94L can effectively stimulate MLR to secrete IL-2 and IFN-γ. For stimulating MLR to secrete IL-2, the EC50s were 0.2571 nM, 0.3703 nM and 0.7554 nM, respectively, and for stimulating MLR to secrete IFN-γ, the EC50s were 0.1426 nM, 0.247 nM and 1.036 nM, respectively.

### Example 1.7.4 Determination of the ability of bispecific antibodies against PD-1 and VEGF to simultaneously bind to two antigens

The microplate was coated with VEGF165 (purchased from Acrobiosystems, Cat. No.: VE5-H4210). The antibody to be tested was serially diluted with PBST containing 1% bovine serum albumin, then transferred to the above plate and incubated at room temperature for about half an hour; the plate was washed three times with PBST; biotinylated human PD-1 extracellular recombinant protein (purchased from Sino Biological, Cat. No.: 10377-H08H-B) was diluted to 200 ng/ml, transferred to the microtiter plate, and incubated at room temperature for about half an hour; the plate was washed three times with PBST; Streptavidin-HRP (purchased from BD Biosciences, Cat. No.: 554066) was diluted 1000 times, transferred to the microtiter plate, incubated at room temperature for about half an hour; the plate was washed three times with PBST; TMB chromogenic solution was added (100 µl/well), and incubated at room temperature for 1~5 min; stop solution was added (50 µl/well) to stop the chromogenic reaction. OD450 was read with a microplate reader. Graphing and data analysis were performed using GraphPad Prism6, and EC50 was calculated.

As shown in Fig. 10, 20-Fab-0313-IgG4-V94L can further effectively bind to human PD-1 after binding to VEGF165, with an EC50 of 0.3293 nM. Neither 601 nor 20-Hu-HC+601-LC-V94L can simultaneously bind to PD-1 and VEGF165.

### Example 1.7.5 Determination of pharmacokinetics of bispecific antibodies against PD-1 and VEGF in rats

SD (Sprague-Dawley) rats (purchased from Zhejiang Vital River Laboratory Animal Technology Co., Ltd) were used to study the pharmacokinetics of the bispecific antibodies. There were four rats in each group, weighing about 200 g. Each rat was administrated with a dose of 1 mg of antibody by intravenous injection (I.V); blood was taken from the orbit at a specific time after the administration, and the serum was collected by centrifugation after the blood coagulates naturally.

The method for measuring the concentration of the target antibody in the serum is as follows: ELISA plate was coated with the two related antigens corresponding to the bispecific antibody (VEGF165, purchased from Acrobiosystems, Cat. No.: VE5-H4210; the source of recombinant protein of the extracellular domain of human PD-1 with 6^{∗}His tag is as described in Example 1.2), respectively, and then blocked with PBST containing 1% bovine serum albumin. An appropriately diluted rat serum was transferred to the above plate coated with the two related antigens, incubated at room temperature for 1 hour, and the plate was washed, and then HRP-labeled goat anti-human (Fc-Specific) antibody (purchased from Sigma; this antibody has been treated with species crossadsorption and does not recognize rat antibodies) was added, incubated at room temperature for half an hour, and the plate was washed; chromogenic solution with TMB as a substrate was added at 100 µl/well, incubated at room temperature for 1~5 min; 50 µl of stop solution (2M H₂SO₄) was added to stop the reaction. OD450 was read with a microplate reader, and the OD450 was converted into antibody serum concentration using standard curve. Graphing and data analysis were performed using GraphPad Prism6. The half-life of the antibody in rats was calculated using the Phoenix software.

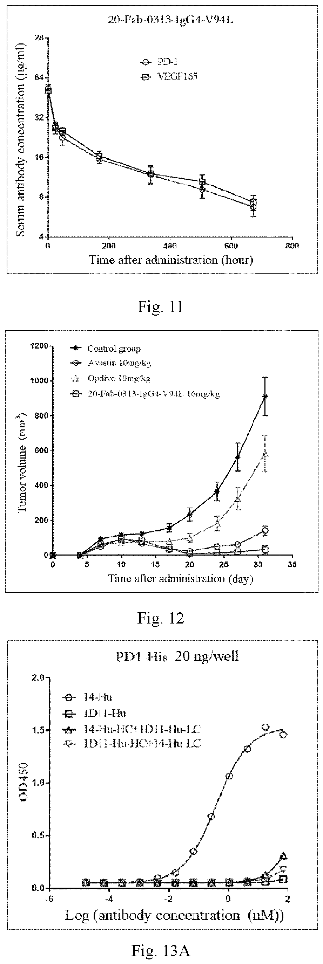

As shown in Fig. 11, the half-life of 20-Fab-0313-IgG4-V94L was calculated to be 16.9 days (detection result with PD-1 as the antigen) and 17.3 days (detection result with VEGF165 as the antigen). The above results indicate that 20-Fab-0313-IgG4-V94L has good pharmacokinetic properties.

### Example 1.7.6 Anti-tumor effect of bispecific antibodies against PD-1 and VEGF in mice

Human peripheral blood mononuclear cells (PBMC) were used to rebuild the human immune system in NSG mice, and establish a human lung cancer NCI-H460 subcutaneous xenograft model on the mice. The mouse model had both T cells expressing human PD-1 and human tumor cells expressing human VEGF, so it can be used to evaluate the anti-tumor activity of the bispecific antibody against PD-1 and VEGF in vivo. The specific implementation steps are as follows: human non-small cell lung cancer NCI-H460 cells (ATCC^{®}HTB-177^{™}) cultured in vitro were collected, the cell suspension was adjusted to a concentration of 1 × 10⁸/ml, and then mixed with Matrigel (purchased from BD Biosciences, Cat. No.: 356234) in equal volume. The purchased PBMCs (purchased from Allcells, Cat. No.: PB005-C) were resuscitated in vitro, and PBMCs were resuspended in PBS, and the PBMC suspension was adjusted to a concentration of 1×10⁷/ml. The mixed tumor cell suspension and the PBMC suspension were mixed in equal volume. Under sterile conditions, 200 µl of the mixed cell suspension was inoculated subcutaneously on the right upper back of M-NSG mice (purchased from Shanghai Model Organisms Center, Inc.). On the same day, the mice inoculated with the mixed cells were randomly divided into groups according to their body weight, with 10 mice in each group. The drug treatment of mice in each group is as follows: Control group, only injected with normal saline; Avastin group, injected with 10 mg/kg of anti-VEGF positive control antibody Avastin (produced by Roche Pharmaceuticals); Opdivo group, injected with 10 mg/kg of anti-PD-1 positive control antibody Opdivo (produced by Bristol-Myers Squibb); 20-Fab-0313-IgG4-V94L group, injected with 16 mg/kg of 20-Fab-0313-IgG4-V94L. Taking into account the difference in molecular weight between bispecific antibodies and monoclonal antibodies, the dose of the drug in this experiment was provided according to the rule of equal amount of substance. Subsequently, the drugs were administered according to the above-designed regimen, twice a week for a total of 10 times, and the tumor volumes were measured twice a week. Finally, the growth curve of tumor of each group determined over time is shown in Fig. 12.

The results showed that at the end of the experiment on the 31st day, the tumor inhibition rates of Avastin, Opdivo and 20-Fab-0313-IgG4-V94L were 84.5%, 35.8% and 96.6%, respectively (tumor inhibition rate = (mean volume of control group - mean volume of experimental group) / mean volume of control group× 100%). Compared to Avastin and Opdivo, 20-Fab-0313-IgG4-V94L can inhibit tumor growth more effectively.

### Example 2 Construction of bispecific antibodies against PD-1 and TGF-Beta

### Example 2.1 Sequences

US5571714A discloses a series of anti-TGF-β (Transforming growth factor beta) monoclonal antibodies, in which murine monoclonal antibody 1D11.16 (hereinafter referred to as 1D11) can effectively bind to TGF-β1 and -β2. The hybridoma corresponding to 1D11 has been deposited in the American Type Culture Collection (ATCC^{®} HB-9849^{™}) by the inventors. The murine 1D11 sequence was obtained from US20180244763A1.

The amino acid sequences of the heavy chain variable region and the light chain variable region of the antibody No. 1D11 were analyzed, and the antigen complementarity determining regions and framework regions of the heavy chain and light chain of the antibody No. 1D11 were determined according to the Kabat scheme. The antibody No. 1D11 comprises the amino acid sequences of the heavy chain CDRs of H-CDR1: TYWMN (SEQ ID NO: 23), H-CDR2: QIFPASGSTNYNEMFEG (SEQ ID NO: 24) and H-CDR3: GDGNYALDAMDY (SEQ ID NO: 25), and the amino acid sequences of the light chain CDRs of L-CDR1: RASESVDSYGNSFMH (SEQ ID NO: 26), L-CDR2: LASNLES (SEQ ID NO: 27) and L-CDR3: QQNNEDPLT (SEQ ID NO: 28).

The homology comparison of the heavy chain variable region of the murine antibody No. 1D11 with the human IgG germline sequence was performed at https://www.ncbi.nlm.nih.gov/ igblast/. IGHV1-46^{∗}01 was selected as the heavy chain CDR grafting template, the heavy chain CDRs of the murine antibody No. 1D11 were transplanted into the framework regions of IGHV1-46^{∗}01, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody No. 1D11 with the human IgG germline sequence was performed. IGKV7-3^{∗}01 was selected as the light chain CDR grafting template, the light chain CDRs of the murine antibody No. 1D11 were transplanted into the framework regions of IGKV7-3^{∗}01, and FGGGTKVELK (SEQ ID NO: 153) was added following the L-CDR3 as the fourth framework region, to obtain a CDR-grafted light chain variable region sequence. On the basis of the CDR-grafted variable regions, some amino acid sites in the framework region were subjected to back-mutation. When back-mutation was performed, the amino acid sequence was numbered according to Kabat and the position of each site was indicated by Kabat numbering.

Preferably, for the CDR-grafted heavy chain variable region, T at position 28 was back-mutated to murine I, T at position 30 was back-mutated to I, and M at position 48 was back-mutated to I, V at position 67 was back-mutated to A, M at position 69 was back-mutated to L, R at position 71 was back-mutated to V, V at position 78 was back-mutated to A. For the CDR-grafted light chain variable region, N at position 81 was back-mutated to D.

The DNAs encoding the above humanized heavy chain variable region and light chain variable region (SEQ ID NOs: 29 and 30) were synthesized by Shanghai Sangon Biotech Co., Ltd.. The synthetic humanized heavy chain variable region was connected to the human IgG4 (with the S228P mutation in the hinge region) heavy chain constant region (SEQ ID NO: 148) to obtain a full-length humanized heavy chain gene, named as 1D11-Hu-HC; the humanized light chain variable region was connected to the human Kappa light chain constant region (SEQ ID NO: 149), to obtain a full-length humanized light chain gene, named as 1D11-Hu-LC.

The heavy chain and light chain genes of the above antibody were constructed into the pcDNA3.4 expression vector, respectively, and the antibody was expressed and purified. The antibody obtained from the combination of 1D11-Hu-HC and 1D11-Hu-LC was named as 1D11-Hu.

US20100136021A1 also discloses a series of anti-TGF-β antibodies, in which the monoclonal antibody mAb12.7 (hereinafter referred to as mAb127) can effectively bind and neutralize TGF-β1, TGF-β2 and TGF-β3. The sequences of the heavy chain variable region and light chain variable region of mAb127 were obtained from US20100136021A1 (SEQ ID NOs: 31 and 32).

The DNAs encoding the above variable regions were synthesized by Shanghai Sangon Biotech Co., Ltd.. The synthetic heavy chain variable region was connected to the human IgG4 (with S228P mutation in the hinge region) heavy chain constant region (SEQ ID NO: 148), to obtain a full-length humanized heavy chain gene, named as mAb127-HC; the light chain variable region was connected to the human Kappa light chain constant region (SEQ ID NO: 149), to obtain a full-length light chain gene, named as mAb127-LC.

According to the description of Examples 1-5 in WO2018/137576A1, the murine monoclonal antibody No. 14 was selected as the lead antibody, and the nucleotide sequence of the heavy chain variable region and the nucleotide sequence of the light chain variable region were obtained and translated into amino acid sequences (SEQ ID NOs: 33 and 34).

The amino acid sequences of the heavy chain variable region and the light chain variable region of the antibody No. 14 were analyzed, and according to the Kabat scheme, the antigen complementarity determining regions and framework regions of the heavy chain and light chain of the antibody No. 14 were determined. The antibody No. 14 comprises the amino acid sequences of the heavy chain CDRs of H-CDR1: GYTMN (SEQ ID NO: 35), H-CDR2: LINPYNGDTSYNQKFKG (SEQ ID NO: 36) and H-CDR3: WRYTMDY (SEQ ID NO: 37), and the amino acid sequences of the light chain CDRs of L-CDR1: RASESVDNYGNSFMN (SEQ ID NO: 38), L-CDR2: FASNLES (SEQ ID NO: 39) and L-CDR3: QQNNEAPPT (SEQ ID NO: 40).

The homology comparison of the heavy chain variable region of the murine antibody No. 14 with the human IgG germline sequence was performed at https://www.ncbi.nlm.nih.gov/igblast/. IGHV1-46^{∗}01 was selected as the heavy chain CDR grafting template, the heavy chain CDRs of the murine antibody No. 14 were transplanted into the framework regions of IGHV1-46^{∗}01, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody No. 14 with the human IgG germline sequence was performed. IGKV7-3^{∗}01 was selected as the light chain CDR grafting template, the light chain CDRs of the murine antibody No. 14 were transplanted into the framework regions of IGKV7-3^{∗}01, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework region were subjected to back-mutation. When back-mutation was performed, the amino acid sequence was numbered according to Kabat and the position of each site was indicated by Kabat numbering.

Preferably, for the CDR-grafted heavy chain variable region, T at position 28 (Kabat numbering) was back-mutated to murine S, M at position 48 was back-mutated to I, V at position 67 was back-mutated to A, M at position 69 was back-mutated to V, R at position 71 was back-mutated to V, T at position 73 was back-mutated to K, V at position 78 was back-mutated to A. For the CDR-grafted light chain variable region, L at position 46 was back-mutated to P, G at position 68 was back-mutated to R, and N at position 81 was back-mutated to D.

The above heavy chain and light chain variable regions with back mutation sites were defined as humanized heavy chain variable region and light chain variable region, respectively (SEQ ID NOs: 41 and 42). The DNAs encoding the humanized heavy chain and light chain variable regions were synthesized by Shanghai Sangon Biotech Co., Ltd.. The synthetic humanized heavy chain variable region was connected to the human IgG4 (with the S228P mutation in the hinge region) heavy chain constant region (SEQ ID NO: 148) to obtain a full-length humanized heavy chain gene, named as 14-Hu-HC; the humanized light chain variable region was connected to the human Kappa chain constant region (SEQ ID NO: 149), to obtain a full-length humanized light chain gene, named as 14-Hu-LC.

The heavy chain and light chain genes of the above antibody were constructed into the pcDNA3.4 expression vector, respectively, and the antibody was expressed and purified. The antibody obtained from the combination of 14-Hu-HC and 14-Hu-LC was named as 14-Hu.

### Example 2.2 Selection of common light chain

BLAST was used to compare and analyze the amino acid sequences of the light chain variable region of 14-Hu and the light chain variable region of 1D11-Hu. The results showed that between them, the identical amino acids accounted for 92% (Identities) and the amino acids with similar properties accounted for 94% (Positives).

The expression vectors of 14-Hu-HC, 14-Hu-LC, 1D11-Hu-HC and 1D11-Hu-LC were combined in the following manner: 14-Hu-HC+14-Hu-LC, 1D11-Hu-HC+1D11-Hu-LC, 14-Hu-HC+1D11-Hu-LC and 1D11-Hu-HC+14-Hu-LC, and the antibodies were expressed and purified. The obtained antibodies were named as 14-Hu, 1D11-Hu, 14-Hu-HC+1D11-Hu-LC and 1D11-Hu-HC+14-Hu-LC, respectively.

The ELISA detection method is as follows: the extracellular domain protein of human PD-1 with a 6^{∗}His tag was prepared by the inventors (the source of the extracellular domain of PD-1 is as described in WO2018/137576A1), and this recombinant protein was denoted as PD1-His, which was used to coat an ELISA plate (20 ng/well); TGF-β1 (purchased from Sino Biological) was used to coat the ELISA plate (5 ng/well).

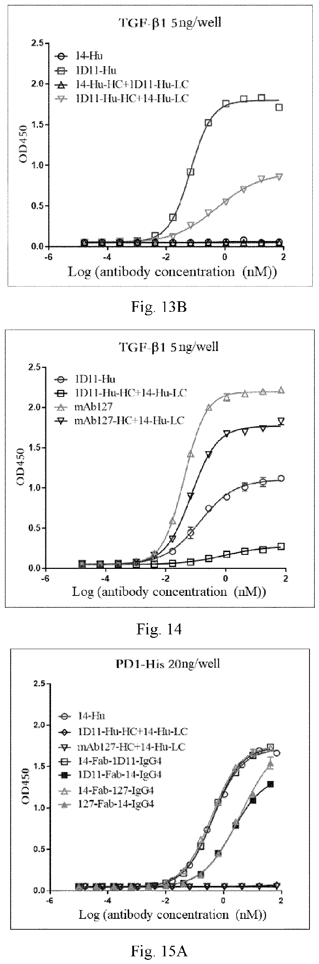

As shown in Fig. 13A, 14-Hu can effectively bind to PD1-His, with an EC50 of 0.3924 nM; while 1D11-Hu, 14-Hu-HC+1D11-Hu-LC and 1D11-Hu-HC+14-Hu-LC cannot effectively bind to PD1-His. As shown in Fig. 13B, 1D11-Hu can effectively bind to TGF-β1, with an EC50 of 0.06624 nM; 1D11-Hu-HC+14-Hu-LC can also bind to TGF-β1, with an EC50 of 0.5255 nM, while 14-Hu and 14-Hu-HC+1D11-Hu-LC cannot bind to TGF-β1. Herein, 14-Hu-LC (SEQ ID NOs: 43 and 44) was selected as the common light chain to construct a bispecific antibody.

BLAST was used to compare and analyze the amino acid sequences of the light chain variable region of 14-Hu and the light chain variable region of mAb127. The results showed that between them, the identical amino acids accounted for 75% (Identities) and the amino acids with similar properties accounted for 83% (Positives).

The expression vectors of 14-Hu-LC, mAb127-HC and mAb127-LC were combined in the following manner: mAb127-HC+mAb127-LC and mAb127-HC+14-Hu-LC, and the antibodies were expressed and purified. The obtained antibodies were named as mAb127 and mAb127-HC+14-Hu-LC, respectively.

The relative affinity of 1D11-Hu, 1D11-Hu-HC+14-Hu-LC, mAb127 and mAb127-HC+14-Hu-LC to TGF-β1 was determined by the ELISA described in the above examples. Graphing and data analysis were performed using GraphPad Prism6 and EC50 was calculated.

As shown in Fig. 14, 1D11-Hu, mAb127 and mAb127-HC+14-Hu-LC can effectively bind to TGF-β1, with EC50s of 0.1338 nM, 0.04136 nM and 0.07105 nM, respectively. Compared to 1D11-Hu, mAb127 and mAb127-HC+14-Hu-LC had a lower EC50 and a higher platform, so both of them had a higher affinity to TGF-β1. Herein, 14-Hu-LC (SEQ ID NOs: 43 and 44) was selected as the common light chain to construct a bispecific antibody.

### Example 2.3 Construction of bispecific antibodies

The heavy chain variable region of 14-Hu was connected to the CH1 domain of human IgG4, and then connected to the heavy chain variable region of 1D11 through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 14-Fab-1D11-IgG4 (SEQ ID NOs: 45 and 46). Similarly, the heavy chain variable region of 1D11 was connected to the CH1 domain of human IgG4, and then connected to the heavy chain variable region of 14-Hu through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 1D11-Fab-14-IgG4 (SEQ ID NOs: 47 and 48).

According to the method described in the above example, the heavy chain variable region of 14-Hu was connected to the CH1 domain of human IgG4, and then connected to the heavy chain variable region of mAb127 through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 14-Fab-127-IgG4 (SEQ ID NOs: 49 and 50). Similarly, the heavy chain variable region of mAb127 was connected to the CH1 domain of human IgG4, and then connected to the heavy chain variable region of 14-Hu through an artificial linker (the linker used here is three GGGGS in tandem), and finally connected to the heavy chain constant region of human IgG4 (CH1+CH2+CH3, with the S228P mutation in the hinge region). The long heavy chain gene containing two heavy chain variable regions and two CH1 domains constructed by such procedures was named as 127-Fab-14-IgG4 (SEQ ID NOs: 51 and 52).

The above sequences were constructed into the pcDNA3.4 expression vector, respectively, the 14-Fab-1D11-IgG4, 1D11-Fab-14-IgG4, 14-Fab-127-IgG4 and 127-Fab-14-IgG4 expression vectors were combined with the 14-Hu-LC expression vector, and the antibodies were expressed and purified. The obtained antibodies were named as 14-Fab-1D11-IgG4, 1D11-Fab-14-IgG4, 14-Fab-127-IgG4 and 127-Fab-14-IgG4, respectively.

### Example 2.4 Determination of the relative affinity by ELISA

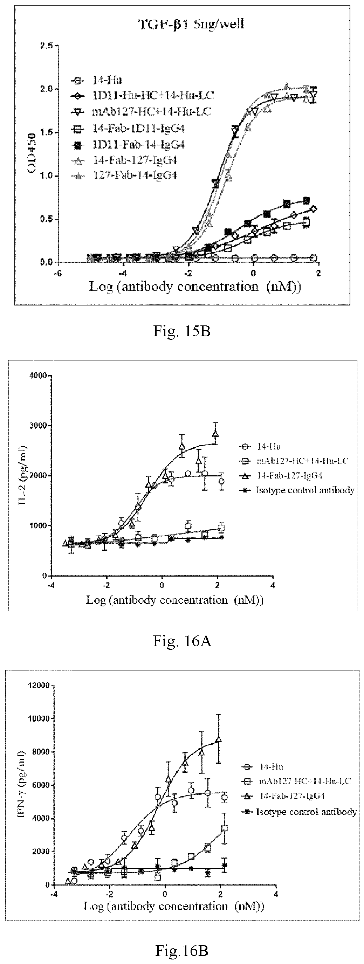

As shown in Fig. 15A, 14-Hu, 14-Fab-1D11-IgG4, 1D11-Fab-14-IgG4, 14-Fab-127-IgG4 and 127-Fab-14-IgG4 can bind to PD1-His, with EC50s of 0.4321 nM, 0.4367 nM, 1.996 nM, 0.3873 nM and 3.955 nM, respectively; compared to 14-Fab-1D11-IgG4 and 14-Fab-127-IgG4, 1D11-Fab-14-IgG4 and 127-Fab-14-IgG4 had a weaker relative affinity to PD1-His, which may be caused by steric hindrance. As shown in Fig. 15B, 1D11-Hu-HC+14-Hu-LC, mAb127-HC+14-Hu-LC, 14-Fab-1D11-IgG4, 1D11-Fab-14-IgG4, 14-Fab-127-IgG4 and 127-Fab-14-IgG4 can bind to TGF-β1, with EC50s of 1.267 nM, 0.0803 nM, 0.6985 nM, 0.3628 nM, 0.1525 nM and 0.1083 nM, respectively. Compared to 1D11-Hu-HC+14-Hu-LC, 14-Fab-1D11-IgG4 and 1D11-Fab-14-IgG4, mAb127-HC+14-Hu-LC, 14-Fab-127-IgG4 and 127-Fab-14-IgG4 had a stronger relative affinity to TGF-β1.

### Example 2.5 Evaluation of the functional activity to enhance MLR

As shown in Figs. 16A and 16B, both 14-Hu and 14-Fab-127-IgG4 can effectively stimulate MLR to secrete IL-2 and IFN-γ. For stimulating MLR to secrete IL-2, the EC50s were 0.1008 nM and 0.3185 nM, respectively, and for stimulating MLR to secrete IFN-γ, the EC50s were 0.04716 nM and 0.5871 nM, respectively. In addition, the experimental results showed that, at higher concentrations, compared to 14-Hu, 14-Fab-127-IgG4 can stimulate MLR to secrete more IL-2 and IFN-γ.

### Example 2.6 Determination of the ability of bispecific antibodies against PD-1 and TGF-Beta to simultaneously bind to two antigens

The microplate was coated with TGF-β1 (purchased from Sino Biological, Cat. No.: 10804-HNAC). The antibody to be tested was serially diluted with PBST containing 1% bovine serum albumin, then transferred to the above microplate and incubated at room temperature for about half an hour. The subsequent experimental steps were the same as described in Example 1.7.4.

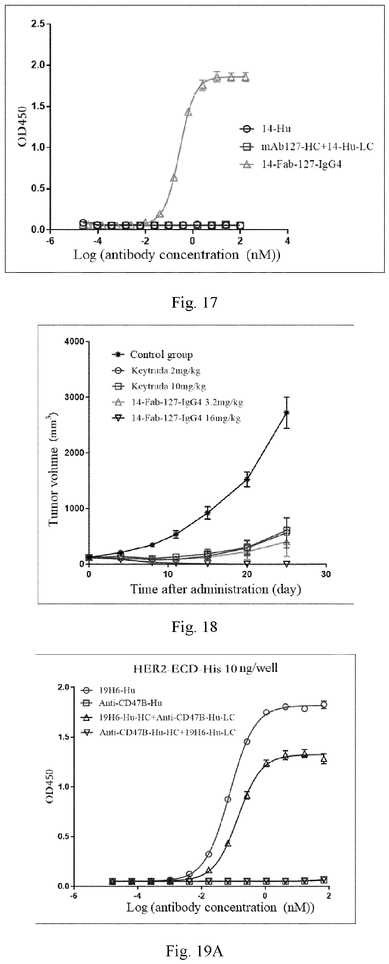

As shown in Fig. 17, 14-Fab-127-IgG4 can further effectively bind to human PD-1 after binding to TGF-β1, with an EC50 of 0.2784 nM. Neither 14-Hu nor mAb127-HC+14-Hu-LC can simultaneously bind to PD-1 and TGF-β1.

### Example 2.7 Anti-tumor effect of bispecific antibodies against PD-1 and TGF-beta in mice

Human PD-1 transgenic mice (germline background is C57BL/6) and MC38 mouse colorectal cancer cells were purchased from the Shanghai Model Organisms Center, Inc.. In the transgenic mice, the extracellular segment of the human PD-1 gene was used to replace the homologous part of the mice. The bispecific antibody of the present invention can recognize the PD-1 molecule in the transgenic mice, and can also bind to the endogenous TGF-beta of the mice. The specific implementation steps are as follows: MC38 cells were cultured in vitro with 10% serum-containing DMEM (serum and medium purchased from Gibco); the cultured MC38 cells were inoculated into human PD-1 transgenic mice, each mouse was inoculated with 2×10⁶ cells subcutaneously; when the tumor cells to be inoculated grew to a volume close to 100 mm³, the mice were randomly divided into groups, with 8 mice in each group. The drug treatment of mice in each group is as follows: Control group, only injected with normal saline; Keytruda group (two dose groups), injected with 2 mg/kg or 10 mg/kg of anti-PD-1 positive control antibody Keytruda (produced by Merck); 14-Fab-127-IgG4 group (two dose groups), injected with 3.2 mg/kg or 16 mg/kg of 14-Fab-127-IgG4. Taking into account the difference in molecular weight between bispecific antibodies and monoclonal antibodies, the doses of the drug in this experiment was provided according to the rule of equal amount of substance. Subsequently, the drugs were administered according to the above-designed regimen, twice a week for a total of 6 times, and the tumor volume was measured twice a week. Finally, the growth curve of tumor in each group determined over time is shown in Fig. 18.

The results showed that at the end of the experiment on the 25^{th} day, the tumor inhibition rates of Keytruda (two dose groups) and 14-Fab-127-IgG4 (two dose groups) were 79.2% (2 mg/kg) and 77.7% (10 mg/kg), 85.1% (3.2 mg/kg) and 100% (16 mg/kg), respectively (tumor inhibition rate = (mean volume of control group - mean volume of experimental group) / mean volume of control group × 100%). Compared to Keytruda, 14-Fab-127-IgG4 can inhibit tumor growth more effectively, and 14-Fab-127-IgG4 at high doses can cause complete tumor regression in all mice.

### Example 3 Construction of bispecific antibodies against HER-2 and CD47

### Example 3.1 Sequences

19H6-Hu is a humanized anti-human HER2 monoclonal antibody, whose heavy chain variable region and light chain variable region sequences were from WO2020/025013A1. The humanized heavy chain variable region and light chain variable region were named as 19H6-Hu-VH and 19H6-Hu-VL (SEQ ID NOs: 53 and 54), respectively.

The DNAs encoding the humanized heavy chain variable region and light chain variable region were synthesized by Shanghai Sangon Biotech Co., Ltd.. The synthetic humanized heavy chain variable region was connected to the human IgG1 heavy chain constant region (SEQ ID NO: 147), to obtain a full-length humanized heavy chain gene, named as 19H6-Hu-HC; the humanized light chain variable region was connected to the human Kappa chain constant region (SEQ ID NO: 149), to obtain a full-length humanized light chain gene, named as 19H6-Hu-LC. The 19H6-Hu-HC and 19H6-Hu-LC genes were constructed into the pcDNA3.4 expression vector, respectively, the antibody was expressed and purified, and the obtained antibody was named as 19H6-Hu.

MABL-2 (hereinafter referred to as Anti-CD47B) is a murine monoclonal antibody against human CD47, whose amino acid sequences of heavy chain variable region and light chain variable region were derived from SEQ ID NO: 12 and SEQ ID NO: 10 in US20030108546A.

The amino acid sequences of the heavy chain variable region and the light chain variable region of the antibody Anti-CD47B were analyzed, and according to the Kabat scheme, the antigen complementarity determining regions and framework regions of the heavy chain and light chain of the antibody Anti-CD47B were determined. The antibody Anti-CD47B comprises the amino acid sequences of the heavy chain CDRs of H-CDR1: NHVIH (SEQ ID NO: 55), H-CDR2: YIYPYNDGTKYNEKFKD (SEQ ID NO: 56) and H-CDR3: GGYYTYDD (SEQ ID NO: 57), and the amino acid sequences of the light chain CDRs of L-CDR1: RSSQSLVHSNGKTYLH (SEQ ID NO: 58), L-CDR2: KVSNRFS (SEQ ID NO: 59) and L-CDR3: SQSTHVPYT (SEQ ID NO: 60).

The homology comparison of the heavy chain variable region of the murine antibody Anti-CD47B with the human IgG germline sequence was performed at https://www.ncbi.nlm.nih.gov/igblast/. IGHV1-46^{∗}01 was selected as the heavy chain CDR grafting template, the heavy chain CDRs of the murine antibody Anti-CD47B were transplanted into the framework regions of IGHV1-46^{∗}01, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody Anti-CD47B with the human IgG germline sequence was performed. IGKV2-30^{∗}01 was selected as the light chain CDR grafting template, the light chain CDRs of the murine antibody Anti-CD47B were transplanted into the framework regions of IGKV2-30^{∗}01, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework region were subjected to back-mutation. When mutation was performed, the amino acid sequence was numbered according to Kabat and the position of each site was indicated by Kabat numbering.

Preferably, for the CDR-grafted heavy chain variable region, according to Kabat numbering, T at position 30 was mutated to A, M at position 69 was mutated to L, R at position 71 was mutated to S, and T at position 73 was mutated to K. For the CDR-grafted light chain variable region, F at position 36 was mutated to Y, and R at position 46 was mutated to L.

The above heavy chain variable region and light chain variable region with mutation sites were defined as humanized heavy chain variable region and light chain variable region, which were named as Anti-CD47B-Hu-VH and Anti-CD47B -Hu-VL (SEQ ID NOs: 61 and 62), respectively.

The DNAs encoding the humanized heavy chain variable region and light chain variable region were synthesized by Shanghai Sangon Biotech Co., Ltd.. The synthetic humanized heavy chain variable region was connected to the human IgG1 heavy chain constant region (SEQ ID NO: 147), to obtain a full-length humanized heavy chain gene, named as Anti-CD47B-Hu-HC; the humanized light chain variable region was connected to the human Kappa chain constant region (SEQ ID NO: 149), to obtain a full-length humanized light chain gene, named as Anti-CD47B-Hu-LC. The Anti-CD47B-Hu-HC and Anti-CD47B-Hu-LC genes were constructed into the pcDNA3.4 expression vector, respectively, the antibody was expressed and purified, and the obtained antibody was named as Anti-CD47B-Hu.

### Example 3.2 Selection of common light chain

BLAST was used to compare and analyze the amino acid sequences of the light chain variable region of 19H6-Hu and the light chain variable region of Anti-CD47B-Hu. The results showed that between them, the identical amino acids accounted for 96% (Identities) and the amino acids with similar properties accounted for 99% (Positives).

The heavy chain and light chain genes of 19H6-Hu and Anti-CD47B-Hu were combined in the following manner: 19H6-Hu-HC+Anti-CD47B-Hu-LC and Anti-CD47B-Hu-HC+19H6-Hu-LC, the antibodies were expressed and purified, and the obtained antibodies were named as 19H6-Hu-HC+Anti-CD47B-Hu-LC and Anti-CD47B-Hu-HC+19H6-Hu-LC, respectively.

The ELISA detection method is as follows: The recombinant protein of the extracellular segment of human Her-2 with a polyhistidine tag was purchased from ACROBiosystems (Cat. No.: HE2-H5225), and the recombinant protein of the extracellular segment of human CD47 with a polyhistidine tag was purchased from Sino Biological (Cat. No.: 12283-H08H), and the two recombinant proteins were named as HER2-ECD-His and CD47-ECD-His. An ELISA plate was coated with HER2-ECD-His and CD47-ECD-His, respectively, both with a coating concentration of 10 ng/well.

As shown in Fig. 19A, 19H6-Hu and 19H6-Hu-HC+Anti-CD47B-Hu-LC can effectively bind to HER2-ECD-His, with EC50s of 0.07701 nM and 0.1388 nM, respectively; while Anti-CD47B-Hu and Anti-CD47B-Hu-HC+19H6-Hu-LC cannot effectively bind to HER2-ECD-His. As shown in Fig. 19B, both Anti-CD47B-Hu and Anti-CD47B-Hu-HC+19H6-Hu-LC can effectively bind to CD47-ECD-His, with EC50s of 0.04276 nM and 0.0541 nM, respectively, while 19H6-Hu and 19H6-Hu-HC+Anti-CD47B-Hu-LC cannot effectively bind to CD47-ECD-His. Herein, 19H6-Hu-LC (SEQ ID NOs: 63 and 64) was selected as the common light chain to construct a bispecific antibody.

### Example 3.3 Construction of bispecific antibodies

The heavy chain variable region of Anti-CD47B-Hu was connected to the CH1 domain of human IgG4, and then connected to the heavy chain variable region of 19H6-Hu through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as CD47B-Fab-19H6-IgG1 (SEQ ID NOs: 65 and 66). Similarly, the heavy chain variable region of 19H6-Hu was connected to the CH1 domain of human IgG4, and then connected to the heavy chain variable region of Anti-CD47B-Hu through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as 19H6-Fab-CD47B-IgG1 (SEQ ID NOs: 67 and 68).

The above sequences were constructed into the pcDNA3.4 expression vector, respectively, the CD47B-Fab-19H6-IgG1 and 19H6-Fab-CD47B-IgG1 expression vectors were combined with the 19H6-Hu-LC expression vector, respectively, and the antibodies were expressed and purified. The obtained antibodies were named as CD47B-Fab-19H6-IgG1 and 19H6-Fab-CD47B-IgG1, respectively (for brevity, only the name of the heavy chain was used as the name of the antibody).

### Example 3.4 Determination of the relative affinity by ELISA

As shown in Fig. 20A, 19H6-Hu, CD47B-Fab-19H6-IgG1 and 19H6-Fab-CD47B-IgG1 can effectively bind to HER2-ECD-His, with EC50s of 0.1262 nM, 0.1057 nM and 0.1543 nM, respectively. As shown in Fig. 20B, Anti-CD47B-Hu-HC+19H6-Hu-LC, CD47B-Fab-19H6-IgG1 and 19H6-Fab-CD47B-IgG1 can effectively bind to CD47-ECD-His, with EC50s of 0.06166 nM, 0.07817 nM and 0.1945 nM, respectively. The above results show that CD47B-Fab-19H6-IgG1 and 19H6-Fab-CD47B-IgG1 can bind to both HER-2 and CD47, indicating that they are bispecific antibodies.

### Example 4 Construction of bispecific antibodies against HER-2 and CD137

### Example 4.1 Sequences

19H6-Hu is a humanized anti-human HER2 monoclonal antibody, the source of which is as described in Example 3.1.

Antibody No. 94 is a mouse-derived anti-human CD137 antibody prepared in our company's laboratory, according to the preparation method described in Examples 1-5 in WO2018/137576A1, the difference is that the mice were immunized using human CD137 recombinant protein and hybridoma cells were selected by ELISA using human CD137 recombinant protein. The amino acid sequence of the heavy chain variable region and the amino acid sequence of the light chain variable region are shown in SEQ ID NOs: 69 and 70, respectively.

The amino acid sequences of the heavy chain variable region and the light chain variable region of the antibody No. 94 were analyzed, and according to the Kabat scheme, the antigen complementarity determining regions and framework regions of the heavy chain and light chain of the antibody No. 94 were determined. The antibody No. 94 comprises the amino acid sequences of the heavy chain CDRs of H-CDR1: SYDIS (SEQ ID NO: 71), H-CDR2: VIWTGGGTNYNSAFMS (SEQ ID NO: 72) and H-CDR3: MDY (SEQ ID NO: 73), and the amino acid sequences of the light chain CDRs of L-CDR1: RSSQSLLHSNGNTYLH (SEQ ID NO: 74), L-CDR2: KVSNRFS (SEQ ID NO: 75) and L-CDR3: SQSTHVPWT (SEQ ID NO: 76).

The homology comparison of the heavy chain variable region of the murine antibody No. 94 with the human IgG germline sequence was performed at https://www.ncbi.nlm.nih.gov/igblast/. IGHV4-59^{∗}01 was selected as the heavy chain CDR grafting template, the heavy chain CDRs of the murine antibody No. 94 were transplanted into the framework regions of IGHV4-59^{∗}01, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody No. 94 with the human IgG germline sequence was performed. IGKV2-30^{∗}01 was selected as the light chain CDR grafting template, the light chain CDRs of the murine antibody Anti-CD47B were transplanted into the framework regions of IGKV2-30^{∗}01, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework region were subjected to back-mutation. When mutation was performed, the amino acid sequence was numbered according to Kabat and the position of each site was indicated by Kabat numbering.

Preferably, for the CDR-grafted heavy chain variable region, according to Kabat numbering, G at position 27 was mutated to F, I at position 29 was mutated to L, I at position 48 was mutated to L, R at position 71 was mutated to S, V at position 71 was mutated to K, T at position 73 was mutated to N, N at position 76 was mutated to S, F at position 78 was mutated to V, A at position 93 was mutated to V. For the CDR-grafted light chain variable region, F at position 36 was mutated to Y, R at position 46 was mutated to L, I at position 48 was mutated to F, and Y at position 87 was mutated to F.

The above heavy chain variable region and light chain variable region with mutation sites were defined as humanized heavy chain variable region and light chain variable region, which were named as 94-Hu-VH and 94-Hu-VL (SEQ ID NOs: 77 and 78), respectively.

The DNAs encoding the above humanized heavy chain variable region and light chain variable region were synthesized by Shanghai Sangon Biotech Co., Ltd.. The synthetic humanized heavy chain variable region was connected to the human IgG1 heavy chain constant region (SEQ ID NO: 147), to obtain a full-length humanized heavy chain gene, named as 94-Hu-HC; the humanized light chain variable region was connected to the human Kappa chain constant region (SEQ ID NO: 149), to obtain a full-length humanized light chain gene, named as 94-Hu-LC. The 94-Hu-HC and 94-Hu-LC genes were constructed into the pcDNA3.4 expression vector, respectively, and the antibody was expressed and purified. The obtained antibody was named as 94-Hu.

### Example 4.2 Selection of common light chain

BLAST was used to compare and analyze the amino acid sequences of the light chain variable region of 19H6-Hu and the light chain variable region of 94-Hu. The results show that between them, the identical amino acids accounted for 97% (Identities) and the amino acids with similar properties accounted for 99% (Positives).

The heavy chain and light chain genes of 19H6-Hu and 94-Hu were combined in the following manner: 19H6-Hu-HC+94-Hu-LC and 94-Hu-HC+19H6-Hu-LC, and the antibodies were expressed and purified. The obtained antibodies were named as 19H6-Hu-HC+94-Hu-LC and 94-Hu-HC+19H6-Hu-LC, respectively.

The ELISA detection method is as follows: the recombinant protein of the extracellular segment of human Her-2 with a polyhistidine tag was purchased from ACROBiosystems (Cat. No.: HE2-H5225), and the recombinant protein of the extracellular segment of human CD137 with Fc tags was purchased from Sino Biological (Cat. No.: 10041-H02H), the two recombinant proteins were named as HER2-ECD-His and CD137-ECD-Fc, respectively. An ELISA plate was coated with HER2-ECD-His and CD137-ECD-Fc, respectively, both with the coating concentration of 10 ng/well.

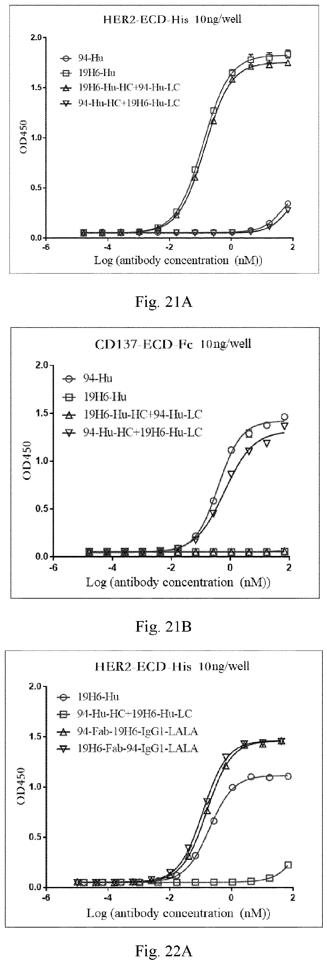

As shown in Fig. 21A, both 19H6-Hu and 19H6-Hu-HC+94-Hu-LC can effectively bind to HER2-ECD-His, with EC50s of 0.1222 nM and 0.1391 nM, respectively; while 94-Hu and 94-Hu-HC+19H6-Hu-LC cannot effectively bind to HER2-ECD-His. As shown in Fig. 21B, both 94-Hu and 94-Hu-HC+19H6-Hu-LC can effectively bind to CD137-ECD-Fc, with EC50s of 0.3913 nM and 0.634 nM, respectively, while 19H6-Hu and 19H6-Hu-HC+94-Hu-LC cannot effectively bind to CD137-ECD-Fc. Herein, 19H6-Hu-LC (SEQ ID NOs: 63 and 64) was selected as the common light chain to construct a bispecific antibody (actually, 94-Hu-LC can also be selected as the common light chain to construct a bispecific antibody).

### Example 4.3 Construction of bispecific antibodies

The heavy chain variable region of 94-Hu was connected to the CH1 domain of human IgG4, and then connected to the heavy chain variable region of 19H6-Hu through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as 94-Fab-19H6-IgG1. Similarly, the heavy chain variable region of 19H6-Hu was connected to the CH1 domain of human IgG4, and then connected to the heavy chain variable region of 94-Hu through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as 19H6-Fab-94-IgG1.

In order to reduce the interaction between the Fc segment of the above bispecific antibody molecules and FcγRs (Fc gamma receptors), both the leucine at position 234 and the leucine at position 235 of their Fc segment were mutated to alanine, and the mutation was marked as LALA (Reference: Wang X, Mathieu M, Brezski R J. IgG Fc engineering to modulate antibody effector functions[J]. Protein & cell, 2018, 9(1): 63-73.). The above BsAb molecules after mutation were named as 94-Fab-19H6-IgG1-LALA (SEQ ID NOs: 79 and 80) and 19H6-Fab-94-IgG1-LALA (SEQ ID NOs: 81 and 82), respectively. Herein, the purpose of LALA mutation is mainly to reduce potential toxicity in vivo (Reference: Ho SK, Xu Z, Thakur A, et al. Epitope and Fc-mediated Crosslinking, but not High Affinity, Are Critical for Antitumor Activity of CD137 Agonist Antibody with Reduced Liver Toxicity[J]. Molecular Cancer Therapeutics, 2020.pp. 1040-1051.).

The above sequences were constructed into the pcDNA3.4 expression vector, respectively, the 94-Fab-19H6-IgG1-LALA and 19H6-Fab-94-IgG1-LALA expression vectors were combined with the 19H6-Hu-LC expression vector, respectively, and the antibodies were expressed and purified. The obtained antibodies were named as 94-Fab-19H6-IgG1-LALA and 19H6-Fab-94-IgG1-LALA, respectively (for brevity, only the name of the heavy chain was used as the name of the antibody).

### Example 4.4 Determination of the relative affinity by ELISA

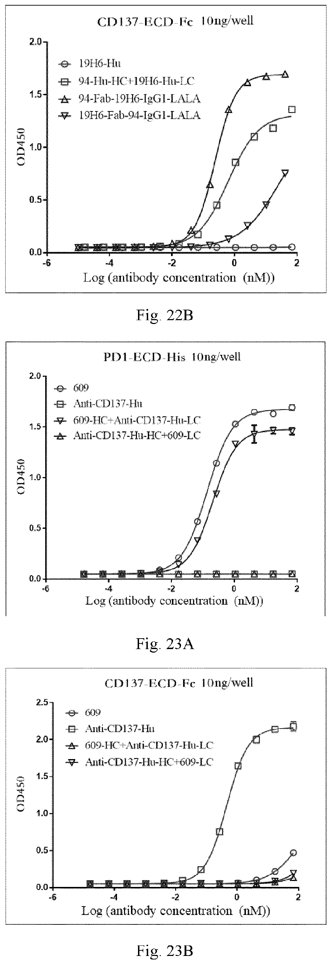

As shown in Fig. 22A, 19H6-Hu, 94-Fab-19H6-IgG1-LALA and 19H6-Fab-94-IgG1-LALA can effectively bind to HER2-ECD-His, with EC50s of 0.1933 nM, 0.1579 nM and 0.1201 nM, respectively. As shown in Fig. 22B, both 94-Hu-HC+19H6-Hu-LC and 94-Fab-19H6-IgG1-LALA can effectively bind to CD137-ECD-Fc, with EC50s of 0.634 nM and 0.2411 nM, respectively; 19H6-Fab-94-IgG1-LALA has weak binding to CD137-ECD-Fc, with an EC50 of 27.56 nM. The above results showed that 94-Fab-19H6-IgG1-LALA and 19H6-Fab-94-IgG1-LALA can bind to both HER-2 and CD137, indicating that they are bispecific antibodies.

### Example 5 Construction of bispecific antibodies against PD-1 and CD137

### Example 5.1 Sequences

MAb1-25-Hu (hereinafter referred to as 609) is a humanized anti-human PD-1 monoclonal antibody, whose heavy chain variable region and light chain variable region sequences are derived from WO2018/137576A1. The humanized heavy chain variable region and light chain variable region (SEQ ID NOs: 83 and 84) were connected to the human IgG4 (S228P) heavy chain constant region (SEQ ID NO: 148) and Kappa light chain constant region (SEQ ID NO: 149), respectively, and finally the heavy chain and light chain genes of complete humanized mAb1-25-Hu monoclonal antibody (609) were obtained.

4B4-1-1 (hereinafter referred to as Anti-CD137) is a murine monoclonal antibody against human CD137, whose heavy chain variable region and light chain variable region amino acid sequences are derived from SEQ ID NO: 10 and SEQ ID NO: 11 in US5928893.

The amino acid sequences of the heavy chain variable region and the light chain variable region of the antibody Anti-CD137 were analyzed, and according to the Kabat scheme, the antigen complementarity determining regions and framework regions of the heavy chain and light chain of the antibody Anti-CD137 were determined. The antibody Anti-CD137 comprises the amino acid sequences of the heavy chain CDRs of H-CDR1: SYWMH (SEQ ID NO: 85), H-CDR2: EINPGNGHTNYNEKFKS (SEQ ID NO: 86) and H-CDR3: SFTTARGFAY (SEQ ID NO:87), and the amino acid sequences of the light chain CDRs of L-CDR1: RASQTISDYLH (SEQ ID NO: 88), L-CDR2: YASQSIS (SEQ ID NO: 89) and L-CDR3: QDGHSFPPT (SEQ ID NO: 90).

The homology comparison of the heavy chain variable region of the murine antibody Anti-CD137 with the human IgG germline sequence was performed at https://www.ncbi.nlm.nih.gov/igblast/. IGHV1-46^{∗}01 was selected as the heavy chain CDR grafting template, the heavy chain CDRs of the murine antibody Anti-CD137 were transplanted into the framework regions of IGHV1-46^{∗}01, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody Anti-CD137 with the human IgG germline sequence was performed. IGKV6-21^{∗}02 was selected as the light chain CDR grafting template, the light chain CDRs of the murine antibody Anti-CD137 were transplanted into the framework regions of IGKV6-21^{∗}02, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework region were subjected to back-mutation. When mutation was performed, the amino acid sequence was numbered according to Kabat and the position of each site was indicated by Kabat numbering.

Preferably, for the CDR-grafted heavy chain variable region, according to Kabat numbering, T at position 30 was mutated to S, M at position 69 was mutated to L, R at position 71 was mutated to V, and T at position 73 was mutated to K. For the CDR-grafted light chain variable region, L at position 4 was mutated to M, and V at position 58 was mutated to I, and T at position 69 was mutated to S.

The above heavy chain variable region and light chain variable region with mutation sites are defined as humanized heavy chain variable region and light chain variable region, which were named as Anti-CD 137-Hu-VH and Anti-CD 137-Hu-VL (SEQ ID NOs: 91 and 92), respectively.

The DNAs encoding the humanized heavy chain variable region and light chain variable region were synthesized by Shanghai Sangon Biotech Co., Ltd.. The synthetic humanized heavy chain variable region was connected to the human IgG4 (S228P) heavy chain constant region (SEQ ID NO: 148), to obtain a full-length humanized heavy chain gene, named as Anti-CD137-Hu-HC; the humanized light chain variable region was connected to the human Kappa chain constant region (SEQ ID NO: 149), to obtain a full-length humanized light chain gene, named as Anti-CD137-Hu-LC. The Anti-CD137-Hu-HC and Anti-CD137-Hu-LC genes were constructed into the pcDNA3.4 expression vector, respectively, the antibody was expressed and purified, and the obtained antibody was named as Anti-CD 137-Hu.

### Example 5.2 Selection of common light chain

BLAST was used to compare and analyze the amino acid sequences of the light chain variable region of 609 and the light chain variable region of Anti-CD137-Hu. The results show that between them, the identical amino acids accounted for 73% (Identities) and the amino acids with similar properties accounted for 88% (Positives).

The heavy chain and light chain genes of 609 and Anti-CD137-Hu were combined in the following manner: 609-HC+Anti-CD137-Hu-LC and Anti-CD137-Hu-HC+609-LC, the antibodies were expressed and purified, and the obtained antibodies were named as 609-HC+Anti-CD137-Hu-LC and Anti-CD 13 7-Hu-HC+609-LC, respectively.

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human CD137 with Fc tags was purchased from Sino Biological (Cat. No.: 10041-H02H), and the two recombinant proteins were named as PD1-ECD-His and CD137-ECD-Fc, respectively. An ELISA plate was coated with PD1-ECD-His and CD137-ECD-Fc, respectively, both with a coating concentration of 10 ng/well.

As shown in Fig. 23A, both 609 and 609-HC+Anti-CD137-Hu-LC can effectively bind to PD1-ECD-His, with EC50s of 0.1358 nM and 0.2067 nM, respectively; while Anti-CD137-Hu and Anti-CD137-Hu-HC+609-LC cannot effectively bind to PD1-ECD-His. As shown in Fig. 23B, Anti-CD137-Hu can effectively bind to CD137-ECD-Fc, with an EC50 of 0.461 nM, while 609, 609-HC+Anti-CD137-Hu-LC and Anti-CD137-Hu-HC+609-LC cannot effectively bind to CD137-ECD-Fc. Herein, Anti-CD137-Hu-LC (SEQ ID NOs: 93 and 94) was selected as the common light chain to construct a bispecific antibody.

### Example 5.3 Construction of bispecific antibodies

The heavy chain variable region of 609 was connected to the CH1 domain of human IgG4, and then connected to the heavy chain variable region of Anti-CD137-Hu through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-137-IgG4 (SEQ ID NOs: 95 and 96). Similarly, the heavy chain variable region of Anti-CD137-Hu was connected to the CH1 domain of human IgG4, and then connected to the heavy chain variable region of 609 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 137-Fab-609-IgG4 (SEQ ID NOs: 97 and 98).

The above sequences were constructed into the pcDNA3.4 expression vector, respectively, the 609-Fab-137-IgG4 and 137-Fab-609-IgG4 expression vectors were combined with the Anti-CD 137-Hu-LC expression vector, and the antibodies were expressed and purified. The obtained antibodies were named as 609-Fab-137-IgG4 and 137-Fab-609-IgG4, respectively (for brevity, only the name of the heavy chain is used as the name of the antibody).

### Example 5.4 Determination of the relative affinity by ELISA

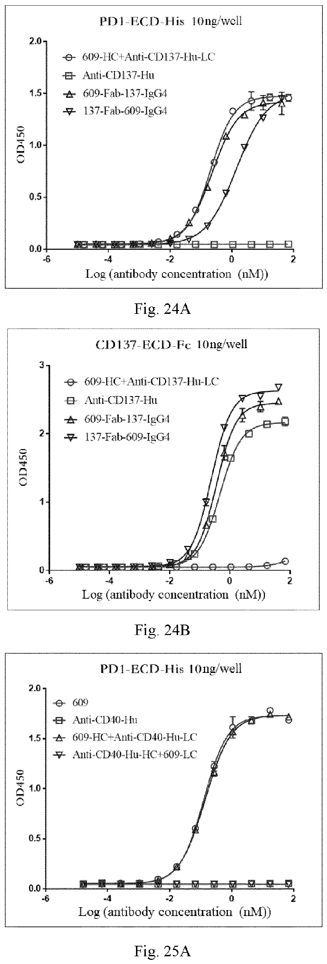

As shown in Fig. 24A, 609-HC+Anti-CD137-Hu-LC, 609-Fab-137-IgG4 and 137-Fab-609-IgG4 can effectively bind to PD1-ECD-His, with EC50s of 0.2067 nM, 0.2293 nM and 1.415 nM, respectively. As shown in Fig. 24B, Anti-CD 137-Hu, 609-Fab-137-IgG4 and 137-Fab-609-IgG4 can effectively bind to CD137-ECD-Fc, with EC50s of 0.461 nM, 0.3572 nM and 0.2424 nM, respectively. The above results showed that 609-Fab-137-IgG4 and 137-Fab-609-IgG4 can bind to both PD-1 and CD137, indicating that they are bispecific antibodies.

### Example 6 Construction of bispecific antibodies against PD-1 and CD40

### Example 6.1 Sequences

609 is a humanized anti-human PD-1 monoclonal antibody, the source of which is described in Example 5.1.

MAb2.220 (hereinafter referred to as Anti-CD40) is a murine monoclonal antibody against human CD40, whose heavy chain variable region and light chain variable region amino acid sequences are derived from SEQ ID NO: 2 and SEQ ID NO: 1 in US6312693.

The amino acid sequences of the heavy chain variable region and the light chain variable region of the antibody Anti-CD40 were analyzed, and according to the Kabat scheme, the antigen complementarity determining regions and framework regions of the heavy chain and light chain of the antibody Anti-CD40 were determined. The antibody Anti-CD40 comprises the amino acid sequences of the heavy chain CDRs of H-CDR1: TTGMQ (SEQ ID NO: 99), H-CDR2: WINTHSGVPKYVEDFKG (SEQ ID NO: 100) and H-CDR3: SGNGNYDLAYFAY (SEQ ID NO: 101), and the amino acid sequences of the light chain CDRs of L-CDR1: RASQSISDYLH (SEQ ID NO: 102), L-CDR2: YASHSIS (SEQ ID NO: 103) and L-CDR3: QHGHSFPWT (SEQ ID NO: 104).

The homology comparison of the heavy chain variable region of the murine antibody Anti-CD40 with the human IgG germline sequence was performed at https://www.ncbi.nlm.nih.gov/ igblast/. IGHV7-4-1^{∗}02 was selected as the heavy chain CDR grafting template, the heavy chain CDRs of the murine antibody Anti-CD40 were transplanted into the framework regions of IGHV7-4-1^{∗}02, and WGQGTLVTVSS (SEQ ID NO: 151) was added following the H-CDR3 as the fourth framework region, to obtain a CDR-grafted heavy chain variable region sequence. Similarly, the homology comparison of the light chain variable region of the murine antibody Anti-CD40 with the human IgG germline sequence was performed. IGKV3-11^{∗}01 was selected as the light chain CDR grafting template, the light chain CDRs of the murine antibody Anti-CD40 were transplanted into the framework regions of IGKV3-11^{∗}01, and FGQGTKVEIK (SEQ ID NO: 152) was added following the L-CDR3 as the fourth framework region, to obtain a CDR-grafted light chain variable region sequence. On the basis of the CDR-grafted variable regions, some amino acid sites in the framework region were subjected to back-mutation. When mutation was performed, the amino acid sequence was numbered according to Kabat and the position of each site was indicated by Kabat numbering.

Preferably, for the CDR-grafted heavy chain variable region, according to Kabat numbering, V at position 2 was mutated to I, T at position 28 was mutated to A, Q at position 39 was mutated to E, and M at position 48 was mutated to I, S at position 76 is mutated to N, and A at position 93 was mutated to V. For the CDR-grafted light chain variable region, A at position 43 was mutated to S, Y at position 49 was mutated to K, and T at position 69 was mutated to S.

The above heavy chain variable region and light chain variable region with mutation sites were defined as humanized heavy chain variable region and light chain variable region, which were named as Anti-CD40-Hu-VH and Anti-CD40-Hu-VL (SEQ ID NOs: 105 and 106), respectively.

The DNAs encoding the humanized heavy chain variable region and light chain variable region were synthesized by Shanghai Sangon Biotech Co., Ltd.. The synthetic humanized heavy chain variable region was connected to the human IgG4 (S228P) heavy chain constant region (SEQ ID NO: 148), to obtain a full-length humanized heavy chain gene, named as Anti-CD40-Hu-HC; the humanized light chain variable region was connected to the human Kappa chain constant region (SEQ ID NO: 149), to obtain a full-length humanized light chain gene, named as Anti-CD40-Hu-LC. The Anti-CD40-Hu-HC and Anti-CD40-Hu-LC genes were constructed into the pcDNA3.4 expression vector, respectively, the antibody was expressed and purified, and the obtained antibody was named as Anti-CD40-Hu.

### Example 6.2 Selection of common light chain

BLAST was used to compare and analyze the amino acid sequences of the light chain variable region of 609 and the light chain variable region of Anti-CD40-Hu. The results showed that between them, the identical amino acids accounted for 90% (Identities) and the amino acids with similar properties accounted for 96% (Positives).

The heavy chain and light chain genes of 609 and Anti-CD40-Hu were combined in the following manner: 609-HC+Anti-CD40-Hu-LC and Anti-CD40-Hu-HC+609-LC, the antibodies were expressed and purified, and the obtained antibodies were named as 609-HC+Anti-CD40-Hu-LC and Anti-CD40-Hu-HC+609-LC, respectively.

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human CD40 with a polyhistidine tag was purchased from Sino Biological (Cat. No.: 10774-H08H), and the two recombinant proteins were named as PD1-ECD-His and CD40-ECD-His, respectively. An ELISA plate was coated with PD1-ECD-His and CD40-ECD-His, respectively, both with a coating concentration of 10 ng/well.

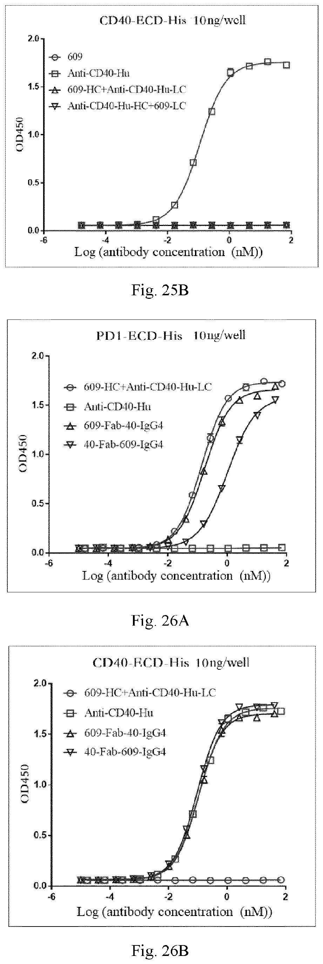

As shown in Fig. 25A, both 609 and 609-HC+Anti-CD40-Hu-LC can effectively bind to PD1-ECD-His, with EC50s of 0.1263 nM and 0.1387 nM, respectively; while Anti-CD40-Hu and Anti-CD40-Hu-HC+609-LC cannot effectively bind to PD1-ECD-His. As shown in Fig. 25B, Anti-CD40-Hu can effectively bind to CD40-ECD-His, with an EC50 of 0.1104 nM, while 609, 609-HC+Anti-CD40-Hu-LC and Anti-CD40-Hu-HC+609-LC cannot effectively bind to CD40-ECD-His. Herein, Anti-CD40-Hu-LC (SEQ ID NOs: 107 and 108) was selected as the common light chain to construct a bispecific antibody.

### Example 6.3 Construction of bispecific antibodies

The heavy chain variable region of 609 was connected to the CH1 domain of human IgG4, and then connected to the heavy chain variable region of Anti-CD40-Hu through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-40-IgG4 (SEQ ID NOs: 109 and 110). Similarly, the heavy chain variable region of Anti-CD40-Hu was connected to the CH1 domain of human IgG4, and then connected to the heavy chain variable region of 609 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 40-Fab-609-IgG4 (SEQ ID NOs: 111 and 112).

The above sequences were constructed into the pcDNA3.4 expression vector, respectively, the 609-Fab-40-IgG4 and 40-Fab-609-IgG4 expression vectors were combined with the Anti-CD40-Hu-LC expression vector, and the antibodies were expressed and purified. The obtained antibodies were named as 609-Fab-40-IgG4 and 40-Fab-609-IgG4, respectively (for brevity, only the name of the heavy chain is used as the name of the antibody).

### Example 6.4 Determination of the relative affinity by ELISA

As shown in Fig. 26A, 609-HC+Anti-CD40-Hu-LC, 609-Fab-40-IgG4 and 40-Fab-609-IgG4 can bind to PD1-ECD-His, with EC50s of 0.1387 nM, 0.1723 nM and 1.017 nM, respectively. As shown in Fig. 26B, Anti-CD40-Hu, 609-Fab-40-IgG4 and 40-Fab-609-IgG4 can effectively bind to CD40-ECD-His, with EC50s of 0.1104 nM, 0.1047 nM and 0.09556 nM, respectively. The above results showed that 609-Fab-40-IgG4 and 40-Fab-609-IgG4 can bind to both PD-1 and CD40, indicating that they are bispecific antibodies.

### Example 7 Construction of bispecific antibodies against PD-1 and other targets

### Example 7.1 Sequences

609 is a humanized anti-human PD-1 monoclonal antibody, the source of which is as described in Example 5.1.

The amino acid sequences (SEQ ID NO: 1-2, 113-118) of the heavy chain variable region and the light chain variable region of the antibodies such as Cetuximab, Bevacizumab, Trastuzumab, Pertuzumab were obtained from the public literature (Magdelaine-Beuzelin C, Kaas Q, Wehbi V, et al. Structure-function relationships of the variable domains of monoclonal antibodies approved for cancer treatment[J]. Critical reviews in oncology/hematology, 2007, 64 (3): 210-225). 10D1 (hereinafter referred to as Ipilimumab) is an anti-human CTLA-4 monoclonal antibody, whose amino acid sequences of the heavy chain variable region and light chain variable region are derived from SEQ ID NO: 17 and SEQ ID NO: 7 in US20020086014A1 (i.e., SEQ ID NOs: 119 and 120 in the present invention).

The DNAs encoding the heavy chain variable region and the light chain variable region were synthesized by Shanghai Sangon Biotech Co., Ltd.. The coding sequences of the heavy chain variable region and the light chain variable region were connected to the human IgG1 heavy chain constant region (SEQ ID NO: 147) and the human Kappa light chain constant region (SEQ ID NO: 149) to construct a full-length heavy chain and light chain genes of an antibody, respectively. The heavy chain genes of the above antibodies were named as Cetuximab-HC, Bevacizumab-HC, Trastuzumab-HC, Pertuzumab-HC and Ipilimumab-HC, respectively, and the light chain genes of the above antibodies were named as Cetuximab-LC, Bevacizumab-LC, Trastuzumab-LC, Pertuzumab-LC and Ipilimumab-LC, respectively. The above heavy chain and light chain genes were constructed into the pcDNA3.4 expression vector, respectively, and the above corresponding heavy chain and light chain genes were combined, and the antibodies were expressed and purified. The obtained antibodies were named as Cetuximab-IgG1, Bevacizumab-IgG1, Trastuzumab-IgG1, Pertuzumab-IgG1 and Ipilimumab-IgG1, respectively.

5E7-Hu is a humanized anti-human LAG-3 antibody, whose heavy chain variable region and light chain variable region sequences are derived from SEQ ID NO: 26 and SEQ ID NO: 28 in PCT/CN2020/076023 (i.e., SEQ ID NOs: 121 and 122 in the present invention). The DNAs encoding the humanized heavy chain variable region and light chain variable region were synthesized by Shanghai Sangon Biotech Co., Ltd.. The synthetic humanized heavy chain variable region was connected to the human IgG4 (S228P) heavy chain constant region (SEQ ID NO: 148) to obtain the full-length humanized heavy chain gene, named as 5E7-Hu-HC; the humanized light chain variable region was connected to the human Kappa chain constant region (SEQ ID NO: 149) to obtain a full-length humanized light chain gene, named as 5E7-Hu-LC. The 5E7-Hu-HC and 5E7-Hu-LC genes were constructed into the pcDNA3.4 expression vector, respectively, and the antibodies were expressed and purified. The obtained antibody was named as 5E7-Hu.

### Example 7.2 Selection of common light chain

### Example 7.2.1 Determination of the binding affinity of hybrid antibodies to antigen

The genes of the heavy chain of 609 and the light chain of the above antibodies were combined, and the antibodies were expressed and purified. The obtained antibodies were named as 609-HC+Cetuximab-LC, 609-HC+Bevacizumab-LC, 609-HC+Trastuzumab-LC, 609-HC+ Pertuzumab-LC, 609-HC+Ipilimumab-LC and 609-HC+5E7-Hu-LC, respectively.

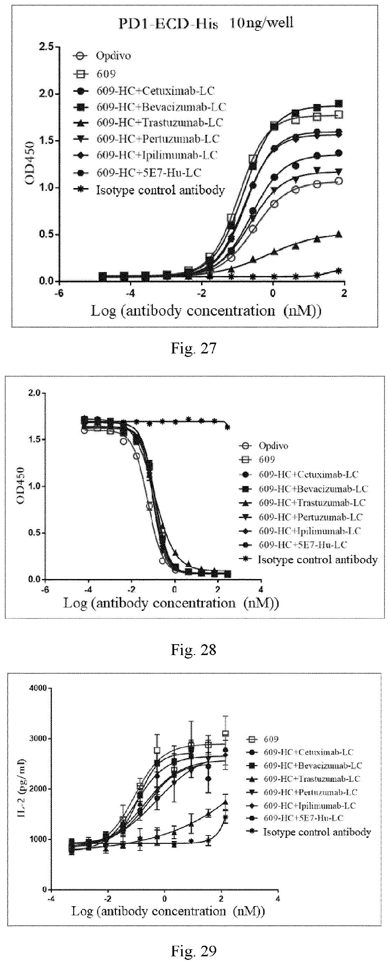

The ELISA detection method is as follows: The recombinant protein of the extracellular segment of human PD-1 with a polyhistidine tag was purchased from Sino Biological (Cat. No.: 10377-H08H), which was named as PD1-ECD-His. An ELISA plate was coated with PD1-ECD-His, with a coating concentration of 10 ng/well. As shown in Fig. 27, Opdivo (purchased from Bristol-Myers Squibb), 609, 609-HC+Cetuximab-LC, 609-HC+Bevacizumab-LC, 609-HC+Pertuzumab-LC, 609-HC+Ipilimumab-LC and 609-HC+5E7-Hu-LC can effectively bind to PD1-ECD-His, with EC50s of 0.2887 nM, 0.1100 nM, 0.2424 nM, 0.1530 nM, 0.2244 nM, 0.1547 nM and 0.1709 nM; while 609-HC+Trastuzumab-LC has a relatively weak binding affinity to PD1-ECD-His, with an EC50 of 0.7219 nM. Therefore, herein Cetuximab-LC (SEQ ID NOs: 123 and 124), Bevacizumab-LC, Pertuzumab-LC (SEQ ID NOs: 125 and 126), Ipilimumab-LC (SEQ ID NOs: 127 and 128) and 5E7-Hu-LC (SEQ ID NOs: 129 and 130) can be selected as a common light chain to construct the corresponding bispecific antibody. Among them, the isotype control antibody is a human IgG4 antibody that does not bind to PD-1.

### Example 7.2.2 Determination of the ability of hybrid antibodies to block PD-1/PD-L1 interaction

The method for detecting the ability of antibodies to block the PD-1/PD-L1 interaction is as follows: The fusion proteins of the extracellular domain of human PD-1 and PD-L1 with human Fc tags were prepared by the inventors (according to the preparation method described in WO2018/137576A1). The two recombinant proteins were named as PD1-ECD-hFc and PD-L1-ECD-hFc, respectively. Biotinylated labeling reagent Biotin N-hydroxysuccinimide ester (purchased from Sigma, Cat. No./Specification: H1759-100MG) was prepared into 100 mM mother liquor with anhydrous DMSO; the corresponding amount of substance concentration was calculated based on the molecular weight and concentration of PD-L1-ECD-hFc; a proper volume of PD-L1-ECD-hFc fusion protein was taken, and after calculating the amount of substance, it was mixed with Biotin N-hydroxysuccinimide ester at a ratio of 1:20, and labeled at room temperature for 1 hour; after dialysis, the protein concentration was determined by ultraviolet spectrophotometry. Human PD-1-ECD-hFc was diluted to 2 µg/ml with coating buffer, added to a 96-well ELISA plate with a multi-channel pipette, 100 µl/well, incubated at room temperature for 4 h; the plate was washed once with PBST, blocked with PBST containing 1% BSA, 200 µl/well, incubated at room temperature for 2h; the blocking solution was discarded, the plate was pat dried, and stored at 4°C for later use. Biotinized PD-L1-ECD-hFc was diluted with PBST+1%BSA (PBST solution containing 1% bovine serum albumin) to 500 ng/ml in a 96-well plate; the anti-PD-1 antibody was serially diluted with the above diluted biotinized fusion protein; the mixed solution of the above diluted antibody and biotinylated fusion protein was transferred to the above ELISA plate coated with human PD-1-ECD-hFc, and incubated at room temperature for 1 hour; the plate was washed 3 times with PBST; Streptavidin-HRP (purchased from BD Biosciences) diluted 1:1000 with PBST+1%BSA was added, incubated at room temperature for 45 min; the plate was washed 3 times with PBST; chromogenic solution (TMB as a substrate) was added, 100 µl/well, incubated at room temperature for 1~5min; stop solution was added to stop the chromogenic reaction, 50 µl/well; OD450 values were read with a microplate reader; data analysis and graphing were performed using GraphPad Prism6, and IC50 was calculated.

As shown in Fig. 28, Opdivo, 609, 609-HC+Cetuximab-LC, 609-HC+Bevacizumab-LC, 609-HC+Trastuzumab-LC, 609-HC+Pertuzumab-LC, 609-HC+Ipilimumab-LC and 609-HC+ 5E7-Hu-LC can effectively block the interaction between PD-1 and PD-L1, with IC50s of 0.05729 nM, 0.1309 nM, 0.1199 nM, 0.1191 nM, 0.1162 nM, 0.09876 nM, 0.1052 nM, 0.1312 nM, respectively. Among them, the isotype control antibody is a human IgG4 antibody that does not bind to PD-1.

### Example 7.2.3 Determination of the ability of hybrid antibodies to enhance mixed lymphocyte reaction

The ability of the above antibodies to enhance the mixed lymphocyte reaction was then determined. As shown in Fig. 29, 609, 609-HC+Cetuximab-LC, 609-HC+Bevacizumab-LC, 609-HC+Pertuzumab-LC, 609-HC+Ipilimumab-LC and 609-HC+5E7-Hu-LC can all effectively stimulate mixed lymphocyte reaction to secrete IL-2, with EC50s of 0.08623 nM, 0.2510 nM, 0.1211 nM, 0.5171 nM, 0.2040 nM and 0.09101 nM, respectively. Among them, 609-HC+ Trastuzumab-LC cannot effectively stimulate mixed lymphocyte reaction to secrete IL-2. The isotype control antibody is a human IgG4 antibody that does not bind to PD-1.

### Example 7.2.4 Determination of the ability of hybrid antibodies to bind to cell surface PD-1 by flow cytometry

The ability of hybrid antibodies to bind to PD-1 on the cell surface was determined by flow cytometry. The establishment process of TF-1 cells expressing PD-1 is as follows: the full-length human PD-1 gene (sequence from UniProt, Entry: Q15116) was constructed into the lentiviral expression vector pLVX-Puro (purchased from Clontech). The lentiviral packaging vector and pLVX-Puro loaded with the target gene were transfected into HEK293FT cells (purchased from Thermo Fisher Scientific, Cat. No.: R70007) with Lipofectamine 3000 (purchased from Thermo Fisher Scientific, Cat. No.: L3000001), incubated in a cell incubator for 48 hours and then the cell culture supernatant was collected and filtered with a 0.45 µm filter membrane to remove cell debris. TF-1 cells (purchased from ATCC, Cat. No. CRL-2003^{™}) were infected with the above supernatant containing virus particles. After 48 hours, the cells were treated with Puromycin to select a cell population that stably expresses the target gene. The TF-1 cell line stably expressing PD-1 was named as TF1-PD1.

The method for detecting the binding of antibodies to cells by flow cytometry is as follows: TF1-PD1 cells were inoculated into a round-bottom 96-well plate (200,000 cells per well); after centrifugation, the supernatant was aspirated, and serially diluted antibodies were added and incubated at room temperature for about half an hour; the cells were washed twice with PBS; after centrifugation, the supernatant was aspirated, and an appropriately diluted anti-human IgG (Fc specific)-FITC antibody (purchased from Sigma, Cat. No.: F9512) was added to each well, and incubated at room temperature for about half an hour; the cells were washed twice with PBS; after aspirating the supernatant, Fix Buffer I (purchased from BD Biosciences) was added to fix the cells and incubated at room temperature for 5 min; the cells were washed twice with PBS, and finally resuspended in 200 µ1 of PBS. The fluorescence intensity of the FITC channel was detected on the flow cytometer; the experimental data was processed with the software of the flow cytometer and exported to Excel; data analysis and graphing were performed using GraphPad Prism6, and EC50 was calculated.

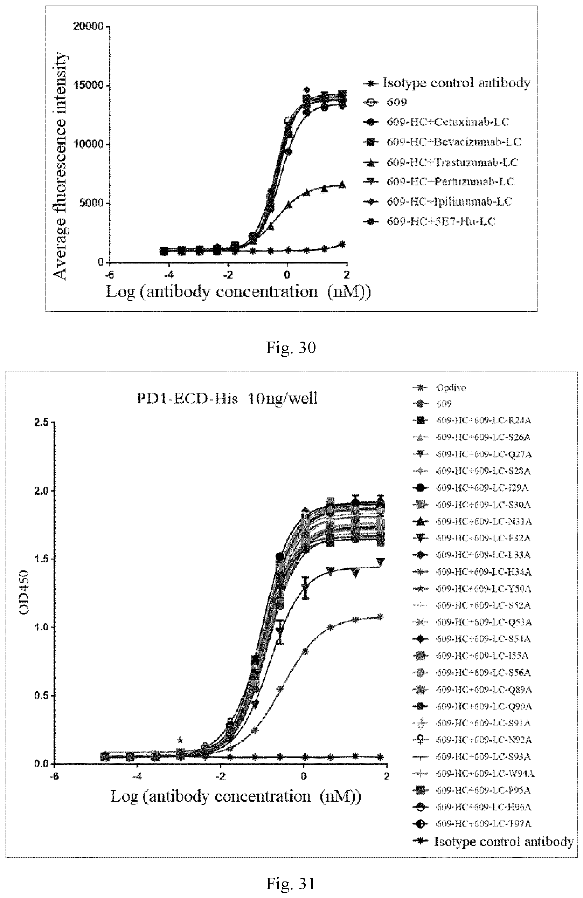

As shown in Fig. 30, 609, 609-HC+Cetuximab-LC, 609-HC+Bevacizumab-LC, 609-HC+ Pertuzumab-LC, 609-HC+Ipilimumab-LC and 609-HC+5E7-Hu-LC can effectively bind to PD-1 on the cell surface, with EC50s of 0.3761 nM, 0.577 nM, 0.5193 nM, 0.4302 nM, 0.4773 nM and 0.3864 nM, respectively. Among them, the binding effect of 609-HC+Trastuzumab-LC to TF1-PD1 was significantly weaker than other hybrid antibodies. The isotype control antibody is a human IgG4 antibody that does not bind to PD-1.

### Example 7.2.5 Alanine scanning study of the effect of light chain variable region CDR of 609 on binding of 609 to PD-1

The above experimental results showed that the hybrid antibodies produced by the combination of the heavy chain of 609 with the light chain of many other target antibodies can further effectively bind to PD-1 molecules, and has the ability to block PD-1/PD-L1 interaction, stimulate mixed lymphocyte reaction and the ability to bind to PD-1 on the cell surface. Herein, alanine scanning was used to study the effect of light chain variable region CDRs of 609 in the binding of 609 to PD-1. The method is as follows: the amino acids in the light chain CDR of 609 were mutated to alanine, respectively (the original alanine in the CDRs not changed), and then the heavy chain of 609 was combined with these light chain mutants, respectively, and the antibodies were expressed and purified according to the method described in the above examples, and then the relative affinity of the antibodies to PD-1 was determined according to the ELISA method described in the above examples. As shown in Table 3, R24A in 609-HC+609-LC-R24A means that the arginine at position 24 was mutated to alanine, the position of the mutated amino acid was indicated by Kabat numbering, and the rest can be deduced by analogy.

**Table 3. Alanine scanning results of light chain variable region of 609**

| **Antibody name** | **EC50 (nM)** |
|---|---|
| Opdiv o | 0.3054 |
| 609 | 0.0980 |
| 609-HC+609-LC- R24A | 0.0967 |
| 609-HC+609-LC-S26A | 0.1094 |
| 609-HC+609-LC-Q27A | 0.1105 |
| 609-HC+609-LC-S28A | 0.1059 |
| 609-HC+609-LC-I29A | 0.0969 |
| 609-HC+609-LC-S3 0A | 0.1068 |
| 609-HC+609-LC-N31A | 0.1148 |
| 609-HC+609-LC-F32A | 0.1547 |
| 609-HC+609-LC-L33A | 0.1060 |
| 609-HC+609-LC- H34A | 0.1112 |
| 609-HC+609-LC- Y50A | 0.1272 |
| 609-HC+609-LC-S52A | 0.1074 |
| 609-HC+609-LC-Q53A | 0.1103 |
| 609-HC+609-LC-S54A | 0.1237 |
| 609-HC+609-LC-I55A | 0.1140 |
| 609-HC+609-LC-S56A | 0.1240 |
| 609-HC+609-LC-Q89A | 0.1322 |
| 609-HC+609-LC-Q90A | 0.1457 |
| 609-HC+609-LC-S91A | 0.1334 |
| 609-HC+609-LC-N92A | 0.0971 |
| 609-HC+609-LC-S93A | 0.1106 |
| 609-HC+609-LC-W94A | 0.1045 |
| 609-HC+609-LC-P95A | 0.1317 |
| 609-HC+609-LC-H96A | 0.1266 |
| 609-HC+609-LC-T 97A | 0.1143 |

As shown in Fig. 31 and Table 3, the alanine scanning results showed that the mutation of the light chain CDR amino acids to alanine did not significantly affect the binding of the antibody to PD-1, indicating that 609 mainly binds to PD-1 molecules through the heavy chain, and is less dependent on the light chain.

### Example 7.3 Construction of bispecific antibodies against PD-1 and EGFR

The heavy chain variable region of 609 was connected to the CH1 domain of human IgG4, and then connected to the heavy chain variable region of Cetuximab-IgG1 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-Cetuximab-IgG4 (SEQ ID NOs: 131 and 132).

The above sequences were constructed into the pcDNA3.4 expression vector, respectively, the 609-Fab-Cetuximab-IgG4 was combined with the Cetuximab-LC expression vector, and the antibody was expressed and purified. The obtained antibody was named as 609-Fab-Cetuximab-IgG4 (for brevity, only the name of the heavy chain is used as the name of the antibody).

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human EGFR with a polyhistidine tag was purchased from Sino Biological (Cat. No.: 10001-H08H), and the two recombinant proteins were named as PD1-ECD-His and EGFR-ECD-His, respectively. An ELISA plate was coated with PD1-ECD-His and EGFR-ECD-His, with a coating concentration of 10 ng/well and 20 ng/well, respectively.

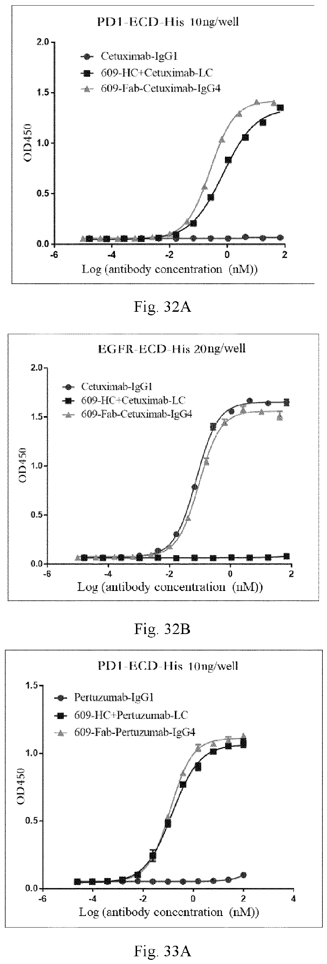

As shown in Fig. 32A, both 609-HC+Cetuximab-LC and 609-Fab-Cetuximab-IgG4 can effectively bind to PD1-ECD-His, with EC50s of 0.7172 nM and 0.2616 nM, respectively. As shown in Fig. 32B, both Cetuximab-IgG1 and 609-Fab-Cetuximab-IgG4 can effectively bind to EGFR-ECD-His, with EC50s of 0.07609 nM and 0.09327 nM, respectively. The above results showed that 609-Fab-Cetuximab-IgG4 can bind to both PD-1 and EGFR, indicating that it is a bispecific antibody.

### Example 7.4 Construction of bispecific antibodies against PD-1 and HER-2

The heavy chain variable region of 609 was connected to the CH1 domain of human IgG4, and then connected to the heavy chain variable region of Pertuzumab-IgG1 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-Pertuzumab-IgG4 (SEQ ID NOs: 133 and 134).

The above sequences were constructed into the pcDNA3.4 expression vector, respectively, the 609-Fab-Pertuzumab-IgG4 was combined with the Pertuzumab-LC expression vector, and the antibodies were expressed and purified. The obtained antibody was named as 609-Fab-Pertuzumab-IgG4 (for brevity, only the name of the heavy chain is used as the name of the antibody).

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human Her-2 with a polyhistidine tag was purchased from ACROBiosystems (Cat. No.: HE2-H5225), and the two recombinant proteins were named as PD1-ECD-His and HER2-ECD-His. An ELISA plate was coated with PD1-ECD-His and HER2-ECD-His, both with a coating concentration of 10 ng/well.

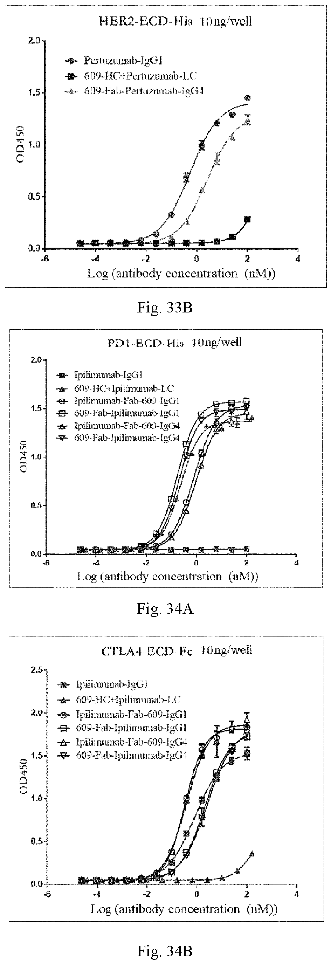

As shown in Fig. 33A, both 609-HC+Pertuzumab-LC and 609-Fab-Pertuzumab-IgG4 can effectively bind to PD1-ECD-His, with EC50s of 0.1422 nM and 0.1196 nM, respectively. As shown in Fig. 33B, both Pertuzumab-IgG1 and 609-Fab-Pertuzumab-IgG4 can effectively bind to HER2-ECD-His, with EC50s of 0.5352 nM and 2.616 nM, respectively. The above results showed that 609-Fab-Pertuzumab-IgG4 can bind to both PD-1 and HER-2, indicating that it is a bispecific antibody.

### Example 7.5 Construction of bispecific antibodies against PD-1 and CTLA-4

### Example 7.5.1 Construction of bispecific antibodies

The heavy chain variable region of 609 was connected to the CH1 domain of human IgG4, and then connected to the heavy chain variable region of Ipilimumab-IgG1 through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as 609-Fab-Ipilimumab-IgG1 (SEQ ID NOs: 135 and 136). Similarly, the heavy chain variable region of Ipilimumab-IgG1 was connected to the CH1 domain of human IgG4, and then connected to the heavy chain variable region of 609 through an artificial linker (the linker used here is three GGGGS in tandem), and finally connected to the heavy chain constant region of human IgG1 (CH1+CH2+CH3). The long heavy chain containing two heavy chain variable regions and two CH1 domains constructed by such procedures was named as Ipilimumab-Fab-609-IgG1 (SEQ ID NOs: 137 and 138).

The heavy chain variable region of 609 was connected to the CH1 domain of human IgG4, and then connected to the heavy chain variable region of Ipilimumab-IgG1 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-Ipilimumab-IgG4 (SEQ ID NOs: 139 and 140). Similarly, the heavy chain variable region of Ipilimumab-IgG1 was connected to the CH1 domain of human IgG4, and then connected to the heavy chain variable region of 609 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as Ipilimumab-Fab-609-IgG4 (SEQ ID NOs: 141 and 142).

The above sequences were constructed into the pcDNA3.4 expression vector, respectively. 609-Fab-Ipilimumab-IgG1, Ipilimumab-Fab-609-IgG1, 609-Fab-Ipilimumab-IgG4 and Ipilimumab-Fab-609-IgG4 were combined with the Ipilimumab-LC expression vector, respectively, and the antibodies were expressed and purified. The obtained antibodies were named as 609-Fab-Ipilimumab-IgG1, Ipilimumab-Fab-609-IgG1, 609-Fab-Ipilimumab-IgG4 and Ipilimumab-Fab-609-IgG4 (for brevity, only the name of the heavy chain is used as the name of the antibody).

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human CTLA-4 with human Fc tags was purchased from Sino Biological (Cat. No.: 11159-H31H5), and the two recombinant proteins were named as PD1-ECD-His and CTLA4-ECD-Fc. An ELISA plate was coated with PD1-ECD-His and CTLA4-ECD-Fc, respectively, both with a coating concentration of 10 ng/well.

As shown in Fig. 34A, 609-HC+Ipilimumab-LC, 609-Fab-Ipilimumab-IgG1, Ipilimumab-Fab-609-IgG1, 609-Fab-Ipilimumab-IgG4 and Ipilimumab-Fab-609-IgG4 can effectively bind to PD1-ECD-His, with EC50s of 0.2337 nM, 0.1734 nM, 0.7954 nM, 0.2078 nM and 0.9643 nM, respectively. As shown in Fig. 34B, Ipilimumab-IgG1, 609-Fab-Ipilimumab-IgG1, Ipilimumab-Fab-609-IgG1, 609-Fab-Ipilimumab-IgG4 and Ipilimumab-Fab-609-IgG4 can effectively bind to CTLA4-ECD-Fc, with EC50s of 0.8354 nM, 2.123 nM, 0.3376 nM, 2.626 nM and 0.392 nM, respectively. The above results showed that 609-Fab-Ipilimumab-IgG1, Ipilimumab- Fab-609-IgG1, 609-Fab-Ipilimumab-IgG4 and Ipilimumab-Fab-609-IgG4 can bind to both PD-1 and CTLA-4, indicating that they are bispecific antibodies.

### Example 7.5.2 Determination of the functional activity of bispecific antibodies against PD-1 and CTLA-4

The following additives were added to RPMI 1640 to prepare RPMI 1640 complete medium: 10% Fetal Bovine Serum; 1% MEM Non-Essential Amino Acids Solution; 1% Sodium Pyruvate; 1% HEPES; 1‰ 2-Mercaptoethanol; 1% Penicillin-Streptomycin; 1% GlutaMAX (the above additives were purchased from Thermo Fisher Scientific). Freshly isolated PBMCs (purchased from Allcells, Cat. No.: PB005-C) was washed and resuspended with the above RPMI 1640 complete medium, and a certain amount of superantigen staphylococcal enterotoxin B (SEB) was added. The superantigen was prepared using E. coli by the inventors (SEB amino acid sequence is derived from https://www.uniprot.org/uniprot/P01552). The PBMC cell suspension was inoculated into a roundbottomed 96-well cell culture plate, with 150 µ1 suspension and 200,000 cells per well; 50 µ1 of serially diluted antibody was added to the above 96-well plate; the 96-well plate was placed in a 37°C cell incubator and incubated for 4 days. An appropriate amount of cell culture supernatant was taken from the 96-well plate, and then IL-2 secretion was determined in accordance with standard operating procedures. The IL-2 in the supernatant was determined by sandwich ELISA (the paired antibodies for related detection were purchased from BD Biosciences). OD450 was read with a microplate reader (SpectraMax 190), graphing was performed using graphPad Prism6 and EC50 was calculated.

**Table 4. Functional activity parameters of bispecific antibodies against PD-1 and CTLA-4**

| Antibody | EC50 (nM) | Top |
|---|---|---|
| 609 | 0.0682 | 9269 |
| 609-HC+Ipilimumab-LC | 0.06578 | 8546 |
| Ipilimumab-IgG1 | 0.07534 | 9700 |
| Ipilimumab-Fab-609-IgG4 | 0.5292 | 9115 |
| 609-Fab-Ipilimumab-IgG4 | 0.1267 | 10441 |
| Ipilimumab-Fab-609-IgG1 | 0.1173 | 11416 |
| 609-Fab-Ipilimumab-IgG1 | 0.05802 | 15237 |
| 609-HC+Ipilimumab-LC/Ipilimumab-IgG1 | 0.06863 | 14447 |

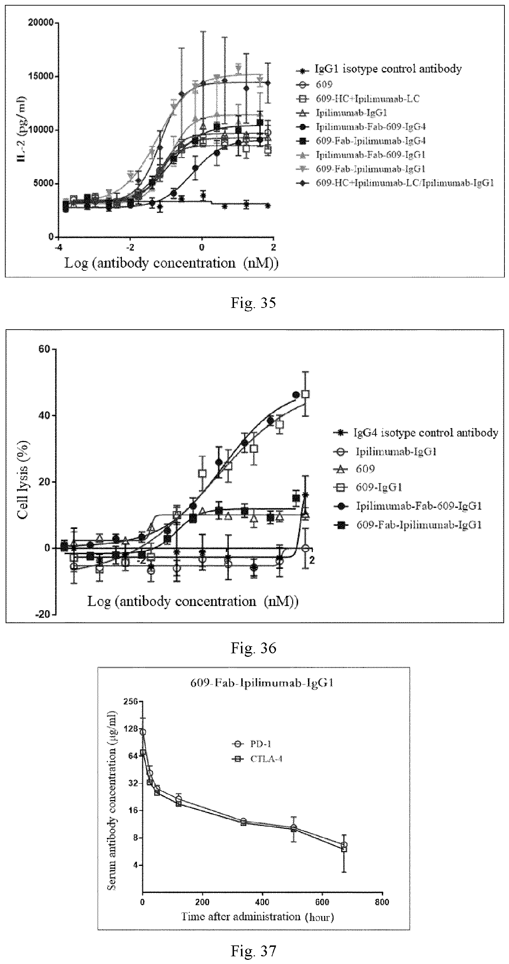

As shown in Fig. 35 and Table 4, 609, 609-HC+Ipilimumab-LC and Ipilimumab-IgG1 have comparable EC50 and Top (high platform), indicating that these three antibodies have similar functional activities. From high to low, the functional activities of anti-PD-1 and CTLA-4 bispecific antibodies are ranked as follows: 609-Fab-Ipilimumab-IgG1>Ipilimumab-Fab-609-IgG1> 609-Fab-Ipilimumab-IgG4>Ipilimumab-Fab-609-IgG4. The functional activity of 609-Fab-Ipilimumab-IgG1 and Ipilimumab-Fab-609-IgG1 was significantly better than that of monoclonal antibodies 609 and Ipilimumab-IgG1, and the hybrid antibody 609-HC+Ipilimumab-LC, but was similar to the effect of the combination application of 609-HC+Ipilimumab-LC and Ipilimumab-IgG1 (609-HC+ Ipilimumab-LC/Ipilimumab-IgG1 means that the two antibodies are used in combination at a ratio of amount of substance of 1:1).

### Example 7.5.3 Determination of the ADCC activity of bispecific antibodies against PD-1 and CTLA-4

Antibody-dependent cell-mediated cytotoxicity (ADCC) is a universal function of human IgG antibodies, and the strength of ADCC is related to the antibody subtype. Bispecific antibodies against PD-1 and CTLA-4 of IgG1 subtype may have potential cytotoxicity to the cells expressing PD-1. Herein, freshly isolated PBMCs (purchased from Allcells, Cat. No.: PB005-C) were used as effector cells, and TF-1 cells expressing PD-1 were used as target cells to determine ADCC.

As shown in Fig. 36, the IgG4 isotype control antibody (a monoclonal antibody that does not bind to PD-1 and CTLA-4) did not show significant ADCC. Ipilimumab-IgG1 did not show significant ADCC effect, because TF1-PD1 cells do not express CTLA-4. 609 had weak ADCC (with heavy chain constant region of IgG4 subtype, target cell lysis of up to about 10%), 609-IgG1 had a strong ADCC (replacing the heavy chain constant region of 609 with IgG1 subtype, with the target cell lysis of up to about 50%), because the heavy chain constant regions of the two are IgG4 and IgG1, respectively, and IgG1 usually has a stronger ADCC activity than IgG4. The ADCC of 609-Fab-Ipilimumab-IgG1 was similar to that of 609, and the ADCC of Ipilimumab-Fab-609-IgG1 was similar to that of 609-IgG1. The ADCC of 609-Fab-Ipilimumab-IgG1 was significantly weaker than that of Ipilimumab-Fab-609-IgG1, which may be related to the spatial ordering of bispecific antibodies.

### Example 7.5.4 Pharmacokinetics of bispecific antibodies against PD-1 and CTLA-4 in rats

The pharmacokinetics of the bispecific antibodies against PD-1 and CTLA-4 in rats was determined by the method described in Example 1.7.5. The difference is that the ELISA plate was coated with the two related antigens corresponding to the bispecific antibodies against PD-1 and CTLA-4 (the sources of PD1-ECD-His and CTLA4-ECD-Fc are as described in Example 7.5.1).

As shown in Fig. 37, it was calculated that the half-life of 609-Fab-Ipilimumab-IgG1 was 15.2 days (detection result with PD-1 as the antigen) and 14.6 days (detection result with CTLA-4 as the antigen). The above results indicate that 609-Fab-Ipilimumab-IgG1 has good pharmacokinetic properties.

### Example 7.5.5 Anti-tumor effect of bispecific antibodies against PD-1 and CTLA-4 in mice

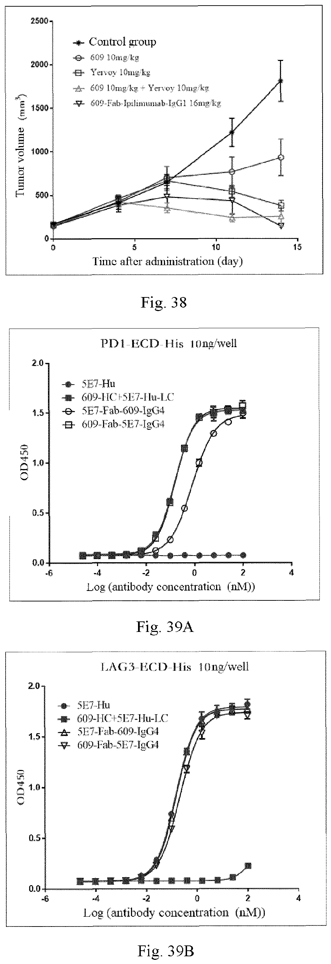

The human PD-1/CTLA-4 double transgenic mice (germline background is C57BL/6) were purchased from Beijing Biocytogen Technology Co. Ltd., and MC38 mouse colorectal cancer cells were purchased from Guangzhou Jennio Biotech Co., Ltd.. In the PD-1/CTLA-4 double transgenic mice, the extracellular segments of human PD-1 and CTLA-4 genes were used to replace the homologous parts of the mouse, so the bispecific antibody of the present invention can recognize PD-1 and CTLA-4 in the transgenic mice. The specific implementation steps are as follows: MC38 cells were cultured in vitro with 10% serum-containing DMEM (serum and medium purchased from Gibco); the cultured MC38 cells were inoculated into human PD-1 transgenic mice, each mouse was inoculated 2×10⁶ cells subcutaneously; when the tumor cells to be inoculated grew to a volume close to 100 mm³, the mice were randomly divided into groups, with 8 mice in each group. The drug treatment of mice in each group is as follows: Control group, only injected with normal saline; 609 group, injected with 10 mg/kg of anti-PD-1 antibody 609; Yervoy group, injected with 10 mg/kg of anti-CTLA-4 positive control antibody Yervoy (produced by Bristol-Myers Squibb); 609+Yervoy group, injected with 10 mg/kg of 609 and 10 mg/kg of Yervoy; 609-Fab-Ipilimumab-IgG1 group, injected with 16mg/kg of 609-Fab-Ipilimumab-IgG1. Taking into account the difference in molecular weight between bispecific antibodies and monoclonal antibodies, the dose of the drug in this experiment was provided according to the rule of equal amount of substance. Subsequently, the drugs were administered according to the above-designed regimen, twice a week for a total of 4 times, and the tumor volume was measured twice a week. Finally, the growth curves of tumors in each group determined over time are shown in Fig. 38.

The results showed that at the end of the 14th day, the tumor inhibition rates of 609, Yervoy, 609 + Yervoy and 609-Fab-Ipilimumab-IgG1 groups were 48.6%, 79.1%, 85.9% and 92.2%, respectively (tumor inhibition rate = (mean volume of control group - mean volume of experimental group) / mean volume of control group × 100%). Compared to single agent 609 or Yervoy, 609-Fab-Ipilimumab-IgG1 can inhibit tumor growth more effectively. The therapeutic effect of 609-Fab-Ipilimumab-IgG1 was similar to that of the combination of 609 and Yervoy.

### Example 7.6 Construction of bispecific antibodies against PD-1 and LAG-3

### Example 7.6.1 Construction of bispecific antibodies

The heavy chain variable region of 609 was connected to the CH1 domain of human IgG4, and then connected to the heavy chain variable region of 5E7-Hu through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 609-Fab-5E7-IgG4 (SEQ ID NOs: 143 and 144). Similarly, the heavy chain variable region of 5E7-Hu was connected to the CH1 domain of human IgG4, and then connected to the heavy chain variable region of 609 through an artificial linker (the linker used here is three GGGGS in tandem), and finally connected to the heavy chain constant region of human IgG4 (CH1+CH2+CH3, with the S228P mutation in the hinge region). The long heavy chain containing two heavy chain variable regions and two CH1 domains constructed by such procedures was named as 5E7-Fab-609-IgG4 (SEQ ID NOs: 145 and 146).

The above sequences were constructed into the pcDNA3.4 expression vector, 609-Fab-5E7-IgG4 and 5E7-Fab-609-IgG4 were combined with the 5E7-Hu-LC expression vector, respectively. The antibodies were expressed and purified using the method described in the above examples. The obtained antibodies were named as 609-Fab-5E7-IgG4 and 5E7-Fab-609-IgG4, respectively (for brevity, only the name of the heavy chain is used as the name of the antibody).

The ELISA detection method is as follows: The recombinant protein of the extracellular segment of human PD-1 with a polyhistidine tag was purchased from Sino Biological (Cat. No.: 10377-H08H), and the recombinant protein of the extracellular segment of human LAG-3 with a polyhistidine tag were purchased from Sino Biological (Cat. No.: 16498-H08H). The two recombinant proteins were named as PD1-ECD-His and LAG3-ECD-His, respectively. An ELISA plate was coated with PD1-ECD-His and LAG3-ECD-His, respectively, both with the coating concentration of 10 ng/well.

As shown in Fig. 39A, 609-HC+5E7-Hu-LC, 609-Fab-5E7-IgG4 and 5E7-Fab-609-IgG4 can effectively bind to PD1-ECD-His, with EC50s of 0.1523 nM, 0.161 nM and 0.8138 nM, respectively. As shown in Fig. 39B, 5E7-Hu, 609-Fab-5E7-IgG4 and 5E7-Fab-609-IgG4 can effectively bind to LAG3-ECD-His, with EC50s of 0.1472 nM, 0.2082 nM and 0.1529 nM, respectively. The above results showed that 609-Fab-5E7-IgG4 and 5E7-Fab-609-IgG4 can bind to both PD-1 and LAG-3, indicating that they are bispecific antibodies.

### Example 7.6.2 Determination of the ability of bispecific antibodies against PD-1 and LAG-3 to simultaneously bind to two antigens

A microplate was coated with LAG3-ECD-His. The antibody to be tested was serially diluted with PBST containing 1% bovine serum albumin, then transferred to the above microplate, and incubated at room temperature for about half an hour. The subsequent experimental steps were the same as described in Example 1.7.4.

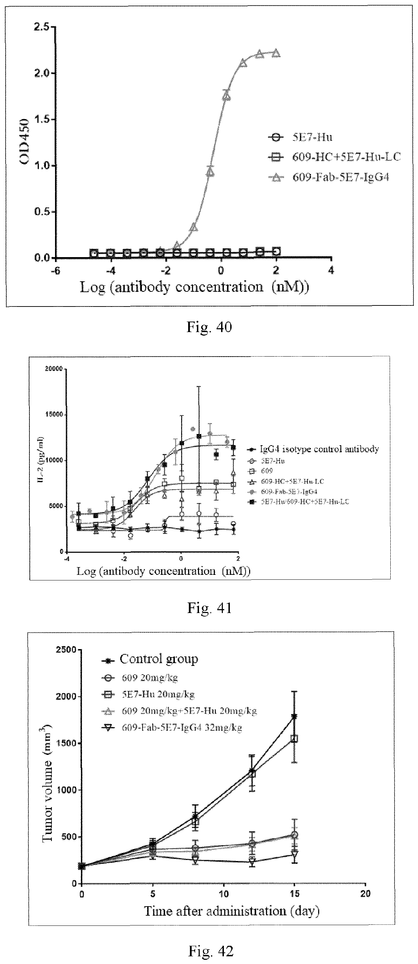

As shown in Fig. 40, 609-Fab-5E7-IgG4 can further effectively bind to human PD-1 after binding to LAG-3, with an EC50 of 0.5294 nM. Neither 5E7-Hu nor 609-HC+5E7-Hu-LC can simultaneously bind to PD-1 and LAG-3.

### Example 7.6.3 Determination of the functional activity of bispecific antibodies against PD-1 and LAG-3

Herein, the functional activity of the bispecific antibodies against PD-1 and LAG-3 was determined according to the method described in Example 7.5.2.

**Table 5. Functional activity parameters of bispecific antibodies against PD-1 and LAG-3**

| Antibody | EC50(nM) | Top |
|---|---|---|
| 5E7-Hu | 0.2835 | 3851 |
| 609 | 0.0271 | 7473 |
| 609-HC+5E7-Hu-LC | 0.0247 | 6836 |
| 609-Fab-5E7-IgG4 | 0.1333 | 12765 |
| 5E7-Hu/609-HC+5E7-Hu-LC | 0.0719 | 11661 |

As shown in Fig. 41 and Table 5, 5E7-Hu can only weakly stimulate the secretion of IL-2. 609 and 609-HC+5E7-Hu-LC had comparable EC50 and Top (high platform), indicating that the two have similar functional activity. When the concentration was greater than 1 nM, the ability of 609-Fab-5E7-IgG4 to stimulate IL-2 secretion was significantly higher than that of the monoclonal antibodies 5E7-Hu and 609 and the hybrid antibody 609-HC+5E7-Hu-LC, but was similar to the effect of the combination of 5E7-Hu and 609-HC+5E7-Hu-LC (5E7-Hu/609-HC+5E7-Hu-LC means that the two antibodies are used in combination at a ratio of 1:1).

### Example 7.6.4 Anti-tumor effect of bispecific antibodies against PD-1 and LAG-3 in mice

Human PD-1/LAG-3 double transgenic mice (germline background is C57BL/6) were purchased from Beijing Biocytogen Technology Co. Ltd., and MC38 mouse colorectal cancer cells were purchased from Guangzhou Jennio Biotech Co., Ltd.. In the PD-1/LAG-3 double transgenic mice, the extracellular segments of human PD-1 and LAG-3 genes were used to replace the homologous parts of the mouse, so the bispecific antibody of the present invention can recognize PD-1 and LAG-3 in the transgenic mice. The specific implementation steps are as follows: MC38 cells were cultured in vitro with 10% serum-containing DMEM (serum and medium purchased from Gibco); the cultured MC38 cells were inoculated into human PD-1 transgenic mice, each mouse was inoculated with 2×10⁶ cells subcutaneously; when the tumor cells to be inoculated grew to a volume close to 100 mm³, the mice were randomly divided into groups, with 8 mice in each group. The drug treatment of mice in each group is as follows: Control group, only injected with normal saline; 609 group, injected with 20 mg/kg of anti-PD-1 antibody 609; 5E7-Hu group, injected with 20 mg/kg of anti-LAG-3 antibody 5E7-Hu; 609 + 5E7-Hu group, injected with 20 mg/kg of 609 and 20 mg/kg of 5E7-Hu; 609-Fab-5E7-IgG4 group, injected with 32 mg/kg of 609-Fab-5E7-IgG4. Taking into account the difference in molecular weight between bispecific antibodies and monoclonal antibodies, the dose of the drug in this experiment was provided according to the rule of equal amount of substance. Subsequently, the drugs were administered according to the above-designed regimen, twice a week for a total of 4 times, and the tumor volume was measured twice a week. Finally, the growth curves of tumors in each group determined over time are shown in Fig. 42.

The results showed that at the end of the 14th day of the experiment, the tumor inhibition rates of 609, 5E7-Hu, 609 + 5E7-Hu and 609-Fab-5E7-IgG4 groups were 70.8%, 13.1%, 71.5% and 82.8%, respectively (Tumor inhibition rate = (mean volume of control group - mean volume of experimental group) / mean volume of control group × 100%). Compared to single agent 609 or 5E7-Hu, 609-Fab-5E7-IgG4 can inhibit tumor growth more effectively. The combination of 609 and 5E7-Hu was not more effective than the single agent 609. Therefore, it can be speculated that 609-Fab-5E7-IgG4 as a bispecific antibody against PD-1 and LAG-3 exhibits a synergistic effect.

### Example 7.7 Determination of the specificity of hybrid antibodies

The ELISA detection method is as follows: the relevant antigens mentioned in the above examples (EGFR-ECD-His, VEGF165-His, HER2-ECD-His, LAG3-ECD-His and CTLA4-ECD-Fc) were used to coat an ELISA plate, respectively, according to the experimental conditions described above, and then detected whether the hybrid antibodies comprising the heavy chain of 609 and the light chain of other target antibodies can recognize such targets. The sources of the antibodies such as Cetuximab-IgG1, 601, Trastuzumab-IgG1, 5E7-Hu and Ipilimumab-IgG1 were described in the above examples (the source of the variable region of Trastuzumab-IgG1 is as described in Example 7.1, the constant region is the same as that of Ipilimumab-IgG1, and the preparation method is the same as that of other antibodies). Herein, they were used as positive control antibodies that bind to various antigens, respectively.

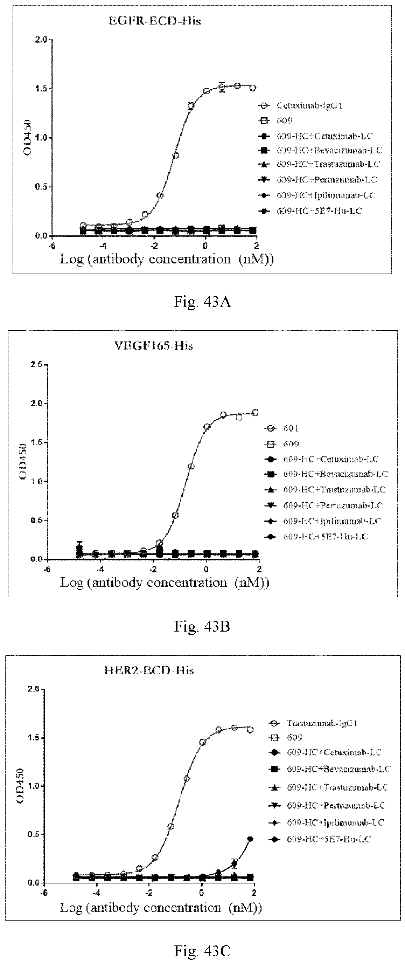

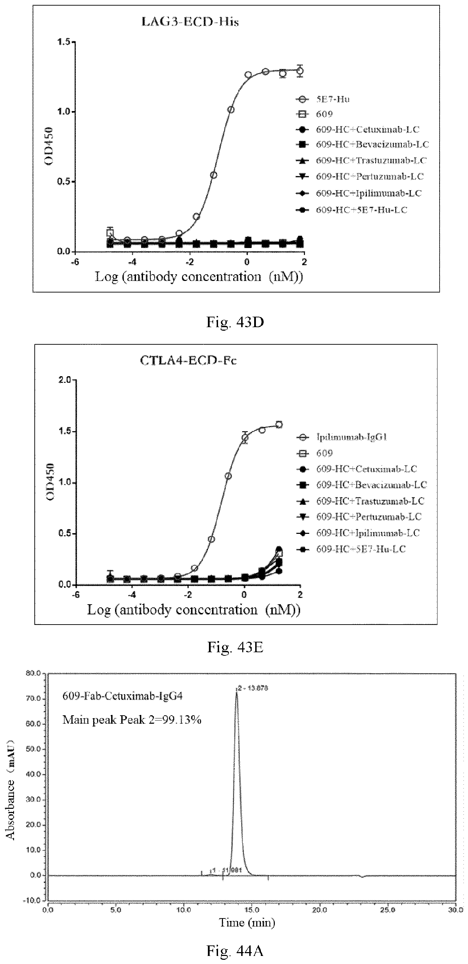

Figs. 43A to 43E showed that the hybrid antibodies comprising the heavy chain of 609 and the light chain of other target antibodies cannot recognize other targets, indicating that these hybrid antibodies have good specificity.

### Example 7.8 HPLC-SEC

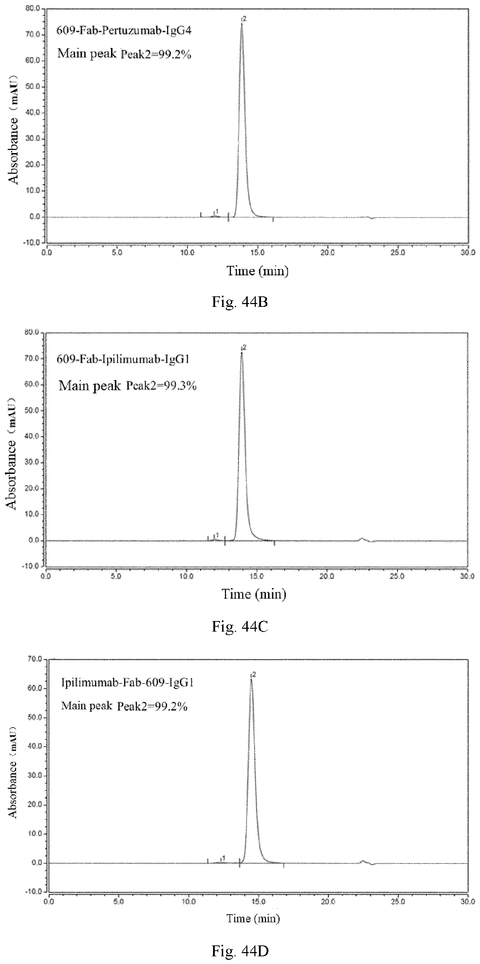

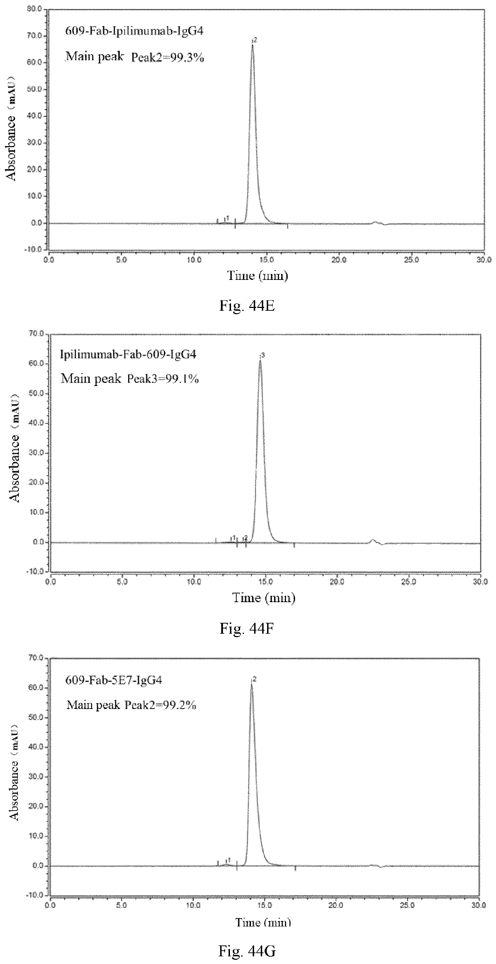

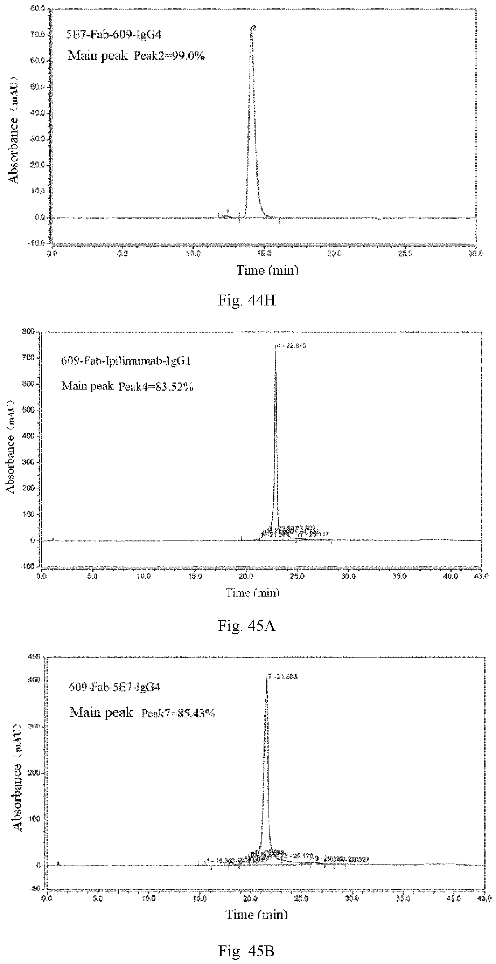

Fig. 44A shows the HPLC-SEC pattern of 609-Fab-Cetuximab-IgG4, with the main peak accounting for 99.13%. Fig. 44B shows the HPLC-SEC pattern of 609-Fab-Pertuzumab-IgG4, with the main peak accounting for 99.2%. Fig. 44C shows the HPLC-SEC pattern of 609-Fab-Ipilimumab-IgG1, with the main peak accounting for 99.3%. Fig. 44D shows the HPLC-SEC pattern of Ipilimumab-Fab-609-IgG1, with the main peak accounting for 99.2%. Fig. 44E shows the HPLC-SEC pattern of 609-Fab-Ipilimumab-IgG4, with the main peak accounting for 99.3%. Fig. 44F shows the HPLC-SEC pattern of Ipilimumab-Fab-609-IgG4, with the main peak accounting for 99.1%. Fig. 44G shows the HPLC-SEC pattern of 609-Fab-5E7-IgG4, with the main peak accounting for 99.2%. Fig. 44H shows the HPLC-SEC pattern of 5E7-Fab-609-IgG4, with the main peak accounting for 99.0%.

### Example 7.9 HPLC-IEC

Fig. 45A shows the HPLC-IEC pattern of 609-Fab-Ipilimumab-IgG1, with the main peak accounting for 83.52%. The results indicate that 609-Fab-Ipilimumab-IgG1 has good charge heterogeneity.

Fig. 45B shows the HPLC-IEC pattern of 609-Fab-5E7-IgG4, with the main peak accounting for 85.43%. The results indicate that 609-Fab-5E7-IgG4 has good charge heterogeneity.

### Example 7.10 CE-SDS

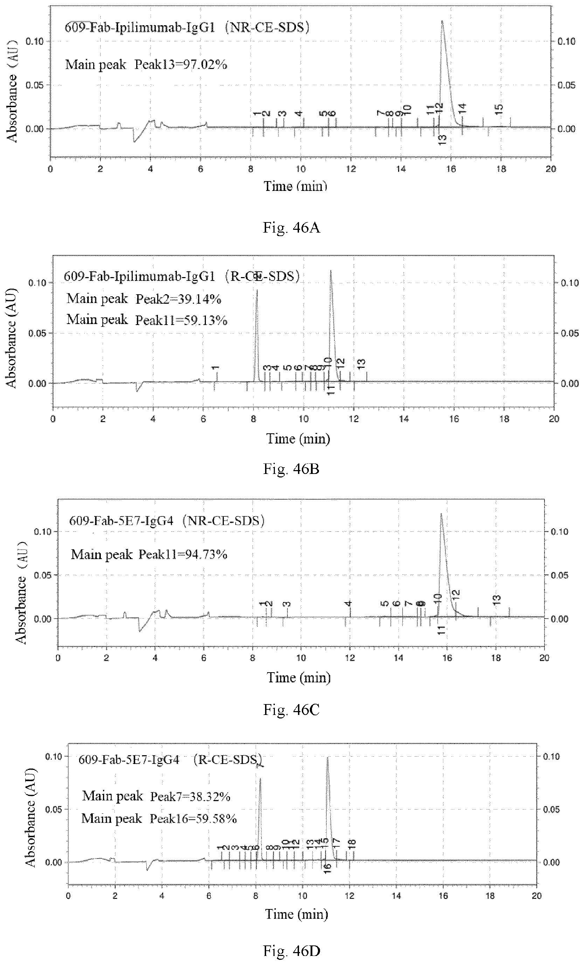

Figs. 46A and 46B show the patterns of NR-CE-SDS and R-CE-SDS of 609-Fab-Ipilimumab-IgG1, respectively. In the NR-CE-SDS pattern, the main peak Peak13 accounted for 97.02%; in the R-CE-SDS pattern, the two main peaks Peak 2 (corresponding to the light chain) and Peak 12 (corresponding to the long heavy chain) accounted for 39.14% and 59.13%, respectively, and the ratio of the two peak areas was 2:3.0. In the R-CE-SDS pattern, the ratio of the peak areas of the light chain and the long heavy chain of 609-Fab-Ipilimumab-IgG1 was consistent with expectations.

Figs. 46C and 46D show the patterns of NR-CE-SDS and R-CE-SDS of 609-Fab-5E7-IgG4, respectively. In the NR-CE-SDS pattern, the main peak Peak 11 accounted for 94.73%; in the R-CE-SDS pattern, the two main peaks Peak 7 (corresponding to the light chain) and Peak 16 (corresponding to the long heavy chain) accounted for 38.32% and 59.58%, respectively, and the ratio of the two peak areas was 2:3.1. In the R-CE-SDS pattern, the ratio of the peak areas of the light chain and the long heavy chain of 609-Fab-5E7-IgG4 was consistent with expectations.

## Claims

1. A tetravalent bispecific antibody, **characterized in that**, the tetravalent bispecific antibody comprises:
two polypeptide chains, wherein the polypeptide chain comprises VH-B-CH1-peptide linker-VH-A-CH1-CH2-CH3 from N-terminus to C-terminus, the VH-A is a heavy chain variable region of a first antibody, the VH-B is a heavy chain variable region of a second antibody, the CH1 is a first domain of a heavy chain constant region, the CH2 is a second domain of the heavy chain constant region, the CH3 is a third domain of the heavy chain constant region, the first antibody specifically binds to a first antigen, the second antibody specifically binds to a second antigen;
four common light chains, wherein the common light chain comprises VL-CL from N-terminus to C-terminus, the VL is a light chain variable region, the CL is a light chain constant region, and the VH-A-CH1 of the polypeptide chain and the VH-B-CH1 of the polypeptide chain are paired with the VL-CL of the common light chain, respectively, the VH-A and the VL form a first antigen binding site, and the VH-B and the VL form a second antigen binding site.

2. The tetravalent bispecific antibody of claim 1, **characterized in that**, the common light chain is selected and obtained by the following method:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and:
(a) selecting the light chain of the second antibody as the common light chain, if the hybrid antibody formed by the combination of the heavy chain of the first antibody and the light chain of the second antibody can specifically bind to the first antigen;
(b) selecting the light chain of the first antibody as the common light chain, if the hybrid antibody formed by the combination of the heavy chain of the second antibody and the light chain of the first antibody can specifically bind to the second antigen;
(c) back-mutating the light chain of the first antibody so that the hybrid antibody formed by the combination of the heavy chain of the second antibody and the mutant light chain of the first antibody can specifically bind to the second antigen, and then selecting the mutant light chain of the first antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen; or
(d) back-mutating the light chain of the second antibody so that the hybrid antibody formed by the combination of the heavy chain of the first antibody and the mutant light chain of the second antibody can specifically bind to the first antigen, and then selecting the mutant light chain of the second antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen.

3. The tetravalent bispecific antibody of claim 2, **characterized in that**, the common light chain is selected and obtained by the following method:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and
(a') back-mutating the light chain of the second antibody so that the binding affinity of the hybrid antibody formed by the combination of the heavy chain of the first antibody and the mutant light chain of the second antibody to the first antigen is better than the binding affinity of the hybrid antibody formed by the combination of the heavy chain of the first antibody and the light chain of the second antibody to the first antigen, and slecting the mutant light chain of the second antibody as the common light chain, if the hybrid antibody formed by the combination of the heavy chain of the first antibody and the light chain of the second antibody can specifically bind to the first antigen;
(b') back-mutating the light chain of the first antibody so that the binding affinity of the hybrid antibody formed by the combination of the heavy chain of the second antibody and the mutant light chain of the first antibody to the second antigen is better than the binding affinity of the hybrid antibody formed by the combination of the heavy chain of the second antibody and the light chain of the first antibody to the second antigen, and then selecting the mutant light chain of the first antibody as the common light chain, if the hybrid antibody formed by the combination of the heavy chain of the second antibody and the light chain of the first antibody can specifically bind to the second antigen.

4. The tetravalent bispecific antibody of claim 1, **characterized in that**, the peptide linker is an artificial linker selected from the group consisting of: G, GS, SG, GGS, GSG, SGG, GGG, GGGS, SGGG, GGGGS, GGGGSGS, GGGGSGGS, GGGGSGGGGS, GGGGSGGGGSGGGGS, AKTTPKLEEGEFSEAR, AKTTPKLEEGEFSEARV, AKTTPKLGG, SAKTTPKLGG, SAKTTP, RADAAP, RADAAPTVS, RADAAAAGGPGS, SAKTTPKLEEGEFSEARV, ADAAP, ADAAPTVSIFPP, TVAAP, TVAAPSVFIFPP, QPKAAP, QPKAAPSVTLFPP, AKTTPP, AKTTPPSVTPLAP, AKTTAPSVYPLAP, ASTKGP, ASTKGPSVFPLAP, GENKVEYAPALMALS, GPAKELTPLKEAKVS and GHEAAAVMQVQYPAS.

5. The tetravalent bispecific antibody of claim 4, **characterized in that**, the artificial linker is GGGGSGGGGSGGGGS.

6. The tetravalent bispecific antibody of claim 1, **characterized in that**, the CH1 domain which is near the N-terminus of the polypeptide chain and CH1-CH2-CH3 are derived from heavy chain constant regions of the same or different subtypes.

7. The tetravalent bispecific antibody of claim 6, **characterized in that**, the CH1 domain which is near the N-terminus of the polypeptide chain and CH1-CH2-CH3 are derived from the heavy chain constant region of the group consisting of: IgG1, IgG2, IgG3 and IgG4.

8. The tetravalent bispecific antibody of claim 7, **characterized in that**, the CH1 domain which is near the N-terminus of the polypeptide chain and CH1-CH2-CH3 are derived from the heavy chain constant region of IgG1 or IgG4.

9. The tetravalent bispecific antibody of claim 7, **characterized in that**, the IgG4 contains the S228P mutation.

10. The tetravalent bispecific antibody of claim 1, **characterized in that**, the CL is κ light chain constant region or λ light chain constant region.

11. The tetravalent bispecific antibody of claim 1, **characterized in that**, the first antigen and the second antigen are selected from the group consisting of: VEGF/PD-1, PD-1/VEGF, TGF-β/PD-1, PD-1/TGF-β, HER2/CD47, CD47/HER2, HER2/CD137, CD137/HER2, PD-1/CD137, CD137/PD-1, PD-1/CD40, CD40/PD-1, PD-1/EGFR, EGFR/PD-1, PD-1/HER2, HER2/PD-1, PD-1/CTLA-4, CTLA-4/PD-1, PD-1/LAG-3 and LAG-3/PD-1.

12. The tetravalent bispecific antibody of claim 1, **characterized in that**, the VH-A or the VH-B has the sequence as shown in SEQ ID NO: 83.

13. The tetravalent bispecific antibody of claim 11 or 12, **characterized in that**, the sequences of the VH-A, the VH-B and the VL are selected from the group consisting of: SEQ ID NOs: 1,11, 15; SEQ ID NOs: 11, 1, 15; SEQ ID NOs: 20, 11, 15; SEQ ID NOs: 11, 20, 15; SEQ ID NOs: 29, 41, 42; SEQ ID NOs: 41, 29, 42; SEQ ID NOs: 31, 41, 42; SEQ ID NOs: 41, 31, 42; SEQ ID NOs: 53, 61, 54; SEQ ID NOs: 61, 53, 54; SEQ ID NOs: 53, 77, 54; SEQ ID NOs: 77, 53, 54; SEQ ID NOs: 83, 91, 92; SEQ ID NOs: 91, 83, 92; SEQ ID NOs: 83, 105, 106; SEQ ID NOs: 105, 83, 106; SEQ ID NOs: 83, 113, 114; SEQ ID NOs: 113,83, 114; SEQ ID NOs: 83, 117, 118; SEQ ID NOs: 117,83, 118; SEQ ID NOs: 83, 119, 120; SEQ ID NOs: 119,83, 120; SEQ ID NOs: 83, 121, 122 and SEQ ID NOs: 121, 83, 122.

14. The tetravalent bispecific antibody of claim 13, **characterized in that**, the sequences of the polypeptide chain and the common light chain are selected from the group consisting of: SEQ ID NOs: 16, 13; SEQ ID NOs: 18, 13; SEQ ID NOs: 21, 13; SEQ ID NOs: 45, 43; SEQ ID NOs: 47, 43; SEQ ID NOs: 49, 43; SEQ ID NOs: 51,43; SEQ ID NOs: 65, 63; SEQ ID NOs: 67,63; SEQ ID NOs: 79,63; SEQ ID NOs: 81,63; SEQ ID NOs: 95, 93; SEQ ID NOs: 97,93; SEQ ID NOs: 109, 107; SEQ ID NOs: 111, 107; SEQ ID NOs: 131, 123; SEQ ID NOs: 133, 125; SEQ ID NOs: 135, 127; SEQ ID NOs: 137, 127; SEQ ID NOs: 139, 127; SEQ ID NOs: 141, 127; SEQ ID NOs: 143, 129 and SEQ ID NOs: 145, 129.

15. An isolated nucleotide, **characterized in that**, the nucleotide encodes the tetravalent bispecific antibody of any one of claims 1-14.

16. The nucleotide of claim 15, **characterized in that**, the nucleotide encodes the polypeptide chain and the common light chain, and has the sequence selected from the group consisting of: SEQ ID NOs: 17, 14; SEQ ID NOs: 19, 14; SEQ ID NOs: 22, 14; SEQ ID NOs: 46, 44; SEQ ID NOs: 48, 44; SEQ ID NOs: 50, 44; SEQ ID NOs: 52, 44; SEQ ID NOs: 66, 64; SEQ ID NOs: 68, 64; SEQ ID NOs: 80, 64; SEQ ID NOs: 82, 64; SEQ ID NOs: 96, 94; SEQ ID NOs: 98, 94; SEQ ID NOs: 110, 108; SEQ ID NOs: 112, 108; SEQ ID NOs: 132, 124; SEQ ID NOs: 134, 126; SEQ ID NOs: 136, 128; SEQ ID NOs: 138, 128; SEQ ID NOs: 140, 128; SEQ ID NOs: 142, 128; SEQ ID NOs: 144, 130 and SEQ ID NOs: 146, 130.

17. An expression vector, **characterized in that**, the expression vector comprises the nucleotide of claim 15 or 16.

18. A host cell, **characterized in that**, the host cell comprises the expression vector of claim 17.

19. A method for preparing the tetravalent bispecific antibody of any one of claims 1-14, **characterized in that**, the method comprises the following steps of:
a) culturing the host cell of claim 18 under expression conditions, to express the tetravalent bispecific antibody;
b) isolating and purifying the tetravalent bispecific antibody of step a).

20. A pharmaceutical composition, **characterized in that**, the pharmaceutical composition comprises the tetravalent bispecific antibody of any one of claims 1-14 and a pharmaceutically acceptable carrier.

21. Use of the tetravalent bispecific antibody of any one of claims 1-14 or the pharmaceutical composition of claim 20 in the preparation of a medicine for the treatment of cancer and inflammatory diseases, autoimmune diseases and other disorders.

22. The use of claim 21, **characterized in that**, the cancer is selected from the group consisting of melanoma, kidney cancer, prostate cancer, pancreatic cancer, breast cancer, colon cancer, lung cancer, esophageal cancer, head and neck squamous cell carcinoma, liver cancer, ovarian cancer, cervical cancer, thyroid cancer, glioblastoma, glioma, leukemia, lymphoma and other neoplastic malignant diseases; the inflammatory diseases, autoimmune diseases and other disorders are selected from the group consisting of ophthalmological disorders, fibrosis, asthma, rheumatoid arthritis, systemic lupus erythematosus, multiple sclerosis, psoriasis and atopic dermatitis.

23. A method for treating cancer and inflammatory diseases, autoimmune diseases and other disorders, **characterized in that**, it comprises administering the tetravalent bispecific antibody of any one of claims 1-14 or the pharmaceutical composition of claim 20 to a subject in need.

24. The method of claim 23, **characterized in that**, the cancer is selected from the group consisting of melanoma, kidney cancer, prostate cancer, pancreatic cancer, breast cancer, colon cancer, lung cancer, esophageal cancer, head and neck squamous cell carcinoma, liver cancer, ovarian cancer, cervical cancer, thyroid cancer, glioblastoma, glioma, leukemia, lymphoma and other neoplastic malignant diseases; the inflammatory diseases, autoimmune diseases and other disorders are selected from the group consisting of ophthalmological disorders, fibrosis, asthma, rheumatoid arthritis, systemic lupus erythematosus, multiple sclerosis, psoriasis and atopic dermatitis .

25. A method for constructing a tetravalent bispecific antibody, **characterized in that**, the method comprises the following steps of:
(a) connecting a heavy chain variable region VH-B of a second antibody with a first domain CH1 of a heavy chain constant region, and the second antibody specifically binding to a second antigen;
(b) connecting (a) with a heavy chain variable region VH-A of a first antibody through a peptide linker, and the first antibody specifically binding to a first antigen;
(c) connecting (b) with a heavy chain constant region CH1-CH2-CH3, to form a polypeptide chain;
(d) respectively constructing (c) and a common light chain VL-CL into an expression vector, for combination expression, so as to obtain the tetravalent bispecific antibody of interest;
wherein, the CH1 is the first domain of the heavy chain constant region, the CH2 is a second domain of the heavy chain constant region, and the CH3 is a third domain of the heavy chain constant region, the VL is a light chain variable region, and the CL is a the light chain constant region, the VH-A-CH1 and the VH-B-CH1 are paired with the VL-CL, respectively, the VH-A and the VL form a first antigen binding site, the VH-B and the VL form a second antigen binding site.

26. The method of claim 25, **characterized in that**, the common light chain is selected and obtained by the following method of:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and
(a) selecting the light chain of the second antibody as the common light chain, if the hybrid antibody formed by the combination of the heavy chain of the first antibody and the light chain of the second antibody can specifically bind to the first antigen;
(b) selecting the light chain of the first antibody as the common light chain, if the hybrid antibody formed by the combination of the heavy chain of the second antibody and the light chain of the first antibody can specifically bind to the second antigen;
(c) back-mutating the light chain of the first antibody so that the hybrid antibody formed by the combination of the heavy chain of the second antibody and the mutant light chain of the first antibody can specifically bind to the second antigen, and then selecting the mutant light chain of the first antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen; or
(d) back-mutating the light chain of the second antibody so that the hybrid antibody formed by the combination of the heavy chain of the first antibody and the mutant light chain of the second antibody can specifically bind to the first antigen, and then selecting the mutant light chain of the second antibody as the common light chain, if the hybrid antibody formed by the combination of the heavy chain of the first antibody and the light chain of the second antibody cannot specifically bind to the first antigen, and the hybrid antibody formed by the combination of the heavy chain of the second antibody and the light chain of the first antibody cannot specifically bind to the second antigen.

27. The method of claim 26, **characterized in that**, the common light chain is selected and obtained by the following method of:
exchanging the heavy chain and light chain of the first antibody and the second antibody, respectively, to obtain a hybrid antibody formed by the combination of the heavy chain of the first antibody and the light chain of the second antibody, and a hybrid antibody formed by the combination of the heavy chain of the second antibody and the light chain of the first antibody, and
(a') back-mutating the light chain of the second antibody so that the binding affinity of the hybrid antibody formed by the combination of the heavy chain of the first antibody and the mutant light chain of the second antibody to the first antigen is better than the binding affinity of the hybrid antibody formed by the combination of the heavy chain of the first antibody and the light chain of the second antibody to the first antigen, and then selecting the mutant light chain of the second antibody as the common light chain, if the hybrid antibody formed by the combination of the heavy chain of the first antibody and the light chain of the second antibody can specifically bind to the first antigen;
(b') back-mutating the light chain of the first antibody so that the binding affinity of the hybrid antibody formed by the combination of the heavy chain of the second antibody and the mutant light chain of the first antibody to the second antigen is better than the binding affinity of the hybrid antibody formed by the combination of the heavy chain of the second antibody and the light chain of the first antibody to the second antigen, then selecting the mutant light chain of the first antibody as the common light chain, if the hybrid antibody formed by the combination of the heavy chain of the second antibody and the light chain of the first antibody can specifically bind to the second antigen.

28. The method of claim 25, **characterized in that**, the peptide linker is an artificial linker selected from the group consisting of: G, GS, SG, GGS, GSG, SGG, GGG, GGGS, SGGG, GGGGS, GGGGSGS, GGGGSGGS, GGGGSGGGGS, GGGGSGGGGSGGGGS, AKTTPKLEEGEFSEAR, AKTTPKLEEGEFSEARV, AKTTPKLGG, SAKTTPKLGG, SAKTTP, RADAAP, RADAAPTVS, RADAAAAGGPGS, SAKTTPKLEEGEFSEARV, ADAAP, ADAAPTVSIFPP, TVAAP, TVAAPSVFIFPP, QPKAAP, QPKAAPSVTLFPP, AKTTPP, AKTTPPSVTPLAP, AKTTAPSVYPLAP, ASTKGP, ASTKGPSVFPLAP, GENKVEYAPALMALS, GPAKELTPLKEAKVS and GHEAAAVMQVQYPAS.

29. The method of claim 28, **characterized in that**, the artificial linker is GGGGSGGGGSGGGGS.

30. The method of claim 25, **characterized in that**, the CH1 domain which is near the N-terminus of the polypeptide chain and CH1-CH2-CH3 are derived from heavy chain constant regions of same or different subtypes.

31. The method of claim 30, **characterized in that**, the CH1 domain which is near the N-terminus of the polypeptide chain and CH1-CH2-CH3 are derived from the heavy chain constant region of the group consisting of: IgG1, IgG2, IgG3 and IgG4.

32. The method of claim 31, **characterized in that**, the CH1-CH2-CH3 of the polypeptide chain is derived from the heavy chain constant region of IgG1 or IgG4.

33. The method of claim 31 or 32, **characterized in that**, the IgG4 comprises the S228P mutation.

34. The method of claim 25, **characterized in that**, the CL is κ light chain constant region or λ light chain constant region.

35. The method of claim 25, **characterized in that**, the first antigen and the second antigen are selected from the group consisting of: VEGF/PD-1, PD-1/VEGF, TGF-β/PD-1, PD-1/TGF-β, HER2/CD47, CD47/HER2, HER2/CD137, CD137/HER2, PD-1/CD137, CD137/PD-1, PD-1/CD40, CD40/PD-1, PD-1/EGFR, EGFR/PD-1, PD-1/HER2, HER2/PD-1, PD-1/CTLA-4, CTLA-4/PD-1, PD-1/LAG-3 and LAG-3/PD-1.

36. The method of claim 25, **characterized in that**, the VH-A or the VH-B has the sequence as shown in SEQ ID NO: 83.

37. The method of claim 35 or 36, **characterized in that**, the sequences of the VH-A, the VH-B and the VL are selected from the group consisting of: SEQ ID NOs: 1, 11, 15; SEQ ID NOs: 11, 1, 15; SEQ ID NOs: 20, 11,15; SEQ ID NOs: 11,20, 15; SEQ ID NOs: 29, 41, 42; SEQ ID NOs: 41, 29,42; SEQ ID NOs: 31,41,42; SEQ ID NOs: 41, 31, 42; SEQ ID NOs: 53, 61, 54; SEQ ID NOs: 61, 53, 54; SEQ ID NOs: 53, 77, 54; SEQ ID NOs: 77, 53, 54; SEQ ID NOs: 83, 91, 92; SEQ ID NOs: 91, 83, 92; SEQ ID NOs: 83, 105, 106; SEQ ID NOs: 105, 83, 106; SEQ ID NOs: 83, 113, 114; SEQ ID NOs: 113, 83, 114; SEQ ID NOs: 83, 117, 118; SEQ ID NOs: 117, 83, 118; SEQ ID NOs: 83, 119, 120; SEQ ID NOs: 119, 83, 120; SEQ ID NOs: 83, 121, 122 and SEQ ID NOs: 121, 83, 122.

38. The method of claim 37, **characterized in that**, the sequences of the polypeptide chain and the common light chain are selected from the group consisting of: SEQ ID NOs: 16, 13; SEQ ID NOs: 18, 13; SEQ ID NOs: 21, 13; SEQ ID NOs: 45, 43; SEQ ID NOs: 47, 43; SEQ ID NOs: 49, 43; SEQ ID NOs: 51,43; SEQ ID NOs: 65, 63; SEQ ID NOs: 67,63; SEQ ID NOs: 79,63; SEQ ID NOs: 81,63; SEQ ID NOs: 95, 93; SEQ ID NOs: 97, 93; SEQ ID NOs: 109, 107; SEQ ID NOs: 111, 107; SEQ ID NOs: 131, 123; SEQ ID NOs: 133, 125; SEQ ID NOs: 135, 127; SEQ ID NOs: 137, 127; SEQ ID NOs: 139, 127; SEQ ID NOs: 141, 127; SEQ ID NOs: 143, 129 and SEQ ID NOs: 145, 129.

39. A bispecific antibody, **characterized in that**, it comprises at least two different heavy chain variable regions and at least two common light chains, the common light chains comprise a same light chain variable region, and the heavy chain variable regions and the light chain variable regions form antigen binding sites, wherein the heavy chain variable regions comprise the amino acid sequence as shown in SEQ ID NO:83.

40. Use of a heavy chain variable region comprising an amino acid sequence as shown in SEQ ID NO: 83 in the construction of a bispecific antibody, **characterized in that**, the bispecific antibody comprises at least two different heavy chain variable regions and at least two common light chains, the common light chains comprise a same light chain variable region, and the heavy chain variable regions and the light chain variable regions form antigen binding sites.


SEQUENCE LISTING

<110>  SUNSHINE GUOJIAN PHARMACEUTICAL (SHANGHAI) CO., LTD.

<120>  TETRAVALENT BISPECIFIC ANTIBODY, PREPARATION METHOD THEREFOR, AND USE
THEREOF

<130>  SH363-21P450327

<160>  153

<170>  PatentIn version 3.5

<210>  1
<211>  123
<212>  PRT
<213>  Homo

<400>  1

<210>  2
<211>  107
<212>  PRT
<213>  Homo

<400>  2

<210>  3
<211>  118
<212>  PRT
<213>  Mus musculus

<400>  3

Glu Val Lys Leu Val Glu Ser Gly Gly Gly Leu Val Glu Pro Gly Gly
1               5                   10                  15

Ser Leu Lys Leu Ser Cys Ala Ala Ser Gly Phe Val Phe Ser Asn Tyr
            20                  25                  30

Asp Met Ser Trp Val Arg Gln Thr Pro Glu Lys Arg Leu Glu Trp Val
        35                  40                  45

Ala Thr Ile Ser Gly Gly Gly Gly Tyr Thr Tyr Tyr Ser Asp Ser Val
    50                  55                  60

Lys Gly Arg Phe Thr Ile Ser Arg Asp Asn Ala Arg Asn Thr Leu Tyr
65                  70                  75                  80

Leu Gln Met Thr Ser Leu Arg Ser Glu Asp Thr Ala Leu Phe Tyr Cys
                85                  90                  95

Ala Ser Pro Tyr Gly His Tyr Gly Phe Glu Tyr Trp Gly Gln Gly Thr
            100                 105                 110

Thr Leu Thr Val Ser Ser
        115

<210>  4
<211>  107
<212>  PRT
<213>  Mus musculus

<400>  4

Asp Ile Gln Met Thr Gln Thr Thr Ser Ser Leu Ser Ala Ser Leu Gly
1               5                   10                  15

Asp Arg Val Thr Ile Ser Cys Ser Ala Ser Gln Gly Ile Ser Asn Phe
            20                  25                  30

Leu Ser Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Asn Leu Pro Trp
                85                  90                  95

Thr Phe Gly Gly Gly Thr Lys Leu Glu Ile Arg
            100                 105

<210>  5
<211>  5
<212>  PRT
<213>  Mus musculus

<400>  5

Asn Tyr Asp Met Ser
1               5

<210>  6
<211>  17
<212>  PRT
<213>  Mus musculus

<400>  6

Thr Ile Ser Gly Gly Gly Gly Tyr Thr Tyr Tyr Ser Asp Ser Val Lys
1               5                   10                  15

Gly

<210>  7
<211>  9

<212>  PRT
<213>  Mus musculus

<400>  7

Pro Tyr Gly His Tyr Gly Phe Glu Tyr
1               5


<210>  8
<211>  11
<212>  PRT
<213>  Mus musculus

<400>  8

Ser Ala Ser Gln Gly Ile Ser Asn Phe Leu Ser
1               5                   10


<210>  9
<211>  7
<212>  PRT
<213>  Mus musculus

<400>  9

Tyr Thr Ser Ser Leu His Ser
1               5


<210>  10
<211>  9
<212>  PRT
<213>  Mus musculus

<400>  10

Gln Gln Tyr Ser Asn Leu Pro Trp Thr
1               5


<210>  11
<211>  118
<212>  PRT
<213>  Homo

<400>  11

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Val Phe Ser Asn Tyr
            20                  25                  30


Asp Met Ser Trp Val Arg Gln Ala Pro Gly Lys Arg Leu Glu Trp Val
        35                  40                  45


Ala Thr Ile Ser Gly Gly Gly Gly Tyr Thr Tyr Tyr Ser Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Pro Tyr Gly His Tyr Gly Phe Glu Tyr Trp Gly Gln Gly Thr
            100                 105                 110

Leu Val Thr Val Ser Ser
        115

<210>  12
<211>  107
<212>  PRT
<213>  Homo

<400>  12

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Gly Ile Ser Asn Phe
            20                  25                  30

Leu Ser Trp Tyr Gln Gln Lys Pro Gly Lys Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Tyr Cys Gln Gln Tyr Ser Asn Leu Pro Trp
                85                  90                  95

Thr Phe Gly Gln Gly Thr Lys Val Glu Ile Lys
            100                 105

<210>  13
<211>  214
<212>  PRT
<213>  Homo

<400>  13

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 14
<211> 642
<212> DNA
<213> Homo

<400> 14
gacatccaga tgacccagtc ccccagcagc ctgagcgcca gcgtgggcga cagggtgacc 60

atcacctgct ccgcctccca ggacatctcc aactacctga actggtacca gcagaagccc 120

ggcaaggccc ccaaggtgct gatctacttc acctcctccc tgcactccgg cgtgcccagc 180

aggttctccg gcagcggctc cggcaccgac ttcaccctga ccatctccag cctgcagccc 240

gaggacttcg ccacctacta ctgccagcag tactccaccc tgccctggac cttcggccag 300

ggcaccaagg tggagatcaa gaggaccgtg gccgccccct ccgtgttcat cttccccccc 360

tccgacgagc agctgaagag cggcaccgcc agcgtggtgt gcctgctgaa caacttctac 420

cccagggagg ccaaggtgca gtggaaggtg gacaacgccc tgcagagcgg caactcccag 480

gagagcgtga ccgagcagga ctccaaggac tccacctaca gcctgagctc caccctgacc 540

ctgtccaagg ccgactacga gaagcacaag gtgtacgcct gcgaggtgac ccaccagggc 600

ctgtcctccc ccgtgaccaa gtccttcaac aggggcgagt gc 642

<210> 15
<211> 107
<212> PRT
<213> Homo

<400> 15

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Leu Pro Trp
                85                  90                  95

Thr Phe Gly Gln Gly Thr Lys Val Glu Ile Lys
            100                 105

<210> 16
<211> 681
<212> PRT
<213> Homo

<400> 16

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Val Phe Ser Asn Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Gly Tyr Thr Tyr Tyr Ser Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Pro Tyr Gly His Tyr Gly Phe Glu Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gly Gly Gly Gly Ser Gly Gly Gly
    210                 215                 220

Gly Ser Gly Gly Gly Gly Ser Glu Val Gln Leu Val Glu Ser Gly Gly
225                 230                 235                 240

Gly Leu Val Gln Pro Gly Gly Ser Leu Arg Leu Ser Cys Ala Ala Ser
                245                 250                 255

Gly Tyr Thr Phe Thr Asn Tyr Gly Met Asn Trp Val Arg Gln Ala Pro

260                 265                 270

Gly Lys Gly Leu Glu Trp Val Gly Trp Ile Asn Thr Tyr Thr Gly Glu
        275                 280                 285

Pro Thr Tyr Ala Ala Asp Phe Lys Arg Arg Phe Thr Phe Ser Leu Asp
    290                 295                 300

Thr Ser Lys Ser Thr Ala Tyr Leu Gln Met Asn Ser Leu Arg Ala Glu
305                 310                 315                 320

Asp Thr Ala Val Tyr Tyr Cys Ala Lys Tyr Pro His Tyr Tyr Gly Ser
                325                 330                 335

Ser His Trp Tyr Phe Asp Val Trp Gly Gln Gly Thr Leu Val Thr Val
            340                 345                 350

Ser Ser Ala Ser Thr Lys Gly Pro Ser Val Phe Pro Leu Ala Pro Cys
        355                 360                 365

Ser Arg Ser Thr Ser Glu Ser Thr Ala Ala Leu Gly Cys Leu Val Lys
    370                 375                 380

Asp Tyr Phe Pro Glu Pro Val Thr Val Ser Trp Asn Ser Gly Ala Leu
385                 390                 395                 400

Thr Ser Gly Val His Thr Phe Pro Ala Val Leu Gln Ser Ser Gly Leu
                405                 410                 415

Tyr Ser Leu Ser Ser Val Val Thr Val Pro Ser Ser Ser Leu Gly Thr
            420                 425                 430

Lys Thr Tyr Thr Cys Asn Val Asp His Lys Pro Ser Asn Thr Lys Val
        435                 440                 445

Asp Lys Arg Val Glu Ser Lys Tyr Gly Pro Pro Cys Pro Pro Cys Pro
    450                 455                 460

Ala Pro Glu Phe Leu Gly Gly Pro Ser Val Phe Leu Phe Pro Pro Lys
465                 470                 475                 480

Pro Lys Asp Thr Leu Met Ile Ser Arg Thr Pro Glu Val Thr Cys Val
                485                 490                 495

Val Val Asp Val Ser Gln Glu Asp Pro Glu Val Gln Phe Asn Trp Tyr
            500                 505                 510

Val Asp Gly Val Glu Val His Asn Ala Lys Thr Lys Pro Arg Glu Glu
        515                 520                 525

Gln Phe Asn Ser Thr Tyr Arg Val Val Ser Val Leu Thr Val Leu His
    530                 535                 540

Gln Asp Trp Leu Asn Gly Lys Glu Tyr Lys Cys Lys Val Ser Asn Lys
545                 550                 555                 560

Gly Leu Pro Ser Ser Ile Glu Lys Thr Ile Ser Lys Ala Lys Gly Gln
                565                 570                 575

Pro Arg Glu Pro Gln Val Tyr Thr Leu Pro Pro Ser Gln Glu Glu Met
            580                 585                 590

Thr Lys Asn Gln Val Ser Leu Thr Cys Leu Val Lys Gly Phe Tyr Pro
        595                 600                 605

Ser Asp Ile Ala Val Glu Trp Glu Ser Asn Gly Gln Pro Glu Asn Asn
    610                 615                 620

Tyr Lys Thr Thr Pro Pro Val Leu Asp Ser Asp Gly Ser Phe Phe Leu
625                 630                 635                 640

Tyr Ser Arg Leu Thr Val Asp Lys Ser Arg Trp Gln Glu Gly Asn Val
                645                 650                 655

Phe Ser Cys Ser Val Met His Glu Ala Leu His Asn His Tyr Thr Gln
            660                 665                 670

Lys Ser Leu Ser Leu Ser Leu Gly Lys
        675                 680

<210>  17
<211>  2043
<212>  DNA
<213>  Homo

<400>  17
gaggtccagc tggtggagag cggaggagga ctggtgcagc ccggcggctc tctgaggctg      60

agctgtgccg ctagcggctt cgtcttctcc aactacgaca tgagctgggt gaggcaagcc     120

cccggcaaga ggctggagtg ggtggctaca atcagcggcg gcggaggcta cacatactac     180

tccgactccg tgaagggaag gttcactatc tctagggaca acgccaagaa ctctctgtat     240

ctgcagatga actctctgag agccgaggat acagctgtgt actactgtgc ctccccatac     300

ggccactacg gcttcgagta ctggggccaa ggcactctgg tcactgtgag cagcgcaagt     360

accaagggac ctagtgtttt ccctcttgca ccttgctcca ggtcaacatc agagtccaca     420

gctgctcttg gatgtctcgt taaggactac ttcccagagc cagttaccgt atcctggaac 480

tccggagctt tgacaagcgg cgttcataca ttcccagctg tgttgcagag ttctgggttg 540

tacagtttga gctcagtggt gaccgtgcct tcatcttctt tgggcactaa gacctacacc 600

tgcaacgtgg atcacaagcc aagcaacacc aaggtggata agagggtggg tggaggcggt 660

tcaggcggag gtggcagcgg aggtggcggg agtgaggtgc agctggtgga gtctggcggc 720

ggactggtgc agcccggcgg cagcctgcgc ctgtcctgcg ctgcctccgg ctacaccttc 780

accaactacg gcatgaactg ggtgaggcag gcccccggaa agggcctgga gtgggtgggc 840

tggatcaaca cctacaccgg cgagcccacc tacgccgctg actttaagcg caggtttacc 900

ttctccctgg acacctccaa gtccaccgcc tacctgcaga tgaacagcct gagggccgag 960

gacaccgccg tgtactactg cgccaagtac ccccactact atggctccag ccactggtac 1020

tttgacgtgt ggggccaggg caccctggtc acagtgtcta gtgcctccac caagggccca 1080

tcggtcttcc ccctggcacc ctgctccagg agcacctctg agtccacagc ggccctgggc 1140

tgcctggtca aggactactt ccccgaaccg gtgacggtgt cgtggaactc aggcgccctg 1200

accagcggcg tgcacacctt cccggctgtc ctacagtcct caggactcta ctccctcagc 1260

agcgtggtga ccgtgccctc cagcagcttg ggcaccaaga catatacctg taatgtggat 1320

cacaagcctt ccaatacaaa agtggacaag agagttgagt ccaagtacgg cccaccatgt 1380

cctccatgtc cagcccctga atttttgggc gggccttctg tctttctgtt tcctcctaaa 1440

cctaaagata ccctgatgat cagccgcaca cccgaagtca cttgtgtggt cgtggatgtg 1500

tctcaggaag atcccgaagt gcagtttaac tggtatgtcg atggcgtgga agtgcataat 1560

gccaaaacta agccccgcga agaacagttc aacagcactt atcgggtcgt gtctgtgctc 1620

acagtcctcc atcaggattg gctgaatggg aaagaatata agtgcaaggt gagcaataag 1680

ggcctcccca gcagcatcga gaagactatt agcaaagcca aagggcagcc acgggaaccc 1740

caggtgtaca ctctgccccc ctctcaggag gagatgacta aaaatcaggt ctctctgact 1800

tgtctggtga aagggtttta tcccagcgac attgccgtgg agtgggagtc taatggccag 1860

cccgagaata attataagac aactcccccc gtcctggact ctgacggcag cttttttctg 1920

tattctcggc tgacagtgga caaaagtcgc tggcaggagg gcaatgtctt tagttgcagt 1980

gtcatgcatg aggccctgca caatcactat acacagaaaa gcctgtctct gagtctgggc 2040

aaa 2043

<210> 18
<211> 681
<212> PRT
<213> Homo

<400>  18

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Lys Thr Tyr Thr Cys Asn Val
        195                 200                 205

Asp His Lys Pro Ser Asn Thr Lys Val Asp Lys Arg Val Gly Gly Gly
    210                 215                 220

Gly Ser Gly Gly Gly Gly Ser Gly Gly Gly Gly Ser Glu Val Gln Leu
225                 230                 235                 240

Val Glu Ser Gly Gly Gly Leu Val Gln Pro Gly Gly Ser Leu Arg Leu

                245                 250                 255

Ser Cys Ala Ala Ser Gly Phe Val Phe Ser Asn Tyr Asp Met Ser Trp
            260                 265                 270

Val Arg Gln Ala Pro Gly Lys Arg Leu Glu Trp Val Ala Thr Ile Ser
        275                 280                 285

Gly Gly Gly Gly Tyr Thr Tyr Tyr Ser Asp Ser Val Lys Gly Arg Phe
    290                 295                 300

Thr Ile Ser Arg Asp Asn Ala Lys Asn Ser Leu Tyr Leu Gln Met Asn
305                 310                 315                 320

Ser Leu Arg Ala Glu Asp Thr Ala Val Tyr Tyr Cys Ala Ser Pro Tyr
                325                 330                 335

Gly His Tyr Gly Phe Glu Tyr Trp Gly Gln Gly Thr Leu Val Thr Val
            340                 345                 350

Ser Ser Ala Ser Thr Lys Gly Pro Ser Val Phe Pro Leu Ala Pro Cys
        355                 360                 365

Ser Arg Ser Thr Ser Glu Ser Thr Ala Ala Leu Gly Cys Leu Val Lys
    370                 375                 380

Asp Tyr Phe Pro Glu Pro Val Thr Val Ser Trp Asn Ser Gly Ala Leu
385                 390                 395                 400

Thr Ser Gly Val His Thr Phe Pro Ala Val Leu Gln Ser Ser Gly Leu
                405                 410                 415

Tyr Ser Leu Ser Ser Val Val Thr Val Pro Ser Ser Ser Leu Gly Thr
            420                 425                 430

Lys Thr Tyr Thr Cys Asn Val Asp His Lys Pro Ser Asn Thr Lys Val
        435                 440                 445

Asp Lys Arg Val Glu Ser Lys Tyr Gly Pro Pro Cys Pro Pro Cys Pro
    450                 455                 460

Ala Pro Glu Phe Leu Gly Gly Pro Ser Val Phe Leu Phe Pro Pro Lys
465                 470                 475                 480

Pro Lys Asp Thr Leu Met Ile Ser Arg Thr Pro Glu Val Thr Cys Val
                485                 490                 495

Val Val Asp Val Ser Gln Glu Asp Pro Glu Val Gln Phe Asn Trp Tyr
                500                 505                 510

Val Asp Gly Val Glu Val His Asn Ala Lys Thr Lys Pro Arg Glu Glu
            515                 520                 525

Gln Phe Asn Ser Thr Tyr Arg Val Val Ser Val Leu Thr Val Leu His
    530                 535                 540

Gln Asp Trp Leu Asn Gly Lys Glu Tyr Lys Cys Lys Val Ser Asn Lys
545                 550                 555                 560

Gly Leu Pro Ser Ser Ile Glu Lys Thr Ile Ser Lys Ala Lys Gly Gln
                565                 570                 575

Pro Arg Glu Pro Gln Val Tyr Thr Leu Pro Pro Ser Gln Glu Glu Met
                580                 585                 590

Thr Lys Asn Gln Val Ser Leu Thr Cys Leu Val Lys Gly Phe Tyr Pro
            595                 600                 605

Ser Asp Ile Ala Val Glu Trp Glu Ser Asn Gly Gln Pro Glu Asn Asn
    610                 615                 620

Tyr Lys Thr Thr Pro Pro Val Leu Asp Ser Asp Gly Ser Phe Phe Leu
625                 630                 635                 640

Tyr Ser Arg Leu Thr Val Asp Lys Ser Arg Trp Gln Glu Gly Asn Val
                645                 650                 655

Phe Ser Cys Ser Val Met His Glu Ala Leu His Asn His Tyr Thr Gln
                660                 665                 670

Lys Ser Leu Ser Leu Ser Leu Gly Lys
            675                 680

<210>  19
<211>  2043
<212>  DNA
<213>  Homo

<400>  19
gaggtgcagc tggtggagtc tggcggcgga ctggtgcagc ccggcggcag cctgcgcctg      60

tcctgcgctg cctccggcta caccttcacc aactacggca tgaactgggt gaggcaggcc     120

cccggaaagg gcctggagtg ggtgggctgg atcaacacct acaccggcga gcccacctac     180

gccgctgact ttaagcgcag gtttaccttc tccctggaca cctccaagtc caccgcctac     240

ctgcagatga acagcctgag ggccgaggac accgccgtgt actactgcgc caagtacccc     300

cactactatg gctccagcca ctggtacttt gacgtgtggg gccagggcac cctggtgact 360

gtgagcagcg caagtaccaa gggacctagt gttttccctc ttgcaccttg ctccaggtca 420

acatcagagt ccacagctgc tcttggatgt ctcgttaagg actacttccc agagccagtt 480

accgtatcct ggaactccgg agctttgaca agcggcgttc atacattccc agctgtgttg 540

cagagttctg ggttgtacag tttgagctca gtggtgaccg tgccttcatc ttctttgggc 600

actaagacct acacctgcaa cgtggatcac aagccaagca acaccaaggt ggataagagg 660

gtgggtggag gcggttcagg cggaggtggc agcggaggtg gcgggagtga ggtccagctg 720

gtggagagcg gaggaggact ggtgcagccc ggcggctctc tgaggctgag ctgtgccgct 780

agcggcttcg tcttctccaa ctacgacatg agctgggtga ggcaagcccc cggcaagagg 840

ctggagtggg tggctacaat cagcggcggc ggaggctaca catactactc cgactccgtg 900

aagggaaggt tcactatctc tagggacaac gccaagaact ctctgtatct gcagatgaac 960

tctctgagag ccgaggatac agctgtgtac tactgtgcct ccccatacgg ccactacggc 1020

ttcgagtact ggggccaagg cactctggtc acagtgtcta gtgcctccac caagggccca 1080

tcggtcttcc ccctggcacc ctgctccagg agcacctctg agtccacagc ggccctgggc 1140

tgcctggtca aggactactt ccccgaaccg gtgacggtgt cgtggaactc aggcgccctg 1200

accagcggcg tgcacacctt cccggctgtc ctacagtcct caggactcta ctccctcagc 1260

agcgtggtga ccgtgccctc cagcagcttg ggcaccaaga catatacctg taatgtggat 1320

cacaagcctt ccaatacaaa agtggacaag agagttgagt ccaagtacgg cccaccatgt 1380

cctccatgtc cagcccctga atttttgggc gggccttctg tctttctgtt tcctcctaaa 1440

cctaaagata ccctgatgat cagccgcaca cccgaagtca cttgtgtggt cgtggatgtg 1500

tctcaggaag atcccgaagt gcagtttaac tggtatgtcg atggcgtgga agtgcataat 1560

gccaaaacta agccccgcga agaacagttc aacagcactt atcgggtcgt gtctgtgctc 1620

acagtcctcc atcaggattg gctgaatggg aaagaatata agtgcaaggt gagcaataag 1680

ggcctcccca gcagcatcga gaagactatt agcaaagcca aagggcagcc acgggaaccc 1740

caggtgtaca ctctgccccc ctctcaggag gagatgacta aaaatcaggt ctctctgact 1800

tgtctggtga aagggtttta tcccagcgac attgccgtgg agtgggagtc taatggccag 1860

cccgagaata attataagac aactcccccc gtcctggact ctgacggcag ctttttcctg 1920

tattctcggc tgacagtgga caaaagtcgc tggcaggagg gcaatgtctt tagttgcagt 1980

gtcatgcatg aggccctgca caatcactat acacagaaaa gcctgtctct gagtctgggc 2040

aaa 2043

<210> 20

<211>  123
<212>  PRT
<213>  Homo

<400>  20


<210>  21
<211>  681
<212>  PRT
<213>  Homo

<400>  21

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Val Phe Ser Asn Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Gly Tyr Thr Tyr Tyr Ser Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Pro Tyr Gly His Tyr Gly Phe Glu Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gly Gly Gly Gly Ser Gly Gly Gly
    210                 215                 220

Gly Ser Gly Gly Gly Gly Ser Glu Val Gln Leu Val Glu Ser Gly Gly
225                 230                 235                 240

Gly Leu Val Gln Pro Gly Gly Ser Leu Arg Leu Ser Cys Ala Ala Ser
                245                 250                 255

Gly Tyr Asp Phe Thr His Tyr Gly Met Asn Trp Val Arg Gln Ala Pro
            260                 265                 270

Gly Lys Gly Leu Glu Trp Val Gly Trp Ile Asn Thr Tyr Thr Gly Glu
        275                 280                 285

Pro Thr Tyr Ala Ala Asp Phe Lys Arg Arg Phe Thr Phe Ser Leu Asp
    290                 295                 300

Thr Ser Lys Ser Thr Ala Tyr Leu Gln Met Asn Ser Leu Arg Ala Glu
305                 310                 315                 320

Asp Thr Ala Val Tyr Tyr Cys Ala Lys Tyr Pro Tyr Tyr Tyr Gly Thr

                325                 330                 335

Ser His Trp Tyr Phe Asp Val Trp Gly Gln Gly Thr Leu Val Thr Val
            340                 345                 350

Ser Ser Ala Ser Thr Lys Gly Pro Ser Val Phe Pro Leu Ala Pro Cys
        355                 360                 365

Ser Arg Ser Thr Ser Glu Ser Thr Ala Ala Leu Gly Cys Leu Val Lys
    370                 375                 380

Asp Tyr Phe Pro Glu Pro Val Thr Val Ser Trp Asn Ser Gly Ala Leu
385                 390                 395                 400

Thr Ser Gly Val His Thr Phe Pro Ala Val Leu Gln Ser Ser Gly Leu
                405                 410                 415

Tyr Ser Leu Ser Ser Val Val Thr Val Pro Ser Ser Ser Leu Gly Thr
            420                 425                 430

Lys Thr Tyr Thr Cys Asn Val Asp His Lys Pro Ser Asn Thr Lys Val
        435                 440                 445

Asp Lys Arg Val Glu Ser Lys Tyr Gly Pro Pro Cys Pro Pro Cys Pro
    450                 455                 460

Ala Pro Glu Phe Leu Gly Gly Pro Ser Val Phe Leu Phe Pro Pro Lys
465                 470                 475                 480

Pro Lys Asp Thr Leu Met Ile Ser Arg Thr Pro Glu Val Thr Cys Val
                485                 490                 495

Val Val Asp Val Ser Gln Glu Asp Pro Glu Val Gln Phe Asn Trp Tyr
            500                 505                 510

Val Asp Gly Val Glu Val His Asn Ala Lys Thr Lys Pro Arg Glu Glu
        515                 520                 525

Gln Phe Asn Ser Thr Tyr Arg Val Val Ser Val Leu Thr Val Leu His
    530                 535                 540

Gln Asp Trp Leu Asn Gly Lys Glu Tyr Lys Cys Lys Val Ser Asn Lys
545                 550                 555                 560

Gly Leu Pro Ser Ser Ile Glu Lys Thr Ile Ser Lys Ala Lys Gly Gln
                565                 570                 575

Pro Arg Glu Pro Gln Val Tyr Thr Leu Pro Pro Ser Gln Glu Glu Met
            580                 585                 590

Thr Lys Asn Gln Val Ser Leu Thr Cys Leu Val Lys Gly Phe Tyr Pro
        595                 600                 605

Ser Asp Ile Ala Val Glu Trp Glu Ser Asn Gly Gln Pro Glu Asn Asn
    610                 615                 620

Tyr Lys Thr Thr Pro Pro Val Leu Asp Ser Asp Gly Ser Phe Phe Leu
625                 630                 635                 640

Tyr Ser Arg Leu Thr Val Asp Lys Ser Arg Trp Gln Glu Gly Asn Val
                645                 650                 655

Phe Ser Cys Ser Val Met His Glu Ala Leu His Asn His Tyr Thr Gln
            660                 665                 670

Lys Ser Leu Ser Leu Ser Leu Gly Lys
        675                 680

<210>  22
<211>  2043
<212>  DNA
<213>  Homo

<400>  22
gaggtccagc tggtggagag cggaggagga ctggtgcagc ccggcggctc tctgaggctg      60

agctgtgccg ctagcggctt cgtcttctcc aactacgaca tgagctgggt gaggcaagcc     120

cccggcaaga ggctggagtg ggtggctaca atcagcggcg gcggaggcta cacatactac     180

tccgactccg tgaagggaag gttcactatc tctagggaca acgccaagaa ctctctgtat     240

ctgcagatga actctctgag agccgaggat acagctgtgt actactgtgc ctccccatac     300

ggccactacg gcttcgagta ctggggccaa ggcactctgg tcactgtgag cagcgcaagt     360

accaagggac ctagtgtttt ccctcttgca ccttgctcca ggtcaacatc agagtccaca     420

gctgctcttg gatgtctcgt taaggactac ttcccagagc cagttaccgt atcctggaac     480

tccggagctt tgacaagcgg cgttcataca ttcccagctg tgttgcagag ttctgggttg     540

tacagtttga gctcagtggt gaccgtgcct tcatcttctt tgggcactaa gacctacacc     600

tgcaacgtgg atcacaagcc aagcaacacc aaggtggata agagggtggg tggaggcggt     660

tcaggcggag gtggcagcgg aggtggcggg agtgaggtgc agctggtgga gtctggagga     720

ggcctggtgc agcctggcgg ctctctgaga ctgtcttgcg ctgctagtgg atatgatttt     780

acacattatg gcatgaactg ggtgagacag gctccaggca agggcctgga atgggtgggc     840

tggattaata cctatacagg cgaacctacc tacgccgctg attttaagag aagattcacc     900

ttttctctgg atacatctaa gagcacagct tacctgcaga tgaacagcct gcgggccgag 960

gacaccgccg tgtactactg cgccaagtac ccctactact acggcacctc ccactggtac 1020

ttcgacgtgt ggggccaggg caccctggtg accgtgtcct ccgcctccac caagggccca 1080

tcggtcttcc ccctggcacc ctgctccagg agcacctctg agtccacagc ggccctgggc 1140

tgcctggtca aggactactt ccccgaaccg gtgacggtgt cgtggaactc aggcgccctg 1200

accagcggcg tgcacacctt cccggctgtc ctacagtcct caggactcta ctccctcagc 1260

agcgtggtga ccgtgccctc cagcagcttg ggcaccaaga catatacctg taatgtggat 1320

cacaagcctt ccaatacaaa agtggacaag agagttgagt ccaagtacgg cccaccatgt 1380

cctccatgtc cagcccctga atttttgggc gggccttctg tctttctgtt tcctcctaaa 1440

cctaaagata ccctgatgat cagccgcaca cccgaagtca cttgtgtggt cgtggatgtg 1500

tctcaggaag atcccgaagt gcagtttaac tggtatgtcg atggcgtgga agtgcataat 1560

gccaaaacta agccccgcga agaacagttc aacagcactt atcgggtcgt gtctgtgctc 1620

acagtcctcc atcaggattg gctgaatggg aaagaatata agtgcaaggt gagcaataag 1680

ggcctcccca gcagcatcga gaagactatt agcaaagcca aagggcagcc acgggaaccc 1740

caggtgtaca ctctgccccc ctctcaggag gagatgacta aaaatcaggt ctctctgact 1800

tgtctggtga aagggtttta tcccagcgac attgccgtgg agtgggagtc taatggccag 1860

cccgagaata attataagac aactcccccc gtcctggact ctgacggcag ctttttcctg 1920

tattctcggc tgacagtgga caaaagtcgc tggcaggagg gcaatgtctt tagttgcagt 1980

gtcatgcatg aggccctgca caatcactat acacagaaaa gcctgtctct gagtctgggc 2040

aaa 2043

<210> 23
<211> 5
<212> PRT
<213> Mus musculus

<400> 23

Thr Tyr Trp Met Asn
1               5

<210> 24
<211> 17
<212> PRT
<213> Mus musculus

<400> 24

Gln Ile Phe Pro Ala Ser Gly Ser Thr Asn Tyr Asn Glu Met Phe Glu
1               5                   10                  15

Gly

<210> 25
<211> 12
<212> PRT
<213> Mus musculus

<400> 25

Gly Asp Gly Asn Tyr Ala Leu Asp Ala Met Asp Tyr
1               5                   10

<210> 26
<211> 15
<212> PRT
<213> Mus musculus

<400> 26

Arg Ala Ser Glu Ser Val Asp Ser Tyr Gly Asn Ser Phe Met His
1               5                   10                  15

<210> 27
<211> 7
<212> PRT
<213> Mus musculus

<400> 27

Leu Ala Ser Asn Leu Glu Ser
1               5

<210> 28
<211> 9
<212> PRT
<213> Mus musculus

<400> 28

Gln Gln Asn Asn Glu Asp Pro Leu Thr
1               5

<210> 29
<211> 121
<212> PRT
<213> Homo

<400> 29

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Phe Ile Thr Tyr
            20                  25                  30

Trp Met Asn Trp Val Arg Gln Ala Pro Gly Gln Gly Leu Glu Trp Ile
        35                  40                  45

Gly Gln Ile Phe Pro Ala Ser Gly Ser Thr Asn Tyr Asn Glu Met Phe
    50                  55                  60

Glu Gly Arg Ala Thr Leu Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Asp Gly Asn Tyr Ala Leu Asp Ala Met Asp Tyr Trp Gly
            100                 105                 110

Gln Gly Thr Leu Val Thr Val Ser Ser
        115                 120

<210>  30
<211>  111
<212>  PRT
<213>  Homo

<400>  30

Asp Ile Val Leu Thr Gln Ser Pro Ala Ser Leu Ala Val Ser Pro Gly
1               5                   10                  15

Gln Arg Ala Thr Ile Thr Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Asn
65                  70                  75                  80

Pro Val Glu Ala Asp Asp Thr Ala Asn Tyr Tyr Cys Gln Gln Asn Asn
                85                  90                  95

Glu Asp Pro Leu Thr Phe Gly Gly Gly Thr Lys Val Glu Leu Lys
            100                 105                 110

<210>  31
<211>  121
<212>  PRT

<213>  Homo

<400>  31

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Glu
            20                  25                  30

Trp Met Asn Trp Val Arg Gln Ala Pro Gly Gln Gly Leu Glu Trp Met
        35                  40                  45

Gly Gln Ile Phe Pro Ala Leu Gly Ser Thr Asn Tyr Asn Glu Met Tyr
    50                  55                  60

Glu Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Gly Ile Gly Asn Tyr Ala Leu Asp Ala Met Asp Tyr Trp Gly
            100                 105                 110

Gln Gly Thr Leu Val Thr Val Ser Ser
        115                 120

<210>  32
<211>  111
<212>  PRT
<213>  Homo

<400>  32

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Phe Tyr
            20                  25                  30

Gly Asn Ser Phe Met His Trp Tyr Gln Gln Lys Pro Gly Lys Ala Pro
        35                  40                  45

Lys Leu Leu Ile Tyr Leu Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Asn Ile
                85                  90                  95

Glu Asp Pro Leu Thr Phe Gly Gly Gly Thr Lys Val Glu Ile Lys
            100                 105                 110

<210>  33
<211>  116
<212>  PRT
<213>  Mus musculus

<400>  33

Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
            20                  25                  30

Thr Met Asn Trp Val Lys Gln Ser His Gly Lys Thr Leu Glu Trp Ile
        35                  40                  45

Gly Leu Ile Asn Pro Tyr Asn Gly Asp Thr Ser Tyr Asn Gln Lys Phe
    50                  55                  60

Lys Gly Lys Ala Thr Val Thr Val Asp Lys Ser Ser Ser Thr Ala Tyr
65                  70                  75                  80

Met Glu Leu Leu Asn Leu Thr Ser Glu Asp Ser Ala Val Tyr Tyr Cys
                85                  90                  95

Ala Arg Trp Arg Tyr Thr Met Asp Tyr Trp Gly Gln Gly Thr Ser Val
            100                 105                 110

Thr Val Ser Ser
        115

<210>  34
<211>  111
<212>  PRT
<213>  Mus musculus

<400>  34

Asn Ile Ala Leu Thr Gln Ser Pro Ala Ser Val Ala Val Ser Leu Gly
1               5                   10                  15

Gln Arg Ala Thr Ile Ser Cys Arg Ala Ser Glu Ser Val Asp Asn Tyr
            20                  25                  30

Gly Asn Ser Phe Met Asn Trp Tyr Gln Gln Lys Pro Gly Gln Pro Pro

35                  40                  45

Lys Pro Leu Ile Tyr Phe Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Ala Pro Pro Thr Phe Gly Gly Gly Thr Lys Leu Glu Ile Lys
            100                 105                 110

<210>  35
<211>  5
<212>  PRT
<213>  Mus musculus

<400>  35

Gly Tyr Thr Met Asn
1               5

<210>  36
<211>  17
<212>  PRT
<213>  Mus musculus

<400>  36

Leu Ile Asn Pro Tyr Asn Gly Asp Thr Ser Tyr Asn Gln Lys Phe Lys
1               5                   10                  15

Gly

<210>  37
<211>  7
<212>  PRT
<213>  Mus musculus

<400>  37

Trp Arg Tyr Thr Met Asp Tyr
1               5

<210>  38
<211>  15
<212>  PRT
<213>  Mus musculus

<400>  38

Arg Ala Ser Glu Ser Val Asp Asn Tyr Gly Asn Ser Phe Met Asn
1               5                   10                  15

<210>  39
<211>  7
<212>  PRT
<213>  Mus musculus

<400>  39

Phe Ala Ser Asn Leu Glu Ser
1               5

<210>  40
<211>  9
<212>  PRT
<213>  Mus musculus

<400>  40

Gln Gln Asn Asn Glu Ala Pro Pro Thr
1               5

<210>  41
<211>  116
<212>  PRT
<213>  Homo

<400>  41

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
            20                  25                  30

Thr Met Asn Trp Val Arg Gln Ala Pro Gly Gln Gly Leu Glu Trp Ile
        35                  40                  45

Gly Leu Ile Asn Pro Tyr Asn Gly Asp Thr Ser Tyr Asn Gln Lys Phe
    50                  55                  60

Lys Gly Arg Ala Thr Val Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Trp Arg Tyr Thr Met Asp Tyr Trp Gly Gln Gly Thr Leu Val
            100                 105                 110

Thr Val Ser Ser
        115

<210>  42
<211>  111
<212>  PRT
<213>  Homo

<400>  42

Asp Ile Val Leu Thr Gln Ser Pro Ala Ser Leu Ala Val Ser Pro Gly
1               5                   10                  15

Gln Arg Ala Thr Ile Thr Cys Arg Ala Ser Glu Ser Val Asp Asn Tyr
            20                  25                  30

Gly Asn Ser Phe Met Asn Trp Tyr Gln Gln Lys Pro Gly Gln Pro Pro
        35                  40                  45

Lys Pro Leu Ile Tyr Phe Ala Ser Asn Leu Glu Ser Gly Val Pro Ala
    50                  55                  60

Arg Phe Ser Gly Ser Gly Ser Arg Thr Asp Phe Thr Leu Thr Ile Asn
65                  70                  75                  80

Pro Val Glu Ala Asp Asp Thr Ala Asn Tyr Tyr Cys Gln Gln Asn Asn
                85                  90                  95

Glu Ala Pro Pro Thr Phe Gly Gln Gly Thr Lys Val Glu Ile Lys
            100                 105                 110


<210>  43
<211>  218
<212>  PRT
<213>  Homo

<400>  43

Asp Ile Val Leu Thr Gln Ser Pro Ala Ser Leu Ala Val Ser Pro Gly
1               5                   10                  15

Gln Arg Ala Thr Ile Thr Cys Arg Ala Ser Glu Ser Val Asp Asn Tyr
            20                  25                  30

Gly Asn Ser Phe Met Asn Trp Tyr Gln Gln Lys Pro Gly Gln Pro Pro
        35                  40                  45

Lys Pro Leu Ile Tyr Phe Ala Ser Asn Leu Glu Ser Gly Val Pro Ala
    50                  55                  60

Arg Phe Ser Gly Ser Gly Ser Arg Thr Asp Phe Thr Leu Thr Ile Asn
65                  70                  75                  80

Pro Val Glu Ala Asp Asp Thr Ala Asn Tyr Tyr Cys Gln Gln Asn Asn
                85                  90                  95

Glu Ala Pro Pro Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  44
<211>  654
<212>  DNA
<213>  Homo

<400>  44
gatatcgtgc tgactcagag cccagcctct ctggctgtga gccccggcca gagagccaca      60

atcacttgta gggccagcga gagcgtggac aactacggca actccttcat gaattggtac     120

cagcagaagc ccggccagcc tccaaagcct ctgatctact tcgcctccaa tctggaaagc     180

ggcgtgccag ctaggtttag cggctccggc tctaggacag acttcactct gactatcaac     240

ccagtggagg ccgatgacac agccaactac tactgccagc agaacaacga ggcccctcca     300

actttcggcc aaggcactaa ggtcgagatc aagagaaccg tcgccgctcc cagcgtcttc     360

atcttccccc ccagcgatga gcagctgaag agcggaaccg ccagcgtggt gtgcctgctg     420

aacaacttct accccaggga ggccaaggtg caatggaagg tggacaacgc cctacagagc     480

ggcaactccc aggagagcgt gaccgagcag gacagcaagg atagcaccta cagcctgagc     540

agcaccctca ccctgagcaa ggccgactac gagaagcaca aggtgtacgc ctgcgaggtg     600

acccatcagg gcctgagcag ccctgtgacc aagagcttca acaggggcga gtgc 654

<210> 45
<211> 677
<212> PRT
<213> Homo

<400> 45

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
            20                  25                  30

Thr Met Asn Trp Val Arg Gln Ala Pro Gly Gln Gly Leu Glu Trp Ile
        35                  40                  45

Gly Leu Ile Asn Pro Tyr Asn Gly Asp Thr Ser Tyr Asn Gln Lys Phe
    50                  55                  60

Lys Gly Arg Ala Thr Val Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Trp Arg Tyr Thr Met Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Gly Gly Gly Gly Ser Gly Gly Gly Gly Ser

210                 215                 220

Gly Gly Gly Gly Ser Gln Val Gln Leu Val Gln Ser Gly Ala Glu Val
225                 230                 235                 240

Lys Lys Pro Gly Ala Ser Val Lys Val Ser Cys Lys Ala Ser Gly Tyr
                245                 250                 255

Ile Phe Ile Thr Tyr Trp Met Asn Trp Val Arg Gln Ala Pro Gly Gln
            260                 265                 270

Gly Leu Glu Trp Ile Gly Gln Ile Phe Pro Ala Ser Gly Ser Thr Asn
        275                 280                 285

Tyr Asn Glu Met Phe Glu Gly Arg Ala Thr Leu Thr Val Asp Thr Ser
    290                 295                 300

Thr Ser Thr Ala Tyr Met Glu Leu Ser Ser Leu Arg Ser Glu Asp Thr
305                 310                 315                 320

Ala Val Tyr Tyr Cys Ala Arg Gly Asp Gly Asn Tyr Ala Leu Asp Ala
                325                 330                 335

Met Asp Tyr Trp Gly Gln Gly Thr Leu Val Thr Val Ser Ser Ala Ser
            340                 345                 350

Thr Lys Gly Pro Ser Val Phe Pro Leu Ala Pro Cys Ser Arg Ser Thr
        355                 360                 365

Ser Glu Ser Thr Ala Ala Leu Gly Cys Leu Val Lys Asp Tyr Phe Pro
    370                 375                 380

Glu Pro Val Thr Val Ser Trp Asn Ser Gly Ala Leu Thr Ser Gly Val
385                 390                 395                 400

His Thr Phe Pro Ala Val Leu Gln Ser Ser Gly Leu Tyr Ser Leu Ser
                405                 410                 415

Ser Val Val Thr Val Pro Ser Ser Ser Leu Gly Thr Lys Thr Tyr Thr
            420                 425                 430

Cys Asn Val Asp His Lys Pro Ser Asn Thr Lys Val Asp Lys Arg Val
        435                 440                 445

Glu Ser Lys Tyr Gly Pro Pro Cys Pro Pro Cys Pro Ala Pro Glu Phe
    450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser Gln Glu Asp Pro Glu Val Gln Phe Asn Trp Tyr Val Asp Gly Val
            500                 505                 510

Glu Val His Asn Ala Lys Thr Lys Pro Arg Glu Glu Gln Phe Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Gly Leu Pro Ser
545                 550                 555                 560

Ser Ile Glu Lys Thr Ile Ser Lys Ala Lys Gly Gln Pro Arg Glu Pro
                565                 570                 575

Gln Val Tyr Thr Leu Pro Pro Ser Gln Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Arg Leu
625                 630                 635                 640

Thr Val Asp Lys Ser Arg Trp Gln Glu Gly Asn Val Phe Ser Cys Ser
                645                 650                 655

Val Met His Glu Ala Leu His Asn His Tyr Thr Gln Lys Ser Leu Ser
            660                 665                 670

Leu Ser Leu Gly Lys
        675

<210>  46
<211>  2031
<212>  DNA
<213>  Homo

<400>  46
   caagtgcagc tggtccagag cggcgctgag gtgaagaagc ccggcgccag cgtgaaagtc        60
   agctgcaaag ctagcggcta ctccttcact ggctacacta tgaactgggt gaggcaagcc 120
   cccggccaag gcctcgagtg gatcggactg atcaacccat acaacggcga cacaagctac 180
   aaccagaagt tcaagggaag ggccacagtg actgtggaca agtccacaag cactgcctac 240
   atggaactga gctctctgag gagcgaggat acagccgtgt actactgcgc taggtggaga 300
   tacacaatgg actattgggg ccaaggcact ctggtcactg tgagcagcgc aagtaccaag 360
   ggacctagtg ttttccctct tgcaccttgc tccaggtcaa catcagagtc cacagctgct 420
   cttggatgtc tcgttaagga ctacttccca gagccagtta ccgtatcctg gaactccgga 480
   gctttgacaa gcggcgttca tacattccca gctgtgttgc agagttctgg gttgtacagt 540
   ttgagctcag tggtgaccgt gccttcatct tctttgggca ctaagaccta cacctgcaac 600
   gtggatcaca agccaagcaa caccaaggtg gataagaggg tgggtggagg cggttcaggc 660
   ggaggtggca gcggaggtgg cgggagtcag gtgcagctgg tgcagtctgg agctgaggtg 720
   aagaagcctg gcgcttctgt gaaggtgtct tgtaaggctt ctggatatat ctttattacc 780
   tattggatga attgggtgag acaggctcct ggccagggcc tggagtggat cggacagatt 840
   tttccagctt ctggctccac aaattataat gagatgtttg agggcagagc tacactgaca 900
   gtggatacat ctacatctac cgcctacatg gaactgtctt ctctgagatc tgaggataca 960
   gctgtgtact attgtgctag aggcgatggc aattatgctc tggatgctat ggattattgg 1020
   ggccagggaa cactggtgac cgtgtcttct gcctccacca agggcccatc ggtcttcccc 1080
   ctggcaccct gctccaggag cacctctgag tccacagcgg ccctgggctg cctggtcaag 1140
   gactacttcc ccgaaccggt gacggtgtcg tggaactcag gcgccctgac cagcggcgtg 1200
   cacaccttcc cggctgtcct acagtcctca ggactctact ccctcagcag cgtggtgacc 1260
   gtgccctcca gcagcttggg caccaagaca tatacctgta atgtggatca caagccttcc 1320
   aatacaaaag tggacaagag agttgagtcc aagtacggcc caccatgtcc tccatgtcca 1380
   gcccctgaat ttttgggcgg gccttctgtc tttctgtttc ctcctaaacc taaagatacc 1440
   ctgatgatca gccgcacacc cgaagtcact tgtgtggtcg tggatgtgtc tcaggaagat 1500
   cccgaagtgc agtttaactg gtatgtcgat ggcgtggaag tgcataatgc caaaactaag 1560
   ccccgcgaag aacagttcaa cagcacttat cgggtcgtgt ctgtgctcac agtcctccat 1620
   caggattggc tgaatgggaa agaatataag tgcaaggtga gcaataaggg cctccccagc 1680
   agcatcgaga agactattag caaagccaaa gggcagccac gggaacccca ggtgtacact 1740
   ctgcccccct ctcaggagga gatgactaaa aatcaggtct ctctgacttg tctggtgaaa 1800
   gggttttatc ccagcgacat tgccgtggag tgggagtcta atggccagcc cgagaataat 1860
   tataagacaa ctccccccgt cctggactct gacggcagct ttttcctgta ttctcggctg 1920
   acagtggaca aaagtcgctg gcaggagggc aatgtcttta gttgcagtgt catgcatgag 1980
   gccctgcaca atcactatac acagaaaagc ctgtctctga gtctgggcaa a 2031

<210> 47
<211> 677
<212> PRT
<213> Homo

<400> 47

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Phe Ile Thr Tyr
            20                  25                  30

Trp Met Asn Trp Val Arg Gln Ala Pro Gly Gln Gly Leu Glu Trp Ile
        35                  40                  45

Gly Gln Ile Phe Pro Ala Ser Gly Ser Thr Asn Tyr Asn Glu Met Phe
    50                  55                  60

Glu Gly Arg Ala Thr Leu Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Asp Gly Asn Tyr Ala Leu Asp Ala Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Gly Gly Gly Gly Ser
    210                 215                 220

Gly Gly Gly Gly Ser Gly Gly Gly Gly Ser Gln Val Gln Leu Val Gln
225                 230                 235                 240

Ser Gly Ala Glu Val Lys Lys Pro Gly Ala Ser Val Lys Val Ser Cys
                245                 250                 255

Lys Ala Ser Gly Tyr Ser Phe Thr Gly Tyr Thr Met Asn Trp Val Arg
            260                 265                 270

Gln Ala Pro Gly Gln Gly Leu Glu Trp Ile Gly Leu Ile Asn Pro Tyr
        275                 280                 285

Asn Gly Asp Thr Ser Tyr Asn Gln Lys Phe Lys Gly Arg Ala Thr Val
    290                 295                 300

Thr Val Asp Lys Ser Thr Ser Thr Ala Tyr Met Glu Leu Ser Ser Leu
305                 310                 315                 320

Arg Ser Glu Asp Thr Ala Val Tyr Tyr Cys Ala Arg Trp Arg Tyr Thr
                325                 330                 335

Met Asp Tyr Trp Gly Gln Gly Thr Leu Val Thr Val Ser Ser Ala Ser
            340                 345                 350

Thr Lys Gly Pro Ser Val Phe Pro Leu Ala Pro Cys Ser Arg Ser Thr
        355                 360                 365

Ser Glu Ser Thr Ala Ala Leu Gly Cys Leu Val Lys Asp Tyr Phe Pro
    370                 375                 380

Glu Pro Val Thr Val Ser Trp Asn Ser Gly Ala Leu Thr Ser Gly Val
385                 390                 395                 400

His Thr Phe Pro Ala Val Leu Gln Ser Ser Gly Leu Tyr Ser Leu Ser
                405                 410                 415

Ser Val Val Thr Val Pro Ser Ser Ser Leu Gly Thr Lys Thr Tyr Thr
            420                 425                 430

Cys Asn Val Asp His Lys Pro Ser Asn Thr Lys Val Asp Lys Arg Val
        435                 440                 445

Glu Ser Lys Tyr Gly Pro Pro Cys Pro Pro Cys Pro Ala Pro Glu Phe
    450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser Gln Glu Asp Pro Glu Val Gln Phe Asn Trp Tyr Val Asp Gly Val
            500                 505                 510

Glu Val His Asn Ala Lys Thr Lys Pro Arg Glu Glu Gln Phe Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Gly Leu Pro Ser
545                 550                 555                 560

Ser Ile Glu Lys Thr Ile Ser Lys Ala Lys Gly Gln Pro Arg Glu Pro
                565                 570                 575

Gln Val Tyr Thr Leu Pro Pro Ser Gln Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Arg Leu
625                 630                 635                 640

Thr Val Asp Lys Ser Arg Trp Gln Glu Gly Asn Val Phe Ser Cys Ser
                645                 650                 655

Val Met His Glu Ala Leu His Asn His Tyr Thr Gln Lys Ser Leu Ser
            660                 665                 670

Leu Ser Leu Gly Lys
        675

<210>  48
<211>  2031
<212>  DNA
<213>  Homo

<400> 48
caggtgcagc tggtgcagtc tggagctgag gtgaagaagc ctggcgcttc tgtgaaggtg 60

tcttgtaagg cttctggata tatctttatt acctattgga tgaattgggt gagacaggct 120

cctggccagg gcctggagtg gatcggacag atttttccag cttctggctc cacaaattat 180

aatgagatgt ttgagggcag agctacactg acagtggata catctacatc taccgcctat 240

atggagctgt cttctctgag atctgaagat accgctgtgt attattgtgc tagaggagat 300

ggcaactatg ctctggatgc catggattac tggggccagg gcaccctggt gactgtgagc 360

agcgcaagta ccaagggacc tagtgttttc cctcttgcac cttgctccag gtcaacatca 420

gagtccacag ctgctcttgg atgtctcgtt aaggactact tcccagagcc agttaccgta 480

tcctggaact ccggagcttt gacaagcggc gttcatacat tcccagctgt gttgcagagt 540

tctgggttgt acagtttgag ctcagtggtg accgtgcctt catcttcttt gggcactaag 600

acctacacct gcaacgtgga tcacaagcca agcaacacca aggtggataa gagggtgggt 660

ggaggcggtt caggcggagg tggcagcgga ggtggcggga gtcaagtgca gctggtccag 720

agcggcgctg aggtgaagaa gcccggcgcc agcgtgaaag tcagctgcaa agctagcggc 780

tactccttca ctggctacac tatgaactgg gtgaggcaag cccccggcca aggcctcgag 840

tggatcggac tgatcaaccc atacaacggc gacacaagct acaaccagaa gttcaaggga 900

agggccacag tgactgtgga caagtccaca agcactgcct acatggaact gagctctctg 960

aggagcgagg atacagccgt gtactactgc gctaggtgga gatacacaat ggactattgg 1020

ggccaaggca ctctggtcac agtgtctagt gcctccacca agggcccatc ggtcttcccc 1080

ctggcaccct gctccaggag cacctctgag tccacagcgg ccctgggctg cctggtcaag 1140

gactacttcc ccgaaccggt gacggtgtcg tggaactcag gcgccctgac cagcggcgtg 1200

cacaccttcc cggctgtcct acagtcctca ggactctact ccctcagcag cgtggtgacc 1260

gtgccctcca gcagcttggg caccaagaca tatacctgta atgtggatca caagccttcc 1320

aatacaaaag tggacaagag agttgagtcc aagtacggcc caccatgtcc tccatgtcca 1380

gcccctgaat ttttgggcgg gccttctgtc tttctgtttc ctcctaaacc taaagatacc 1440

ctgatgatca gccgcacacc cgaagtcact tgtgtggtcg tggatgtgtc tcaggaagat 1500

cccgaagtgc agtttaactg gtatgtcgat ggcgtggaag tgcataatgc caaaactaag 1560

ccccgcgaag aacagttcaa cagcacttat cgggtcgtgt ctgtgctcac agtcctccat 1620

caggattggc tgaatggaaa agaatataag tgcaaggtga gcaataaggg cctccccagc 1680

agcatcgaga agactattag caaagccaaa gggcagccac gggaacccca ggtgtacact 1740

ctgcccccct ctcaggagga gatgactaaa aatcaggtct ctctgacttg tctggtgaaa 1800

gggttttatc ccagcgacat tgccgtggag tgggagtcta atggccagcc cgagaataat 1860

tataagacaa ctccccccgt cctggactct gacggcagct ttttcctgta ttctcggctg 1920

acagtggaca aaagtcgctg gcaggagggc aatgtcttta gttgcagtgt catgcatgag 1980

gccctgcaca atcactatac acagaaaagc ctgtctctga gtctgggcaa a 2031

<210> 49
<211> 677
<212> PRT
<213> Homo

<400> 49

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
            20                  25                  30

Thr Met Asn Trp Val Arg Gln Ala Pro Gly Gln Gly Leu Glu Trp Ile
        35                  40                  45

Gly Leu Ile Asn Pro Tyr Asn Gly Asp Thr Ser Tyr Asn Gln Lys Phe
    50                  55                  60

Lys Gly Arg Ala Thr Val Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Trp Arg Tyr Thr Met Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr

195                 200                 205

Lys Val Asp Lys Arg Val Gly Gly Gly Gly Ser Gly Gly Gly Gly Ser
    210                 215                 220

Gly Gly Gly Gly Ser Gln Val Gln Leu Val Gln Ser Gly Ala Glu Val
225                 230                 235                 240

Lys Lys Pro Gly Ala Ser Val Lys Val Ser Cys Lys Ala Ser Gly Tyr
                245                 250                 255

Thr Phe Thr Ser Glu Trp Met Asn Trp Val Arg Gln Ala Pro Gly Gln
            260                 265                 270

Gly Leu Glu Trp Met Gly Gln Ile Phe Pro Ala Leu Gly Ser Thr Asn
        275                 280                 285

Tyr Asn Glu Met Tyr Glu Gly Arg Val Thr Met Thr Thr Asp Thr Ser
    290                 295                 300

Thr Ser Thr Ala Tyr Met Glu Leu Arg Ser Leu Arg Ser Asp Asp Thr
305                 310                 315                 320

Ala Val Tyr Tyr Cys Ala Arg Gly Ile Gly Asn Tyr Ala Leu Asp Ala
                325                 330                 335

Met Asp Tyr Trp Gly Gln Gly Thr Leu Val Thr Val Ser Ser Ala Ser
            340                 345                 350

Thr Lys Gly Pro Ser Val Phe Pro Leu Ala Pro Cys Ser Arg Ser Thr
        355                 360                 365

Ser Glu Ser Thr Ala Ala Leu Gly Cys Leu Val Lys Asp Tyr Phe Pro
    370                 375                 380

Glu Pro Val Thr Val Ser Trp Asn Ser Gly Ala Leu Thr Ser Gly Val
385                 390                 395                 400

His Thr Phe Pro Ala Val Leu Gln Ser Ser Gly Leu Tyr Ser Leu Ser
                405                 410                 415

Ser Val Val Thr Val Pro Ser Ser Ser Leu Gly Thr Lys Thr Tyr Thr
            420                 425                 430

Cys Asn Val Asp His Lys Pro Ser Asn Thr Lys Val Asp Lys Arg Val
        435                 440                 445

Glu Ser Lys Tyr Gly Pro Pro Cys Pro Pro Cys Pro Ala Pro Glu Phe
    450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser Gln Glu Asp Pro Glu Val Gln Phe Asn Trp Tyr Val Asp Gly Val
            500                 505                 510

Glu Val His Asn Ala Lys Thr Lys Pro Arg Glu Glu Gln Phe Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Gly Leu Pro Ser
545                 550                 555                 560

Ser Ile Glu Lys Thr Ile Ser Lys Ala Lys Gly Gln Pro Arg Glu Pro
                565                 570                 575

Gln Val Tyr Thr Leu Pro Pro Ser Gln Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Arg Leu
625                 630                 635                 640

Thr Val Asp Lys Ser Arg Trp Gln Glu Gly Asn Val Phe Ser Cys Ser
                645                 650                 655

Val Met His Glu Ala Leu His Asn His Tyr Thr Gln Lys Ser Leu Ser
            660                 665                 670

Leu Ser Leu Gly Lys
        675

<210>  50
<211>  2031
<212>  DNA

<213>  Homo

<400>  50
caagtgcagc tggtccagag cggcgctgag gtgaagaagc ccggcgccag cgtgaaagtc      60

agctgcaaag ctagcggcta ctccttcact ggctacacta tgaactgggt gaggcaagcc     120

cccggccaag gcctcgagtg gatcggactg atcaacccat acaacggcga cacaagctac     180

aaccagaagt tcaagggaag ggccacagtg actgtggaca agtccacaag cactgcctac     240

atggaactga gctctctgag gagcgaggat acagccgtgt actactgcgc taggtggaga     300

tacacaatgg actattgggg ccaaggcact ctggtcactg tgagcagcgc aagtaccaag     360

ggacctagtg ttttccctct tgcaccttgc tccaggtcaa catcagagtc cacagctgct     420

cttggatgtc tcgttaagga ctacttccca gagccagtta ccgtatcctg gaactccgga     480

gctttgacaa gcggcgttca tacattccca gctgtgttgc agagttctgg gttgtacagt     540

ttgagctcag tggtgaccgt gccttcatct tctttgggca ctaagaccta cacctgcaac     600

gtggatcaca agccaagcaa caccaaggtg gataagaggg tgggtggagg cggttcaggc     660

ggaggtggca gcggaggtgg cgggagtcaa gtgcagctgg tgcagagcgg cgctgaggtc     720

aaaaagcccg gcgcctccgt gaaggtgagc tgtaaggcca gcggctacac attcactagc     780

gagtggatga actgggtgag acaagccccc ggccaaggac tggaatggat gggccagatc     840

ttcccagctc tgggctccac taactacaac gagatgtacg agggaagggt cactatgact     900

acagacacta gcactagcac tgcctacatg gaactgaggt ctctgagaag cgacgataca     960

gccgtgtact actgcgccag aggcatcggc aactatgctc tggatgccat ggactactgg    1020

ggccaaggca ctctcgtgac tgtgagctcc gcctccacca agggcccatc ggtcttcccc    1080

ctggcaccct gctccaggag cacctctgag tccacagcgg ccctgggctg cctggtcaag    1140

gactacttcc ccgaaccggt gacggtgtcg tggaactcag gcgccctgac cagcggcgtg    1200

cacaccttcc cggctgtcct acagtcctca ggactctact ccctcagcag cgtggtgacc    1260

gtgccctcca gcagcttggg caccaagaca tatacctgta atgtggatca caagccttcc    1320

aatacaaaag tggacaagag agttgagtcc aagtacggcc caccatgtcc tccatgtcca    1380

gcccctgaat ttttgggcgg gccttctgtc tttctgtttc ctcctaaacc taaagatacc    1440

ctgatgatca gccgcacacc cgaagtcact tgtgtggtcg tggatgtgtc tcaggaagat    1500

cccgaagtgc agtttaactg gtatgtcgat ggcgtggaag tgcataatgc caaaactaag    1560

ccccgcgaag aacagttcaa cagcacttat cgggtcgtgt ctgtgctcac agtcctccat    1620

caggattggc tgaatggaaa agaatataag tgcaaggtga gcaataaggg cctccccagc    1680

agcatcgaga agactattag caaagccaaa gggcagccac gggaacccca ggtgtacact    1740

ctgcccccct ctcaggagga gatgactaaa aatcaggtct ctctgacttg tctggtgaaa    1800

gggttttatc ccagcgacat tgccgtggag tgggagtcta atggccagcc cgagaataat 1860

tataagacaa ctccccccgt cctggactct gacggcagct ttttcctgta ttctcggctg 1920

acagtggaca aaagtcgctg gcaggagggc aatgtcttta gttgcagtgt catgcatgag 1980

gccctgcaca atcactatac acagaaaagc ctgtctctga gtctgggcaa a 2031

<210> 51
<211> 677
<212> PRT
<213> Homo

<400> 51

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Glu
            20                  25                  30

Trp Met Asn Trp Val Arg Gln Ala Pro Gly Gln Gly Leu Glu Trp Met
        35                  40                  45

Gly Gln Ile Phe Pro Ala Leu Gly Ser Thr Asn Tyr Asn Glu Met Tyr
    50                  55                  60

Glu Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Gly Ile Gly Asn Tyr Ala Leu Asp Ala Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Gly Gly Gly Gly Ser
    210                 215                 220

Gly Gly Gly Gly Ser Gly Gly Gly Gly Ser Gln Val Gln Leu Val Gln
225                 230                 235                 240

Ser Gly Ala Glu Val Lys Lys Pro Gly Ala Ser Val Lys Val Ser Cys
                245                 250                 255

Lys Ala Ser Gly Tyr Ser Phe Thr Gly Tyr Thr Met Asn Trp Val Arg
            260                 265                 270

Gln Ala Pro Gly Gln Gly Leu Glu Trp Ile Gly Leu Ile Asn Pro Tyr
        275                 280                 285

Asn Gly Asp Thr Ser Tyr Asn Gln Lys Phe Lys Gly Arg Ala Thr Val
    290                 295                 300

Thr Val Asp Lys Ser Thr Ser Thr Ala Tyr Met Glu Leu Ser Ser Leu
305                 310                 315                 320

Arg Ser Glu Asp Thr Ala Val Tyr Tyr Cys Ala Arg Trp Arg Tyr Thr
                325                 330                 335

Met Asp Tyr Trp Gly Gln Gly Thr Leu Val Thr Val Ser Ser Ala Ser
            340                 345                 350

Thr Lys Gly Pro Ser Val Phe Pro Leu Ala Pro Cys Ser Arg Ser Thr
        355                 360                 365

Ser Glu Ser Thr Ala Ala Leu Gly Cys Leu Val Lys Asp Tyr Phe Pro
    370                 375                 380

Glu Pro Val Thr Val Ser Trp Asn Ser Gly Ala Leu Thr Ser Gly Val
385                 390                 395                 400

His Thr Phe Pro Ala Val Leu Gln Ser Ser Gly Leu Tyr Ser Leu Ser
                405                 410                 415

Ser Val Val Thr Val Pro Ser Ser Ser Leu Gly Thr Lys Thr Tyr Thr
            420                 425                 430

Cys Asn Val Asp His Lys Pro Ser Asn Thr Lys Val Asp Lys Arg Val
        435                 440                 445

Glu Ser Lys Tyr Gly Pro Pro Cys Pro Pro Cys Pro Ala Pro Glu Phe
    450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser Gln Glu Asp Pro Glu Val Gln Phe Asn Trp Tyr Val Asp Gly Val
            500                 505                 510

Glu Val His Asn Ala Lys Thr Lys Pro Arg Glu Glu Gln Phe Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Gly Leu Pro Ser
545                 550                 555                 560

Ser Ile Glu Lys Thr Ile Ser Lys Ala Lys Gly Gln Pro Arg Glu Pro
                565                 570                 575

Gln Val Tyr Thr Leu Pro Pro Ser Gln Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Arg Leu
625                 630                 635                 640

Thr Val Asp Lys Ser Arg Trp Gln Glu Gly Asn Val Phe Ser Cys Ser
                645                 650                 655

Val Met His Glu Ala Leu His Asn His Tyr Thr Gln Lys Ser Leu Ser
            660                 665                 670

Leu Ser Leu Gly Lys
        675

<210>  52

<211>  2031
<212>  DNA
<213>  Homo

<400>  52
caagtgcagc tggtgcagag cggcgctgag gtcaaaaagc ccggcgcctc cgtgaaggtg      60

agctgtaagg ccagcggcta cacattcact agcgagtgga tgaactgggt gagacaagcc     120

cccggccaag gactggaatg gatgggccag atcttcccag ctctgggctc cactaactac     180

aacgagatgt acgagggaag ggtcactatg actacagaca ctagcactag cactgcctac     240

atggaactga ggtctctgag aagcgacgat acagccgtgt actactgcgc cagaggcatc     300

ggcaactatg ctctggatgc catggactac tggggccaag gcactctcgt gactgtgagc     360

tccgcaagta ccaagggacc tagtgttttc cctcttgcac cttgctccag gtcaacatca     420

gagtccacag ctgctcttgg atgtctcgtt aaggactact tcccagagcc agttaccgta     480

tcctggaact ccggagcttt gacaagcggc gttcatacat tcccagctgt gttgcagagt     540

tctgggttgt acagtttgag ctcagtggtg accgtgcctt catcttcttt gggcactaag     600

acctacacct gcaacgtgga tcacaagcca agcaacacca aggtggataa gagggtgggt     660

ggaggcggtt caggcggagg tggcagcgga ggtggcggga gtcaagtgca gctggtccag     720

agcggcgctg aggtgaagaa gcccggcgcc agcgtgaaag tcagctgcaa agctagcggc     780

tactccttca ctggctacac tatgaactgg gtgaggcaag cccccggcca aggcctcgag     840

tggatcggac tgatcaaccc atacaacggc gacacaagct acaaccagaa gttcaaggga     900

agggccacag tgactgtgga caagtccaca agcactgcct acatggaact gagctctctg     960

aggagcgagg atacagccgt gtactactgc gctaggtgga gatacacaat ggactattgg    1020

ggccaaggca ctctggtcac agtgtctagt gcctccacca agggcccatc ggtcttcccc    1080

ctggcaccct gctccaggag cacctctgag tccacagcgg ccctgggctg cctggtcaag    1140

gactacttcc ccgaaccggt gacggtgtcg tggaactcag gcgccctgac cagcggcgtg    1200

cacaccttcc cggctgtcct acagtcctca ggactctact ccctcagcag cgtggtgacc    1260

gtgccctcca gcagcttggg caccaagaca tatacctgta atgtggatca caagccttcc    1320

aatacaaaag tggacaagag agttgagtcc aagtacggcc caccatgtcc tccatgtcca    1380

gcccctgaat ttttgggcgg gccttctgtc tttctgtttc ctcctaaacc taaagatacc    1440

ctgatgatca gccgcacacc cgaagtcact tgtgtggtcg tggatgtgtc tcaggaagat    1500

cccgaagtgc agtttaactg gtatgtcgat ggcgtggaag tgcataatgc caaaactaag    1560

ccccgcgaag aacagttcaa cagcacttat cgggtcgtgt ctgtgctcac agtcctccat    1620

caggattggc tgaatggaaa agaatataag tgcaaggtga gcaataaggg cctccccagc    1680

agcatcgaga agactattag caaagccaaa gggcagccac gggaacccca ggtgtacact    1740

ctgcccccct ctcaggagga gatgactaaa aatcaggtct ctctgacttg tctggtgaaa 1800

gggttttatc ccagcgacat tgccgtggag tgggagtcta atggccagcc cgagaataat 1860

tataagacaa ctccccccgt cctggactct gacggcagct ttttcctgta ttctcggctg 1920

acagtggaca aaagtcgctg gcaggagggc aatgtcttta gttgcagtgt catgcatgag 1980

gccctgcaca atcactatac acagaaaagc ctgtctctga gtctgggcaa a 2031

<210> 53
<211> 117
<212> PRT
<213> Homo

<400> 53

Gln Val Gln Leu Val Gln Ser Gly Ala Glu Val Lys Lys Pro Gly Ala
1               5                   10                  15

Ser Val Lys Val Ser Cys Lys Gly Ser Gly Tyr Thr Phe Thr Asp Tyr
            20                  25                  30

Ala Ile His Trp Val Arg Gln Ala Pro Gly Gln Ser Leu Glu Trp Ile
        35                  40                  45

Gly Val Phe Ser Ile Tyr Tyr Glu Asn Ile Asn Tyr Asn Gln Lys Phe
    50                  55                  60

Lys Gly Arg Ala Thr Met Thr Val Asp Lys Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Arg Asp Gly Gly Thr Ile Asn Tyr Trp Gly Gln Gly Thr Leu
            100                 105                 110

Val Thr Val Ser Ser
        115

<210> 54
<211> 112
<212> PRT
<213> Homo

<400> 54

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Phe Cys Ser Gln Ser
                85                  90                  95

Thr His Ile Pro Trp Thr Phe Gly Gln Gly Thr Lys Val Glu Ile Lys
            100                 105                 110

<210>  55
<211>  5
<212>  PRT
<213>  Mus musculus

<400>  55

Asn His Val Ile His
1               5

<210>  56
<211>  17
<212>  PRT
<213>  Mus musculus

<400>  56

Tyr Ile Tyr Pro Tyr Asn Asp Gly Thr Lys Tyr Asn Glu Lys Phe Lys
1               5                   10                  15

Asp

<210>  57
<211>  8
<212>  PRT
<213>  Mus musculus

<400>  57

Gly Gly Tyr Tyr Thr Tyr Asp Asp
1               5

<210>  58
<211>  16
<212>  PRT
<213>  Mus musculus

<400>  58

Arg Ser Ser Gln Ser Leu Val His Ser Asn Gly Lys Thr Tyr Leu His
1               5                   10                  15


<210>  59
<211>  7
<212>  PRT
<213>  Mus musculus

<400>  59

Lys Val Ser Asn Arg Phe Ser
1               5


<210>  60
<211>  9
<212>  PRT
<213>  Mus musculus

<400>  60

Ser Gln Ser Thr His Val Pro Tyr Thr
1               5


<210>  61
<211>  117
<212>  PRT
<213>  Homo

<400>  61

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Ala Asn His
            20                  25                  30


Val Ile His Trp Val Arg Gln Ala Pro Gly Gln Gly Leu Glu Trp Met
        35                  40                  45


Gly Tyr Ile Tyr Pro Tyr Asn Asp Gly Thr Lys Tyr Asn Glu Lys Phe
    50                  55                  60


Lys Asp Arg Val Thr Leu Thr Ser Asp Lys Ser Thr Ser Thr Val Tyr
65                  70                  75                  80


Met Glu Leu Ser Ser Leu Arg Ser Glu Asp Thr Ala Val Tyr Tyr Cys
                85                  90                  95


Ala Arg Gly Gly Tyr Tyr Thr Tyr Asp Asp Trp Gly Gln Gly Thr Leu
            100                 105                 110

Val Thr Val Ser Ser
        115

<210>  62
<211>  112
<212>  PRT
<213>  Homo

<400>  62

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Lys Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Tyr Thr Phe Gly Gln Gly Thr Lys Val Glu Ile Lys
            100                 105                 110

<210>  63
<211>  219
<212>  PRT
<213>  Homo

<400>  63

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Phe Cys Ser Gln Ser
                85                  90                  95

Thr His Ile Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  64
<211>  657
<212>  DNA
<213>  Homo

<400>  64
gacgtggtga tgacccagag ccctttatct ttacccgtta cactgggaca gcccgccagc      60

atcagctgtc gtagcagcca gtctttagtg cacagcaacg gcaacaccta tttacactgg     120

taccagcaga gacccggcca gagccccaga ctgctgatct acaaggtgag caatcgtttc     180

tccggcgtgc ccgacagatt cagcggcagc ggaagcggca ccgacttcac tttaaagatc     240

agcagagtgg aggccgagga cgtgggcgtg tacttctgca gccagagcac ccacatccct     300

tggaccttcg gccaaggtac caaggtggag atcaagagaa ccgtcgccgc tcccagcgtc     360

ttcatcttcc cccccagcga tgagcagctg aagagcggaa ccgccagcgt ggtgtgcctg     420

ctgaacaact tctaccccag ggaggccaag gtgcaatgga aggtggacaa cgccctacag     480

agcggcaact cccaggagag cgtgaccgag caggacagca aggatagcac ctacagcctg     540

agcagcaccc tcaccctgag caaggccgac tacgagaagc acaaggtgta cgcctgcgag 600

gtgacccatc agggcctgag cagccctgtg accaagagct tcaacagggg cgagtgc 657

<210> 65
<211> 677
<212> PRT
<213> Homo

<400> 65

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ala Asn His
            20                  25                  30

Val Ile His Trp Val Arg Gln Ala Pro Gly Gln Gly Leu Glu Trp Met
        35                  40                  45

Gly Tyr Ile Tyr Pro Tyr Asn Asp Gly Thr Lys Tyr Asn Glu Lys Phe
    50                  55                  60

Lys Asp Arg Val Thr Leu Thr Ser Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Tyr Thr Tyr Asp Asp Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Val Gln Ser Gly Ala Glu
225                 230                 235                 240

Val Lys Lys Pro Gly Ala Ser Val Lys Val Ser Cys Lys Gly Ser Gly
                245                 250                 255

Tyr Thr Phe Thr Asp Tyr Ala Ile His Trp Val Arg Gln Ala Pro Gly
            260                 265                 270

Gln Ser Leu Glu Trp Ile Gly Val Phe Ser Ile Tyr Tyr Glu Asn Ile
        275                 280                 285

Asn Tyr Asn Gln Lys Phe Lys Gly Arg Ala Thr Met Thr Val Asp Lys
    290                 295                 300

Ser Thr Ser Thr Ala Tyr Met Glu Leu Arg Ser Leu Arg Ser Asp Asp
305                 310                 315                 320

Thr Ala Val Tyr Tyr Cys Ala Arg Arg Asp Gly Gly Thr Ile Asn Tyr
                325                 330                 335

Trp Gly Gln Gly Thr Leu Val Thr Val Ser Ser Ala Ser Thr Lys Gly
            340                 345                 350

Pro Ser Val Phe Pro Leu Ala Pro Ser Ser Lys Ser Thr Ser Gly Gly
        355                 360                 365

Thr Ala Ala Leu Gly Cys Leu Val Lys Asp Tyr Phe Pro Glu Pro Val
    370                 375                 380

Thr Val Ser Trp Asn Ser Gly Ala Leu Thr Ser Gly Val His Thr Phe
385                 390                 395                 400

Pro Ala Val Leu Gln Ser Ser Gly Leu Tyr Ser Leu Ser Ser Val Val
                405                 410                 415

Thr Val Pro Ser Ser Ser Leu Gly Thr Gln Thr Tyr Ile Cys Asn Val
            420                 425                 430

Asn His Lys Pro Ser Asn Thr Lys Val Asp Lys Lys Val Glu Pro Lys
        435                 440                 445

Ser Cys Asp Lys Thr His Thr Cys Pro Pro Cys Pro Ala Pro Glu Leu

450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser His Glu Asp Pro Glu Val Lys Phe Asn Trp Tyr Val Asp Gly Val
            500                 505                 510

Glu Val His Asn Ala Lys Thr Lys Pro Arg Glu Glu Gln Tyr Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Ala Leu Pro Ala
545                 550                 555                 560

Pro Ile Glu Lys Thr Ile Ser Lys Ala Lys Gly Gln Pro Arg Glu Pro
                565                 570                 575

Gln Val Tyr Thr Leu Pro Pro Ser Arg Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Lys Leu
625                 630                 635                 640

Thr Val Asp Lys Ser Arg Trp Gln Gln Gly Asn Val Phe Ser Cys Ser
                645                 650                 655

Val Met His Glu Ala Leu His Asn His Tyr Thr Gln Lys Ser Leu Ser
            660                 665                 670

Leu Ser Pro Gly Lys
        675

<210>  66
<211>  2031
<212>  DNA
<213>  Homo

<400> 66
caggtgcagc tggtgcagtc cggcgccgag gtgaagaagc ccggcgcctc cgtgaaagtg 60

agctgcaagg catccggcta caccttcgcc aaccacgtga tccactgggt gcggcaggcc 120

cccggccagg gcctggagtg gatgggctac atctatcctt ataatgatgg cacaaagtat 180

aatgagaagt ttaaggatag ggtaacactg acatctgata agtctacatc tacagtgtac 240

atggagctgt cctctctgag gtccgaggac accgccgtgt actactgcgc cagaggcggc 300

tactacacct acgacgactg gggccagggc accctggtga ccgtgtcctc cgcaagtacc 360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt caagttcagc tggtgcagag cggagctgag 720

gtgaagaagc ccggcgccag cgtgaaggtg agctgtaagg gcagcggcta caccttcacc 780

gactacgcca tccactgggt gagacaagct cccggtcagt ctttagaatg gatcggcgtg 840

ttcagcatct actacgagaa catcaactat aaccagaagt tcaagggtcg tgccaccatg 900

accgtggaca agagcaccag caccgcctac atggagctga ggtctttaag gagcgacgac 960

accgccgtgt actactgcgc tcgtagggac ggcggcacca tcaactactg gggccaaggt 1020

actttagtga cagtgagcag cgcgagcacc aagggacctt ccgtgtttcc cctcgccccc 1080

agctccaaaa gcaccagcgg cggaacagct gctctcggct gtctcgtcaa ggattacttc 1140

cccgagcccg tgaccgtgag ctggaacagc ggagccctga caagcggcgt ccacaccttc 1200

cctgctgtcc tacagtcctc cggactgtac agcctgagca gcgtggtgac agtccctagc 1260

agctccctgg gcacccagac atatatttgc aacgtgaatc acaagcccag caacaccaag 1320

gtcgataaga aggtggagcc taagtcctgc gacaagaccc acacatgtcc cccctgtccc 1380

gctcctgaac tgctgggagg cccttccgtg ttcctgttcc cccctaagcc caaggacacc 1440

ctgatgattt ccaggacacc cgaggtgacc tgtgtggtgg tggacgtcag ccacgaggac 1500

cccgaggtga aattcaactg gtacgtcgat ggcgtggagg tgcacaacgc taagaccaag 1560

cccagggagg agcagtacaa ttccacctac agggtggtgt ccgtgctgac cgtcctccat 1620

caggactggc tgaacggcaa agagtataag tgcaaggtga gcaacaaggc cctccctgct 1680

cccatcgaga agaccatcag caaagccaag ggccagccca gggaacctca agtctatacc 1740

ctgcctccca gcagggagga gatgaccaag aaccaagtga gcctcacatg cctcgtcaag 1800

ggcttctatc cttccgatat tgccgtcgag tgggagtcca acggacagcc cgagaacaac 1860

tacaagacaa caccccccgt gctcgattcc gatggcagct tcttcctgta ctccaagctg 1920

accgtggaca agtccagatg gcaacaaggc aacgtcttca gttgcagcgt catgcatgag 1980

gccctccaca accactacac ccagaaaagc ctgtctctga gtcctggcaa a 2031

<210> 67
<211> 677
<212> PRT
<213> Homo

<400> 67

Gln Val Gln Leu Val Gln Ser Gly Ala Glu Val Lys Lys Pro Gly Ala
1               5                   10                  15

Ser Val Lys Val Ser Cys Lys Gly Ser Gly Tyr Thr Phe Thr Asp Tyr
            20                  25                  30

Ala Ile His Trp Val Arg Gln Ala Pro Gly Gln Ser Leu Glu Trp Ile
        35                  40                  45

Gly Val Phe Ser Ile Tyr Tyr Glu Asn Ile Asn Tyr Asn Gln Lys Phe
    50                  55                  60

Lys Gly Arg Ala Thr Met Thr Val Asp Lys Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Arg Asp Gly Gly Thr Ile Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Val Gln Ser Gly Ala Glu
225                 230                 235                 240

Val Lys Lys Pro Gly Ala Ser Val Lys Val Ser Cys Lys Ala Ser Gly
                245                 250                 255

Tyr Thr Phe Ala Asn His Val Ile His Trp Val Arg Gln Ala Pro Gly
            260                 265                 270

Gln Gly Leu Glu Trp Met Gly Tyr Ile Tyr Pro Tyr Asn Asp Gly Thr
        275                 280                 285

Lys Tyr Asn Glu Lys Phe Lys Asp Arg Val Thr Leu Thr Ser Asp Lys
    290                 295                 300

Ser Thr Ser Thr Val Tyr Met Glu Leu Ser Ser Leu Arg Ser Glu Asp
305                 310                 315                 320

Thr Ala Val Tyr Tyr Cys Ala Arg Gly Gly Tyr Tyr Thr Tyr Asp Asp
                325                 330                 335

Trp Gly Gln Gly Thr Leu Val Thr Val Ser Ser Ala Ser Thr Lys Gly
            340                 345                 350

Pro Ser Val Phe Pro Leu Ala Pro Ser Ser Lys Ser Thr Ser Gly Gly
        355                 360                 365

Thr Ala Ala Leu Gly Cys Leu Val Lys Asp Tyr Phe Pro Glu Pro Val
    370                 375                 380

Thr Val Ser Trp Asn Ser Gly Ala Leu Thr Ser Gly Val His Thr Phe
385                 390                 395                 400

Pro Ala Val Leu Gln Ser Ser Gly Leu Tyr Ser Leu Ser Ser Val Val
                405                 410                 415

Thr Val Pro Ser Ser Ser Leu Gly Thr Gln Thr Tyr Ile Cys Asn Val
            420                 425                 430

Asn His Lys Pro Ser Asn Thr Lys Val Asp Lys Lys Val Glu Pro Lys
        435                 440                 445

Ser Cys Asp Lys Thr His Thr Cys Pro Pro Cys Pro Ala Pro Glu Leu
    450                 455                 460

Leu Gly Gly Pro Ser Val Phe Leu Phe Pro Pro Lys Pro Lys Asp Thr
465                 470                 475                 480

Leu Met Ile Ser Arg Thr Pro Glu Val Thr Cys Val Val Val Asp Val
                485                 490                 495

Ser His Glu Asp Pro Glu Val Lys Phe Asn Trp Tyr Val Asp Gly Val
            500                 505                 510

Glu Val His Asn Ala Lys Thr Lys Pro Arg Glu Glu Gln Tyr Asn Ser
        515                 520                 525

Thr Tyr Arg Val Val Ser Val Leu Thr Val Leu His Gln Asp Trp Leu
    530                 535                 540

Asn Gly Lys Glu Tyr Lys Cys Lys Val Ser Asn Lys Ala Leu Pro Ala
545                 550                 555                 560

Pro Ile Glu Lys Thr Ile Ser Lys Ala Lys Gly Gln Pro Arg Glu Pro
                565                 570                 575

Gln Val Tyr Thr Leu Pro Pro Ser Arg Glu Glu Met Thr Lys Asn Gln
            580                 585                 590

Val Ser Leu Thr Cys Leu Val Lys Gly Phe Tyr Pro Ser Asp Ile Ala
        595                 600                 605

Val Glu Trp Glu Ser Asn Gly Gln Pro Glu Asn Asn Tyr Lys Thr Thr
    610                 615                 620

Pro Pro Val Leu Asp Ser Asp Gly Ser Phe Phe Leu Tyr Ser Lys Leu
625                 630                 635                 640

Thr Val Asp Lys Ser Arg Trp Gln Gln Gly Asn Val Phe Ser Cys Ser
                645                 650                 655

Val Met His Glu Ala Leu His Asn His Tyr Thr Gln Lys Ser Leu Ser
            660                 665                 670

Leu Ser Pro Gly Lys
        675

<210>  68
<211>  2031

<212>  DNA
<213>  Homo

<400>  68
caagttcagc tggtgcagag cggagctgag gtgaagaagc ccggcgccag cgtgaaggtg      60

agctgtaagg gcagcggcta caccttcacc gactacgcca tccactgggt gagacaagct     120

cccggtcagt ctttagaatg gatcggcgtg ttcagcatct actacgagaa catcaactat     180

aaccagaagt tcaagggtcg tgccaccatg accgtggaca agagcaccag caccgcctac     240

atggagctga ggtctttaag gagcgacgac accgccgtgt actactgcgc tcgtagggac     300

ggcggcacca tcaactactg gggccaaggt actttagtga cagtgagcag cgcaagtacc     360

aagggaccta gtgttttccc tcttgcacct tgctccaggt caacatcaga gtccacagct     420

gctcttggat gtctcgttaa ggactacttc ccagagccag ttaccgtatc ctggaactcc     480

ggagctttga caagcggcgt tcatacattc ccagctgtgt tgcagagttc tgggttgtac     540

agtttgagct cagtggtgac cgtgccttca tcttctttgg gcactaagac ctacacctgc     600

aacgtggatc acaagccaag caacaccaag gtggataaga gggtgggtgg aggcggttca     660

ggcggaggtg gcagcggagg tggcgggagt caggtgcagc tggtgcagtc cggcgccgag     720

gtgaagaagc ccggcgcctc cgtgaaagtg agctgcaagg catccggcta caccttcgcc     780

aaccacgtga tccactgggt gcggcaggcc cccggccagg gcctggagtg gatgggctac     840

atctatcctt ataatgatgg cacaaagtat aatgagaagt ttaaggatag ggtaacactg     900

acatctgata agtctacatc tacagtgtac atggagctgt cctctctgag gtccgaggac     960

accgccgtgt actactgcgc cagaggcggc tactacacct acgacgactg gggccagggc    1020

accctggtga ccgtgtcctc cgcgagcacc aagggacctt ccgtgtttcc cctcgccccc    1080

agctccaaaa gcaccagcgg cggaacagct gctctcggct gtctcgtcaa ggattacttc    1140

cccgagcccg tgaccgtgag ctggaacagc ggagccctga caagcggcgt ccacaccttc    1200

cctgctgtcc tacagtcctc cggactgtac agcctgagca gcgtggtgac agtccctagc    1260

agctccctgg gcacccagac atatatttgc aacgtgaatc acaagcccag caacaccaag    1320

gtcgataaga aggtggagcc taagtcctgc gacaagaccc acacatgtcc cccctgtccc     1380

gctcctgaac tgctgggagg cccttccgtg ttcctgttcc cccctaagcc caaggacacc    1440

ctgatgattt ccaggacacc cgaggtgacc tgtgtggtgg tggacgtcag ccacgaggac    1500

cccgaggtga aattcaactg gtacgtcgat ggcgtggagg tgcacaacgc taagaccaag    1560

cccagggagg agcagtacaa ttccacctac agggtggtgt ccgtgctgac cgtcctccat    1620

caggactggc tgaacggcaa agagtataag tgcaaggtga gcaacaaggc cctccctgct    1680

cccatcgaga agaccatcag caaagccaag ggccagccca gggaacctca agtctatacc    1740

ctgcctccca gcagggagga gatgaccaag aaccaagtga gcctcacatg cctcgtcaag    1800

ggcttctatc cttccgatat tgccgtcgag tgggagtcca acggacagcc cgagaacaac 1860

tacaagacaa caccccccgt gctcgattcc gatggcagct tcttcctgta ctccaagctg 1920

accgtggaca agtccagatg gcaacaaggc aacgtcttca gttgcagcgt catgcatgag 1980

gccctccaca accactacac ccagaaaagc ctgtctctga gtcctggcaa a 2031

<210> 69
<211> 111
<212> PRT
<213> Mus musculus

<400> 69

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Ser Ser Tyr
            20                  25                  30

Asp Ile Ser Trp Ile Arg Gln Pro Pro Gly Lys Gly Leu Glu Trp Leu
        35                  40                  45

Gly Val Ile Trp Thr Gly Gly Gly Thr Asn Tyr Asn Ser Ala Phe Met
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Lys Tyr Tyr Cys Val
                85                  90                  95

Arg Met Asp Tyr Trp Gly Gln Gly Thr Ser Val Thr Val Ser Ser
            100                 105                 110

<210> 70
<211> 112
<212> PRT
<213> Mus musculus

<400> 70

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Leu His Ser
            20                  25                  30

Asn Gly Asn Thr Tyr Leu His Trp Tyr Leu Gln Lys Pro Gly Gln Ser
        35                  40                  45

Pro Lys Leu Leu Phe Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

<210>  71
<211>  5
<212>  PRT
<213>  Mus musculus

<400>  71

Ser Tyr Asp Ile Ser
1               5

<210>  72
<211>  16
<212>  PRT
<213>  Mus musculus

<400>  72

Val Ile Trp Thr Gly Gly Gly Thr Asn Tyr Asn Ser Ala Phe Met Ser
1               5                   10                  15

<210>  73
<211>  3
<212>  PRT
<213>  Mus musculus

<400>  73

Met Asp Tyr
1

<210>  74
<211>  16
<212>  PRT
<213>  Mus musculus

<400>  74

Arg Ser Ser Gln Ser Leu Leu His Ser Asn Gly Asn Thr Tyr Leu His
1               5                   10                  15

<210>  75
<211>  7

<212>  PRT
<213>  Mus musculus

<400>  75

Lys Val Ser Asn Arg Phe Ser
1               5


<210>  76
<211>  9
<212>  PRT
<213>  Mus musculus

<400>  76

Ser Gln Ser Thr His Val Pro Trp Thr
1               5


<210>  77
<211>  111
<212>  PRT
<213>  Homo

<400>  77

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Ser Ser Tyr
            20                  25                  30

Asp Ile Ser Trp Ile Arg Gln Pro Pro Gly Lys Gly Leu Glu Trp Leu
        35                  40                  45

Gly Val Ile Trp Thr Gly Gly Gly Thr Asn Tyr Asn Ser Ala Phe Met
    50                  55                  60

Ser Arg Leu Thr Ile Ser Lys Asp Asn Ser Lys Ser Gln Val Ser Leu
65                  70                  75                  80

Lys Leu Ser Ser Val Thr Ala Ala Asp Thr Ala Val Tyr Tyr Cys Val
                85                  90                  95

Arg Met Asp Tyr Trp Gly Gln Gly Thr Leu Val Thr Val Ser Ser
            100                 105                 110


<210>  78
<211>  112
<212>  PRT
<213>  Homo

<400>  78

Asp Val Val Met Thr Gln Ser Pro Leu Ser Leu Pro Val Thr Leu Gly

1               5                   10                  15

Gln Pro Ala Ser Ile Ser Cys Arg Ser Ser Gln Ser Leu Leu His Ser
            20                  25                  30

Asn Gly Asn Thr Tyr Leu His Trp Tyr Gln Gln Arg Pro Gly Gln Ser
        35                  40                  45

Pro Arg Leu Leu Phe Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Phe Cys Ser Gln Ser
                85                  90                  95

Thr His Val Pro Trp Thr Phe Gly Gln Gly Thr Lys Val Glu Ile Lys
            100                 105                 110

<210>  79
<211>  671
<212>  PRT
<213>  Homo

<400>  79

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Ser Ser Tyr
            20                  25                  30

Asp Ile Ser Trp Ile Arg Gln Pro Pro Gly Lys Gly Leu Glu Trp Leu
        35                  40                  45

Gly Val Ile Trp Thr Gly Gly Gly Thr Asn Tyr Asn Ser Ala Phe Met
    50                  55                  60

Ser Arg Leu Thr Ile Ser Lys Asp Asn Ser Lys Ser Gln Val Ser Leu
65                  70                  75                  80

Lys Leu Ser Ser Val Thr Ala Ala Asp Thr Ala Val Tyr Tyr Cys Val
                85                  90                  95

Arg Met Asp Tyr Trp Gly Gln Gly Thr Leu Val Thr Val Ser Ser Ala
            100                 105                 110

Ser Thr Lys Gly Pro Ser Val Phe Pro Leu Ala Pro Cys Ser Arg Ser

115                 120                 125

Thr Ser Glu Ser Thr Ala Ala Leu Gly Cys Leu Val Lys Asp Tyr Phe
    130                 135                 140

Pro Glu Pro Val Thr Val Ser Trp Asn Ser Gly Ala Leu Thr Ser Gly
145                 150                 155                 160

Val His Thr Phe Pro Ala Val Leu Gln Ser Ser Gly Leu Tyr Ser Leu
                165                 170                 175

Ser Ser Val Val Thr Val Pro Ser Ser Ser Leu Gly Thr Lys Thr Tyr
            180                 185                 190

Thr Cys Asn Val Asp His Lys Pro Ser Asn Thr Lys Val Asp Lys Arg
        195                 200                 205

Val Gly Gly Gly Gly Ser Gly Gly Gly Gly Ser Gly Gly Gly Gly Ser
    210                 215                 220

Gln Val Gln Leu Val Gln Ser Gly Ala Glu Val Lys Lys Pro Gly Ala
225                 230                 235                 240

Ser Val Lys Val Ser Cys Lys Gly Ser Gly Tyr Thr Phe Thr Asp Tyr
                245                 250                 255

Ala Ile His Trp Val Arg Gln Ala Pro Gly Gln Ser Leu Glu Trp Ile
            260                 265                 270

Gly Val Phe Ser Ile Tyr Tyr Glu Asn Ile Asn Tyr Asn Gln Lys Phe
        275                 280                 285

Lys Gly Arg Ala Thr Met Thr Val Asp Lys Ser Thr Ser Thr Ala Tyr
    290                 295                 300

Met Glu Leu Arg Ser Leu Arg Ser Asp Asp Thr Ala Val Tyr Tyr Cys
305                 310                 315                 320

Ala Arg Arg Asp Gly Gly Thr Ile Asn Tyr Trp Gly Gln Gly Thr Leu
                325                 330                 335

Val Thr Val Ser Ser Ala Ser Thr Lys Gly Pro Ser Val Phe Pro Leu
            340                 345                 350

Ala Pro Ser Ser Lys Ser Thr Ser Gly Gly Thr Ala Ala Leu Gly Cys
        355                 360                 365

Leu Val Lys Asp Tyr Phe Pro Glu Pro Val Thr Val Ser Trp Asn Ser
    370                 375                 380

Gly Ala Leu Thr Ser Gly Val His Thr Phe Pro Ala Val Leu Gln Ser
385                 390                 395                 400

Ser Gly Leu Tyr Ser Leu Ser Ser Val Val Thr Val Pro Ser Ser Ser
                405                 410                 415

Leu Gly Thr Gln Thr Tyr Ile Cys Asn Val Asn His Lys Pro Ser Asn
            420                 425                 430

Thr Lys Val Asp Lys Lys Val Glu Pro Lys Ser Cys Asp Lys Thr His
        435                 440                 445

Thr Cys Pro Pro Cys Pro Ala Pro Glu Ala Ala Gly Gly Pro Ser Val
    450                 455                 460

Phe Leu Phe Pro Pro Lys Pro Lys Asp Thr Leu Met Ile Ser Arg Thr
465                 470                 475                 480

Pro Glu Val Thr Cys Val Val Val Asp Val Ser His Glu Asp Pro Glu
                485                 490                 495

Val Lys Phe Asn Trp Tyr Val Asp Gly Val Glu Val His Asn Ala Lys
            500                 505                 510

Thr Lys Pro Arg Glu Glu Gln Tyr Asn Ser Thr Tyr Arg Val Val Ser
        515                 520                 525

Val Leu Thr Val Leu His Gln Asp Trp Leu Asn Gly Lys Glu Tyr Lys
    530                 535                 540

Cys Lys Val Ser Asn Lys Ala Leu Pro Ala Pro Ile Glu Lys Thr Ile
545                 550                 555                 560

Ser Lys Ala Lys Gly Gln Pro Arg Glu Pro Gln Val Tyr Thr Leu Pro
                565                 570                 575

Pro Ser Arg Glu Glu Met Thr Lys Asn Gln Val Ser Leu Thr Cys Leu
            580                 585                 590

Val Lys Gly Phe Tyr Pro Ser Asp Ile Ala Val Glu Trp Glu Ser Asn
        595                 600                 605

Gly Gln Pro Glu Asn Asn Tyr Lys Thr Thr Pro Pro Val Leu Asp Ser
    610                 615                 620

Asp Gly Ser Phe Phe Leu Tyr Ser Lys Leu Thr Val Asp Lys Ser Arg
625                 630                 635                 640

Trp Gln Gln Gly Asn Val Phe Ser Cys Ser Val Met His Glu Ala Leu
                645                 650                 655

His Asn His Tyr Thr Gln Lys Ser Leu Ser Leu Ser Pro Gly Lys
            660                 665                 670

<210>  80
<211>  2013
<212>  DNA
<213>  Homo

<400>  80
caggtgcagc tgcaggaatc tggcccaggc ctggtgaagc cttctgagac actgtctttg      60

acctgtacag tgtctggctt ttctctgtct tcttatgata tttcctggat tagacagcct     120

cctggcaagg gcctggagtg gctgggagtg atttggacag gaggcggaac aaattataac     180

tctgctttta tgtctaggct gaccatctct aaggataata gtaaatctca ggtgtctctg     240

aagctgtctt ctgtgaccgc tgctgataca gccgtgtact attgtgttag aatggattat     300

tggggccagg gcacactggt gaccgtgtct tctgcaagta ccaagggacc tagtgttttc     360

cctcttgcac cttgctccag gtcaacatca gagtccacag ctgctcttgg atgtctcgtt     420

aaggactact tcccagagcc agttaccgta tcctggaact ccggagcttt gacaagcggc     480

gttcatacat tcccagctgt gttgcagagt tctgggttgt acagtttgag ctcagtggtg     540

accgtgcctt catcttcttt gggcactaag acctacacct gcaacgtgga tcacaagcca     600

agcaacacca aggtggataa gagggtgggt ggaggcggtt caggcggagg tggcagcgga     660

ggtggcggga gtcaagttca gctggtgcag agcggagctg aggtgaagaa gcccggcgcc     720

agcgtgaagg tgagctgtaa gggcagcggc tacaccttca ccgactacgc catccactgg     780

gtgagacaag ctcccggtca gtctttagaa tggatcggcg tgttcagcat ctactacgag     840

aacatcaact ataaccagaa gttcaagggt cgtgccacca tgaccgtgga caagagcacc     900

agcaccgcct acatggagct gaggtcttta aggagcgacg acaccgccgt gtactactgc     960

gctcgtaggg acggcggcac catcaactac tggggccaag gtactttagt gacagtgagc    1020

agcgcgagca ccaagggacc ttccgtgttt cccctcgccc ccagctccaa aagcaccagc    1080

ggcggaacag ctgctctcgg ctgtctcgtc aaggattact tccccgagcc cgtgaccgtg    1140

agctggaaca gcggagccct gacaagcggc gtccacacct tccctgctgt cctacagtcc    1200

tccggactgt acagcctgag cagcgtggtg acagtcccta gcagctccct gggcacccag    1260

acatatattt gcaacgtgaa tcacaagccc agcaacacca aggtcgataa gaaggtggag    1320

cctaagtcct gcgacaagac ccacacatgt cccccctgtc ccgctcctga agctgctgga 1380

ggcccttccg tgttcctgtt cccccctaag cccaaggaca ccctgatgat ttccaggaca 1440

cccgaggtga cctgtgtggt ggtggacgtc agccacgagg accccgaggt gaaattcaac 1500

tggtacgtcg atggcgtgga ggtgcacaac gctaagacca agcccaggga ggagcagtac 1560

aattccacct acagggtggt gtccgtgctg accgtcctcc atcaggactg gctgaacggc 1620

aaagagtata agtgcaaggt gagcaacaag gccctccctg ctcccatcga gaagaccatc 1680

agcaaagcca agggccagcc cagggaacct caagtctata ccctgcctcc cagcagggag 1740

gagatgacca agaaccaagt gagcctcaca tgcctcgtca agggcttcta tccttccgat 1800

attgccgtcg agtgggagtc caacggacag cccgagaaca actacaagac aacacccccc 1860

gtgctcgatt ccgatggcag cttcttcctg tactccaagc tgaccgtgga caagtccaga 1920

tggcaacaag gcaacgtctt cagttgcagc gtcatgcatg aggccctcca caaccactac 1980

acccagaaga gcctctccct gagccctgga aag 2013

<210> 81
<211> 671
<212> PRT
<213> Homo

<400> 81

Gln Val Gln Leu Val Gln Ser Gly Ala Glu Val Lys Lys Pro Gly Ala
1               5                   10                  15

Ser Val Lys Val Ser Cys Lys Gly Ser Gly Tyr Thr Phe Thr Asp Tyr
            20                  25                  30

Ala Ile His Trp Val Arg Gln Ala Pro Gly Gln Ser Leu Glu Trp Ile
        35                  40                  45

Gly Val Phe Ser Ile Tyr Tyr Glu Asn Ile Asn Tyr Asn Gln Lys Phe
    50                  55                  60

Lys Gly Arg Ala Thr Met Thr Val Asp Lys Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Arg Asp Gly Gly Thr Ile Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Gln Glu Ser Gly Pro Gly
225                 230                 235                 240

Leu Val Lys Pro Ser Glu Thr Leu Ser Leu Thr Cys Thr Val Ser Gly
                245                 250                 255

Phe Ser Leu Ser Ser Tyr Asp Ile Ser Trp Ile Arg Gln Pro Pro Gly
            260                 265                 270

Lys Gly Leu Glu Trp Leu Gly Val Ile Trp Thr Gly Gly Gly Thr Asn
        275                 280                 285

Tyr Asn Ser Ala Phe Met Ser Arg Leu Thr Ile Ser Lys Asp Asn Ser
    290                 295                 300

Lys Ser Gln Val Ser Leu Lys Leu Ser Ser Val Thr Ala Ala Asp Thr
305                 310                 315                 320

Ala Val Tyr Tyr Cys Val Arg Met Asp Tyr Trp Gly Gln Gly Thr Leu
                325                 330                 335

Val Thr Val Ser Ser Ala Ser Thr Lys Gly Pro Ser Val Phe Pro Leu
            340                 345                 350

Ala Pro Ser Ser Lys Ser Thr Ser Gly Gly Thr Ala Ala Leu Gly Cys
        355                 360                 365

Leu Val Lys Asp Tyr Phe Pro Glu Pro Val Thr Val Ser Trp Asn Ser
    370                 375                 380

Gly Ala Leu Thr Ser Gly Val His Thr Phe Pro Ala Val Leu Gln Ser
385                 390                 395                 400

Ser Gly Leu Tyr Ser Leu Ser Ser Val Val Thr Val Pro Ser Ser Ser
                405                 410                 415

Leu Gly Thr Gln Thr Tyr Ile Cys Asn Val Asn His Lys Pro Ser Asn
            420                 425                 430

Thr Lys Val Asp Lys Lys Val Glu Pro Lys Ser Cys Asp Lys Thr His
        435                 440                 445

Thr Cys Pro Pro Cys Pro Ala Pro Glu Ala Ala Gly Gly Pro Ser Val
    450                 455                 460

Phe Leu Phe Pro Pro Lys Pro Lys Asp Thr Leu Met Ile Ser Arg Thr
465                 470                 475                 480

Pro Glu Val Thr Cys Val Val Val Asp Val Ser His Glu Asp Pro Glu
                485                 490                 495

Val Lys Phe Asn Trp Tyr Val Asp Gly Val Glu Val His Asn Ala Lys
            500                 505                 510

Thr Lys Pro Arg Glu Glu Gln Tyr Asn Ser Thr Tyr Arg Val Val Ser
        515                 520                 525

Val Leu Thr Val Leu His Gln Asp Trp Leu Asn Gly Lys Glu Tyr Lys
    530                 535                 540

Cys Lys Val Ser Asn Lys Ala Leu Pro Ala Pro Ile Glu Lys Thr Ile
545                 550                 555                 560

Ser Lys Ala Lys Gly Gln Pro Arg Glu Pro Gln Val Tyr Thr Leu Pro
                565                 570                 575

Pro Ser Arg Glu Glu Met Thr Lys Asn Gln Val Ser Leu Thr Cys Leu
            580                 585                 590

Val Lys Gly Phe Tyr Pro Ser Asp Ile Ala Val Glu Trp Glu Ser Asn
        595                 600                 605

Gly Gln Pro Glu Asn Asn Tyr Lys Thr Thr Pro Pro Val Leu Asp Ser
    610                 615                 620

Asp Gly Ser Phe Phe Leu Tyr Ser Lys Leu Thr Val Asp Lys Ser Arg

625                 630                 635                 640

Trp Gln Gln Gly Asn Val Phe Ser Cys Ser Val Met His Glu Ala Leu
                645                 650                 655

His Asn His Tyr Thr Gln Lys Ser Leu Ser Leu Ser Pro Gly Lys
            660                 665                 670

<210>  82
<211>  2013
<212>  DNA
<213>  Homo

<400>  82
caagttcagc tggtgcagag cggagctgag gtgaagaagc ccggcgccag cgtgaaggtg      60

agctgtaagg gcagcggcta caccttcacc gactacgcca tccactgggt gagacaagct     120

cccggtcagt ctttagaatg gatcggcgtg ttcagcatct actacgagaa catcaactat     180

aaccagaagt tcaagggtcg tgccaccatg accgtggaca agagcaccag caccgcctac     240

atggagctga ggtctttaag gagcgacgac accgccgtgt actactgcgc tcgtagggac     300

ggcggcacca tcaactactg gggccaaggt actttagtga cagtgagcag cgcaagtacc     360

aagggaccta gtgttttccc tcttgcacct tgctccaggt caacatcaga gtccacagct     420

gctcttggat gtctcgttaa ggactacttc ccagagccag ttaccgtatc ctggaactcc     480

ggagctttga caagcggcgt tcatacattc ccagctgtgt tgcagagttc tgggttgtac     540

agtttgagct cagtggtgac cgtgccttca tcttctttgg gcactaagac ctacacctgc     600

aacgtggatc acaagccaag caacaccaag gtggataaga gggtgggtgg aggcggttca     660

ggcggaggtg gcagcggagg tggcgggagt caggtgcagc tgcaggaatc tggcccaggc     720

ctggtgaagc cttctgagac actgtctttg acctgtacag tgtctggctt ttctctgtct     780

tcttatgata tttcctggat tagacagcct cctggcaagg gcctggagtg gctgggagtg     840

atttggacag gaggcggaac aaattataac tctgctttta tgtctaggct gaccatctct     900

aaggataata gtaaatctca ggtgtctctg aagctgtctt ctgtgaccgc tgctgataca     960

gccgtgtact attgtgttag aatggattat tggggccagg gcacactggt gaccgtgtct    1020

tctgcgagca ccaagggacc ttccgtgttt cccctcgccc ccagctccaa aagcaccagc    1080

ggcggaacag ctgctctcgg ctgtctcgtc aaggattact tccccgagcc cgtgaccgtg    1140

agctggaaca gcggagccct gacaagcggc gtccacacct tccctgctgt cctacagtcc    1200

tccggactgt acagcctgag cagcgtggtg acagtcccta gcagctccct gggcacccag    1260

acatatattt gcaacgtgaa tcacaagccc agcaacacca aggtcgataa gaaggtggag    1320

cctaagtcct gcgacaagac ccacacatgt cccccctgtc ccgctcctga agctgctgga    1380

ggcccttccg tgttcctgtt cccccctaag cccaaggaca ccctgatgat ttccaggaca 1440

cccgaggtga cctgtgtggt ggtggacgtc agccacgagg accccgaggt gaaattcaac 1500

tggtacgtcg atggcgtgga ggtgcacaac gctaagacca agcccaggga ggagcagtac 1560

aattccacct acagggtggt gtccgtgctg accgtcctcc atcaggactg gctgaacggc 1620

aaagagtata agtgcaaggt gagcaacaag gccctccctg ctcccatcga gaagaccatc 1680

agcaaagcca agggccagcc cagggaacct caagtctata ccctgcctcc cagcagggag 1740

gagatgacca agaaccaagt gagcctcaca tgcctcgtca agggcttcta tccttccgat 1800

attgccgtcg agtgggagtc caacggacag cccgagaaca actacaagac aacacccccc 1860

gtgctcgatt ccgatggcag cttcttcctg tactccaagc tgaccgtgga caagtccaga 1920

tggcaacaag gcaacgtctt cagttgcagc gtcatgcatg aggccctcca caaccactac 1980

acccagaaga gcctctccct gagccctgga aag 2013

<210> 83
<211> 117
<212> PRT
<213> Homo

<400> 83

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser
        115

<210> 84

<211>  107
<212>  PRT
<213>  Homo

<400>  84

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Ile Ser Asn Phe
            20                  25                  30

Leu His Trp Tyr Gln Gln Lys Pro Gly Gln Ala Pro Arg Leu Leu Ile
        35                  40                  45

Lys Tyr Ala Ser Gln Ser Ile Ser Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Phe Cys Gln Gln Ser Asn Ser Trp Pro His
                85                  90                  95

Thr Phe Gly Gln Gly Thr Lys Val Glu Ile Lys
            100                 105

<210>  85
<211>  5
<212>  PRT
<213>  Mus musculus

<400>  85

Ser Tyr Trp Met His
1               5

<210>  86
<211>  17
<212>  PRT
<213>  Mus musculus

<400>  86

Glu Ile Asn Pro Gly Asn Gly His Thr Asn Tyr Asn Glu Lys Phe Lys
1               5                   10                  15

Ser

<210>  87
<211>  10
<212>  PRT

<213>  Mus musculus

<400>  87

Ser Phe Thr Thr Ala Arg Gly Phe Ala Tyr
1               5                   10


<210>  88
<211>  11
<212>  PRT
<213>  Mus musculus

<400>  88

Arg Ala Ser Gln Thr Ile Ser Asp Tyr Leu His
1               5                   10


<210>  89
<211>  7
<212>  PRT
<213>  Mus musculus

<400>  89

Tyr Ala Ser Gln Ser Ile Ser
1               5


<210>  90
<211>  9
<212>  PRT
<213>  Mus musculus

<400>  90

Gln Asp Gly His Ser Phe Pro Pro Thr
1               5


<210>  91
<211>  119
<212>  PRT
<213>  Homo

<400>  91

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Ser Ser Tyr
            20                  25                  30


Trp Met His Trp Val Arg Gln Ala Pro Gly Gln Gly Leu Glu Trp Met
        35                  40                  45


Gly Glu Ile Asn Pro Gly Asn Gly His Thr Asn Tyr Asn Glu Lys Phe
    50                  55                  60

Lys Ser Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Ser Phe Thr Thr Ala Arg Gly Phe Ala Tyr Trp Gly Gln Gly
            100                 105                 110

Thr Leu Val Thr Val Ser Ser
        115

<210>  92
<211>  107
<212>  PRT
<213>  Homo

<400>  92

Glu Ile Val Met Thr Gln Ser Pro Asp Phe Gln Ser Val Thr Pro Lys
1               5                   10                  15

Glu Lys Val Thr Ile Thr Cys Arg Ala Ser Gln Thr Ile Ser Asp Tyr
            20                  25                  30

Leu His Trp Tyr Gln Gln Lys Pro Asp Gln Ser Pro Lys Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Ser Asp Phe Thr Leu Thr Ile Asn Ser Leu Glu Ala
65                  70                  75                  80

Glu Asp Ala Ala Thr Tyr Tyr Cys Gln Asp Gly His Ser Phe Pro Pro
                85                  90                  95

Thr Phe Gly Gln Gly Thr Lys Val Glu Ile Lys
            100                 105

<210>  93
<211>  214
<212>  PRT
<213>  Homo

<400>  93

Glu Ile Val Met Thr Gln Ser Pro Asp Phe Gln Ser Val Thr Pro Lys
1               5                   10                  15

Glu Lys Val Thr Ile Thr Cys Arg Ala Ser Gln Thr Ile Ser Asp Tyr
            20                  25                  30

Leu His Trp Tyr Gln Gln Lys Pro Asp Gln Ser Pro Lys Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Ser Asp Phe Thr Leu Thr Ile Asn Ser Leu Glu Ala
65                  70                  75                  80

Glu Asp Ala Ala Thr Tyr Tyr Cys Gln Asp Gly His Ser Phe Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  94
<211>  642
<212>  DNA
<213>  Homo

<400>  94
gagatcgtga tgacccagtc tcctgatttt cagtctgtga cccctaagga gaaggtgaca      60

atcacctgta gagcttctca gaccatctct gattatctgc attggtatca gcagaagcct     120

gatcagtctc ctaagctgct gatcaagtat gcttctcagt ctatctctgg cattccttct     180

aggttttccg gctctggctc tggctctgat tttacactga ccatcaattc tctggaggct 240

gaggatgctg ccacctatta ttgtcaggac ggccactctt ttcctcctac ctttggccag 300

ggaaccaagg tggagatcaa gagaaccgtc gccgctccca gcgtcttcat cttccccccc 360

agcgatgagc agctgaagag cggaaccgcc agcgtggtgt gcctgctgaa caacttctac 420

cccagggagg ccaaggtgca atggaaggtg gacaacgccc tacagagcgg caactcccag 480

gagagcgtga ccgagcagga cagcaaggat agcacctaca gcctgagcag caccctcacc 540

ctgagcaagg ccgactacga gaagcacaag gtgtacgcct gcgaggtgac ccatcagggc 600

ctgagcagcc ctgtgaccaa gagcttcaac aggggcgagt gc 642

<210> 95
<211> 676
<212> PRT
<213> Homo

<400> 95

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Val Gln Ser Gly Ala Glu
225                 230                 235                 240

Val Lys Lys Pro Gly Ala Ser Val Lys Val Ser Cys Lys Ala Ser Gly
                245                 250                 255

Tyr Thr Phe Ser Ser Tyr Trp Met His Trp Val Arg Gln Ala Pro Gly
            260                 265                 270

Gln Gly Leu Glu Trp Met Gly Glu Ile Asn Pro Gly Asn Gly His Thr
        275                 280                 285

Asn Tyr Asn Glu Lys Phe Lys Ser Arg Val Thr Leu Thr Val Asp Lys
    290                 295                 300

Ser Thr Ser Thr Val Tyr Met Glu Leu Ser Ser Leu Arg Ser Glu Asp
305                 310                 315                 320

Thr Ala Val Tyr Tyr Cys Ala Arg Ser Phe Thr Thr Ala Arg Gly Phe
                325                 330                 335

Ala Tyr Trp Gly Gln Gly Thr Leu Val Thr Val Ser Ser Ala Ser Thr
            340                 345                 350

Lys Gly Pro Ser Val Phe Pro Leu Ala Pro Cys Ser Arg Ser Thr Ser
        355                 360                 365

Glu Ser Thr Ala Ala Leu Gly Cys Leu Val Lys Asp Tyr Phe Pro Glu
    370                 375                 380

Pro Val Thr Val Ser Trp Asn Ser Gly Ala Leu Thr Ser Gly Val His
385                 390                 395                 400

Thr Phe Pro Ala Val Leu Gln Ser Ser Gly Leu Tyr Ser Leu Ser Ser

                405                 410                 415

Val Val Thr Val Pro Ser Ser Ser Leu Gly Thr Lys Thr Tyr Thr Cys
            420                 425                 430

Asn Val Asp His Lys Pro Ser Asn Thr Lys Val Asp Lys Arg Val Glu
        435                 440                 445

Ser Lys Tyr Gly Pro Pro Cys Pro Pro Cys Pro Ala Pro Glu Phe Leu
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

Gln Glu Asp Pro Glu Val Gln Phe Asn Trp Tyr Val Asp Gly Val Glu
            500                 505                 510

Val His Asn Ala Lys Thr Lys Pro Arg Glu Glu Gln Phe Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Gly Leu Pro Ser Ser
545                 550                 555                 560

Ile Glu Lys Thr Ile Ser Lys Ala Lys Gly Gln Pro Arg Glu Pro Gln
                565                 570                 575

Val Tyr Thr Leu Pro Pro Ser Gln Glu Glu Met Thr Lys Asn Gln Val
            580                 585                 590

Ser Leu Thr Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Arg Leu Thr
625                 630                 635                 640

Val Asp Lys Ser Arg Trp Gln Glu Gly Asn Val Phe Ser Cys Ser Val
                645                 650                 655

Met His Glu Ala Leu His Asn His Tyr Thr Gln Lys Ser Leu Ser Leu
            660                 665                 670

Ser Leu Gly Lys
        675

<210>  96
<211>  2028
<212>  DNA
<213>  Homo

<400>  96
gaggtcaagc tggtggaaag cggcggcggc ctggtgcagc ctggaggatc cctgcggctg      60

agctgcgctg cctccggctt cgctttcagc tcctatgaca tgtcctgggt gaggcaggcc     120

cctggaaaga ggctggagtg ggtggctacc atctccggag gcggaaggta cacctactac     180

cccgacacag tgaagggaag gttcaccatc agccgggata acgccaaaaa cagccactat     240

ctccagatga actccctgag ggccgaagat acagccgtgt atttctgtgc ctccccctac      300

ggaggctatt ttgacgtgtg gggacagggc accctggtga ccgtctcctc cgcaagtacc     360

aagggaccta gtgttttccc tcttgcacct tgctccaggt caacatcaga gtccacagct     420

gctcttggat gtctcgttaa ggactacttc ccagagccag ttaccgtatc ctggaactcc     480

ggagctttga caagcggcgt tcatacattc ccagctgtgt tgcagagttc tgggttgtac     540

agtttgagct cagtggtgac cgtgccttca tcttctttgg gcactaagac ctacacctgc     600

aacgtggatc acaagccaag caacaccaag gtggataaga gggtgggtgg aggcggttca     660

ggcggaggtg gcagcggagg tggcgggagt caggtgcagc tggtgcagag cggcgctgaa     720

gtgaagaagc caggcgcttc tgtaaaggtg tcttgtaagg cttctggcta tacattttct     780

tcttattgga tgcactgggt gagacaggct cctggccagg gactggagtg gatgggtgag     840

atcaatcctg gcaatggcca tactaactac aacgagaagt tcaagtccag ggtgaccctg     900

accgtggaca agtccacctc caccgtgtac atggagctga gctccctgag gtccgaggac     960

accgccgtgt actactgcgc ccggagcttc actaccgccc gcggcttcgc ctattggggc    1020

cagggcaccc tggtgaccgt gagctccgcc tccaccaagg gcccatcggt cttccccctg    1080

gcaccctgct ccaggagcac ctctgagtcc acagcggccc tgggctgcct ggtcaaggac    1140

tacttccccg aaccggtgac ggtgtcgtgg aactcaggcg ccctgaccag cggcgtgcac    1200

accttcccgg ctgtcctaca gtcctcagga ctctactccc tcagcagcgt ggtgaccgtg    1260

ccctccagca gcttgggcac caagacatat acctgtaatg tggatcacaa gccttccaat    1320

acaaaagtgg acaagagagt tgagtccaag tacggcccac catgtcctcc atgtccagcc    1380

cctgaatttt tgggcgggcc ttctgtcttt ctgtttcctc ctaaacctaa agataccctg    1440

atgatcagcc gcacacccga agtcacttgt gtggtcgtgg atgtgtctca ggaagatccc    1500

gaagtgcagt ttaactggta tgtcgatggc gtggaagtgc ataatgccaa aactaagccc 1560

cgcgaagaac agttcaacag cacttatcgg gtcgtgtctg tgctcacagt cctccatcag 1620

gattggctga atgggaaaga atataagtgc aaggtgagca ataagggcct ccccagcagc 1680

atcgagaaga ctattagcaa agccaaaggg cagccacggg aaccccaggt gtacactctg 1740

cccccctctc aggaggagat gactaaaaat caggtctctc tgacttgtct ggtgaaaggg 1800

ttttatccca gcgacattgc cgtggagtgg gagtctaatg gccagcccga gaataattat 1860

aagacaactc cccccgtcct ggactctgac ggcagctttt tcctgtattc tcggctgaca 1920

gtggacaaaa gtcgctggca ggagggcaat gtctttagtt gcagtgtcat gcatgaggcc 1980

ctgcacaatc actatacaca gaaaagcctg tctctgagtc tgggcaaa 2028

<210> 97
<211> 676
<212> PRT
<213> Homo

<400> 97

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Ser Tyr
            20                  25                  30

Trp Met His Trp Val Arg Gln Ala Pro Gly Gln Gly Leu Glu Trp Met
        35                  40                  45

Gly Glu Ile Asn Pro Gly Asn Gly His Thr Asn Tyr Asn Glu Lys Phe
    50                  55                  60

Lys Ser Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Ser Phe Thr Thr Ala Arg Gly Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Gly Gly Gly Gly Ser Gly Gly
    210                 215                 220

Gly Gly Ser Gly Gly Gly Gly Ser Glu Val Lys Leu Val Glu Ser Gly
225                 230                 235                 240

Gly Gly Leu Val Gln Pro Gly Gly Ser Leu Arg Leu Ser Cys Ala Ala
                245                 250                 255

Ser Gly Phe Ala Phe Ser Ser Tyr Asp Met Ser Trp Val Arg Gln Ala
            260                 265                 270

Pro Gly Lys Arg Leu Glu Trp Val Ala Thr Ile Ser Gly Gly Gly Arg
        275                 280                 285

Tyr Thr Tyr Tyr Pro Asp Thr Val Lys Gly Arg Phe Thr Ile Ser Arg
    290                 295                 300

Asp Asn Ala Lys Asn Ser His Tyr Leu Gln Met Asn Ser Leu Arg Ala
305                 310                 315                 320

Glu Asp Thr Ala Val Tyr Phe Cys Ala Ser Pro Tyr Gly Gly Tyr Phe
                325                 330                 335

Asp Val Trp Gly Gln Gly Thr Leu Val Thr Val Ser Ser Ala Ser Thr
            340                 345                 350

Lys Gly Pro Ser Val Phe Pro Leu Ala Pro Cys Ser Arg Ser Thr Ser
        355                 360                 365

Glu Ser Thr Ala Ala Leu Gly Cys Leu Val Lys Asp Tyr Phe Pro Glu
    370                 375                 380

Pro Val Thr Val Ser Trp Asn Ser Gly Ala Leu Thr Ser Gly Val His
385                 390                 395                 400

Thr Phe Pro Ala Val Leu Gln Ser Ser Gly Leu Tyr Ser Leu Ser Ser
                405                 410                 415

Val Val Thr Val Pro Ser Ser Ser Leu Gly Thr Lys Thr Tyr Thr Cys
            420                 425                 430

Asn Val Asp His Lys Pro Ser Asn Thr Lys Val Asp Lys Arg Val Glu
        435                 440                 445

Ser Lys Tyr Gly Pro Pro Cys Pro Pro Cys Pro Ala Pro Glu Phe Leu
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

Gln Glu Asp Pro Glu Val Gln Phe Asn Trp Tyr Val Asp Gly Val Glu
            500                 505                 510

Val His Asn Ala Lys Thr Lys Pro Arg Glu Glu Gln Phe Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Gly Leu Pro Ser Ser
545                 550                 555                 560

Ile Glu Lys Thr Ile Ser Lys Ala Lys Gly Gln Pro Arg Glu Pro Gln
                565                 570                 575

Val Tyr Thr Leu Pro Pro Ser Gln Glu Glu Met Thr Lys Asn Gln Val
            580                 585                 590

Ser Leu Thr Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Arg Leu Thr
625                 630                 635                 640

Val Asp Lys Ser Arg Trp Gln Glu Gly Asn Val Phe Ser Cys Ser Val
                645                 650                 655

Met His Glu Ala Leu His Asn His Tyr Thr Gln Lys Ser Leu Ser Leu
            660                 665                 670

Ser Leu Gly Lys
        675

<210>  98
<211>  2028
<212>  DNA
<213>  Homo

<400>  98
caggtgcagc tggtgcagag cggcgctgaa gtgaagaagc caggcgcttc tgtaaaggtg      60

tcttgtaagg cttctggcta tacattttct tcttattgga tgcactgggt gagacaggct     120

cctggccagg gactggagtg gatgggtgag atcaatcctg gcaatggcca tactaactac     180

aacgagaagt tcaagtccag ggtgaccctg accgtggaca agtccacctc caccgtgtac     240

atggagctga gctccctgag gtccgaggac accgccgtgt actactgcgc ccggagcttc     300

actaccgccc gcggcttcgc ctattggggc cagggcaccc tggtgaccgt gagctccgca     360

agtaccaagg gacctagtgt tttccctctt gcaccttgct ccaggtcaac atcagagtcc     420

acagctgctc ttggatgtct cgttaaggac tacttcccag agccagttac cgtatcctgg     480

aactccggag ctttgacaag cggcgttcat acattcccag ctgtgttgca gagttctggg     540

ttgtacagtt tgagctcagt ggtgaccgtg ccttcatctt ctttgggcac taagacctac     600

acctgcaacg tggatcacaa gccaagcaac accaaggtgg ataagagggt gggtggaggc     660

ggttcaggcg gaggtggcag cggaggtggc gggagtgagg tcaagctggt ggaaagcggc     720

ggcggcctgg tgcagcctgg aggatccctg cggctgagct gcgctgcctc cggcttcgct     780

ttcagctcct atgacatgtc ctgggtgagg caggcccctg gaaagaggct ggagtgggtg     840

gctaccatct ccggaggcgg aaggtacacc tactaccccg acacagtgaa gggaaggttc     900

accatcagcc gggataacgc caaaaacagc cactatctcc agatgaactc cctgagggcc     960

gaagatacag ccgtgtattt ctgtgcctcc ccctacggag gctattttga cgtgtgggga    1020

cagggcaccc tggtgaccgt ctcctccgcc tccaccaagg gcccatcggt cttccccctg    1080

gcaccctgct ccaggagcac ctctgagtcc acagcggccc tgggctgcct ggtcaaggac    1140

tacttccccg aaccggtgac ggtgtcgtgg aactcaggcg ccctgaccag cggcgtgcac    1200

accttcccgg ctgtcctaca gtcctcagga ctctactccc tcagcagcgt ggtgaccgtg    1260

ccctccagca gcttgggcac caagacatat acctgtaatg tggatcacaa gccttccaat    1320

acaaaagtgg acaagagagt tgagtccaag tacggcccac catgtcctcc atgtccagcc    1380

cctgaatttt tgggcgggcc ttctgtcttt ctgtttcctc ctaaacctaa agataccctg    1440

atgatcagcc gcacacccga agtcacttgt gtggtcgtgg atgtgtctca ggaagatccc 1500

gaagtgcagt ttaactggta tgtcgatggc gtggaagtgc ataatgccaa aactaagccc 1560

cgcgaagaac agttcaacag cacttatcgg gtcgtgtctg tgctcacagt cctccatcag 1620

gattggctga atgggaaaga atataagtgc aaggtgagca ataagggcct ccccagcagc 1680

atcgagaaga ctattagcaa agccaaaggg cagccacggg aaccccaggt gtacactctg 1740

cccccctctc aggaggagat gactaaaaat caggtctctc tgacttgtct ggtgaaaggg 1800

ttttatccca gcgacattgc cgtggagtgg gagtctaatg gccagcccga gaataattat 1860

aagacaactc cccccgtcct ggactctgac ggcagctttt tcctgtattc tcggctgaca 1920

gtggacaaaa gtcgctggca ggagggcaat gtctttagtt gcagtgtcat gcatgaggcc 1980

ctgcacaatc actatacaca gaaaagcctg tctctgagtc tgggcaaa 2028

<210> 99
<211> 5
<212> PRT
<213> Mus musculus

<400> 99

Thr Thr Gly Met Gln
1               5

<210> 100
<211> 17
<212> PRT
<213> Mus musculus

<400> 100

Trp Ile Asn Thr His Ser Gly Val Pro Lys Tyr Val Glu Asp Phe Lys
1               5                   10                  15

Gly

<210> 101
<211> 13
<212> PRT
<213> Mus musculus

<400> 101

Ser Gly Asn Gly Asn Tyr Asp Leu Ala Tyr Phe Ala Tyr
1               5                   10

<210> 102
<211> 11
<212> PRT
<213> Mus musculus

<400>  102

Arg Ala Ser Gln Ser Ile Ser Asp Tyr Leu His
1               5                   10


<210>  103
<211>  7
<212>  PRT
<213>  Mus musculus

<400>  103

Tyr Ala Ser His Ser Ile Ser
1               5


<210>  104
<211>  9
<212>  PRT
<213>  Mus musculus

<400>  104

Gln His Gly His Ser Phe Pro Trp Thr
1               5


<210>  105
<211>  122
<212>  PRT
<213>  Homo

<400>  105

Gln Ile Gln Leu Val Gln Ser Gly Ser Glu Leu Lys Lys Pro Gly Ala
1               5                   10                  15


Ser Val Lys Val Ser Cys Lys Ala Ser Gly Tyr Ala Phe Thr Thr Thr
            20                  25                  30


Gly Met Gln Trp Val Arg Glu Ala Pro Gly Gln Gly Leu Glu Trp Ile
        35                  40                  45


Gly Trp Ile Asn Thr His Ser Gly Val Pro Lys Tyr Val Glu Asp Phe
    50                  55                  60


Lys Gly Arg Phe Val Phe Ser Leu Asp Thr Ser Val Asn Thr Ala Tyr
65                  70                  75                  80


Leu Gln Ile Ser Ser Leu Lys Ala Glu Asp Thr Ala Val Tyr Tyr Cys
                85                  90                  95


Val Arg Ser Gly Asn Gly Asn Tyr Asp Leu Ala Tyr Phe Ala Tyr Trp
            100                 105                 110

Gly Gln Gly Thr Leu Val Thr Val Ser Ser
        115                 120

<210>  106
<211>  107
<212>  PRT
<213>  Homo

<400>  106

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Ile Ser Asp Tyr
            20                  25                  30

Leu His Trp Tyr Gln Gln Lys Pro Gly Gln Ser Pro Arg Leu Leu Ile
        35                  40                  45

Lys Tyr Ala Ser His Ser Ile Ser Gly Ile Pro Ala Arg Phe Ser Gly
    50                  55                  60

Ser Gly Ser Gly Ser Asp Phe Thr Leu Thr Ile Ser Ser Leu Glu Pro
65                  70                  75                  80

Glu Asp Phe Ala Val Tyr Tyr Cys Gln His Gly His Ser Phe Pro Trp
                85                  90                  95

Thr Phe Gly Gln Gly Thr Lys Val Glu Ile Lys
            100                 105

<210>  107
<211>  214
<212>  PRT
<213>  Homo

<400>  107

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Ile Ser Asp Tyr
            20                  25                  30

Leu His Trp Tyr Gln Gln Lys Pro Gly Gln Ser Pro Arg Leu Leu Ile
        35                  40                  45

Lys Tyr Ala Ser His Ser Ile Ser Gly Ile Pro Ala Arg Phe Ser Gly
    50                  55                  60

Ser Gly Ser Gly Ser Asp Phe Thr Leu Thr Ile Ser Ser Leu Glu Pro
65                  70                  75                  80

Glu Asp Phe Ala Val Tyr Tyr Cys Gln His Gly His Ser Phe Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  108
<211>  642
<212>  DNA
<213>  Homo

<400>  108
gaaatcgtgc tgacacagtc tcctgctaca ctgtctctga gtcctggaga aagagctaca      60

ctgtcttgta gagcttccca gtctatctcc gattatctgc actggtacca gcagaagcct     120

ggccagtctc ctagactgct gatcaagtat gcttctcatt ctatttctgg catcccagct     180

aggtttagtg gatctggctc tggaagcgac tttacactga ccatttcttc tctggagcct     240

gaggattttg ctgtgtatta ctgtcagcac ggccattctt ttccttggac ctttggccag     300

ggcacaaagg tggagatcaa gagaaccgtc gccgctccca gcgtcttcat cttccccccc     360

agcgatgagc agctgaagag cggaaccgcc agcgtggtgt gcctgctgaa caacttctac     420

cccagggagg ccaaggtgca atggaaggtg gacaacgccc tacagagcgg caactcccag     480

gagagcgtga ccgagcagga cagcaaggat agcacctaca gcctgagcag caccctcacc     540

ctgagcaagg ccgactacga gaagcacaag gtgtacgcct gcgaggtgac ccatcagggc 600

ctgagcagcc ctgtgaccaa gagcttcaac aggggcgagt gc 642

<210> 109
<211> 679
<212> PRT
<213> Homo

<400> 109

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Ile Gln Leu Val Gln Ser Gly Ser Glu
225                 230                 235                 240

Leu Lys Lys Pro Gly Ala Ser Val Lys Val Ser Cys Lys Ala Ser Gly
                245                 250                 255

Tyr Ala Phe Thr Thr Thr Gly Met Gln Trp Val Arg Glu Ala Pro Gly
            260                 265                 270

Gln Gly Leu Glu Trp Ile Gly Trp Ile Asn Thr His Ser Gly Val Pro
        275                 280                 285

Lys Tyr Val Glu Asp Phe Lys Gly Arg Phe Val Phe Ser Leu Asp Thr
    290                 295                 300

Ser Val Asn Thr Ala Tyr Leu Gln Ile Ser Ser Leu Lys Ala Glu Asp
305                 310                 315                 320

Thr Ala Val Tyr Tyr Cys Val Arg Ser Gly Asn Gly Asn Tyr Asp Leu
                325                 330                 335

Ala Tyr Phe Ala Tyr Trp Gly Gln Gly Thr Leu Val Thr Val Ser Ser
            340                 345                 350

Ala Ser Thr Lys Gly Pro Ser Val Phe Pro Leu Ala Pro Cys Ser Arg
        355                 360                 365

Ser Thr Ser Glu Ser Thr Ala Ala Leu Gly Cys Leu Val Lys Asp Tyr
    370                 375                 380

Phe Pro Glu Pro Val Thr Val Ser Trp Asn Ser Gly Ala Leu Thr Ser
385                 390                 395                 400

Gly Val His Thr Phe Pro Ala Val Leu Gln Ser Ser Gly Leu Tyr Ser
                405                 410                 415

Leu Ser Ser Val Val Thr Val Pro Ser Ser Ser Leu Gly Thr Lys Thr
            420                 425                 430

Tyr Thr Cys Asn Val Asp His Lys Pro Ser Asn Thr Lys Val Asp Lys
        435                 440                 445

Arg Val Glu Ser Lys Tyr Gly Pro Pro Cys Pro Pro Cys Pro Ala Pro

450                 455                 460

Glu Phe Leu Gly Gly Pro Ser Val Phe Leu Phe Pro Pro Lys Pro Lys
465                 470                 475                 480

Asp Thr Leu Met Ile Ser Arg Thr Pro Glu Val Thr Cys Val Val Val
                485                 490                 495

Asp Val Ser Gln Glu Asp Pro Glu Val Gln Phe Asn Trp Tyr Val Asp
            500                 505                 510

Gly Val Glu Val His Asn Ala Lys Thr Lys Pro Arg Glu Glu Gln Phe
        515                 520                 525

Asn Ser Thr Tyr Arg Val Val Ser Val Leu Thr Val Leu His Gln Asp
    530                 535                 540

Trp Leu Asn Gly Lys Glu Tyr Lys Cys Lys Val Ser Asn Lys Gly Leu
545                 550                 555                 560

Pro Ser Ser Ile Glu Lys Thr Ile Ser Lys Ala Lys Gly Gln Pro Arg
                565                 570                 575

Glu Pro Gln Val Tyr Thr Leu Pro Pro Ser Gln Glu Glu Met Thr Lys
            580                 585                 590

Asn Gln Val Ser Leu Thr Cys Leu Val Lys Gly Phe Tyr Pro Ser Asp
        595                 600                 605

Ile Ala Val Glu Trp Glu Ser Asn Gly Gln Pro Glu Asn Asn Tyr Lys
    610                 615                 620

Thr Thr Pro Pro Val Leu Asp Ser Asp Gly Ser Phe Phe Leu Tyr Ser
625                 630                 635                 640

Arg Leu Thr Val Asp Lys Ser Arg Trp Gln Glu Gly Asn Val Phe Ser
                645                 650                 655

Cys Ser Val Met His Glu Ala Leu His Asn His Tyr Thr Gln Lys Ser
            660                 665                 670

Leu Ser Leu Ser Leu Gly Lys
        675

<210>  110
<211>  2037
<212>  DNA
<213>  Homo

<400>  110
gaggtcaagc tggtggaaag cggcggcggc ctggtgcagc ctggaggatc cctgcggctg      60

agctgcgctg cctccggctt cgctttcagc tcctatgaca tgtcctgggt gaggcaggcc     120

cctggaaaga ggctggagtg ggtggctacc atctccggag gcggaaggta cacctactac     180

cccgacacag tgaagggaag gttcaccatc agccgggata acgccaaaaa cagccactat     240

ctccagatga actccctgag ggccgaagat acagccgtgt atttctgtgc ctccccctac     300

ggaggctatt ttgacgtgtg gggacagggc accctggtga ccgtctcctc cgcaagtacc     360

aagggaccta gtgttttccc tcttgcacct tgctccaggt caacatcaga gtccacagct     420

gctcttggat gtctcgttaa ggactacttc ccagagccag ttaccgtatc ctggaactcc     480

ggagctttga caagcggcgt tcatacattc ccagctgtgt tgcagagttc tgggttgtac     540

agtttgagct cagtggtgac cgtgccttca tcttctttgg gcactaagac ctacacctgc     600

aacgtggatc acaagccaag caacaccaag gtggataaga gggtgggtgg aggcggttca     660

ggcggaggtg gcagcggagg tggcgggagt cagatccagc tggtgcagtc cggctccgag     720

ctgaagaagc ccggcgcctc cgtcaaggtg tcctgcaagg ccagtggcta cgcctttacc     780

accaccggca tgcagtgggt gagggaggcc ccaggccagg gcctggagtg gatcggctgg     840

atcaacacac attctggagt gcctaagtat gtggaagact ttaagggcag attcgtgttt     900

tctctggata cctctgtgaa taccgcttac ctgcagattt cttctctgaa ggctgaggac     960

acagctgtgt attactgtgt gagatctgga aatggaaact atgatctggc ttattttgcc    1020

tattggggcc agggcacact ggtgaccgtg tcttctgcct ccaccaaggg cccatcggtc    1080

ttccccctgg caccctgctc caggagcacc tctgagtcca cagcggccct gggctgcctg    1140

gtcaaggact acttccccga accggtgacg gtgtcgtgga actcaggcgc cctgaccagc    1200

ggcgtgcaca ccttcccggc tgtcctacag tcctcaggac tctactccct cagcagcgtg    1260

gtgaccgtgc cctccagcag cttgggcacc aagacatata cctgtaatgt ggatcacaag    1320

ccttccaata caaaagtgga caagagagtt gagtccaagt acggcccacc atgtcctcca    1380

tgtccagccc ctgaattttt gggcgggcct tctgtctttc tgtttcctcc taaacctaaa    1440

gataccctga tgatcagccg cacacccgaa gtcacttgtg tggtcgtgga tgtgtctcag    1500

gaagatcccg aagtgcagtt taactggtat gtcgatggcg tggaagtgca taatgccaaa    1560

actaagcccc gcgaagaaca gttcaacagc acttatcggg tcgtgtctgt gctcacagtc    1620

ctccatcagg attggctgaa tgggaaagaa tataagtgca aggtgagcaa taagggcctc    1680

cccagcagca tcgagaagac tattagcaaa gccaaagggc agccacggga accccaggtg    1740

tacactctgc ccccctctca ggaggagatg actaaaaatc aggtctctct gacttgtctg    1800

gtgaaagggt tttatcccag cgacattgcc gtggagtggg agtctaatgg ccagcccgag    1860

aataattata agacaactcc ccccgtcctg gactctgacg gcagcttttt cctgtattct 1920

cggctgacag tggacaaaag tcgctggcag gagggcaatg tctttagttg cagtgtcatg 1980

catgaggccc tgcacaatca ctatacacag aaaagcctgt ctctgagtct gggcaaa 2037

<210> 111
<211> 679
<212> PRT
<213> Homo

<400> 111

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Ala Phe Thr Thr Thr
            20                  25                  30

Gly Met Gln Trp Val Arg Glu Ala Pro Gly Gln Gly Leu Glu Trp Ile
        35                  40                  45

Gly Trp Ile Asn Thr His Ser Gly Val Pro Lys Tyr Val Glu Asp Phe
    50                  55                  60

Lys Gly Arg Phe Val Phe Ser Leu Asp Thr Ser Val Asn Thr Ala Tyr
65                  70                  75                  80

Leu Gln Ile Ser Ser Leu Lys Ala Glu Asp Thr Ala Val Tyr Tyr Cys
                85                  90                  95

Val Arg Ser Gly Asn Gly Asn Tyr Asp Leu Ala Tyr Phe Ala Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Gly Gly Gly Gly
    210                 215                 220

Ser Gly Gly Gly Gly Ser Gly Gly Gly Gly Ser Glu Val Lys Leu Val
225                 230                 235                 240

Glu Ser Gly Gly Gly Leu Val Gln Pro Gly Gly Ser Leu Arg Leu Ser
                245                 250                 255

Cys Ala Ala Ser Gly Phe Ala Phe Ser Ser Tyr Asp Met Ser Trp Val
            260                 265                 270

Arg Gln Ala Pro Gly Lys Arg Leu Glu Trp Val Ala Thr Ile Ser Gly
        275                 280                 285

Gly Gly Arg Tyr Thr Tyr Tyr Pro Asp Thr Val Lys Gly Arg Phe Thr
    290                 295                 300

Ile Ser Arg Asp Asn Ala Lys Asn Ser His Tyr Leu Gln Met Asn Ser
305                 310                 315                 320

Leu Arg Ala Glu Asp Thr Ala Val Tyr Phe Cys Ala Ser Pro Tyr Gly
                325                 330                 335

Gly Tyr Phe Asp Val Trp Gly Gln Gly Thr Leu Val Thr Val Ser Ser
            340                 345                 350

Ala Ser Thr Lys Gly Pro Ser Val Phe Pro Leu Ala Pro Cys Ser Arg
        355                 360                 365

Ser Thr Ser Glu Ser Thr Ala Ala Leu Gly Cys Leu Val Lys Asp Tyr
    370                 375                 380

Phe Pro Glu Pro Val Thr Val Ser Trp Asn Ser Gly Ala Leu Thr Ser
385                 390                 395                 400

Gly Val His Thr Phe Pro Ala Val Leu Gln Ser Ser Gly Leu Tyr Ser
                405                 410                 415

Leu Ser Ser Val Val Thr Val Pro Ser Ser Ser Leu Gly Thr Lys Thr
            420                 425                 430

Tyr Thr Cys Asn Val Asp His Lys Pro Ser Asn Thr Lys Val Asp Lys
        435                 440                 445

Arg Val Glu Ser Lys Tyr Gly Pro Pro Cys Pro Pro Cys Pro Ala Pro
    450                 455                 460

Glu Phe Leu Gly Gly Pro Ser Val Phe Leu Phe Pro Pro Lys Pro Lys
465                 470                 475                 480

Asp Thr Leu Met Ile Ser Arg Thr Pro Glu Val Thr Cys Val Val Val
                485                 490                 495

Asp Val Ser Gln Glu Asp Pro Glu Val Gln Phe Asn Trp Tyr Val Asp
            500                 505                 510

Gly Val Glu Val His Asn Ala Lys Thr Lys Pro Arg Glu Glu Gln Phe
        515                 520                 525

Asn Ser Thr Tyr Arg Val Val Ser Val Leu Thr Val Leu His Gln Asp
    530                 535                 540

Trp Leu Asn Gly Lys Glu Tyr Lys Cys Lys Val Ser Asn Lys Gly Leu
545                 550                 555                 560

Pro Ser Ser Ile Glu Lys Thr Ile Ser Lys Ala Lys Gly Gln Pro Arg
                565                 570                 575

Glu Pro Gln Val Tyr Thr Leu Pro Pro Ser Gln Glu Glu Met Thr Lys
            580                 585                 590

Asn Gln Val Ser Leu Thr Cys Leu Val Lys Gly Phe Tyr Pro Ser Asp
        595                 600                 605

Ile Ala Val Glu Trp Glu Ser Asn Gly Gln Pro Glu Asn Asn Tyr Lys
    610                 615                 620

Thr Thr Pro Pro Val Leu Asp Ser Asp Gly Ser Phe Phe Leu Tyr Ser
625                 630                 635                 640

Arg Leu Thr Val Asp Lys Ser Arg Trp Gln Glu Gly Asn Val Phe Ser
                645                 650                 655

Cys Ser Val Met His Glu Ala Leu His Asn His Tyr Thr Gln Lys Ser
            660                 665                 670

Leu Ser Leu Ser Leu Gly Lys
        675

<210>  112
<211>  2037

<212>  DNA
<213>  Homo

<400>  112
cagatccagc tggtgcagtc cggctccgag ctgaagaagc ccggcgcctc cgtcaaggtg      60

tcctgcaagg ccagtggcta cgcctttacc accaccggca tgcagtgggt gagggaggcc     120

ccaggccagg gcctggagtg gatcggctgg atcaacacac attctggagt gcctaagtat     180

gtggaagact ttaagggcag attcgtgttt tctctggata cctctgtgaa taccgcttac     240

ctgcagattt cttctctgaa ggctgaggac acagctgtgt attactgtgt gagatctgga     300

aatggaaact atgatctggc ttattttgcc tattggggcc agggcacact ggtgaccgtg     360

tcttctgcaa gtaccaaggg acctagtgtt ttccctcttg caccttgctc caggtcaaca     420

tcagagtcca cagctgctct tggatgtctc gttaaggact acttcccaga gccagttacc     480

gtatcctgga actccggagc tttgacaagc ggcgttcata cattcccagc tgtgttgcag     540

agttctgggt tgtacagttt gagctcagtg gtgaccgtgc cttcatcttc tttgggcact     600

aagacctaca cctgcaacgt ggatcacaag ccaagcaaca ccaaggtgga taagagggtg     660

ggtggaggcg gttcaggcgg aggtggcagc ggaggtggcg ggagtgaggt caagctggtg     720

gaaagcggcg gcggcctggt gcagcctgga ggatccctgc ggctgagctg cgctgcctcc     780

ggcttcgctt tcagctccta tgacatgtcc tgggtgaggc aggcccctgg aaagaggctg     840

gagtgggtgg ctaccatctc cggaggcgga aggtacacct actaccccga cacagtgaag     900

ggaaggttca ccatcagccg ggataacgcc aaaaacagcc actatctcca gatgaactcc     960

ctgagggccg aagatacagc cgtgtatttc tgtgcctccc cctacggagg ctattttgac    1020

gtgtggggac agggcaccct ggtgaccgtc tcctccgcct ccaccaaggg cccatcggtc    1080

ttccccctgg caccctgctc caggagcacc tctgagtcca cagcggccct gggctgcctg    1140

gtcaaggact acttccccga accggtgacg gtgtcgtgga actcaggcgc cctgaccagc    1200

ggcgtgcaca ccttcccggc tgtcctacag tcctcaggac tctactccct cagcagcgtg    1260

gtgaccgtgc cctccagcag cttgggcacc aagacatata cctgtaatgt ggatcacaag    1320

ccttccaata caaaagtgga caagagagtt gagtccaagt acggcccacc atgtcctcca    1380

tgtccagccc ctgaattttt gggcgggcct tctgtctttc tgtttcctcc taaacctaaa    1440

gataccctga tgatcagccg cacacccgaa gtcacttgtg tggtcgtgga tgtgtctcag    1500

gaagatcccg aagtgcagtt taactggtat gtcgatggcg tggaagtgca taatgccaaa    1560

actaagcccc gcgaagaaca gttcaacagc acttatcggg tcgtgtctgt gctcacagtc    1620

ctccatcagg attggctgaa tgggaaagaa tataagtgca aggtgagcaa taagggcctc    1680

cccagcagca tcgagaagac tattagcaaa gccaaaggcc agccacggga accccaggtg    1740

tacactctgc ccccctctca ggaggagatg actaaaaatc aggtctctct gacttgtctg    1800

gtgaaagggt tttatcccag cgacattgcc gtggagtggg agtctaatgg ccagcccgag 1860

aataattata agacaactcc ccccgtcctg gactctgacg gcagcttttt cctgtattct 1920

cggctgacag tggacaaaag tcgctggcag gagggcaatg tctttagttg cagtgtcatg 1980

catgaggccc tgcacaatca ctatacacag aaaagcctgt ctctgagtct gggcaaa 2037

<210> 113
<211> 119
<212> PRT
<213> Homo

<400> 113

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ala
        115

<210> 114
<211> 107
<212> PRT
<213> Homo

<400> 114

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys
            100                 105

<210>  115
<211>  120
<212>  PRT
<213>  Homo

<400>  115

<210>  116
<211>  107
<212>  PRT

<213>  Homo

<400>  116


<210>  117
<211>  119
<212>  PRT
<213>  Homo

<400>  117

<210> 118
<211> 107
<212> PRT
<213> Homo

<400> 118

<210> 119
<211> 118
<212> PRT
<213> Homo

<400> 119

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Thr Met His Trp Val Arg Gln Ala Pro Gly Lys Gly Leu Glu Trp Val
        35                  40                  45

Thr Phe Ile Ser Tyr Asp Gly Asn Asn Lys Tyr Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Thr Gly Trp Leu Gly Pro Phe Asp Tyr Trp Gly Gln Gly Thr
            100                 105                 110

Leu Val Thr Val Ser Ser
        115

<210>  120
<211>  108
<212>  PRT
<213>  Homo

<400>  120

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Gly Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Phe Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys
            100                 105

<210>  121
<211>  116
<212>  PRT
<213>  Homo

<400>  121

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Asp
            20                  25                  30

Tyr Met Ala Trp Phe Arg Gln Ala Pro Gly Lys Arg Leu Glu Trp Val
        35                  40                  45

Ala Ser Ile Ser His Gly Gly Asp Tyr Ile Tyr Tyr Ala Asp Asn Leu
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ser Arg Asp Arg Arg Ser Ile Asp Tyr Trp Gly Gln Gly Thr Leu Val
            100                 105                 110

Thr Val Ser Ser
        115

<210>  122
<211>  107
<212>  PRT
<213>  Homo

<400>  122

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Ser Trp Tyr Gln Gln Lys Pro Gly Lys Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Phe Cys Gln Gln Gly Lys Thr Leu Pro Tyr
                85                  90                  95

Thr Phe Gly Gln Gly Thr Lys Val Glu Ile Lys
            100                 105

<210>  123
<211>  214
<212>  PRT
<213>  Homo

<400>  123

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  124
<211>  642
<212>  DNA
<213>  Homo

<400>  124
gacatcctgc tgacccagtc ccccgtgatc ctgtccgtgt cccctggcga gagggtgtcc        60

ttctcctgca gggcctccca gtccatcggc accaacatcc actggtacca gcagaggacc       120

aacggctccc ccaggctgct gatcaagtac gcctccgaga gcatcagcgg catcccctcc       180

aggttttccg gctccggctc cggaaccgac ttcaccctgt ccatcaactc cgtggagtcc       240

gaggacatcg ccgactacta ctgccagcag aacaacaact ggcccaccac ctttggcgcc       300

ggcaccaagc tggagctgaa gaggaccgtg gccgccccct ccgtgttcat ttttccccct       360

agcgacgagc agctgaagag cggcaccgct agcgtggtgt gcctgctgaa caacttctac       420

cccagggagg ccaaggtgca gtggaaagtg gacaacgccc tgcagagcgg caactcccag       480

gagtccgtga ccgagcagga cagcaaggac agcacctact ccctgtcctc caccctgacc       540

ctgtccaagg ccgactacga gaagcacaag gtgtacgcct gtgaggtgac acaccagggc       600

ctgtcctccc ccgtgacaaa gtccttcaac aggggcgagt gc                          642

<210>  125
<211>  214
<212>  PRT
<213>  Homo

<400>  125

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Gly
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Arg Thr Val Ala Ala

            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 126
<211> 642
<212> DNA
<213> Homo

<400> 126
gacatccaga tgacccagtc tccttcttct ctgtctgctt ctgtgggcga tagagtgacc 60

atcacctgta aggcttctca ggatgtgtct atcggcgtgg cctggtatca gcagaagcct 120

ggaaaggctc ctaagctgct gatctactct gcttcttaca gatatactgg cgtgccttct 180

agattttctg gctctggctc tggcacagat tttacactga ccatctcttc tctgcagcct 240

gaggacttcg ctacctatta ctgtcagcag tactacatct atccttatac ctttggccag 300

ggcaccaagg tggagatcaa gagaaccgtc gccgctccca gcgtcttcat cttccccccc 360

agcgatgagc agctgaagag cggaaccgcc agcgtggtgt gcctgctgaa caacttctac 420

cccagggagg ccaaggtgca atggaaggtg gacaacgccc tacagagcgg caactcccag 480

gagagcgtga ccgagcagga cagcaaggat agcacctaca gcctgagcag caccctcacc 540

ctgagcaagg ccgactacga gaagcacaag gtgtacgcct gcgaggtgac ccatcagggc 600

ctgagcagcc ctgtgaccaa gagcttcaac aggggcgagt gc 642

<210> 127
<211> 215
<212> PRT

<213>  Homo

<400>  127

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Gly Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Phe Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  128
<211>  645
<212>  DNA

<213>  Homo

<400>  128
gagatcgtgc tgactcagag ccccggcact ctgtctctga gccccggcga aagggccaca      60

ctgagctgta gggctagcca gagcgtgggc agcagctatc tggcttggta ccagcagaag      120

cccggccaag cccctagact gctgatctac ggcgccttct ctagggctac tggcatccca      180

gataggttta gcggcagcgg atccggcaca gacttcactc tgacaatctc taggctggag      240

ccagaagact tcgccgtgta ctattgccag cagtacggca gcagcccatg gactttcggc      300

caaggcacaa aggtcgagat caagagaacc gtcgccgctc ccagcgtctt catcttcccc      360

cccagcgatg agcagctgaa gagcggaacc gccagcgtgg tgtgcctgct gaacaacttc      420

taccccaggg aggccaaggt gcaatggaag gtggacaacg ccctacagag cggcaactcc      480

caggagagcg tgaccgagca ggacagcaag gatagcacct acagcctgag cagcaccctc      540

accctgagca aggccgacta cgagaagcac aaggtgtacg cctgcgaggt gacccatcag      600

ggcctgagca gccctgtgac caagagcttc aacaggggcg agtgc                      645

<210>  129
<211>  214
<212>  PRT
<213>  Homo

<400>  129

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Ser Trp Tyr Gln Gln Lys Pro Gly Lys Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Phe Cys Gln Gln Gly Lys Thr Leu Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  130
<211>  642
<212>  DNA
<213>  Homo

<400>  130
gacatccaga tgacccagtc cccttcctcc ctgtccgctt ccgtgggcga cagggtgacc      60

atcacctgca gggccagcca ggacatctcc aactacctgt cctggtacca gcagaagccc     120

ggcaagacca tcaagctgct gatctactac acctccaggc tgcacagcgg cgtgcctagc     180

aggttctccg gttctggctc cggcaccgac tacaccttca ccatcagctc cctgcagccc     240

gaggacatcg ccacctactt ctgccagcag ggcaagaccc tgccctacac cttcggccag     300

ggcaccaagg tggagatcaa gcgcactgtg gctgccccca gtgttttcat atttcccccc     360

agtgatgagc aactgaagtc cggcacagcc tctgttgtat gtctgctgaa taatttttat     420

ccacgggagg ccaaggtgca gtggaaggtg gacaatgccc tgcagtctgg gaactctcaa     480

gagagtgtga cagagcagga cagtaaagac agcacctata gcctcagcag caccctgacc     540

ctgtctaaag ccgactatga aaaacacaaa gtgtatgcct gcgaagtgac ccatcagggg     600

ctcagctctc ccgttaccaa gagctttaac cgaggcgaat gt                        642

<210>  131
<211>  676
<212>  PRT
<213>  Homo

<400>  131

Glu Val Lys Leu Val Glu Ser Gly Gly Gly Leu Val Gln Pro Gly Gly

1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Lys Gln Ser Gly Pro Gly
225                 230                 235                 240

Leu Val Gln Pro Ser Gln Ser Leu Ser Ile Thr Cys Thr Val Ser Gly
                245                 250                 255

Phe Ser Leu Thr Asn Tyr Gly Val His Trp Val Arg Gln Ser Pro Gly
            260                 265                 270

Lys Gly Leu Glu Trp Leu Gly Val Ile Trp Ser Gly Gly Asn Thr Asp
        275                 280                 285

Tyr Asn Thr Pro Phe Thr Ser Arg Leu Ser Ile Asn Lys Asp Asn Ser
    290                 295                 300

Lys Ser Gln Val Phe Phe Lys Met Asn Ser Leu Gln Ser Asn Asp Thr
305                 310                 315                 320

Ala Ile Tyr Tyr Cys Ala Arg Ala Leu Thr Tyr Tyr Asp Tyr Glu Phe
                325                 330                 335

Ala Tyr Trp Gly Gln Gly Thr Leu Val Thr Val Ser Ala Ala Ser Thr
            340                 345                 350

Lys Gly Pro Ser Val Phe Pro Leu Ala Pro Cys Ser Arg Ser Thr Ser
        355                 360                 365

Glu Ser Thr Ala Ala Leu Gly Cys Leu Val Lys Asp Tyr Phe Pro Glu
    370                 375                 380

Pro Val Thr Val Ser Trp Asn Ser Gly Ala Leu Thr Ser Gly Val His
385                 390                 395                 400

Thr Phe Pro Ala Val Leu Gln Ser Ser Gly Leu Tyr Ser Leu Ser Ser
                405                 410                 415

Val Val Thr Val Pro Ser Ser Ser Leu Gly Thr Lys Thr Tyr Thr Cys
            420                 425                 430

Asn Val Asp His Lys Pro Ser Asn Thr Lys Val Asp Lys Arg Val Glu
        435                 440                 445

Ser Lys Tyr Gly Pro Pro Cys Pro Pro Cys Pro Ala Pro Glu Phe Leu
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

Gln Glu Asp Pro Glu Val Gln Phe Asn Trp Tyr Val Asp Gly Val Glu
            500                 505                 510

Val His Asn Ala Lys Thr Lys Pro Arg Glu Glu Gln Phe Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Gly Leu Pro Ser Ser
545                 550                 555                 560

Ile Glu Lys Thr Ile Ser Lys Ala Lys Gly Gln Pro Arg Glu Pro Gln
                565                 570                 575

Val Tyr Thr Leu Pro Pro Ser Gln Glu Glu Met Thr Lys Asn Gln Val
            580                 585                 590

Ser Leu Thr Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Arg Leu Thr
625                 630                 635                 640

Val Asp Lys Ser Arg Trp Gln Glu Gly Asn Val Phe Ser Cys Ser Val
                645                 650                 655

Met His Glu Ala Leu His Asn His Tyr Thr Gln Lys Ser Leu Ser Leu
            660                 665                 670

Ser Leu Gly Lys
        675

<210>  132
<211>  2028
<212>  DNA
<213>  Homo

<400>  132
gaggtcaagc tggtggaaag cggcggcggc ctggtgcagc ctggaggatc cctgcggctg      60

agctgcgctg cctccggctt cgctttcagc tcctatgaca tgtcctgggt gaggcaggcc     120

cctggaaaga ggctggagtg ggtggctacc atctccggag gcggaaggta cacctactac     180

cccgacacag tgaagggaag gttcaccatc agccgggata acgccaaaaa cagccactat     240

ctccagatga actccctgag ggccgaagat acagccgtgt atttctgtgc ctccccctac     300

ggaggctatt ttgacgtgtg gggacagggc accctggtga ccgtctcctc cgcaagtacc     360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt caggtgcagc tgaagcagtc cggacctggc 720

ctggtgcagc cttcccagtc cctgtccatc acctgcaccg tgtccggctt ttccctgacc 780

aactacggcg tgcactgggt gaggcagtcc cctggcaagg gcctggaatg gctgggcgtg 840

atctggtccg gcggcaacac cgactacaac acccccttca cctcccggct gtccatcaac 900

aaggacaaca gcaagtccca ggtgttcttc aagatgaact ccctgcagag caacgacacc 960

gccatctact actgcgccag agccctgacc tattacgact acgagttcgc ctactggggc 1020

cagggcacac tggtgaccgt gtccgccgcc tccaccaagg gcccatcggt cttccccctg 1080

gcaccctgct ccaggagcac ctctgagtcc acagcggccc tgggctgcct ggtcaaggac 1140

tacttccccg aaccggtgac ggtgtcgtgg aactcaggcg ccctgaccag cggcgtgcac 1200

accttcccgg ctgtcctaca gtcctcagga ctctactccc tcagcagcgt ggtgaccgtg 1260

ccctccagca gcttgggcac caagacatat acctgtaatg tggatcacaa gccttccaat 1320

acaaaagtgg acaagagagt tgagtccaag tacggcccac catgtcctcc atgtccagcc 1380

cctgaatttt tgggcgggcc ttctgtcttt ctgtttcctc ctaaacctaa agataccctg 1440

atgatcagcc gcacacccga agtcacttgt gtggtcgtgg atgtgtctca ggaagatccc 1500

gaagtgcagt ttaactggta tgtcgatggc gtggaagtgc ataatgccaa aactaagccc 1560

cgcgaagaac agttcaacag cacttatcgg gtcgtgtctg tgctcacagt cctccatcag 1620

gattggctga atgggaaaga atataagtgc aaggtgagca ataagggcct ccccagcagc 1680

atcgagaaga ctattagcaa agccaaaggg cagccacggg aaccccaggt gtacactctg 1740

cccccctctc aggaggagat gactaaaaat caggtctctc tgacttgtct ggtgaaaggg 1800

ttttatccca gcgacattgc cgtggagtgg gagtctaatg gccagcccga gaataattat 1860

aagacaactc cccccgtcct ggactctgac ggcagctttt tcctgtattc tcggctgaca 1920

gtggacaaaa gtcgctggca ggagggcaat gtctttagtt gcagtgtcat gcatgaggcc 1980

ctgcacaatc actatacaca gaaaagcctg tctctgagtc tgggcaaa 2028

<210> 133
<211> 676
<212> PRT
<213> Homo

<400> 133

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Glu Val Gln Leu Val Glu Ser Gly Gly Gly
225                 230                 235                 240

Leu Val Gln Pro Gly Gly Ser Leu Arg Leu Ser Cys Ala Ala Ser Gly
                245                 250                 255

Phe Thr Phe Thr Asp Tyr Thr Met Asp Trp Val Arg Gln Ala Pro Gly
                260                 265                 270

Lys Gly Leu Glu Trp Val Ala Asp Val Asn Pro Asn Ser Gly Gly Ser
            275                 280                 285

Ile Tyr Asn Gln Arg Phe Lys Gly Arg Phe Thr Leu Ser Val Asp Arg
    290                 295                 300

Ser Lys Asn Thr Leu Tyr Leu Gln Met Asn Ser Leu Arg Ala Glu Asp
305                 310                 315                 320

Thr Ala Val Tyr Tyr Cys Ala Arg Asn Leu Gly Pro Ser Phe Tyr Phe
                325                 330                 335

Asp Tyr Trp Gly Gln Gly Thr Leu Val Thr Val Ser Ser Ala Ser Thr
            340                 345                 350

Lys Gly Pro Ser Val Phe Pro Leu Ala Pro Cys Ser Arg Ser Thr Ser
        355                 360                 365

Glu Ser Thr Ala Ala Leu Gly Cys Leu Val Lys Asp Tyr Phe Pro Glu
    370                 375                 380

Pro Val Thr Val Ser Trp Asn Ser Gly Ala Leu Thr Ser Gly Val His
385                 390                 395                 400

Thr Phe Pro Ala Val Leu Gln Ser Ser Gly Leu Tyr Ser Leu Ser Ser
                405                 410                 415

Val Val Thr Val Pro Ser Ser Ser Leu Gly Thr Lys Thr Tyr Thr Cys
            420                 425                 430

Asn Val Asp His Lys Pro Ser Asn Thr Lys Val Asp Lys Arg Val Glu
        435                 440                 445

Ser Lys Tyr Gly Pro Pro Cys Pro Pro Cys Pro Ala Pro Glu Phe Leu
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

Gln Glu Asp Pro Glu Val Gln Phe Asn Trp Tyr Val Asp Gly Val Glu

500                 505                 510

Val His Asn Ala Lys Thr Lys Pro Arg Glu Glu Gln Phe Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Gly Leu Pro Ser Ser
545                 550                 555                 560

Ile Glu Lys Thr Ile Ser Lys Ala Lys Gly Gln Pro Arg Glu Pro Gln
                565                 570                 575

Val Tyr Thr Leu Pro Pro Ser Gln Glu Glu Met Thr Lys Asn Gln Val
            580                 585                 590

Ser Leu Thr Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Arg Leu Thr
625                 630                 635                 640

Val Asp Lys Ser Arg Trp Gln Glu Gly Asn Val Phe Ser Cys Ser Val
                645                 650                 655

Met His Glu Ala Leu His Asn His Tyr Thr Gln Lys Ser Leu Ser Leu
            660                 665                 670

Ser Leu Gly Lys
        675

<210>  134
<211>  2028
<212>  DNA
<213>  Homo

<400>  134
gaggtcaagc tggtggaaag cggcggcggc ctggtgcagc ctggaggatc cctgcggctg      60

agctgcgctg cctccggctt cgctttcagc tcctatgaca tgtcctgggt gaggcaggcc     120

cctggaaaga ggctggagtg ggtggctacc atctccggag gcggaaggta cacctactac     180

cccgacacag tgaagggaag gttcaccatc agccgggata acgccaaaaa cagccactat     240

ctccagatga actccctgag ggccgaagat acagccgtgt atttctgtgc ctccccctac     300

ggaggctatt ttgacgtgtg gggacagggc accctggtga ccgtctcctc cgcaagtacc 360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt gaagtgcagc tggtggagtc tggaggcgga 720

ctggtgcagc ctggaggctc tctgagactg tcttgtgctg cttctggctt tacctttacc 780

gattatacca tggattgggt gagacaggcc cctggaaagg gactggagtg ggtggctgat 840

gtgaacccta actctggagg atctatctat aatcagaggt ttaagggcag attcaccctg 900

tctgtggata gatctaagaa tacactgtat ctgcagatga actctctgag agctgaagat 960

acagctgtgt attactgcgc tagaaatctg ggcccttcct tttactttga ttactggggc 1020

cagggaacac tggtgaccgt gtcttctgcc tccaccaagg gcccatcggt cttccccctg 1080

gcaccctgct ccaggagcac ctctgagtcc acagcggccc tgggctgcct ggtcaaggac 1140

tacttccccg aaccggtgac ggtgtcgtgg aactcaggcg ccctgaccag cggcgtgcac 1200

accttcccgg ctgtcctaca gtcctcagga ctctactccc tcagcagcgt ggtgaccgtg 1260

ccctccagca gcttgggcac caagacatat acctgtaatg tggatcacaa gccttccaat 1320

acaaaagtgg acaagagagt tgagtccaag tacggcccac catgtcctcc atgtccagcc 1380

cctgaatttt tgggcgggcc ttctgtcttt ctgtttcctc ctaaacctaa agataccctg 1440

atgatcagcc gcacacccga agtcacttgt gtggtcgtgg atgtgtctca ggaagatccc 1500

gaagtgcagt ttaactggta tgtcgatggc gtggaagtgc ataatgccaa aactaagccc 1560

cgcgaagaac agttcaacag cacttatcgg gtcgtgtctg tgctcacagt cctccatcag 1620

gattggctga atgggaaaga atataagtgc aaggtgagca ataagggcct ccccagcagc 1680

atcgagaaga ctattagcaa agccaaaggg cagccacggg aaccccaggt gtacactctg 1740

cccccctctc aggaggagat gactaaaaat caggtctctc tgacttgtct ggtgaaaggg 1800

ttttatccca gcgacattgc cgtggagtgg gagtctaatg gccagcccga gaataattat 1860

aagacaactc cccccgtcct ggactctgac ggcagctttt tcctgtattc tcggctgaca 1920

gtggacaaaa gtcgctggca ggagggcaat gtctttagtt gcagtgtcat gcatgaggcc 1980

ctgcacaatc actatacaca gaaaagcctg tctctgagtc tgggcaaa 2028

<210> 135
<211> 678
<212> PRT
<213> Homo

<400>  135

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Val Glu Ser Gly Gly Gly
225                 230                 235                 240

Val Val Gln Pro Gly Arg Ser Leu Arg Leu Ser Cys Ala Ala Ser Gly
                245                 250                 255

Phe Thr Phe Ser Ser Tyr Thr Met His Trp Val Arg Gln Ala Pro Gly
            260                 265                 270

Lys Gly Leu Glu Trp Val Thr Phe Ile Ser Tyr Asp Gly Asn Asn Lys
        275                 280                 285

Tyr Tyr Ala Asp Ser Val Lys Gly Arg Phe Thr Ile Ser Arg Asp Asn
    290                 295                 300

Ser Lys Asn Thr Leu Tyr Leu Gln Met Asn Ser Leu Arg Ala Glu Asp
305                 310                 315                 320

Thr Ala Ile Tyr Tyr Cys Ala Arg Thr Gly Trp Leu Gly Pro Phe Asp
                325                 330                 335

Tyr Trp Gly Gln Gly Thr Leu Val Thr Val Ser Ser Ala Ser Thr Lys
            340                 345                 350

Gly Pro Ser Val Phe Pro Leu Ala Pro Ser Ser Lys Ser Thr Ser Gly
        355                 360                 365

Gly Thr Ala Ala Leu Gly Cys Leu Val Lys Asp Tyr Phe Pro Glu Pro
    370                 375                 380

Val Thr Val Ser Trp Asn Ser Gly Ala Leu Thr Ser Gly Val His Thr
385                 390                 395                 400

Phe Pro Ala Val Leu Gln Ser Ser Gly Leu Tyr Ser Leu Ser Ser Val
                405                 410                 415

Val Thr Val Pro Ser Ser Ser Leu Gly Thr Gln Thr Tyr Ile Cys Asn
            420                 425                 430

Val Asn His Lys Pro Ser Asn Thr Lys Val Asp Lys Lys Val Glu Pro
        435                 440                 445

Lys Ser Cys Asp Lys Thr His Thr Cys Pro Pro Cys Pro Ala Pro Glu
    450                 455                 460

Leu Leu Gly Gly Pro Ser Val Phe Leu Phe Pro Pro Lys Pro Lys Asp
465                 470                 475                 480

Thr Leu Met Ile Ser Arg Thr Pro Glu Val Thr Cys Val Val Val Asp
                485                 490                 495

Val Ser His Glu Asp Pro Glu Val Lys Phe Asn Trp Tyr Val Asp Gly
            500                 505                 510

Val Glu Val His Asn Ala Lys Thr Lys Pro Arg Glu Glu Gln Tyr Asn
        515                 520                 525

Ser Thr Tyr Arg Val Val Ser Val Leu Thr Val Leu His Gln Asp Trp
    530                 535                 540

Leu Asn Gly Lys Glu Tyr Lys Cys Lys Val Ser Asn Lys Ala Leu Pro
545                 550                 555                 560

Ala Pro Ile Glu Lys Thr Ile Ser Lys Ala Lys Gly Gln Pro Arg Glu
                565                 570                 575

Pro Gln Val Tyr Thr Leu Pro Pro Ser Arg Glu Glu Met Thr Lys Asn
            580                 585                 590

Gln Val Ser Leu Thr Cys Leu Val Lys Gly Phe Tyr Pro Ser Asp Ile
        595                 600                 605

Ala Val Glu Trp Glu Ser Asn Gly Gln Pro Glu Asn Asn Tyr Lys Thr
    610                 615                 620

Thr Pro Pro Val Leu Asp Ser Asp Gly Ser Phe Phe Leu Tyr Ser Lys
625                 630                 635                 640

Leu Thr Val Asp Lys Ser Arg Trp Gln Gln Gly Asn Val Phe Ser Cys
                645                 650                 655

Ser Val Met His Glu Ala Leu His Asn His Tyr Thr Gln Lys Ser Leu
            660                 665                 670

Ser Leu Ser Pro Gly Lys
        675

<210>  136
<211>  2034
<212>  DNA
<213>  Homo

<400>  136
gaggtcaagc tggtggaaag cggcggcggc ctggtgcagc ctggaggatc cctgcggctg      60

agctgcgctg cctccggctt cgctttcagc tcctatgaca tgtcctgggt gaggcaggcc     120

cctggaaaga ggctggagtg ggtggctacc atctccggag gcggaaggta cacctactac     180

cccgacacag tgaagggaag gttcaccatc agccgggata acgccaaaaa cagccactat     240

ctccagatga actccctgag ggccgaagat acagccgtgt atttctgtgc ctccccctac 300

ggaggctatt ttgacgtgtg gggacagggc accctggtga ccgtctcctc cgcaagtacc 360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt caagtgcagc tggtcgaaag cggcggagga 720

gtcgtccagc ccggcagatc tctgagactg agctgtgctg cctccggctt cacattcagc 780

tcctacacta tgcactgggt gaggcaagcc cccggcaagg gactggagtg ggtgactttc 840

atcagctacg acggcaacaa caagtactac gccgacagcg tgaagggaag gttcactatc 900

tctagggaca acagcaagaa cactctgtat ctgcagatga actctctgag ggccgaggat 960

actgccatct actactgcgc taggactggc tggctgggcc ctttcgatta ctggggccaa 1020

ggcactctgg tcactgtgag cagcgcgagc accaagggac cttccgtgtt tcccctcgcc 1080

cccagctcca aaagcaccag cggcggaaca gctgctctcg gctgtctcgt caaggattac 1140

ttccccgagc ccgtgaccgt gagctggaac agcggagccc tgacaagcgg cgtccacacc 1200

ttccctgctg tcctacagtc ctccggactg tacagcctga gcagcgtggt gacagtccct 1260

agcagctccc tgggcaccca gacatatatt tgcaacgtga atcacaagcc cagcaacacc 1320

aaggtcgata agaaggtgga gcctaagtcc tgcgacaaga cccacacatg tcccccctgt 1380

cccgctcctg aactgctggg aggcccttcc gtgttcctgt tcccccctaa gcccaaggac 1440

accctgatga tttccaggac acccgaggtg acctgtgtgg tggtggacgt cagccacgag 1500

gaccccgagg tgaaattcaa ctggtacgtc gatggcgtgg aggtgcacaa cgctaagacc 1560

aagcccaggg aggagcagta caattccacc tacagggtgg tgtccgtgct gaccgtcctc 1620

catcaggact ggctgaacgg caaagagtat aagtgcaagg tgagcaacaa ggccctccct 1680

gctcccatcg agaagaccat cagcaaagcc aagggccagc ccagggaacc tcaagtctat 1740

accctgcctc ccagcaggga ggagatgacc aagaaccaag tgagcctcac atgcctcgtc 1800

aagggcttct atccttccga tattgccgtc gagtgggagt ccaacggaca gcccgagaac 1860

aactacaaga caacaccccc cgtgctcgat tccgatggca gcttcttcct gtactccaag 1920

ctgaccgtgg acaagtccag atggcaacaa ggcaacgtct tcagttgcag cgtcatgcat 1980

gaggccctcc acaaccacta cacccagaaa agcctgtctc tgagtcctgg caaa 2034

<210> 137
<211> 678

<212>   PRT
<213>   Homo

<400>   137

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Thr Met His Trp Val Arg Gln Ala Pro Gly Lys Gly Leu Glu Trp Val
        35                  40                  45

Thr Phe Ile Ser Tyr Asp Gly Asn Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Thr Gly Trp Leu Gly Pro Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gly Gly Gly Gly Ser Gly Gly Gly
    210                 215                 220

Gly Ser Gly Gly Gly Gly Ser Glu Val Lys Leu Val Glu Ser Gly Gly
225                 230                 235                 240

Gly Leu Val Gln Pro Gly Gly Ser Leu Arg Leu Ser Cys Ala Ala Ser
                245                 250                 255

Gly Phe Ala Phe Ser Ser Tyr Asp Met Ser Trp Val Arg Gln Ala Pro
            260                 265                 270

Gly Lys Arg Leu Glu Trp Val Ala Thr Ile Ser Gly Gly Gly Arg Tyr
        275                 280                 285

Thr Tyr Tyr Pro Asp Thr Val Lys Gly Arg Phe Thr Ile Ser Arg Asp
    290                 295                 300

Asn Ala Lys Asn Ser His Tyr Leu Gln Met Asn Ser Leu Arg Ala Glu
305                 310                 315                 320

Asp Thr Ala Val Tyr Phe Cys Ala Ser Pro Tyr Gly Gly Tyr Phe Asp
                325                 330                 335

Val Trp Gly Gln Gly Thr Leu Val Thr Val Ser Ser Ala Ser Thr Lys
            340                 345                 350

Gly Pro Ser Val Phe Pro Leu Ala Pro Ser Ser Lys Ser Thr Ser Gly
        355                 360                 365

Gly Thr Ala Ala Leu Gly Cys Leu Val Lys Asp Tyr Phe Pro Glu Pro
    370                 375                 380

Val Thr Val Ser Trp Asn Ser Gly Ala Leu Thr Ser Gly Val His Thr
385                 390                 395                 400

Phe Pro Ala Val Leu Gln Ser Ser Gly Leu Tyr Ser Leu Ser Ser Val
                405                 410                 415

Val Thr Val Pro Ser Ser Ser Leu Gly Thr Gln Thr Tyr Ile Cys Asn
            420                 425                 430

Val Asn His Lys Pro Ser Asn Thr Lys Val Asp Lys Lys Val Glu Pro
        435                 440                 445

Lys Ser Cys Asp Lys Thr His Thr Cys Pro Pro Cys Pro Ala Pro Glu
    450                 455                 460

Leu Leu Gly Gly Pro Ser Val Phe Leu Phe Pro Pro Lys Pro Lys Asp
465                 470                 475                 480

Thr Leu Met Ile Ser Arg Thr Pro Glu Val Thr Cys Val Val Val Asp

485                 490                 495

Val Ser His Glu Asp Pro Glu Val Lys Phe Asn Trp Tyr Val Asp Gly
            500                 505                 510

Val Glu Val His Asn Ala Lys Thr Lys Pro Arg Glu Glu Gln Tyr Asn
        515                 520                 525

Ser Thr Tyr Arg Val Val Ser Val Leu Thr Val Leu His Gln Asp Trp
    530                 535                 540

Leu Asn Gly Lys Glu Tyr Lys Cys Lys Val Ser Asn Lys Ala Leu Pro
545                 550                 555                 560

Ala Pro Ile Glu Lys Thr Ile Ser Lys Ala Lys Gly Gln Pro Arg Glu
                565                 570                 575

Pro Gln Val Tyr Thr Leu Pro Pro Ser Arg Glu Glu Met Thr Lys Asn
            580                 585                 590

Gln Val Ser Leu Thr Cys Leu Val Lys Gly Phe Tyr Pro Ser Asp Ile
        595                 600                 605

Ala Val Glu Trp Glu Ser Asn Gly Gln Pro Glu Asn Asn Tyr Lys Thr
    610                 615                 620

Thr Pro Pro Val Leu Asp Ser Asp Gly Ser Phe Phe Leu Tyr Ser Lys
625                 630                 635                 640

Leu Thr Val Asp Lys Ser Arg Trp Gln Gln Gly Asn Val Phe Ser Cys
                645                 650                 655

Ser Val Met His Glu Ala Leu His Asn His Tyr Thr Gln Lys Ser Leu
            660                 665                 670

Ser Leu Ser Pro Gly Lys
        675

<210>  138
<211>  2034
<212>  DNA
<213>  Homo

<400>  138
   caagtgcagc tggtcgaaag cggcggagga gtcgtccagc ccggcagatc tctgagactg      60
   agctgtgctg cctccggctt cacattcagc tcctacacta tgcactgggt gaggcaagcc     120
   cccggcaagg gactggagtg ggtgactttc atcagctacg acggcaacaa caagtactac     180
   gccgacagcg tgaagggaag gttcactatc tctagggaca acagcaagaa cactctgtat 240
   ctgcagatga actctctgag ggccgaggat actgccatct actactgcgc taggactggc 300
   tggctgggcc ctttcgatta ctggggccaa ggcactctgg tcactgtgag cagcgcaagt 360
   accaagggac ctagtgtttt ccctcttgca ccttgctcca ggtcaacatc agagtccaca 420
   gctgctcttg gatgtctcgt taaggactac ttcccagagc cagttaccgt atcctggaac 480
   tccggagctt tgacaagcgg cgttcataca ttcccagctg tgttgcagag ttctgggttg 540
   tacagtttga gctcagtggt gaccgtgcct tcatcttctt tgggcactaa gacctacacc 600
   tgcaacgtgg atcacaagcc aagcaacacc aaggtggata agagggtggg tggaggcggt 660
   tcaggcggag gtggcagcgg aggtggcggg agtgaggtca agctggtgga aagcggcggc 720
   ggcctggtgc agcctggagg atccctgcgg ctgagctgcg ctgcctccgg cttcgctttc 780
   agctcctatg acatgtcctg ggtgaggcag gcccctggaa agaggctgga gtgggtggct 840
   accatctccg gaggcggaag gtacacctac taccccgaca cagtgaaggg aaggttcacc 900
   atcagccggg ataacgccaa aaacagccac tatctccaga tgaactccct gagggccgaa 960
   gatacagccg tgtatttctg tgcctccccc tacggaggct attttgacgt gtggggacag 1020
   ggcaccctgg tgaccgtctc ctccgcgagc accaaggacc cttccgtgtt tcccctcgcc 1080
   cccagctcca aaagcaccag cggcggaaca gctgctctcg gctgtctcgt caaggattac 1140
   ttccccgagc ccgtgaccgt gagctggaac agcggagccc tgacaagcgg cgtccacacc 1200
   ttccctgctg tcctacagtc ctccggactg tacagcctga gcagcgtggt gacagtccct 1260
   agcagctccc tgggcaccca gacatatatt tgcaacgtga atcacaagcc cagcaacacc 1320
   aaggtcgata agaaggtgga gcctaagtcc tgcgacaaga cccacacatg tcccccctgt 1380
   cccgctcctg aactgctggg aggcccttcc gtgttcctgt tcccccctaa gcccaaggac 1440
   accctgatga tttccaggac acccgaggtg acctgtgtgg tggtggacgt cagccacgag 1500
   gaccccgagg tgaaattcaa ctggtacgtc gatggcgtgg aggtgcacaa cgctaagacc 1560
   aagcccaggg aggagcagta caattccacc tacagggtgg tgtccgtgct gaccgtcctc 1620
   catcaggact ggctgaacgg caaagagtat aagtgcaagg tgagcaacaa ggccctccct 1680
   gctcccatcg agaagaccat cagcaaagcc aagggccagc ccagggaacc tcaagtctat 1740
   accctgcctc ccagcaggga ggagatgacc aagaaccaag tgagcctcac atgcctcgtc 1800
   aagggcttct atccttccga tattgccgtc gagtgggagt ccaacggaca gcccgagaac 1860
   aactacaaga caacaccccc cgtgctcgat tccgatggca gcttcttcct gtactccaag 1920
   ctgaccgtgg acaagtccag atggcaacaa ggcaacgtct tcagttgcag cgtcatgcat 1980
   gaggccctcc acaaccacta cacccagaaa agcctgtctc tgagtcctgg caaa 2034

<210>  139
<211>  675
<212>  PRT
<213>  Homo

<400>  139

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Gln Val Gln Leu Val Glu Ser Gly Gly Gly
225                 230                 235                 240

Val Val Gln Pro Gly Arg Ser Leu Arg Leu Ser Cys Ala Ala Ser Gly
                245                 250                 255

Phe Thr Phe Ser Ser Tyr Thr Met His Trp Val Arg Gln Ala Pro Gly
            260                 265                 270

Lys Gly Leu Glu Trp Val Thr Phe Ile Ser Tyr Asp Gly Asn Asn Lys
        275                 280                 285

Tyr Tyr Ala Asp Ser Val Lys Gly Arg Phe Thr Ile Ser Arg Asp Asn
    290                 295                 300

Ser Lys Asn Thr Leu Tyr Leu Gln Met Asn Ser Leu Arg Ala Glu Asp
305                 310                 315                 320

Thr Ala Ile Tyr Tyr Cys Ala Arg Thr Gly Trp Leu Gly Pro Phe Asp
                325                 330                 335

Tyr Trp Gly Gln Gly Thr Leu Val Thr Val Ser Ser Ala Ser Thr Lys
            340                 345                 350

Gly Pro Ser Val Phe Pro Leu Ala Pro Cys Ser Arg Ser Thr Ser Glu
        355                 360                 365

Ser Thr Ala Ala Leu Gly Cys Leu Val Lys Asp Tyr Phe Pro Glu Pro
    370                 375                 380

Val Thr Val Ser Trp Asn Ser Gly Ala Leu Thr Ser Gly Val His Thr
385                 390                 395                 400

Phe Pro Ala Val Leu Gln Ser Ser Gly Leu Tyr Ser Leu Ser Ser Val
                405                 410                 415

Val Thr Val Pro Ser Ser Ser Leu Gly Thr Lys Thr Tyr Thr Cys Asn
            420                 425                 430

Val Asp His Lys Pro Ser Asn Thr Lys Val Asp Lys Arg Val Glu Ser
        435                 440                 445

Lys Tyr Gly Pro Pro Cys Pro Pro Cys Pro Ala Pro Glu Phe Leu Gly
    450                 455                 460

Gly Pro Ser Val Phe Leu Phe Pro Pro Lys Pro Lys Asp Thr Leu Met
465                 470                 475                 480

Ile Ser Arg Thr Pro Glu Val Thr Cys Val Val Val Asp Val Ser Gln
                485                 490                 495

Glu Asp Pro Glu Val Gln Phe Asn Trp Tyr Val Asp Gly Val Glu Val
            500                 505                 510

His Asn Ala Lys Thr Lys Pro Arg Glu Glu Gln Phe Asn Ser Thr Tyr
        515                 520                 525

Arg Val Val Ser Val Leu Thr Val Leu His Gln Asp Trp Leu Asn Gly
    530                 535                 540

Lys Glu Tyr Lys Cys Lys Val Ser Asn Lys Gly Leu Pro Ser Ser Ile
545                 550                 555                 560

Glu Lys Thr Ile Ser Lys Ala Lys Gly Gln Pro Arg Glu Pro Gln Val
                565                 570                 575

Tyr Thr Leu Pro Pro Ser Gln Glu Glu Met Thr Lys Asn Gln Val Ser
            580                 585                 590

Leu Thr Cys Leu Val Lys Gly Phe Tyr Pro Ser Asp Ile Ala Val Glu
        595                 600                 605

Trp Glu Ser Asn Gly Gln Pro Glu Asn Asn Tyr Lys Thr Thr Pro Pro
    610                 615                 620

Val Leu Asp Ser Asp Gly Ser Phe Phe Leu Tyr Ser Arg Leu Thr Val
625                 630                 635                 640

Asp Lys Ser Arg Trp Gln Glu Gly Asn Val Phe Ser Cys Ser Val Met
                645                 650                 655

His Glu Ala Leu His Asn His Tyr Thr Gln Lys Ser Leu Ser Leu Ser
            660                 665                 670

Leu Gly Lys
        675

<210>  140
<211>  2025
<212>  DNA
<213>  Homo

<400>  140
gaggtcaagc tggtggaaag cggcggcggc ctggtgcagc ctggaggatc cctgcggctg        60

agctgcgctg cctccggctt cgctttcagc tcctatgaca tgtcctgggt gaggcaggcc       120

cctggaaaga ggctggagtg ggtggctacc atctccggag gcggaaggta cacctactac 180

cccgacacag tgaagggaag gttcaccatc agccgggata acgccaaaaa cagccactat 240

ctccagatga actccctgag ggccgaagat acagccgtgt atttctgtgc ctccccctac 300

ggaggctatt ttgacgtgtg gggacagggc accctggtga ccgtctcctc cgcaagtacc 360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt caagtgcagc tggtcgaaag cggcggagga 720

gtcgtccagc ccggcagatc tctgagactg agctgtgctg cctccggctt cacattcagc 780

tcctacacta tgcactgggt gaggcaagcc cccggcaagg gactggagtg ggtgactttc 840

atcagctacg acggcaacaa caagtactac gccgacagcg tgaagggaag gttcactatc 900

tctagggaca acagcaagaa cactctgtat ctgcagatga actctctgag ggccgaggat 960

actgccatct actactgcgc taggactggc tggctgggcc ctttcgatta ctggggccaa 1020

ggcactctgg tcactgtgag cagcgcctcc accaagggcc catcggtctt ccccctggca 1080

ccctgctcca ggagcacctc tgagtccaca gcggccctgg gctgcctggt caaggactac 1140

ttccccgaac cggtgacggt gtcgtggaac tcaggcgccc tgaccagcgg cgtgcacacc 1200

ttcccggctg tcctacagtc ctcaggactc tactccctca gcagcgtggt gaccgtgccc 1260

tccagcagct tgggcaccaa gacatatacc tgtaatgtgg atcacaagcc ttccaataca 1320

aaagtggaca agagagttga gtccaagtac ggcccaccat gtcctccatg tccagcccct 1380

gaatttttgg gcgggccttc tgtctttctg tttcctccta aacctaaaga taccctgatg 1440

atcagccgca cacccgaagt cacttgtgtg gtcgtggatg tgtctcagga agatcccgaa 1500

gtgcagttta actggtatgt cgatggcgtg gaagtgcata atgccaaaac taagccccgc 1560

gaagaacagt tcaacagcac ttatcgggtc gtgtctgtgc tcacagtcct ccatcaggat 1620

tggctgaatg ggaaagaata taagtgcaag gtgagcaata agggcctccc cagcagcatc 1680

gagaagacta ttagcaaagc caaagggcag ccacgggaac cccaggtgta cactctgccc 1740

ccctctcagg aggagatgac taaaaatcag gtctctctga cttgtctggt gaaagggttt 1800

tatcccagcg acattgccgt ggagtgggag tctaatggcc agcccgagaa taattataag 1860

acaactcccc ccgtcctgga ctctgacggc agctttttcc tgtattctcg gctgacagtg 1920

gacaaaagtc gctggcagga gggcaatgtc tttagttgca gtgtcatgca tgaggccctg 1980

cacaatcact atacacagaa aagcctgtct ctgagtctgg gcaaa 2025

<210>  141
<211>  675
<212>  PRT
<213>  Homo

<400>  141

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Thr Met His Trp Val Arg Gln Ala Pro Gly Lys Gly Leu Glu Trp Val
        35                  40                  45

Thr Phe Ile Ser Tyr Asp Gly Asn Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Thr Gly Trp Leu Gly Pro Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gly Gly Gly Gly Ser Gly Gly Gly
    210                 215                 220

Gly Ser Gly Gly Gly Gly Ser Glu Val Lys Leu Val Glu Ser Gly Gly
225                 230                 235                 240

Gly Leu Val Gln Pro Gly Gly Ser Leu Arg Leu Ser Cys Ala Ala Ser
                245                 250                 255

Gly Phe Ala Phe Ser Ser Tyr Asp Met Ser Trp Val Arg Gln Ala Pro
            260                 265                 270

Gly Lys Arg Leu Glu Trp Val Ala Thr Ile Ser Gly Gly Gly Arg Tyr
        275                 280                 285

Thr Tyr Tyr Pro Asp Thr Val Lys Gly Arg Phe Thr Ile Ser Arg Asp
    290                 295                 300

Asn Ala Lys Asn Ser His Tyr Leu Gln Met Asn Ser Leu Arg Ala Glu
305                 310                 315                 320

Asp Thr Ala Val Tyr Phe Cys Ala Ser Pro Tyr Gly Gly Tyr Phe Asp
                325                 330                 335

Val Trp Gly Gln Gly Thr Leu Val Thr Val Ser Ser Ala Ser Thr Lys
            340                 345                 350

Gly Pro Ser Val Phe Pro Leu Ala Pro Cys Ser Arg Ser Thr Ser Glu
        355                 360                 365

Ser Thr Ala Ala Leu Gly Cys Leu Val Lys Asp Tyr Phe Pro Glu Pro
    370                 375                 380

Val Thr Val Ser Trp Asn Ser Gly Ala Leu Thr Ser Gly Val His Thr
385                 390                 395                 400

Phe Pro Ala Val Leu Gln Ser Ser Gly Leu Tyr Ser Leu Ser Ser Val
                405                 410                 415

Val Thr Val Pro Ser Ser Ser Leu Gly Thr Lys Thr Tyr Thr Cys Asn
            420                 425                 430

Val Asp His Lys Pro Ser Asn Thr Lys Val Asp Lys Arg Val Glu Ser
        435                 440                 445

Lys Tyr Gly Pro Pro Cys Pro Pro Cys Pro Ala Pro Glu Phe Leu Gly
    450                 455                 460

Gly Pro Ser Val Phe Leu Phe Pro Pro Lys Pro Lys Asp Thr Leu Met

465                 470                 475                 480

Ile Ser Arg Thr Pro Glu Val Thr Cys Val Val Val Asp Val Ser Gln
                485                 490                 495

Glu Asp Pro Glu Val Gln Phe Asn Trp Tyr Val Asp Gly Val Glu Val
            500                 505                 510

His Asn Ala Lys Thr Lys Pro Arg Glu Glu Gln Phe Asn Ser Thr Tyr
        515                 520                 525

Arg Val Val Ser Val Leu Thr Val Leu His Gln Asp Trp Leu Asn Gly
    530                 535                 540

Lys Glu Tyr Lys Cys Lys Val Ser Asn Lys Gly Leu Pro Ser Ser Ile
545                 550                 555                 560

Glu Lys Thr Ile Ser Lys Ala Lys Gly Gln Pro Arg Glu Pro Gln Val
                565                 570                 575

Tyr Thr Leu Pro Pro Ser Gln Glu Glu Met Thr Lys Asn Gln Val Ser
            580                 585                 590

Leu Thr Cys Leu Val Lys Gly Phe Tyr Pro Ser Asp Ile Ala Val Glu
        595                 600                 605

Trp Glu Ser Asn Gly Gln Pro Glu Asn Asn Tyr Lys Thr Thr Pro Pro
    610                 615                 620

Val Leu Asp Ser Asp Gly Ser Phe Phe Leu Tyr Ser Arg Leu Thr Val
625                 630                 635                 640

Asp Lys Ser Arg Trp Gln Glu Gly Asn Val Phe Ser Cys Ser Val Met
                645                 650                 655

His Glu Ala Leu His Asn His Tyr Thr Gln Lys Ser Leu Ser Leu Ser
            660                 665                 670

Leu Gly Lys
        675

<210>  142
<211>  2025
<212>  DNA
<213>  Homo

<400>  142
caagtgcagc tggtcgaaag cggcggagga gtcgtccagc ccggcagatc tctgagactg        60

agctgtgctg cctccggctt cacattcagc tcctacacta tgcactgggt gaggcaagcc 120

cccggcaagg gactggagtg ggtgactttc atcagctacg acggcaacaa caagtactac 180

gccgacagcg tgaagggaag gttcactatc tctagggaca acagcaagaa cactctgtat 240

ctgcagatga actctctgag ggccgaggat actgccatct actactgcgc taggactggc 300

tggctgggcc ctttcgatta ctggggccaa ggcactctgg tcactgtgag cagcgcaagt 360

accaagggac ctagtgtttt ccctcttgca ccttgctcca ggtcaacatc agagtccaca 420

gctgctcttg gatgtctcgt taaggactac ttcccagagc cagttaccgt atcctggaac 480

tccggagctt tgacaagcgg cgttcataca ttcccagctg tgttgcagag ttctgggttg 540

tacagtttga gctcagtggt gaccgtgcct tcatcttctt tgggcactaa gacctacacc 600

tgcaacgtgg atcacaagcc aagcaacacc aaggtggata agagggtggg tggaggcggt 660

tcaggcggag gtggcagcgg aggtggcggg agtgaggtca agctggtgga aagcggcggc 720

ggcctggtgc agcctggagg atccctgcgg ctgagctgcg ctgcctccgg cttcgctttc 780

agctcctatg acatgtcctg ggtgaggcag gcccctggaa agaggctgga gtgggtggct 840

accatctccg gaggcggaag gtacacctac taccccgaca cagtgaaggg aaggttcacc 900

atcagccggg ataacgccaa aaacagccac tatctccaga tgaactccct gagggccgaa 960

gatacagccg tgtatttctg tgcctccccc tacggaggct attttgacgt gtggggacag 1020

ggcaccctgg tgaccgtctc ctccgcctcc accaagggcc catcggtctt ccccctggca 1080

ccctgctcca ggagcacctc tgagtccaca gcggccctgg gctgcctggt caaggactac 1140

ttccccgaac cggtgacggt gtcgtggaac tcaggcgccc tgaccagcgg cgtgcacacc 1200

ttcccggctg tcctacagtc ctcaggactc tactccctca gcagcgtggt gaccgtgccc 1260

tccagcagct tgggcaccaa gacatatacc tgtaatgtgg atcacaagcc ttccaataca 1320

aaagtggaca agagagttga gtccaagtac ggcccaccat gtcctccatg tccagcccct 1380

gaatttttgg gcgggccttc tgtctttctg tttcctccta aacctaaaga taccctgatg 1440

atcagccgca cacccgaagt cacttgtgtg gtcgtggatg tgtctcagga agatcccgaa 1500

gtgcagttta actggtatgt cgatggcgtg gaagtgcata atgccaaaac taagccccgc 1560

gaagaacagt tcaacagcac ttatcgggtc gtgtctgtgc tcacagtcct ccatcaggat 1620

tggctgaatg ggaaagaata taagtgcaag gtgagcaata agggcctccc cagcagcatc 1680

gagaagacta ttagcaaagc caaagggcag ccacgggaac cccaggtgta cactctgccc 1740

ccctctcagg aggagatgac taaaaatcag gtctctctga cttgtctggt gaaagggttt 1800

tatcccagcg acattgccgt ggagtgggag tctaatggcc agcccgagaa taattataag 1860

acaactcccc ccgtcctgga ctctgacggc agctttttcc tgtattctcg gctgacagtg 1920

gacaaaagtc gctggcagga gggcaatgtc tttagttgca gtgtcatgca tgaggccctg 1980

cacaatcact atacacagaa aagcctgtct ctgagtctgg gcaaa                    2025

<210>  143
<211>  673
<212>  PRT
<213>  Homo

<400>  143

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Arg Leu Glu Trp Val
        35                  40                  45

Ala Thr Ile Ser Gly Gly Gly Arg Tyr Thr Tyr Tyr Pro Asp Thr Val
    50                  55                  60

Lys Gly Arg Phe Thr Ile Ser Arg Asp Asn Ala Lys Asn Ser His Tyr
65                  70                  75                  80

Leu Gln Met Asn Ser Leu Arg Ala Glu Asp Thr Ala Val Tyr Phe Cys
                85                  90                  95

Ala Ser Pro Tyr Gly Gly Tyr Phe Asp Val Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Gly Gly Gly Gly Ser Gly Gly Gly Gly
    210                 215                 220

Ser Gly Gly Gly Gly Ser Glu Val Gln Leu Val Glu Ser Gly Gly Gly
225                 230                 235                 240

Leu Val Lys Pro Gly Gly Ser Leu Arg Leu Ser Cys Ala Ala Ser Gly
                245                 250                 255

Phe Thr Phe Ser Asp Asp Tyr Met Ala Trp Phe Arg Gln Ala Pro Gly
            260                 265                 270

Lys Arg Leu Glu Trp Val Ala Ser Ile Ser His Gly Gly Asp Tyr Ile
        275                 280                 285

Tyr Tyr Ala Asp Asn Leu Lys Gly Arg Phe Thr Ile Ser Arg Asp Asn
    290                 295                 300

Ala Lys Asn Ser Leu Tyr Leu Gln Met Asn Ser Leu Arg Ala Glu Asp
305                 310                 315                 320

Thr Ala Val Tyr Phe Cys Ser Arg Asp Arg Arg Ser Ile Asp Tyr Trp
                325                 330                 335

Gly Gln Gly Thr Leu Val Thr Val Ser Ser Ala Ser Thr Lys Gly Pro
            340                 345                 350

Ser Val Phe Pro Leu Ala Pro Cys Ser Arg Ser Thr Ser Glu Ser Thr
        355                 360                 365

Ala Ala Leu Gly Cys Leu Val Lys Asp Tyr Phe Pro Glu Pro Val Thr
    370                 375                 380

Val Ser Trp Asn Ser Gly Ala Leu Thr Ser Gly Val His Thr Phe Pro
385                 390                 395                 400

Ala Val Leu Gln Ser Ser Gly Leu Tyr Ser Leu Ser Ser Val Val Thr
                405                 410                 415

Val Pro Ser Ser Ser Leu Gly Thr Lys Thr Tyr Thr Cys Asn Val Asp
            420                 425                 430

His Lys Pro Ser Asn Thr Lys Val Asp Lys Arg Val Glu Ser Lys Tyr
        435                 440                 445

Gly Pro Pro Cys Pro Pro Cys Pro Ala Pro Glu Phe Leu Gly Gly Pro
    450                 455                 460

Ser Val Phe Leu Phe Pro Pro Lys Pro Lys Asp Thr Leu Met Ile Ser
465                 470                 475                 480

Arg Thr Pro Glu Val Thr Cys Val Val Val Asp Val Ser Gln Glu Asp
                485                 490                 495

Pro Glu Val Gln Phe Asn Trp Tyr Val Asp Gly Val Glu Val His Asn
            500                 505                 510

Ala Lys Thr Lys Pro Arg Glu Glu Gln Phe Asn Ser Thr Tyr Arg Val
        515                 520                 525

Val Ser Val Leu Thr Val Leu His Gln Asp Trp Leu Asn Gly Lys Glu
    530                 535                 540

Tyr Lys Cys Lys Val Ser Asn Lys Gly Leu Pro Ser Ser Ile Glu Lys
545                 550                 555                 560

Thr Ile Ser Lys Ala Lys Gly Gln Pro Arg Glu Pro Gln Val Tyr Thr
                565                 570                 575

Leu Pro Pro Ser Gln Glu Glu Met Thr Lys Asn Gln Val Ser Leu Thr
            580                 585                 590

Cys Leu Val Lys Gly Phe Tyr Pro Ser Asp Ile Ala Val Glu Trp Glu
        595                 600                 605

Ser Asn Gly Gln Pro Glu Asn Asn Tyr Lys Thr Thr Pro Pro Val Leu
    610                 615                 620

Asp Ser Asp Gly Ser Phe Phe Leu Tyr Ser Arg Leu Thr Val Asp Lys
625                 630                 635                 640

Ser Arg Trp Gln Glu Gly Asn Val Phe Ser Cys Ser Val Met His Glu
                645                 650                 655

Ala Leu His Asn His Tyr Thr Gln Lys Ser Leu Ser Leu Ser Leu Gly
            660                 665                 670

Lys

<210>  144
<211>  2019
<212>  DNA
<213>  Homo

<400>  144

gaggtcaagc tggtggaaag cggcggcggc ctggtgcagc ctggaggatc cctgcggctg 60

agctgcgctg cctccggctt cgctttcagc tcctatgaca tgtcctgggt gaggcaggcc 120

cctggaaaga ggctggagtg ggtggctacc atctccggag gcggaaggta cacctactac 180

cccgacacag tgaagggaag gttcaccatc agccgggata acgccaaaaa cagccactat 240

ctccagatga actccctgag ggccgaagat acagccgtgt atttctgtgc ctccccctac 300

ggaggctatt ttgacgtgtg gggacagggc accctggtga ccgtctcctc cgcaagtacc 360

aagggaccta gtgttttccc tcttgcacct tgctccaggt caacatcaga gtccacagct 420

gctcttggat gtctcgttaa ggactacttc ccagagccag ttaccgtatc ctggaactcc 480

ggagctttga caagcggcgt tcatacattc ccagctgtgt tgcagagttc tgggttgtac 540

agtttgagct cagtggtgac cgtgccttca tcttctttgg gcactaagac ctacacctgc 600

aacgtggatc acaagccaag caacaccaag gtggataaga gggtgggtgg aggcggttca 660

ggcggaggtg gcagcggagg tggcgggagt gaagtgcagc tggtggagtc cggaggcgga 720

ctggtgaagc ctggaggctc cctgaggctg tcctgtgccg cttccggctt caccttctcc 780

gacgactaca tggcctggtt caggcaggcc cctggaaaga ggctggagtg ggtggcttcc 840

atctcccacg gcggcgacta catctactac gccgacaacc tgaagggcag gttcaccatc 900

tccagggaca acgccaagaa ctccctgtac ctgcagatga actccctgag ggccgaggac 960

accgccgtgt acttctgctc cagggacagg aggtccatcg actattgggg ccagggcacc 1020

ctggtgacag tgtcctccgc ctccaccaag ggcccatcgg tcttccccct ggcaccctgc 1080

tccaggagca cctctgagtc cacagcggcc ctgggctgcc tggtcaagga ctacttcccc 1140

gaaccggtga cggtgtcgtg gaactcaggc gccctgacca gcggcgtgca caccttcccg 1200

gctgtcctac agtcctcagg actctactcc ctcagcagcg tggtgaccgt gccctccagc 1260

agcttgggca ccaagacata tacctgtaat gtggatcaca agccttccaa tacaaaagtg 1320

gacaagagag ttgagtccaa gtacggccca ccatgtcctc catgtccagc ccctgaattt 1380

ttgggcgggc cttctgtctt tctgtttcct cctaaaccta aagataccct gatgatcagc 1440

cgcacacccg aagtcacttg tgtggtcgtg gatgtgtctc aggaagatcc cgaagtgcag 1500

tttaactggt atgtcgatgg cgtggaagtg cataatgcca aaactaagcc ccgcgaagaa 1560

cagttcaaca gcacttatcg ggtcgtgtct gtgctcacag tcctccatca ggattggctg 1620

aatgggaaag aatataagtg caaggtgagc aataagggcc tccccagcag catcgagaag 1680

actattagca aagccaaagg gcagccacgg gaaccccagg tgtacactct gcccccctct 1740

caggaggaga tgactaaaaa tcaggtctct ctgacttgtc tggtgaaagg gttttatccc 1800

agcgacattg ccgtggagtg ggagtctaat ggccagcccg agaataatta taagacaact 1860

ccccccgtcc tggactctga cggcagcttt ttcctgtatt ctcggctgac agtggacaaa 1920

agtcgctggc aggagggcaa tgtctttagt tgcagtgtca tgcatgaggc cctgcacaat 1980

cactatacac agaaaagcct gtctctgagt ctgggcaaa 2019

<210> 145
<211> 673
<212> PRT
<213> Homo

<400> 145

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Asp
            20                  25                  30

Tyr Met Ala Trp Phe Arg Gln Ala Pro Gly Lys Arg Leu Glu Trp Val
        35                  40                  45

Ala Ser Ile Ser His Gly Gly Asp Tyr Ile Tyr Tyr Ala Asp Asn Leu
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ser Arg Asp Arg Arg Ser Ile Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Gly Gly Gly Gly Ser Gly Gly Gly Gly Ser
    210                 215                 220

Gly Gly Gly Gly Ser Glu Val Lys Leu Val Glu Ser Gly Gly Gly Leu
225                 230                 235                 240

Val Gln Pro Gly Gly Ser Leu Arg Leu Ser Cys Ala Ala Ser Gly Phe
                245                 250                 255

Ala Phe Ser Ser Tyr Asp Met Ser Trp Val Arg Gln Ala Pro Gly Lys
            260                 265                 270

Arg Leu Glu Trp Val Ala Thr Ile Ser Gly Gly Gly Arg Tyr Thr Tyr
        275                 280                 285

Tyr Pro Asp Thr Val Lys Gly Arg Phe Thr Ile Ser Arg Asp Asn Ala
    290                 295                 300

Lys Asn Ser His Tyr Leu Gln Met Asn Ser Leu Arg Ala Glu Asp Thr
305                 310                 315                 320

Ala Val Tyr Phe Cys Ala Ser Pro Tyr Gly Gly Tyr Phe Asp Val Trp
                325                 330                 335

Gly Gln Gly Thr Leu Val Thr Val Ser Ser Ala Ser Thr Lys Gly Pro
            340                 345                 350

Ser Val Phe Pro Leu Ala Pro Cys Ser Arg Ser Thr Ser Glu Ser Thr
        355                 360                 365

Ala Ala Leu Gly Cys Leu Val Lys Asp Tyr Phe Pro Glu Pro Val Thr
    370                 375                 380

Val Ser Trp Asn Ser Gly Ala Leu Thr Ser Gly Val His Thr Phe Pro
385                 390                 395                 400

Ala Val Leu Gln Ser Ser Gly Leu Tyr Ser Leu Ser Ser Val Val Thr
                405                 410                 415

Val Pro Ser Ser Ser Leu Gly Thr Lys Thr Tyr Thr Cys Asn Val Asp
            420                 425                 430

His Lys Pro Ser Asn Thr Lys Val Asp Lys Arg Val Glu Ser Lys Tyr
        435                 440                 445

Gly Pro Pro Cys Pro Pro Cys Pro Ala Pro Glu Phe Leu Gly Gly Pro

450                 455                 460

Ser Val Phe Leu Phe Pro Pro Lys Pro Lys Asp Thr Leu Met Ile Ser
465                 470                 475                 480

Arg Thr Pro Glu Val Thr Cys Val Val Val Asp Val Ser Gln Glu Asp
                485                 490                 495

Pro Glu Val Gln Phe Asn Trp Tyr Val Asp Gly Val Glu Val His Asn
            500                 505                 510

Ala Lys Thr Lys Pro Arg Glu Glu Gln Phe Asn Ser Thr Tyr Arg Val
        515                 520                 525

Val Ser Val Leu Thr Val Leu His Gln Asp Trp Leu Asn Gly Lys Glu
    530                 535                 540

Tyr Lys Cys Lys Val Ser Asn Lys Gly Leu Pro Ser Ser Ile Glu Lys
545                 550                 555                 560

Thr Ile Ser Lys Ala Lys Gly Gln Pro Arg Glu Pro Gln Val Tyr Thr
                565                 570                 575

Leu Pro Pro Ser Gln Glu Glu Met Thr Lys Asn Gln Val Ser Leu Thr
            580                 585                 590

Cys Leu Val Lys Gly Phe Tyr Pro Ser Asp Ile Ala Val Glu Trp Glu
        595                 600                 605

Ser Asn Gly Gln Pro Glu Asn Asn Tyr Lys Thr Thr Pro Pro Val Leu
    610                 615                 620

Asp Ser Asp Gly Ser Phe Phe Leu Tyr Ser Arg Leu Thr Val Asp Lys
625                 630                 635                 640

Ser Arg Trp Gln Glu Gly Asn Val Phe Ser Cys Ser Val Met His Glu
                645                 650                 655

Ala Leu His Asn His Tyr Thr Gln Lys Ser Leu Ser Leu Ser Leu Gly
            660                 665                 670

Lys

<210>  146
<211>  2019
<212>  DNA
<213>  Homo

<400> 146
gaagtgcagc tggtggagtc cggaggcgga ctggtgaagc ctggaggctc cctgaggctg 60

tcctgtgccg cttccggctt caccttctcc gacgactaca tggcctggtt caggcaggcc 120

cctggaaaga ggctggagtg ggtggcttcc atctcccacg gcggcgacta catctactac 180

gccgacaacc tgaagggcag gttcaccatc tccagggaca acgccaagaa ctccctgtac 240

ctgcagatga actccctgag ggccgaggac accgccgtgt acttctgctc cagggacagg 300

aggtccatcg actattgggg ccagggcacc ctggtgacag tgtcctccgc aagtaccaag 360

ggacctagtg ttttccctct tgcaccttgc tccaggtcaa catcagagtc cacagctgct 420

cttggatgtc tcgttaagga ctacttccca gagccagtta ccgtatcctg gaactccgga 480

gctttgacaa gcggcgttca tacattccca gctgtgttgc agagttctgg gttgtacagt 540

ttgagctcag tggtgaccgt gccttcatct tctttgggca ctaagaccta cacctgcaac 600

gtggatcaca agccaagcaa caccaaggtg gataagaggg tgggtggagg cggttcaggc 660

ggaggtggca gcggaggtgg cgggagtgag gtcaagctgg tggaaagcgg cggcggcctg 720

gtgcagcctg gaggatccct gcggctgagc tgcgctgcct ccggcttcgc tttcagctcc 780

tatgacatgt cctgggtgag gcaggcccct ggaaagaggc tggagtgggt ggctaccatc 840

tccggaggcg gaaggtacac ctactacccc gacacagtga agggaaggtt caccatcagc 900

cgggataacg ccaaaaacag ccactatctc cagatgaact ccctgagggc cgaagataca 960

gccgtgtatt tctgtgcctc cccctacgga ggctattttg acgtgtgggg acagggcacc 1020

ctggtgaccg tctcctccgc ctccaccaag ggcccatcgg tcttccccct ggcaccctgc 1080

tccaggagca cctctgagtc cacagcggcc ctgggctgcc tggtcaagga ctacttcccc 1140

gaaccggtga cggtgtcgtg gaactcaggc gccctgacca gcggcgtgca caccttcccg 1200

gctgtcctac agtcctcagg actctactcc ctcagcagcg tggtgaccgt gccctccagc 1260

agcttgggca ccaagacata tacctgtaat gtggatcaca agccttccaa tacaaaagtg 1320

gacaagagag ttgagtccaa gtacggccca ccatgtcctc catgtccagc ccctgaattt 1380

ttgggcgggc cttctgtctt tctgtttcct cctaaaccta aagataccct gatgatcagc 1440

cgcacacccg aagtcacttg tgtggtcgtg gatgtgtctc aggaagatcc cgaagtgcag 1500

tttaactggt atgtcgatgg cgtggaagtg cataatgcca aaactaagcc ccgcgaagaa 1560

cagttcaaca gcacttatcg ggtcgtgtct gtgctcacag tcctccatca ggattggctg 1620

aatgggaaag aatataagtg caaggtgagc aataaggccc tccccagcag catcgagaag 1680

actattagca aagccaaagg gcagccacgg gaaccccagg tgtacactct gcccccctct 1740

caggaggaga tgactaaaaa tcaggtctct ctgacttgtc tggtgaaagg gttttatccc 1800

agcgacattg ccgtggagtg ggagtctaat ggccagcccg agaataatta taagacaact 1860

ccccccgtcc tggactctga cggcagcttt ttcctgtatt ctcggctgac agtggacaaa 1920

agtcgctggc aggagggcaa tgtctttagt tgcagtgtca tgcatgaggc cctgcacaat 1980

cactatacac agaaaagcct gtctctgagt ctgggcaaa 2019

<210> 147
<211> 330
<212> PRT
<213> Homo

<400> 147

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  148
<211>  327
<212>  PRT
<213>  Homo

<400>  148

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  149
<211>  107
<212>  PRT
<213>  Homo

<400>  149


<210>  150
<211>  15
<212>  PRT
<213>  Homo

<400>  150

Gly Gly Gly Gly Ser Gly Gly Gly Gly Ser Gly Gly Gly Gly Ser
1               5                   10                  15


<210>  151
<211>  11
<212>  PRT
<213>  Homo

<400>  151

Trp Gly Gln Gly Thr Leu Val Thr Val Ser Ser
1               5                   10


<210>  152
<211>  10
<212>  PRT
<213>  Homo

<400>  152

Phe Gly Gln Gly Thr Lys Val Glu Ile Lys
1               5                   10


<210>  153
<211>  10
<212>  PRT
<213>  Homo

<400>  153

Phe Gly Gly Gly Thr Lys Val Glu Leu Lys
1               5                   10